# EUROPEAN PATENT APPLICATION

(11) **EP 2 264 155 A1**
(43) Date of publication of application: **22.12.2010**
(21) Application number: 10010578.2
(22) Date of filing: 25.07.2003
(51) Int. Cl.: C12N 9/12, C12Q 1/68

(54) **Hybrid polymerase methods and compositions**

(30) Priority: 25.07.2002 US 398687 P; 17.04.2003 US 463781 P; 27.06.2003 US 483287 P
(62) Divisional of application: 03771830.1
(71) Applicant: BIO-RAD LABORATORIES, INC., Hercules California 94547 (US)
(72) Inventor: Vander Horn, Peter, Foster City, California 94404 (US); Wang, Yan, San Francisco, California 94127 (US)
(74) Representative: Campbell, Patrick John Henry

(57) **Abstract**

This invention relates to methods for the facilitated evolution of proteins. The invention provides methods for generating hybrid proteins and improved proteins obtained using the methods.

SEQUENCE LISTING

<110> BIO-RAD LABORATORIES, INC

<120> Hybrid Polymerase Methods and Compositions

<130> N.93844A PJC

<140> EP 03771830.1
<141> 2003-07-25

<150> WO PCT/US03/23278
<151> 2003-07-25

<150> US 60/398,687
<151> 2002-07-25

<150> US 60/463,781
<151> 2003-04-17

<150> US 60/483,287
<151> 2003-06-27

<160> 85

<170> PatentIn Ver. 2.1

<210> 1
<211> 2325
<212> DNA
<213> Artificial Sequence

<220>
<223> Description of Artificial Sequence:full-length
      assembled hybrid polymerase clone Phy1

<400> 1
atgatcctgg atgctgacta catcactgaa gaaggcaaac cggttatccg tctgttcaaa 60
aaagagaacg gcgaatttaa gattgagcat gatcgcacct ttcgtccata catttacgct 120
ctgctgaaag atgattctaa gattgaggaa gttaaaaaaa tcactgctga gcgccatggc 180
aagattgttc gtatcgttga tgcggaaaag gtagaaaaga aatttctggg cagaccaatc 240
accgtgtgga gactgtattt cgaacatcca caagatgttc cgactattcg cgagaaaatt 300
cgcgaacatt ctgcagttgt tgacatcttc gaatacgata ttccatttgc aaagcgttac 360
ctcatcgaca aaggcctgat accaatggag ggcgatgaag aactcaagct cctggcgttc 420
gatatagaaa ccctctatca cgaaggcgaa gagtttggta aaggcccaat tataatgatc 480
agctatgcag atgaagaaga agcaaaggtg attacttgga aaaaaataga tctcccatac 540
gttgaggttg tatcttccga gcgcgagatg attaagcgct ttctcaaaat tatccgcgag 600
aaggatccgg acattatcat tacttataac ggcgactctt ttgacctccc atatctggcg 660
aaacgcgcag aaaaactcgg tattaaactg actatcggcc gtgatggttc cgagccgaag 720
atgcagcgta tcggcgatat gaccgctgta gaagttaagg gtcgtatcca tttcgacctg 780
tatcatgtaa ttcgtcgtac tattaacctc ccgacttaca ctctcgaggc tgtatatgaa 840
gcaatttttg gtaagccgaa ggagaaggta tacgccgatg agattgcaaa ggcgtgggaa 900
accggtgagg gcctcgagcg tgttgcaaaa tactccatgg aagatgcaaa ggcgacttat 960
gaactcggca aagaattctt cccaatggaa gctcagctct ctcgcctggt tggccaacca 1020
ctgtgggatg tttctcgttc ttccaccggt aacctcgtag agtggtttct cctgcgcaaa 1080
gcgtacgaac gcaacgaact ggctccgaac aagccagatg aacgtgagta tgaacgccgt 1140
ctccgcgagt cttacgctgg tggctttgtt aaagagccag aaaagggcct ctgggaaaac 1200
atcgtgtccc tcgattttcg cgctctgtat ccgtctatta tcattaccca caacgtgtct 1260
ccggatactc tcaaccgcga gggctgcaga aactatgatg ttgctccgga agtaggccac 1320
aagttctgca aggacttccc gggctttatt ccgtctctcc tgaaacgtct gctcgatgaa 1380
cgccaaaaga ttaagactaa aatgaaggcg tcccaggatc cgattgaaaa aataatgctc 1440
gactatcgcc aaagagcgat taaaatcctc gcaaactctt attacggcta ttatggctat 1500
gcaaaagcac gctggtactg taaggagtgt gctgagtccg ttactgcttg gggtcgcgaa 1560
tacatcgagt tcgtgtggaa ggagctcgaa gaaaagtttg gctttaaagt tctctacatt 1620
gacactgatg gtctctatgc gactattccg ggtggtaagt ctgaggaaat taagaaaaag 1680
gctctagaat ttgtggatta cattaacgcg aagctcccgg gtctcctgga gctcgaatat 1740
gaaggctttt ataaacgcgg cttcttcgtt accaagaaga aatatgcgct gattgatgaa 1800
gaaggcaaaa ttattactcg tggtctcgag attgtgcgcc gtgattggag cgaaattgcg 1860
aaagaaactc aagctagagt tctcgaggct attctcaaac acggcaacgt tgaagaagct 1920

gtgagaattg taaaagaagt aacccaaaag ctctctaaat atgaaattcc gccagagaag 1980
ctcgcgattt atgagcagat tactcgcccg ctgcatgagt ataaggcgat tggtccgcac 2040
gtggctgttg caaagagact ggctgctaaa ggcgtgaaaa ttaaaccggg tatggtaatt 2100
ggctacattg tactccgcgg cgatggtccg attagcaacc gtgcaattct agctgaggaa 2160
tacgatccga gaaagcacaa gtatgacgca gaatattaca ttgagaacca ggtgctcccg 2220
gcggtactcc gtattctgga gggttttggc taccgtaagg aagacctccg ctggcaaaag 2280
actaaacaga ctggcctcac ttcttggctc aacattaaaa aatcc 2325

<210> 2
<211> 775
<212> PRT
<213> Artificial Sequence

<220>
<223> Description of Artificial Sequence:full-length
      assembled hybrid polymerase clone Phy1

<400> 2
Met Ile Leu Asp Ala Asp Tyr Ile Thr Glu Glu Gly Lys Pro Val Ile
1               5                   10                  15

Arg Leu Phe Lys Lys Glu Asn Gly Glu Phe Lys Ile Glu His Asp Arg
            20                  25                  30

Thr Phe Arg Pro Tyr Ile Tyr Ala Leu Leu Lys Asp Asp Ser Lys Ile
        35                  40                  45

Glu Glu Val Lys Lys Ile Thr Ala Glu Arg His Gly Lys Ile Val Arg
    50                  55                  60

Ile Val Asp Ala Glu Lys Val Glu Lys Lys Phe Leu Gly Arg Pro Ile
65                  70                  75                  80

Thr Val Trp Arg Leu Tyr Phe Glu His Pro Gln Asp Val Pro Thr Ile
                85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190
Arg Phe Leu Lys Ile Ile Arg Glu Lys Asp Pro Asp Ile Ile Ile Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu

        275                 280                 285

Lys Val Tyr Ala Asp Glu Ile Ala Lys Ala Trp Glu Thr Gly Glu Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Phe Val Lys Glu Pro Glu Lys Gly Leu Trp Glu Asn
385                 390                 395                 400

Ile Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Arg Asn Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Lys Ile
    450                 455                 460

Lys Thr Lys Met Lys Ala Ser Gln Asp Pro Ile Glu Lys Ile Met Leu
465                 470                 475                 480

Asp Tyr Arg Gln Arg Ala Ile Lys Ile Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Ser Glu Glu Ile Lys Lys Lys
545                 550                 555                 560

Ala Leu Glu Phe Val Asp Tyr Ile Asn Ala Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Lys Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala
625                 630                 635                 640

Val Arg Ile Val Lys Glu Val Thr Gln Lys Leu Ser Lys Tyr Glu Ile

645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Lys Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Pro Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Thr Gly Leu Thr Ser
        755                 760                 765

Trp Leu Asn Ile Lys Lys Ser
    770                 775

<210> 3
<211> 2535
<212> DNA
<213> Artificial Sequence

<220>
<223> Description of Artificial Sequence:PhS1, Phy1 and
      SSo7d fusion protein

<400> 3
atgatcctgg atgctgacta catcactgaa gaaggcaaac cggttatccg tctgttcaaa 60
aaagagaacg gcgaatttaa gattgagcat gatcgcacct ttcgtccata catttacgct 120
ctgctgaaag atgattctaa gattgaggaa gttaaaaaaa tcactgctga gcgccatggc 180
aagattgttc gtatcgttga tgcggaaaag gtagaaaaga aatttctggg cagaccaatc 240
accgtgtgga gactgtattt cgaacatcca caagatgttc cgactattcg cgagaaaatt 300
cgcgaacatt ctgcagttgt tgacatcttc gaatacgata ttccatttgc aaagcgttac 360
ctcatcgaca aaggcctgat accaatggag ggcgatgaag aactcaagct cctggcgttc 420
gatatagaaa ccctctatca cgaaggcgaa gagtttggta aaggcccaat tataatgatc 480
agctatgcag atgaagaaga agcaaaggtg attacttgga aaaaaataga tctcccatac 540
gttgaggttg tatcttccga gcgcgagatg attaagcgct ttctcaaaat tatccgcgag 600
aaggatccgg acattatcat tacttataac ggcgactctt ttgacctccc atatctggcg 660
aaacgcgcag aaaaactcgg tattaaactg actatcggcc gtgatggttc cgagccgaag 720
atgcagcgta tcggcgatat gaccgctgta gaagttaagg gtcgtatcca tttcgacctg 780
tatcatgtaa ttcgtcgtac tattaacctc ccgacttaca ctctcgaggc tgtatatgaa 840
gcaatttttg gtaagccgaa ggagaaggta tacgccgatg agattgcaaa ggcgtgggaa 900
accggtgagg gcctcgagcg tgttgcaaaa tactccatgg aagatgcaaa ggcgacttat 960
gaactcggca aagaattctt cccaatggaa gctcagctct ctcgcctggt tggccaacca 1020
ctgtgggatg tttctcgttc ttccaccggt aacctcgtag agtggtttct cctgcgcaaa 1080
gcgtacgaac gcaacgaact ggctccgaac aagccagatg aacgtgagta tgaacgccgt 1140
ctccgcgagt cttacgctgg tggctttgtt aaagagccag aaaagggcct ctgggaaaac 1200
atcgtgtccc tcgattttcg cgctctgtat ccgtctatta tcattaccca caacgtgtct 1260
ccggatactc tcaaccgcga gggctgcaga aactatgatg ttgctccgga agtaggccac 1320
aagttctgca aggacttccc gggctttatt ccgtctctcc tgaaacgtct gctcgatgaa 1380
cgccaaaaga ttaagactaa aatgaaggcg tcccaggatc cgattgaaaa aataatgctc 1440
gactatcgcc aaagagcgat taaaatcctc gcaaactctt attacggcta ttatggctat 1500
gcaaaagcac gctggtactg taaggagtgt gctgagtccg ttactgcttg gggtcgcgaa 1560
tacatcgagt tcgtgtggaa ggagctcgaa gaaaagtttg gctttaaagt tctctacatt 1620
gacactgatg gtctctatgc gactattccg ggtggtaagt ctgaggaaat taagaaaaag 1680
gctctagaat ttgtggatta cattaacgcg aagctcccgg gtctcctgga gctcgaatat 1740
gaaggctttt ataaacgcgg cttcttcgtt accaagaaga aatatgcgct gattgatgaa 1800
gaaggcaaaa ttattactcg tggtctcgag attgtgcgcc gtgattggag cgaaattgcg 1860
aaagaaactc aagctagagt tctcgaggct attctcaaac acggcaacgt tgaagaagct 1920
gtgagaattg taaaagaagt aacccaaaag ctctctaaat atgaaattcc gccagagaag 1980

ctcgcgattt atgagcagat tactcgcccg ctgcatgagt ataaggcgat tggtccgcac 2040
gtggctgttg caaagagact ggctgctaaa ggcgtgaaaa ttaaaccggg tatggtaatt 2100
ggctacattg tactccgcgg cgatggtccg attagcaacc gtgcaattct agctgaggaa 2160
tacgatccga gaaagcacaa gtatgacgca gaatattaca ttgagaacca ggtgctcccg 2220
gcggtactcc gtattctgga gggttttggc taccgtaagg aagacctccg ctggcaaaag 2280
actaaacaga ctggcctcac ttcttggctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gcgccgaagg agctgctgca gatgctggag 2520
aagcagaaaa agtga 2535

<210> 4
<211> 844
<212> PRT
<213> Artificial Sequence

<220>
<223> Description of Artificial Sequence:PhS1, Phy1 and
      SSo7d fusion protein

<400> 4
Met Ile Leu Asp Ala Asp Tyr Ile Thr Glu Glu Gly Lys Pro Val Ile
1               5                   10                  15

Arg Leu Phe Lys Lys Glu Asn Gly Glu Phe Lys Ile Glu His Asp Arg
            20                  25                  30
Thr Phe Arg Pro Tyr Ile Tyr Ala Leu Leu Lys Asp Asp Ser Lys Ile
        35                  40                  45

Glu Glu Val Lys Lys Ile Thr Ala Glu Arg His Gly Lys Ile Val Arg
    50                  55                  60

Ile Val Asp Ala Glu Lys Val Glu Lys Lys Phe Leu Gly Arg Pro Ile
65                  70                  75                  80

Thr Val Trp Arg Leu Tyr Phe Glu His Pro Gln Asp Val Pro Thr Ile
                85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Ile Ile Arg Glu Lys Asp Pro Asp Ile Ile Ile Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Arg Arg Thr Ile Asn Leu Pro Thr

            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Lys Ala Trp Glu Thr Gly Glu Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Phe Val Lys Glu Pro Glu Lys Gly Leu Trp Glu Asn
385                 390                 395                 400

Ile Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Arg Asn Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Lys Ile
    450                 455                 460

Lys Thr Lys Met Lys Ala Ser Gln Asp Pro Ile Glu Lys Ile Met Leu
465                 470                 475                 480

Asp Tyr Arg Gln Arg Ala Ile Lys Ile Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Ser Glu Glu Ile Lys Lys Lys
545                 550                 555                 560

Ala Leu Glu Phe Val Asp Tyr Ile Asn Ala Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Lys Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala

625                 630                 635                 640

Val Arg Ile Val Lys Glu Val Thr Gln Lys Leu Ser Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Lys Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Pro Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Thr Gly Leu Thr Ser
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 5
<211> 2535
<212> DNA
<213> Artificial Sequence

<220>
<223> Description of Artificial Sequence:hybrid
      polymerase Sso7d fusion protein PhS2

<400> 5
atgatcctgg atgttgacta catcactgaa gaaggcaaac cggttatccg tctgttcaaa 60
aaagagaacg gcgaatttaa ggttgagtat gatcgcacct ttcgtccata catttacgct 120
ctgctgaaag atgattctaa gattgatgaa gttagaaaaa tcactggtga gcgccatggc 180
aagattgttc gtatcattga tgcggaaaag gtacgtaaga aatttctggg caaaccaatc 240
gaggtgtgga aactgtattt cgaacatcca caagatgttc cgactattcg cgagaaaatt 300
cgcgaacatt ctgcagttgt tgacatcttc gaatacgata ttccatttgc aaagcgttac 360
ctcatcgaca aaggcctgat accaatggag ggcgaggaag aactcaagat cctggcgttc 420
gatatagaaa ccctctatca cgaaggcgaa gagtttggta aaggcccaat tataatgatc 480
agctatgcag atgaaaacga agcaaaggtg attacttgga aaaaaataga tctcccatac 540
gttgaggttg tatcttccga gcgcgagatg attaagcgct ttctcaaagt tatccgcgag 600
aaggatccgg acattatcgt tacttataac ggcgactctt ttgacttccc atatctggcg 660
aaacgcgcag aaaaactcgg tattaaactg cctatcggcc gtgatggttc cgagccgaag 720
atgcagcgta tcggcgatat gaccgctgta gaagttaagg gtcgtatcca tttcgacctg 780
tatcatgtaa ttcgtcgtac tattaacctc ccgacttaca ctctcgaggc tgtatatgaa 840
gcaatttttg gtaagccgaa ggagaaggta tacgcccatg agattgcaga ggcgtgggaa 900
tccggtgagg gcctcgagcg tgttgcaaaa tactccatgg aagatgcaaa ggcgacttat 960
gaactcggca aagaattctt cccaatggaa atccagctct ctcgcctggt tggccaacca 1020

ctgtgggatg tttctcgttc ttccaccggt aacctcgtag agtggtttct cctgcgcaaa 1080
gcgtacgaac gcaacgaact ggctccgaac aagccatctg aacgtgagta tgaacgccgt 1140
ctccgcgagt cttacactgg tggctatgtt aaagagccag aaaagggcct ctgggaaaac 1200
atcgtgtacc tcgattttcg ctctctgtat ccgtctatta tcattaccca caacgtgtct 1260
ccggatactc tcaacctcga gggctgcaaa gagtatgatg ttgctccgga agtaggccac 1320
aagttctgca aggacatccc gggctttatt ccgtctctcc tgggccatct gctcgaggaa 1380
cgccaaaaga ttaagcgtaa aatgaaggcg tccaaggatc cgattgaaaa aatactgctc 1440
gactatcgcc aaagagcgat taaactcctc gcaaactctt tttacggcta ttatggctat 1500
gcaaaagcac gctggtactg taaggagtgt gctgagtccg ttactgcttg gggtcgcgaa 1560
tacatcgagc tcgtgcggaa ggagctcgaa gaaaagtttg gctttaaagt tctctacatt 1620
gacactgatg gtctctatgc gactattccg ggtggtaagt ctgaggaaat taagaaaaag 1680
gctctagaat ttgtggatta cattaactcg aagctcccgg gtctcctgga gctcgaatat 1740
gaaggctttt ataaacgcgg cttcttcgtt accaagaaga gatatgcgct gattgatgaa 1800
gaaggcaaaa ttattactcg tggtctcgag attgtgcgcc gtgattggag cgaaattgcg 1860
aaagaaactc aagctaaagt tctcgagact attctcaaac acggcaacgt tgaagaagct 1920
gtgagaattg taaaagaagt aacccaaaag ctcgctaaat atgaaattcc accagagaag 1980
ctcgcgattt atgagcagat tactcccccc ctgcatgagt ataaggcgat tggtccccac 2040
gtggctgttg caaagagact ggctgctaga ggcgtgaaaa ttaaaccggg tatggtaata 2100
ggctacattg tactccgcgg cgatggtccg attagcaacc gtgcaattct agctgaggaa 2160
tacgatctga aaaagcacaa gtatgacgca gaatattaca ttgagaacca ggtgctcccg 2220
gcggtactcc gtattctgga ggcttttggc taccgtaagg aagacctccg ctaccaaaag 2280
actaaacagg ttgacctcac tgcttgcctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gctccgaagg agctcctgca gatgctggag 2520
aagcagaaaa agtga 2535

<210> 6
<211> 844
<212> PRT
<213> Artificial Sequence

<220>
<223> Description of Artificial Sequence:hybrid
      polymerase Sso7d fusion protein PhS2

<400> 6
Met Ile Leu Asp Val Asp Tyr Ile Thr Glu Glu Gly Lys Pro Val Ile
1               5                   10                  15

Arg Leu Phe Lys Lys Glu Asn Gly Glu Phe Lys Val Glu Tyr Asp Arg
            20                  25                  30

Thr Phe Arg Pro Tyr Ile Tyr Ala Leu Leu Lys Asp Asp Ser Lys Ile
        35                  40                  45

Asp Glu Val Arg Lys Ile Thr Gly Glu Arg His Gly Lys Ile Val Arg
    50                  55                  60

Ile Ile Asp Ala Glu Lys Val Arg Lys Lys Phe Leu Gly Lys Pro Ile
65                  70                  75                  80

Glu Val Trp Lys Leu Tyr Phe Glu His Pro Gln Asp Val Pro Thr Ile
                85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125
Met Glu Gly Glu Glu Glu Leu Lys Ile Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Val Ile Arg Glu Lys Asp Pro Asp Ile Ile Val Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Pro Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala His Glu Ile Ala Glu Ala Trp Glu Ser Gly Glu Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Phe Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Leu Ala
        355                 360                 365

Pro Asn Lys Pro Ser Glu Arg Glu Tyr Glu Arg Arg Leu Arg Glu Ser
    370                 375                 380

Tyr Thr Gly Gly Tyr Val Lys Glu Pro Glu Lys Gly Leu Trp Glu Asn
385                 390                 395                 400

Ile Val Tyr Leu Asp Phe Arg Ser Leu Tyr Pro Ser Ile Ile Ile Thr
                405                 410                 415

His Asn Val Ser Pro Asp Thr Leu Asn Leu Glu Gly Cys Lys Glu Tyr
            420                 425                 430

Asp Val Ala Pro Glu Val Gly His Lys Phe Cys Lys Asp Ile Pro Gly
        435                 440                 445

Phe Ile Pro Ser Leu Leu Gly His Leu Leu Glu Glu Arg Gln Lys Ile
    450                 455                 460

Lys Arg Lys Met Lys Ala Ser Lys Asp Pro Ile Glu Lys Ile Leu Leu
465                 470                 475                 480

Asp Tyr Arg Gln Arg Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Leu Val Arg Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Ser Glu Glu Ile Lys Lys Lys
545                 550                 555                 560

Ala Leu Glu Phe Val Asp Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Thr Ile Leu Lys His Gly Asn Val Glu Glu Ala
625                 630                 635                 640

Val Arg Ile Val Lys Glu Val Thr Gln Lys Leu Ala Lys Tyr Glu Ile
                645                 650                 655

Pro Pro Glu Lys Leu Ala Ile Tyr Glu Gln Ile Thr Pro Pro Leu His
            660                 665                 670

Glu Tyr Lys Ala Ile Gly Pro His Val Ala Val Ala Lys Arg Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Leu Lys Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg
            740                 745                 750

Lys Glu Asp Leu Arg Tyr Gln Lys Thr Lys Gln Val Asp Leu Thr Ala
        755                 760                 765

Cys Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 7
<211> 2535
<212> DNA
<213> Artificial Sequence

<220>
<223> Description of Artificial Sequence:hybrid
      polymerase Sso7d fusion protein PhS5

<400> 7
atgatcctgg atgctgacta catcactgaa gacggcaaac cgattatccg tctgttcaaa 60

aaagagaacg gcgaatttaa ggttgagtat gatcgcaact ttcgtccata catttacgct 120
ctgctgagag atgattctca gattgatgaa gttaaaaaaa tcactgctga gcgccatggc 180
aagattgttc gtatcattga tgcggaaaag gtagaaaaga aatttctggg cagaccaatc 240
accgtgtgga gactgtattt cgaacatcca caagatgttc cggctattcg cgataaagtt 300
cgcgaacatc ctgcagttgt tgacatcttc gaatacgata ttccatttgc aaagcgttac 360
ctcatcgaca aaggcctgat accaatggag ggcgaggaag aactcaagct cctggcgttc 420
gatatagaaa ccctctatca cgaaggcgaa gagtttggta aaggcccaat tataatgatc 480
agctatgcag atgaaaacga agcaaaggtg attacttgga aaaaaataga tctcccatac 540
gttgaggttg tatcttccga gcgcgagatg attaaacgtt ttctcagagt tatccgcgag 600
aaggatccgg acattatcat tacttataac ggcgactctt ttgacttccc atatctggcg 660
aaacgcgcag aaaaactcgg tattaaactg cctctcggcc gtgatggttc cgagccgaag 720
atgcagcgta tcggcgatat gaccgctgta gaaattaagg gtcgtatcca tttcgacctg 780
tatcatgtaa ttactcgtac tattaacctc ccgacttaca ctctcgaggc tgtatatgaa 840
gcaatttttg gtaagccgaa ggagaaggta tacgccgatg agattgcaga ggcgtgggaa 900
tccggtaaga acctcgagcg tgttgcaaaa tactccatgg aagatgcaaa ggcgacttat 960
gaactcggca aagaattcct cccaatggaa atccagctct ctcgcctggt tggccaacca 1020
ctgtgggatg tttctcgttc ttccaccggt aacctcgtag agtggtatct cctgcgcaaa 1080
gcgtacgaac gcaacgaagt ggctccgaac aagccagacg aagaagagta tgaacgccgt 1140
ctccgcgagt cttacactgg tggctatgtt aaagagccag aaaagggcct ctgggaaaac 1200
ctcgtgtccc tcgattttcg cgctctgtat ccgtctatta tcattaccca caacgtgtct 1260
ccggatactc tcaaccgcga gggctgcaaa gagtatgata ttgctccgca agtaggccac 1320
aagttctgca aggacttccc gggctttatt ccgtctctcc tgaaacatct gctcgatgaa 1380
cgccaagaga ttaagcgtaa aatgaaggcg tccaaggatc cgattgaaaa aaaaatgctc 1440
gactatcgcc aaagagcgat taaactcctc gcaaactctt tttacggcta ttatggctat 1500
gcaaaagcac gctggtactg taaggagtgt gctgagtccg ttactgcttg gggtcgcgaa 1560
tacatcgagc tcgtgtggaa ggagctcgaa gaaaagtttg gctttaaagt tctctacatt 1620
gacactgatg gtctctatgc gactattccg ggtggtaagc ctgaggaaat taagaaaaag 1680
gctctagaat ttgtgaaata cattaactcg aagctcccgg gtctcctgga gctcgaatat 1740
gaaggctttt atgttcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctagagt tctcgaggct attctcaaac acggcaacgt tgaagaagct 1920
gtgaaaattg taaaagaagt aacccaaaag ctcgctaaat atgaaattcc gccagagaag 1980
ctcgcgattt atgagcagat tactcgcccg ctgcatgagt ataaggcgat tggtccgcac 2040
gtggctgttg caaagagact ggctgctaga ggcgtgaaag ttagaccggg tatggtaatt 2100
ggctacattg tactccgcgg cgatggtccg attagcaacc gtgcaattct agctgaggaa 2160
tacgatctga aaaagcacaa gtatgacgca gaatattaca ttgagaacca ggtgctcccg 2220
gcggtactcc gtattctgga ggcttttggc taccgtaagg aagacctccg ctggcaaaag 2280
actaaacagg ttggcctcac ttcttggctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gcgccgaagg agctgctgca gatgctggag 2520
aagcagaaaa agtga 2535

<210> 8
<211> 844
<212> PRT
<213> Artificial Sequence

<220>
<223> Description of Artificial Sequence:hybrid
      polymerase Sso7d fusion protein PhS5

<400> 8
Met Ile Leu Asp Ala Asp Tyr Ile Thr Glu Asp Gly Lys Pro Ile Ile
1               5                   10                  15

Arg Leu Phe Lys Lys Glu Asn Gly Glu Phe Lys Val Glu Tyr Asp Arg
            20                  25                  30

Asn Phe Arg Pro Tyr Ile Tyr Ala Leu Leu Arg Asp Asp Ser Gln Ile
        35                  40                  45

Asp Glu Val Lys Lys Ile Thr Ala Glu Arg His Gly Lys Ile Val Arg
    50                  55                  60

Ile Ile Asp Ala Glu Lys Val Glu Lys Lys Phe Leu Gly Arg Pro Ile
65                  70                  75                  80

Thr Val Trp Arg Leu Tyr Phe Glu His Pro Gln Asp Val Pro Ala Ile

                85                  90                  95

Arg Asp Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Ile Ile Ile Thr
        195                 200                 205

Tyr Asn Gly Asp Ser Phe Asp Phe Pro Tyr Leu Ala Lys Arg Ala Glu
    210                 215                 220
Lys Leu Gly Ile Lys Leu Pro Leu Gly Arg Asp Gly Ser Glu Pro Lys
225                 230                 235                 240

Met Gln Arg Ile Gly Asp Met Thr Ala Val Glu Ile Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Glu Ala Trp Glu Ser Gly Lys Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Asp Glu Glu Glu Tyr Glu Arg Arg Leu Arg Glu Ser
    370                 375                 380

Tyr Thr Gly Gly Tyr Val Lys Glu Pro Glu Lys Gly Leu Trp Glu Asn
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Glu Tyr
            420                 425                 430

Asp Ile Ala Pro Gln Val Gly His Lys Phe Cys Lys Asp Phe Pro Gly
        435                 440                 445

Phe Ile Pro Ser Leu Leu Lys His Leu Leu Asp Glu Arg Gln Glu Ile
    450                 455                 460

Lys Arg Lys Met Lys Ala Ser Lys Asp Pro Ile Glu Lys Lys Met Leu
465                 470                 475                 480

Asp Tyr Arg Gln Arg Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Leu Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Val Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala
625                 630                 635                 640

Val Lys Ile Val Lys Glu Val Thr Gln Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Arg Gly Val Lys Val Arg Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Leu Lys Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg
            740                 745                 750

Lys Glu Asp Leu Arg Trp Gln Lys Thr Lys Gln Val Gly Leu Thr Ser
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 9
<211> 2535
<212> DNA
<213> Artificial Sequence

<220>
<223> Description of Artificial Sequence:hybrid
      polymerase Sso7d fusion protein PhS7

<400> 9
atgatcctgg atgctgacta catcactgaa gacggcaaac cgattatccg tctgttcaaa 60
aaagagaacg gcgaatttaa ggttgagtat gatcgcaact ttcgtccata catttacgct 120
ctgctgagag atgattctca gattgatgaa gttaaaaaaa tcactgctga gcgccatggc 180
aagattgttc gtatcattga tgcggaaaag gtagaaaaga aatttctggg cagaccaatc 240
accgtgtgga gactgtattt cgaacatcca caagatgttc cggctattcg cgataaagtt 300
cgcgaacatc ctgcagttgt tgacatcttc gaatacgata ttccatttgc aaagcgttac 360
ctcatcgaca aaggcctgat accaatggag ggcgaggaag aactcaagct cctggcgttc 420
gatatagaaa ccctctatca cgaaggcgaa gagtttggta aaggcccaat tataatgatc 480
agctatgcag atgaaaacga agcaaaggtg attacttgga aaaaaataga tctcccatac 540
gttgaggttg tatcttccga gcgcgagatg attaaacgtt ttctcagagt tatccgcgag 600
aaggatccgg acattatcat tacttataac ggcgactctt ttgacttccc atatctggcg 660
aaacgcgcag aaaaactcgg tattaaactg cctctcggcc gtgatggttc cgagccgaag 720
atgcagcgta tcggcgatat gaccgctgta gaaattaagg gtcgtatcca tttcgacctg 780
tatcatgtaa ttactcgtac tattaacctc ccgacttaca ctctcgaggc tgtatatgaa 840
gcaatttttg gtaagccgaa ggagaaggta tacgccgatg agattgcaga ggcgtgggaa 900
tccggtaaga acctcgagcg tgttgcaaaa tactccatgg aagatgcaaa ggcgacttat 960
gaactcggca aagaattcct cccaatggaa atccagctct ctcgcctggt tggccaacca 1020
ctgtgggatg tttctcgttc ttccaccggt aacctcgtag agtggtatct cctgcgcaaa 1080
gcgtacgaac gcaacgaagt ggctccgaac aagccagacg aagaagagta tgaacgccgt 1140
ctccgcgagt cttacactgg tggctatgtt aaagagccag aaaagggcct ctgggaaaac 1200
ctcgtgtccc tcgattttcg cgctctgtat ccgtctatta tcattaccca caacgtgtct 1260
ccggatactc tcaaccgcga gggctgcaga aactatgatg ttgctccgca agtaggccac 1320
aagttctgca aggacttccc gggctttatt ccgtctctcc tgggccgtct gctcgaggaa 1380
cgccaagaga ttaagactaa aatgaaggcg accaaggatc cgattgaaaa aaaactgctc 1440
gactatcgcc aaaaagcgat taaaatcctc gcaaactctt tttacggcta ttatggctat 1500
gcaaaagcac gctggtactg taaggagtgt gctgagtccg ttactgcttg gggtcgcaaa 1560
tacatcgagt tcgtgcggaa ggagctcgaa gaaaagtttg gctttaaagt tctctacatt 1620
gacactgatg gtctctatgc gactattccg ggtggtaagc ctgaggaaat taagaaaaag 1680
gctctagaat ttgtgaaata cattaactcg aagctcccgg gtctcctgga gctcgaatat 1740
gaaggctttt atgttcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctagagt tctcgaggct attctcaaac acggcaacgt tgaagaagct 1920
gtgaaaattg taaaagaagt aacccaaaag ctcgctaaat atgaaattcc gccagagaag 1980
ctcgcgattt atgagcagat tactcgcccg ctgcatgagt ataaggcgat tggtccgcac 2040
gtggctgttg caaagagact ggctgctaga ggcgtgaaag ttagaccggg tatggtaatt 2100
ggctacattg tactccgcgg cgatggtccg attagcaacc gtgcaattct agctgaggaa 2160
tacgatctga aaaagcacaa gtatgacgca gaatattaca ttgagaacca ggtgctcccg 2220
gcggtactcc gtattctgga ggcttttggc taccgtaagg aagacctccg ctggcaaaag 2280
actaaacagg ttggcctcac ttcttggctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gcgccgaagg agctgctgca gatgctggag 2520
aagcagaaaa agtga                                                  2535

<210> 10
<211> 844
<212> PRT
<213> Artificial Sequence

<220>
<223> Description of Artificial Sequence:hybrid
      polymerase Sso7d fusion protein PhS7

<400> 10
Met Ile Leu Asp Ala Asp Tyr Ile Thr Glu Asp Gly Lys Pro Ile Ile
1               5                   10                  15

Arg Leu Phe Lys Lys Glu Asn Gly Glu Phe Lys Val Glu Tyr Asp Arg
            20                  25                  30

Asn Phe Arg Pro Tyr Ile Tyr Ala Leu Leu Arg Asp Asp Ser Gln Ile
        35                  40                  45

Asp Glu Val Lys Lys Ile Thr Ala Glu Arg His Gly Lys Ile Val Arg
    50                  55                  60

Ile Ile Asp Ala Glu Lys Val Glu Lys Lys Phe Leu Gly Arg Pro Ile
65                  70                  75                  80

Thr Val Trp Arg Leu Tyr Phe Glu His Pro Gln Asp Val Pro Ala Ile
                85                  90                  95

Arg Asp Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Ile Ile Ile Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Ile Gly Asp Met Thr Ala Val Glu Ile Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Glu Ala Trp Glu Ser Gly Lys Asn
    290                 295                 300
Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Asp Glu Glu Glu Tyr Glu Arg Arg Leu Arg Glu Ser

370                 375                 380

Tyr Thr Gly Gly Tyr Val Lys Glu Pro Glu Lys Gly Leu Trp Glu Asn
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Arg Asn Tyr
            420                 425                 430

Asp Val Ala Pro Gln Val Gly His Lys Phe Cys Lys Asp Phe Pro Gly
        435                 440                 445

Phe Ile Pro Ser Leu Leu Gly Arg Leu Leu Glu Glu Arg Gln Glu Ile
    450                 455                 460

Lys Thr Lys Met Lys Ala Thr Lys Asp Pro Ile Glu Lys Lys Leu Leu
465                 470                 475                 480

Asp Tyr Arg Gln Lys Ala Ile Lys Ile Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Lys Tyr Ile Glu Phe Val Arg Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Val Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala
625                 630                 635                 640

Val Lys Ile Val Lys Glu Val Thr Gln Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Arg Gly Val Lys Val Arg Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Leu Lys Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg

740                 745                 750

Lys Glu Asp Leu Arg Trp Gln Lys Thr Lys Gln Val Gly Leu Thr Ser
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 11
<211> 2337
<212> DNA
<213> Artificial Sequence

<220>
<223> Description of Artificial Sequence:hybrid
      polymerase Hyb1

<220>
<221> modified_base
<222> (1416)
<223> n = g, a, c or t

<400> 11
atgatcctgg atgctgacta catcactgaa gacggcaaac cggttatccg tctcttcaaa 60
aaagagaacg gcgaatttaa gattgagtat gatcgcacct ttcgtccata catttacgct 120
ctgctgagag atgattctaa gattgaggaa gttagaaaaa tcactgctga gcgccatggc 180
aagattgttc gtatcgttga tgtggaaaag gtaaggaaga aatttctggg cagaccaatc 240
aaggtgtgga gactgtattt cgaacatcca caagatgttc cgactattcg cgataaagtt 300
cgcgaacatc ctgcagttat tgacatcttc gaatacgata ttgcatttgc aaagcgttac 360
ctcatcgaca aaggcctgat accaatggag ggcgaggaag aactcaagat cctggcgttc 420
gatatagaaa ccctctatca cggaagcgaa gagtttggta aaggcccaat tataatgatc 480
agctatgcag atgaaaacga agcaaaggtg attacttgga aaaacataga tctcccatac 540
gttgaggttg tatcttccga gcgcgagatg attaaacgct ttctcagaat tatccgcgag 600
aaggatccgg acattatcgt tacttataac ggcgactctt ttgacctccc atatctggcg 660
aaacgcgcag aaaaactcgg tattaaactg actctcggcc gtgatggttg cgaggcgaag 720
atgcagcgtc tcggcgatat gaccgctgta gaagttaagg gtcgtatcca tttcgacctg 780
tattatgtaa ttagccgtac tattaacctc ccgacttaca ctctcgaggc tgtatatgaa 840
gcaatttttg gtaagccgaa ggagaaggta tacgccgatg atattgcaga ggcgtgggaa 900
accggtaagg gcctcgagcg tgttgcaaaa tactccatgg aagatgcaaa ggcgacttat 960
gaactcggca aagaattcct cccaatggaa gctcagctct ctcgcctggt tggccaacca 1020
ctgtgggatg tttctcgttc ttccaccggt aacctcgtag agtggtatct cctgcgcaaa 1080
gcgtacgaac gcaacgaagt ggctccgaac aagccatacg aacgagagta tgaacgccgt 1140
ctccgcgagt cttacactgg tggctttgtt aaagagccag aaaagggcct ctgggaaagc 1200
ctcgtgtccc tcgattttcg ctctctgtat ccgtctatta tcattaccca caacgtgtct 1260
ccggatactc tcaaccgcga gggctgcaaa gactatgata ttgctccgga agtaggccac 1320
aagttctgca aggacttcct tggctttatt ccgtctctcc tggggcatct gctcgaggaa 1380
cgccaagaga ttaagaccaa aatgaaggag acccangatc cgattgaaaa aatactgctc 1440
gactatcgcc aaaaagcgat taaactcctc gcaaactctt attacggcta ttatggctat 1500
gcaaaagcac gctggtactg taaggagtgt gctgagtccg ttactgcttg gggtcgcgaa 1560
tacatcgagt tcgtgtggaa ggagctcgaa gaaaagtttg gctttaaagt tctctacatt 1620
gacactgatg gtctctatgc gactattccg ggtggtgagc ctgaggaaat taagaaaaag 1680
gctctagaat ttgtgaaata cattaactcg aagctccccg gtctcttgga gctcgaatat 1740
gaaggctttt ataagcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctaaagt tctcgaggct attctcaaac acggcaacgt tgaagaagct 1920
gtgaaaattg taaaagaaat aatcgaaaag ctcgctaaat atgaaatacc gccagagaag 1980
ctcgcgattt atgagcagat tactcgcccg ctgcatgagt ataaggcgat tggtccgcac 2040

gtggctgttg caaagaaact ggctgctaga ggcgtgaaaa ttaaaccggg tatggtaatt 2100
ggctacattg tactccgcgg cgatggtccg attagcaaac gtgcaattct agctgaggaa 2160
ttcgatccga aaaagcacaa gtatgacgca gaatattaca ttgagaacca ggtgctcccg 2220
gcggtactcc gtattctgga gggttttggc taccgtaagg aagacctccg ttggcaaaag 2280
actaaacagg ctggcctcac tgcttggctc aacattaaaa aatccggtac ccactag 2337

<210> 12
<211> 775
<212> PRT
<213> Artificial Sequence

<220>
<223> Description of Artificial Sequence:hybrid
      polymerase Hyb1

<220>
<221> MOD_RES
<222> (472)
<223> Xaa = unknown amino acid

<400> 12
Met Ile Leu Asp Ala Asp Tyr Ile Thr Glu Asp Gly Lys Pro Val Ile
1               5                   10                  15

Arg Leu Phe Lys Lys Glu Asn Gly Glu Phe Lys Ile Glu Tyr Asp Arg
            20                  25                  30

Thr Phe Arg Pro Tyr Ile Tyr Ala Leu Leu Arg Asp Asp Ser Lys Ile
        35                  40                  45
Glu Glu Val Arg Lys Ile Thr Ala Glu Arg His Gly Lys Ile Val Arg
    50                  55                  60

Ile Val Asp Val Glu Lys Val Arg Lys Lys Phe Leu Gly Arg Pro Ile
65                  70                  75                  80

Lys Val Trp Arg Leu Tyr Phe Glu His Pro Gln Asp Val Pro Thr Ile
                85                  90                  95

Arg Asp Lys Val Arg Glu His Pro Ala Val Ile Asp Ile Phe Glu Tyr
            100                 105                 110

Asp Ile Ala Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Gly Ser Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Leu Gly Arg Asp Gly Cys Glu Ala Lys
225                 230                 235                 240

Met Gln Arg Leu Gly Asp Met Thr Ala Val Glu Val Lys Gly Arg Ile
                245                 250                 255

His Phe Asp Leu Tyr Tyr Val Ile Ser Arg Thr Ile Asn Leu Pro Thr

            260                 265                 270

Tyr Thr Leu Glu Ala Val Tyr Glu Ala Ile Phe Gly Lys Pro Lys Glu
        275                 280                 285

Lys Val Tyr Ala Asp Asp Ile Ala Glu Ala Trp Glu Thr Gly Lys Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Tyr Glu Arg Glu Tyr Glu Arg Arg Leu Arg Glu Ser
    370                 375                 380

Tyr Thr Gly Gly Phe Val Lys Glu Pro Glu Lys Gly Leu Trp Glu Ser
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala

625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Ala Gly Leu Thr Ala
        755                 760                 765

Trp Leu Asn Ile Lys Lys Ser
    770                 775

<210> 13
<211> 2535
<212> DNA
<213> Artificial Sequence

<220>
<223> Description of Artificial Sequence:hybrid
      polymerase Hyb1-Sso7d fusion protein HyS1

<220>
<221> modified_base
<222> (1416)
<223> n = g, a, c or t

<400> 13
atgatcctgg atgctgacta catcactgaa gacggcaaac cggttatccg tctcttcaaa 60
aaagagaacg gcgaatttaa gattgagtat gatcgcacct ttcgtccata catttacgct 120
ctgctgagag atgattctaa gattgaggaa gttagaaaaa tcactgctga gcgccatggc 180
aagattgttc gtatcgttga tgtggaaaag gtaaggaaga aatttctggg cagaccaatc 240
aaggtgtgga gactgtattt cgaacatcca caagatgttc cgactattcg cgataaagtt 300
cgcgaacatc ctgcagttat tgacatcttc gaatacgata ttgcatttgc aaagcgttac 360
ctcatcgaca aaggcctgat accaatggag ggcgaggaag aactcaagat cctggcgttc 420
gatatagaaa ccctctatca cggaagcgaa gagtttggta aaggcccaat tataatgatc 480
agctatgcag atgaaaacga agcaaaggtg attacttgga aaaacataga tctcccatac 540
gttgaggttg tatcttccga gcgcgagatg attaaacgct ttctcagaat tatccgcgag 600
aaggatccgg acattatcgt tacttataac ggcgactctt ttgacctccc atatctggcg 660
aaacgcgcag aaaaactcgg tattaaactg actctcggcc gtgatggttg cgaggcgaag 720
atgcagcgtc tcggcgatat gaccgctgta gaagttaagg gtcgtatcca tttcgacctg 780
tattatgtaa ttagccgtac tattaacctc ccgacttaca ctctcgaggc tgtatatgaa 840
gcaatttttg gtaagccgaa ggagaaggta tacgccgatg atattgcaga ggcgtgggaa 900
accggtaagg gcctcgagcg tgttgcaaaa tactccatgg aagatgcaaa ggcgacttat 960
gaactcggca aagaattcct cccaatggaa gctcagctct ctcgcctggt tggccaacca 1020
ctgtgggatg tttctcgttc ttccaccggt aacctcgtag agtggtatct cctgcgcaaa 1080
gcgtacgaac gcaacgaagt ggctccgaac aagccatacg aacgagagta tgaacgccgt 1140
ctccgcgagt cttacactgg tggctttgtt aaagagccag aaaagggcct ctgggaaagc 1200
ctcgtgtccc tcgattttcg ctctctgtat ccgtctatta tcattaccca caacgtgtct 1260
ccggatactc tcaaccgcga gggctgcaaa gactatgata ttgctccgga agtaggccac 1320
aagttctgca aggacttcct tggctttatt ccgtctctcc tggggcatct gctcgaggaa 1380
cgccaagaga ttaagaccaa aatgaaggag acccangatc cgattgaaaa aatactgctc 1440

gactatcgcc aaaaagcgat taaactcctc gcaaactctt attacggcta ttatggctat 1500
gcaaaagcac gctggtactg taaggagtgt gctgagtccg ttactgcttg ggtcgcgaa 1560
tacatcgagt tcgtgtggaa ggagctcgaa gaaaagtttg gctttaaagt tctctacatt 1620
gacactgatg gtctctatgc gactattccg ggtggtgagc ctgaggaaat taagaaaaag 1680
gctctagaat ttgtgaaata cattaactcg aagctccccg gtctcttgga gctcgaatat 1740
gaaggctttt ataagcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctaaagt tctcgaggct attctcaaac acggcaacgt tgaagaagct 1920
gtgaaaattg taaaagaaat aatcgaaaag ctcgctaaat atgaaatacc gccagagaag 1980
ctcgcgattt atgagcagat tactcgcccg ctgcatgagt ataaggcgat tggtccgcac 2040
gtggctgttg caaagaaact ggctgctaga ggcgtgaaaa ttaaaccggg tatggtaatt 2100
ggctacattg tactccgcgg cgatggtccg attagcaaac gtgcaattct agctgaggaa 2160
ttcgatccga aaaagcacaa gtatgacgca gaatattaca ttgagaacca ggtgctcccg 2220
gcggtactcc gtattctgga gggttttggc taccgtaagg aagacctccg ttggcaaaag 2280
actaaacagg ctggcctcac tgcttggctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gcgccgaagg agctgctgca gatgctggag 2520
aagcagaaaa agtga 2535

<210> 14
<211> 844
<212> PRT
<213> Artificial Sequence

<220>
<223> Description of Artificial Sequence:hybrid
      polymerase Hyb1-Sso7d fusion protein HyS1

<220>
<221> MOD_RES
<222> (472)
<223> Xaa = unknown amino acid

<400> 14
Met Ile Leu Asp Ala Asp Tyr Ile Thr Glu Asp Gly Lys Pro Val Ile
  1               5                  10                  15

Arg Leu Phe Lys Lys Glu Asn Gly Glu Phe Lys Ile Glu Tyr Asp Arg
             20                  25                  30

Thr Phe Arg Pro Tyr Ile Tyr Ala Leu Leu Arg Asp Asp Ser Lys Ile
         35                  40                  45

Glu Glu Val Arg Lys Ile Thr Ala Glu Arg His Gly Lys Ile Val Arg
     50                  55                  60

Ile Val Asp Val Glu Lys Val Arg Lys Lys Phe Leu Gly Arg Pro Ile
 65                  70                  75                  80

Lys Val Trp Arg Leu Tyr Phe Glu His Pro Gln Asp Val Pro Thr Ile
                 85                  90                  95

Arg Asp Lys Val Arg Glu His Pro Ala Val Ile Asp Ile Phe Glu Tyr
            100                 105                 110

Asp Ile Ala Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Gly Ser Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Leu Gly Arg Asp Gly Cys Glu Ala Lys
225                 230                 235                 240

Met Gln Arg Leu Gly Asp Met Thr Ala Val Glu Val Lys Gly Arg Ile
                245                 250                 255

His Phe Asp Leu Tyr Tyr Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Asp Ile Ala Glu Ala Trp Glu Thr Gly Lys Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Tyr Glu Arg Glu Tyr Glu Arg Arg Leu Arg Glu Ser
    370                 375                 380

Tyr Thr Gly Gly Phe Val Lys Glu Pro Glu Lys Gly Leu Trp Glu Ser
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Ala Gly Leu Thr Ala
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 15
<211> 2340
<212> DNA
<213> Artificial Sequence

<220>
<223> Description of Artificial Sequence:truncated
      hybrid polymerase Hyb2

<220>
<221> modified_base

<222> (1416)
<223> n = g, a, c or t

<400> 15
atgatcctgg atgctgacta catcactgaa gaaggcaaac cggttatccg tatcttcaaa 60
aaagagaacg gcgaatttaa ggttgagtat gatcgcaact ttcgtccata catttacgct 120
ctgctggaag atgattctaa gattgatgaa gttagaaaaa tcactgctga gcgccatggc 180
aagattgttc gtatcgttga tgcggaaaag gtagagaaga aatttctggg cagaccaatc 240
acggtgtgga aactgtattt cgaacatcca caagatgttc cgactattcg cgagaaaatt 300
cgcgaacatt ctgcagttgt tggcatcttc gaatacgata ttccatttgc aaagagttac 360
ctcatcgaca aaggcctgat accaatggag ggcgaggaag aactcaagct cctggcgttc 420
gatatagaaa ccctctatca cgaaggcgaa gagtttgcta aaggcccaat tataatgatc 480
agctatgcag atgaagacga agcaaaggtg attacttgga aaaaaataga tctcccatac 540
gttgaggttg tatcttccga gcgcgagatg attaagcgct ttctcagagt tatccgcgag 600
aaggatccgg acgttatcgt tacttataac ggcgactctt ttgacctccc atatctggcg 660
aaacgcgcag aaaaactcgg tattaaactg cctctcggcc gtgatggttc cgagccgaag 720
atgcagcgtc tcggcgatat gaccgctgta gaagttaagg gtcgtatcca tttcgacctg 780
tatcatgtaa ttagccgtac tattaacctc ccgacttaca ctctcgaggc tgtatatgaa 840
gcaatttttg gtaagccgaa ggagaaggta tacgccgatg agattgcagg ggcgtgggaa 900
accggtgagg acctcgagcg tgttgcaaaa tactccatgg aagatgcaaa ggcgatttat 960
gaactcggca aagaattctt cccaatggaa gttcagctcc ctcgcctggt tggccaacca 1020
ctgtgggatg tttctcgttc ttccaccggt aacctcgtag agtggttgct cctgcgcaaa 1080
gcgtacgaac gcaacgaact ggctccgaac aagccagccg aacaagagta tgaacgccgt 1140
ctccgcgagt cttacactgg tggctttgtt aaagagccag aaaagggcct ctgggaagac 1200
ctcgtgtccc tcgattttcg cgctctgtat ccgtctatta tcattaccca caacgtgtct 1260
ccggatactc tcaaccgcga gggctgcaaa gactatgata ttgctccgga agtaggccac 1320
aagttctgca aggacttcct tggctttatt ccgtctctcc tggggcatct gctcgaggaa 1380
cgccaagaga ttaagaccaa aatgaaggag acccangatc cgattgaaaa aatactgctc 1440
gactatcgcc aaaaagcgat taaactcctc gcaaactctt attacggcta ttatggctat 1500
gcaaaagcac gctggtactg taaggagtgt gctgagtccg ttactgcttg gggtcgcgaa 1560
tacatcgagt tcgtgtggaa ggagctcgaa gaaaagtttg gctttaaagt tctctacatt 1620
gacactgatg gtctctatgc gactattccg ggtggtgagc ctgaggaaat taagaaaaag 1680
gctctagaat ttgtgaaata cattaactcg aagctccccg gtctcttgga gctcgaatat 1740
gaaggctttt ataagcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctaaagt tctcgaggct attctcaaac acggcaacgt tgaagaagct 1920
gtgaaaattg taaaagaaat aatcgaaaag ctcgctaaat atgaaatacc gccagagaag 1980
ctcgcgattt atgagcagat tactcgcccg ctgcatgagt ataaggcgat tggtccgcac 2040
gtggctgttg caaagaaact ggctgctaga ggcgtgaaaa ttaaaccggg tatggtaatt 2100
ggctacattg tactccgcgg cgatggtccg attagcaacc gtgcaattct agctgaggaa 2160
ttcgatctga gaaagcacaa gtatgacgca gaatattaca ttgagaacca ggtgctcccg 2220
gcggtactcc gtattctgga gggttttggc taccgtaagg aagacctccg ttagcaaaag 2280
actaaacagg ctggactcac tgcttggctc atcattaaaa aatccggtac ccactagtgc 2340

<210> 16
<211> 757
<212> PRT
<213> Artificial Sequence

<220>
<223> Description of Artificial Sequence:truncated
      hybrid polymerase Hyb2

<220>
<221> MOD_RES
<222> (472)
<223> Xaa = unknown amino acid

<400> 16
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Glu Asp Asp Ser Lys Ile
         35                  40                  45

Asp Glu Val Arg Lys Ile Thr Ala Glu Arg His Gly Lys Ile Val Arg

50                  55                  60

Ile Val Asp Ala Glu Lys Val Glu Lys Lys Phe Leu Gly Arg Pro Ile
65                  70                  75                  80

Thr Val Trp Lys Leu Tyr Phe Glu His Pro Gln Asp Val Pro Thr Ile
                85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205
Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Phe Asp Leu Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg
        755

<210> 17
<211> 2340
<212> DNA
<213> Artificial Sequence

<220>
<223> Description of Artificial Sequence:truncated
      hybrid polymerase Hyb3

<220>
<221> modified_base
<222> (1416)
<223> n = g, a, c or t

<400> 17
atgatcctgg atgctgacta catcactgaa gaaggcaaac cggttatccg tatcttcaaa 60
aaagagaacg gcgaatttaa ggttgagtat gatcgcaact ttcgtccata catttacgct 120
ctgctggaag atgattctaa gattgatgaa gttagaaaaa tcactgctga gcgccatggc 180
aagattgttc gtatcgttga tgcggaaaag gtagagaaga aatttctggg cagaccaatc 240
acggtgtgga aactgtattt cgaacatcca caagatgttc cgactattcg cgagaaaatt 300
cgcgaacatt ctgcagttgt tggcatcttc gaatacgata ttccatttgc aaagagttac 360
ctcatcgaca aaggcctgat accaatggag ggcgaggaag aactcaagct cctggcgttc 420
gatatagaaa ccctctatca cgaaggcgaa gagtttgcta aaggcccaat tataatgatc 480
agctatgcag atgaagacga agcaaaggtg attacttgga aaaaaataga tctcccatac 540
gttgaggttg tatcttccga gcgcgagatg attaagcgct ttctcagagt tatccgcgag 600
aaggatccgg acgttatcgt tacttataac ggcgactctt ttgacctccc atatctggcg 660
aaacgcgcag aaaaactcgg tattaaactg cctctcggcc gtgatggttc cgagccgaag 720
atgcagcgtc tcggcgatat gaccgctgta gaagttaagg gtcgtatcca tttcgacctg 780
tatcatgtaa ttagccgtac tattaacctc ccgacttaca ctctcgaggc tgtatatgaa 840
gcaatttttg gtaagccgaa ggagaaggta tacgccgatg agattgcagg ggcgtgggaa 900
accggtgagg acctcgagcg tgttgcaaaa tactccatgg aagatgcaaa ggcgatttat 960
gaactcggca aagaattctt cccaatggaa gttcagctcc ctcgcctggt tggccaacca 1020
ctgtgggatg tttctcgttc ttccaccggt aacctcgtag agtggttgct cctgcgcaaa 1080
gcgtacgaac gcaacgaact ggctccgaac aagccagccg aacaagagta tgaacgccgt 1140
ctccgcgagt cttacactgg tggctttgtt aaagagccag aaaagggcct ctgggaagac 1200
ctcgtgtccc tcgattttcg cgctctgtat ccgtctatta tcattaccca caacgtgtct 1260
ccggatactc tcaaccgcga gggctgcaaa gactatgata ttgctccgga agtaggccac 1320
aagttctgca aggacttcct tggctttatt ccgtctctcc tggggcatct gctcgaggaa 1380
cgccaagaga ttaagaccaa aatgaaggag acccangatc cgattgaaaa aatactgctc 1440
gactatcgcc aaaaagcgat taaactcctc gcaaactctt attacggcta ttatggctat 1500
gcaaaagcac gctggtactg taaggagtgt gctgagtccg ttactgcttg ggtcgcgaa 1560
tacatcgagt tcgtgtggaa ggagctcgaa gaaaagtttg gctttaaagt tctctacatt 1620
gacactgatg gtctctatgc gactattccg ggtggtgagc ctgaggaaat taagaaaaag 1680
gctctagaat ttgtgaaata cattaactcg aagctccccg gtctcttgga gctcgaatat 1740
gaaggctttt ataagcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctaaagt tctcgaggct attctcaaac acggcaacgt tgaagaagct 1920
gtgaaaattg taaaagaaat aatcgaaaag ctcgctaaat atgaaatacc gccagagaag 1980
ctcgcgattt atgagcagat tactcgcccg ctgcatgagt ataaggcgat tggtccgcac 2040
gtggctgttg caaagaaact ggctgctaga ggcgtgaaaa ttaaaccggg tatggtaatt 2100
ggctacattg tactccgcgg cgatggtccg attagcaacc gtgcaattct agctgaggaa 2160
ttcgatctga gaaagcacaa gtatgacgca gaatattaca ttgagaacca ggtgctcccg 2220
gcggtactcc gtattctgga gggttttggc taccgtaagg aagacctccg ttagcaaaag 2280
actaaacagg ctggactcac tgcttggctc atcattaaaa aatccggtac ccactagtgc 2340

<210> 18
<211> 757
<212> PRT
<213> Artificial Sequence

<220>
<223> Description of Artificial Sequence:truncated
      hybrid polymerase Hyb3

<220>
<221> MOD_RES
<222> (472)
<223> Xaa = unknown amino acid

<400> 18
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
            20                  25                  30

Asn Phe Arg Pro Tyr Ile Tyr Ala Leu Leu Glu Asp Asp Ser Lys Ile
        35                  40                  45

Asp Glu Val Arg Lys Ile Thr Ala Glu Arg His Gly Lys Ile Val Arg
    50                  55                  60

Ile Val Asp Ala Glu Lys Val Glu Lys Lys Phe Leu Gly Arg Pro Ile
65                  70                  75                  80

Thr Val Trp Lys Leu Tyr Phe Glu His Pro Gln Asp Val Pro Thr Ile
                85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Phe Asp Leu Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg
        755

<210> 19
<211> 2535
<212> DNA
<213> Artificial Sequence

<220>
<223> Description of Artificial Sequence:hybrid
      polymerase Sso7d fusion protein HyS4

<220>
<221> modified_base
<222> (1416)
<223> n = g, a, c or t

<400> 19
atgatcctgg atgctgacta catcactgaa gaaggcaaac cggttatccg tatcttcaaa 60
aaagagaacg gcgaatttaa ggttgagtat gatcgcaact ttcgtccata catttacgct 120
ctgctggaag atgattctaa gattgatgaa gttagaaaaa tcactgctga gcgccatggc 180
aagattgttc gtatcgttga tgcggaaaag gtagagaaga aatttctggg cagaccaatc 240
acggtgtgga aactgtattt cgaacatcca caagatgttc cgactattcg cgagaaaatt 300
cgcgaacatt ctgcagttgt tggcatcttc gaatacgata ttccatttgc aaagagttac 360
ctcatcgaca aaggcctgat accaatggag ggcgaggaag aactcaagct cctggcgttc 420
gatatagaaa ccctctatca cgaaggcgaa gagtttgcta aaggcccaat tataatgatc 480
agctatgcag atgaagacga agcaaaggtg attacttgga aaaaaataga tctcccatac 540
gttgaggttg tatcttccga gcgcgagatg attaagcgct ttctcagagt tatccgcgag 600
aaggatccgg acgttatcgt tacttataac ggcgactctt ttgacctccc atatctggcg 660
aaacgcgcag aaaaactcgg tattaaactg cctctcggcc gtgatggttc cgagccgaag 720
atgcagcgtc tcggcgatat gaccgctgta gaagttaagg gtcgtatcca tttcgacctg 780
tatcatgtaa ttagccgtac tattaacctc ccgacttaca ctctcgaggc tgtatatgaa 840
gcaatttttg gtaagccgaa ggagaaggta tacgccgatg agattgcagg ggcgtgggaa 900
accggtgagg acctcgagcg tgttgcaaaa tactccatgg aagatgcaaa ggcgatttat 960
gaactcggca aagaattctt cccaatggaa gttcagctcc ctcgcctggt tggccaacca 1020
ctgtgggatg tttctcgttc ttccaccggt aacctcgtag agtggttgct cctgcgcaaa 1080
gcgtacgaac gcaacgaact ggctccgaac aagccagccg aacaagagta tgaacgccgt 1140
ctccgcgagt cttacactgg tggctttgtt aaagagccag aaaagggcct ctgggaagac 1200
ctcgtgtccc tcgattttcg cgctctgtat ccgtctatta tcattaccca caacgtgtct 1260
ccggatactc tcaaccgcga gggctgcaaa gactatgata ttgctccgga agtaggccac 1320
aagttctgca aggacttcct tggctttatt ccgtctctcc tggggcatct gctcgaggaa 1380
cgccaagaga ttaagaccaa aatgaaggag acccangatc cgattgaaaa aatactgctc 1440
gactatcgcc aaaaagcgat taaactcctc gcaaactctt attacggcta ttatggctat 1500
gcaaaagcac gctggtactg taaggagtgt gctgagtccg ttactgcttg gggtcgcgaa 1560
tacatcgagt tcgtgtggaa ggagctcgaa gaaaagtttg gctttaaagt tctctacatt 1620
gacactgatg gtctctatgc gactattccg ggtggtgagc ctgaggaaat taagaaaaag 1680
gctctagaat ttgtgaaata cattaactcg aagctccccg gtctcttgga gctcgaatat 1740
gaaggctttt ataagcgcgg cttcttcgtt accaagaaga gatatgcggt gattgatgaa 1800
gaaggcaaaa ttattactcg tggtctcgag attgtgcgcc gtgattggag cgaaattgcg 1860
aaagaaactc aagctaaagt tctcgaggct attctcaaac acggcaacgt tgaagaagct 1920
gtgaaaattg taaaagaaat aatcgaaaag ctcgctaaat atgaaatacc gccagagaag 1980
ctcgcgattt atgagcagat tactcgcccg ctgcatgagt ataaggcgat tggtccgcac 2040
gtggctgttg caaagaaact ggctgctaga ggcgtgaaaa ttaaaccggg tatggtaatt 2100
ggctacattg tactccgcgg cgatggtccg attagcaaac gtgcaattct agctgaggaa 2160
ttcgatccga aaaagcacaa gtatgacgca gaatattaca ttgagaacca ggtgctcccg 2220
gcggtactcc gtattctgga gggttttggc taccgtaagg aagacctccg ttggcaaaag 2280
actaaacagg ctggcctcac tgcttggctc aacattaaaa aatccggtac cggcggtggc 2340
ggtgcaaccg taaagttcaa gtacaaaggc gaagaaaaag aggtagacat ctccaagatc 2400
aagaaagtat ggcgtgtggg caagatgatc tccttcacct acgacgaggg cggtggcaag 2460
accggccgtg gtgcggtaag cgaaaaggac gcgccgaagg agctgctgca gatgctggag 2520
aagcagaaaa agtga 2535

<210> 20
<211> 844
<212> PRT
<213> Artificial Sequence

<220>
<223> Description of Artificial Sequence:hybrid
      polymerase Sso7d fusion protein HyS4

<220>
<221> MOD_RES
<222> (472)
<223> Xaa = unknown amino acid

<400> 20
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Glu Asp Asp Ser Lys Ile
         35                  40                  45

Asp Glu Val Arg Lys Ile Thr Ala Glu Arg His Gly Lys Ile Val Arg
     50                  55                  60

Ile Val Asp Ala Glu Lys Val Glu Lys Lys Phe Leu Gly Arg Pro Ile
 65                  70                  75                  80

Thr Val Trp Lys Leu Tyr Phe Glu His Pro Gln Asp Val Pro Thr Ile
                 85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Ala Gly Leu Thr Ala
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys
        835                 840

<210> 21
<211> 189
<212> DNA
<213> Artificial Sequence

<220>
<223> Description of Artificial Sequence:Sso7d coding
      region

<400> 21
accgtaaagt tcaagtacaa aggcgaagaa aaagaggtag acatctccaa gatcaagaaa 60
gtatggcgtg tgggcaagat gatctccttc acctacgacg agggcggtgg caagaccggc 120
cgtggtgcgg taagcgaaaa ggacgcgccg aaggagctgc tgcagatgct ggagaagcag 180
aaaaagtga                                                         189

<210> 22
<211> 63
<212> PRT
<213> Artificial Sequence

<220>
<223> Description of Artificial Sequence:Sso7d binding
      domain

<400> 22
Ala Thr Val Lys Phe Lys Tyr Lys Gly Glu Glu Lys Glu Val Asp Ile
  1               5                  10                  15

Ser Lys Ile Lys Lys Val Trp Arg Val Gly Lys Met Ile Ser Phe Thr
             20                  25                  30

Tyr Asp Glu Gly Gly Gly Lys Thr Gly Arg Gly Ala Val Ser Glu Lys
         35                  40                  45

Asp Ala Pro Lys Glu Leu Leu Gln Met Leu Glu Lys Gln Lys Lys
     50                  55                  60

<210> 23
<211> 25
<212> PRT
<213> Artificial Sequence

<220>
<223> Description of Artificial Sequence:signature
      invariable sequence element in hybrid polymerases
      containing nucleotide binding motif

<400> 23
Tyr Gly Tyr Tyr Gly Tyr Ala Lys Ala Arg Trp Tyr Cys Lys Glu Cys
  1               5                  10                  15

Ala Glu Ser Val Thr Ala Trp Gly Arg
             20                  25

<210> 24
<211> 775
<212> PRT
<213> Artificial Sequence

<220>
<223> Description of Artificial Sequence:parent
      Pyrococcus furiosus family B DNA polymerase (Pfu)

<400> 24
Met Ile Leu Asp Val Asp Tyr Ile Thr Glu Glu Gly Lys Pro Val Ile
  1               5                  10                  15

Arg Leu Phe Lys Lys Glu Asn Gly Lys Phe Lys Ile Glu His Asp Arg
             20                  25                  30

Thr Phe Arg Pro Tyr Ile Tyr Ala Leu Leu Arg Asp Asp Ser Lys Ile
         35                  40                  45

Glu Glu Val Lys Lys Ile Thr Gly Glu Arg His Gly Lys Ile Val Arg
     50                  55                  60

Ile Val Asp Val Glu Lys Val Glu Lys Lys Phe Leu Gly Lys Pro Ile
 65                  70                  75                  80

Thr Val Trp Lys Leu Tyr Leu Glu His Pro Gln Asp Val Pro Thr Ile
                 85                  90                  95

Arg Glu Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205
Tyr Asn Gly Asp Ser Phe Asp Phe Pro Tyr Leu Ala Lys Arg Ala Glu

210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Lys Ala Trp Glu Ser Gly Glu Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Val Ala
        355                 360                 365

Pro Asn Lys Pro Ser Glu Glu Glu Tyr Gln Arg Arg Leu Arg Glu Ser
    370                 375                 380

Tyr Thr Gly Gly Phe Val Lys Glu Pro Glu Lys Gly Leu Trp Glu Asn
385                 390                 395                 400

Ile Val Tyr Leu Asp Phe Arg Ala Leu Tyr Pro Ser Ile Ile Ile Thr
                405                 410                 415

His Asn Val Ser Pro Asp Thr Leu Asn Leu Glu Gly Cys Lys Asn Tyr
            420                 425                 430

Asp Ile Ala Pro Gln Val Gly His Lys Phe Cys Lys Asp Ile Pro Gly
        435                 440                 445

Phe Ile Pro Ser Leu Leu Gly His Leu Leu Glu Glu Arg Gln Lys Ile
    450                 455                 460

Lys Thr Lys Met Lys Glu Thr Gln Asp Pro Ile Glu Lys Ile Leu Leu
465                 470                 475                 480

Asp Tyr Arg Gln Lys Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Lys Tyr Ile Glu Leu Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Ser Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys

                580                 585                 590

Lys Arg Tyr Ala Val Ile Asp Glu Glu Gly Lys Val Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Thr Ile Leu Lys His Gly Asp Val Glu Glu Ala
625                 630                 635                 640

Val Arg Ile Val Lys Glu Val Ile Gln Lys Leu Ala Asn Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Lys Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Tyr Gln Lys Thr Arg Gln Val Gly Leu Thr Ser
        755                 760                 765

Trp Leu Asn Ile Lys Lys Ser
    770                 775


<210> 25
<211> 775
<212> PRT
<213> Artificial Sequence

<220>
<223> Description of Artificial Sequence:parent
      Pyrococcus sp. GD-B (Deep Vent) DNA polymerase

<400> 25
Met Ile Leu Asp Ala Asp Tyr Ile Thr Glu Asp Gly Lys Pro Ile Ile
  1               5                  10                  15
Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Lys Asp Asp Ser Gln Ile
         35                  40                  45

Asp Glu Val Arg Lys Ile Thr Ala Glu Arg His Gly Lys Ile Val Arg
     50                  55                  60

Ile Ile Asp Ala Glu Lys Val Arg Lys Lys Phe Leu Gly Arg Pro Ile
 65                  70                  75                  80

Glu Val Trp Arg Leu Tyr Phe Glu His Pro Gln Asp Val Pro Ala Ile
                 85                  90                  95

Arg Asp Lys Ile Arg Glu His Ser Ala Val Ile Asp Ile Phe Glu Tyr
            100                 105                 110

Asp Ile Pro Phe Ala Lys Arg Tyr Leu Ile Asp Lys Gly Leu Ile Pro

        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Val Ile Arg Glu Lys Asp Pro Asp Val Ile Ile Thr
        195                 200                 205

Tyr Asn Gly Asp Ser Phe Asp Leu Pro Tyr Leu Val Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Ile Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala His Glu Ile Ala Glu Ala Trp Glu Thr Gly Lys Gly
    290                 295                 300

Leu Glu Arg Val Ala Lys Tyr Ser Met Glu Asp Ala Lys Val Thr Tyr
305                 310                 315                 320

Glu Leu Gly Arg Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Tyr Val Lys Glu Pro Glu Lys Gly Leu Trp Glu Gly
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Arg Glu Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Glu Ile
    450                 455                 460

Lys Arg Lys Met Lys Ala Ser Lys Asp Pro Ile Glu Lys Lys Met Leu
465                 470                 475                 480

Asp Tyr Arg Gln Arg Ala Ile Lys Ile Leu Ala Asn Ser Tyr Tyr Gly

485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Arg Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Ala Lys Pro Glu Glu Ile Lys Lys Lys
545                 550                 555                 560

Ala Leu Glu Phe Val Asp Tyr Ile Asn Ala Lys Leu Pro Gly Leu Leu
                565                 570                 575

Glu Leu Glu Tyr Glu Gly Phe Tyr Val Arg Gly Phe Phe Val Thr Lys
            580                 585                 590

Lys Lys Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Val Thr Glu Lys Leu Ser Lys Tyr Glu Ile
                645                 650                 655

Pro Pro Glu Lys Leu Val Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Arg Gly Val Lys Val Arg Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Leu Arg Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg
            740                 745                 750

Lys Glu Asp Leu Arg Trp Gln Lys Thr Lys Gln Thr Gly Leu Thr Ala
        755                 760                 765

Trp Leu Asn Ile Lys Lys Lys
    770                 775

<210> 26
<211> 775
<212> PRT
<213> Artificial Sequence

<220>
<223> Description of Artificial Sequence:designed hybrid
      polymerase

<220>
<221> MOD_RES
<222> (1)..(775)

<223> Xaa = unknown amino acid

<400> 26
Met Ile Leu Asp Xaa Asp Tyr Ile Thr Glu Xaa Gly Lys Pro Xaa Ile
1               5                   10                  15

Arg Xaa Phe Lys Lys Glu Asn Gly Xaa Phe Lys Xaa Glu Xaa Asp Arg
            20                  25                  30

Xaa Phe Arg Pro Tyr Ile Tyr Ala Leu Leu Xaa Asp Asp Ser Xaa Ile
        35                  40                  45

Xaa Glu Val Xaa Lys Ile Thr Xaa Glu Arg His Gly Lys Ile Val Arg
    50                  55                  60

Ile Xaa Asp Xaa Glu Lys Val Xaa Lys Lys Phe Leu Gly Xaa Pro Ile
65                  70                  75                  80

Xaa Val Trp Xaa Leu Tyr Xaa Glu His Pro Gln Asp Val Pro Xaa Ile
                85                  90                  95

Arg Xaa Lys Xaa Arg Glu His Xaa Ala Val Xaa Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Xaa Glu Glu Leu Lys Xaa Leu Ala Phe Asp Ile Glu Thr
    130                 135                 140

Leu Tyr His Glu Gly Glu Glu Phe Xaa Lys Gly Pro Ile Ile Met Ile
145                 150                 155                 160

Ser Tyr Ala Asp Glu Xaa Glu Ala Lys Val Ile Thr Trp Lys Xaa Ile
                165                 170                 175

Asp Leu Pro Tyr Val Glu Val Val Ser Ser Glu Arg Glu Met Ile Lys
            180                 185                 190

Arg Phe Leu Xaa Xaa Ile Arg Glu Lys Asp Pro Asp Xaa Ile Xaa Thr
        195                 200                 205

Tyr Asn Gly Asp Ser Phe Asp Xaa Pro Tyr Leu Xaa Lys Arg Ala Glu
    210                 215                 220

Lys Leu Gly Ile Lys Leu Xaa Xaa Gly Arg Asp Gly Ser Glu Pro Lys
225                 230                 235                 240

Met Gln Arg Xaa Gly Asp Met Thr Ala Val Glu Xaa Lys Gly Arg Ile
                245                 250                 255

His Phe Asp Leu Tyr His Val Ile Xaa Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Xaa Glu Ile Ala Xaa Ala Trp Glu Xaa Gly Xaa Xaa
    290                 295                 300

Leu Glu Arg Val Ala Lys Tyr Ser Met Glu Asp Ala Lys Xaa Thr Tyr
305                 310                 315                 320

Glu Leu Gly Xaa Glu Phe Xaa Pro Met Glu Xaa Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Xaa Leu Leu Arg Lys Ala Tyr Glu Arg Asn Glu Xaa Ala

355                 360                 365

Pro Asn Lys Pro Xaa Glu Xaa Glu Tyr Xaa Arg Arg Leu Arg Glu Ser
    370                 375                 380

Tyr Xaa Gly Gly Xaa Val Lys Glu Pro Glu Lys Gly Leu Trp Glu Xaa
385                 390                 395                 400

Xaa Val Xaa Leu Asp Phe Arg Xaa Leu Tyr Pro Ser Ile Ile Ile Thr
                405                 410                 415

His Asn Val Ser Pro Asp Thr Leu Asn Xaa Glu Gly Cys Xaa Xaa Tyr
            420                 425                 430

Asp Xaa Ala Pro Xaa Val Gly His Lys Phe Cys Lys Asp Xaa Pro Gly
        435                 440                 445

Phe Ile Pro Ser Leu Leu Xaa Xaa Leu Leu Xaa Glu Arg Gln Xaa Ile
    450                 455                 460

Lys Xaa Lys Met Lys Xaa Xaa Xaa Asp Pro Ile Glu Lys Xaa Xaa Leu
465                 470                 475                 480

Asp Tyr Arg Gln Xaa Ala Ile Lys Xaa Leu Ala Asn Ser Xaa Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Xaa Tyr Ile Glu Xaa Val Xaa Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Xaa Xaa Xaa Glu Glu Ile Lys Lys Lys
545                 550                 555                 560

Ala Leu Glu Phe Val Lys Tyr Ile Asn Xaa Lys Leu Pro Gly Leu Leu
                565                 570                 575

Glu Leu Glu Tyr Glu Gly Phe Tyr Xaa Arg Gly Phe Phe Val Thr Lys
            580                 585                 590

Lys Xaa Tyr Ala Xaa Ile Asp Glu Glu Gly Lys Xaa Ile Thr Arg Gly
        595                 600                 605

Leu Glu Ile Val Arg Arg Asp Trp Ser Glu Ile Ala Lys Glu Thr Gln
    610                 615                 620

Ala Xaa Val Leu Glu Xaa Ile Leu Lys His Gly Xaa Val Glu Glu Ala
625                 630                 635                 640

Val Xaa Ile Val Lys Glu Val Xaa Xaa Lys Leu Xaa Xaa Tyr Glu Ile
                645                 650                 655

Pro Pro Glu Lys Leu Xaa Ile Tyr Glu Gln Ile Thr Arg Pro Leu His
            660                 665                 670

Glu Tyr Lys Ala Ile Gly Pro His Val Ala Val Ala Lys Xaa Leu Ala
        675                 680                 685

Ala Xaa Gly Val Lys Xaa Xaa Pro Gly Met Val Ile Gly Tyr Ile Val
    690                 695                 700

Leu Arg Gly Asp Gly Pro Ile Ser Xaa Arg Ala Ile Leu Ala Glu Glu
705                 710                 715                 720

Xaa Asp Xaa Xaa Lys His Lys Tyr Asp Ala Glu Tyr Tyr Ile Glu Asn

                725                 730                 735

Gln Val Leu Pro Ala Val Leu Arg Ile Leu Glu Xaa Phe Gly Tyr Arg
            740                 745                 750

Lys Glu Asp Leu Arg Xaa Gln Lys Thr Xaa Gln Xaa Gly Leu Thr Xaa
        755                 760                 765

Trp Leu Asn Ile Lys Lys Ser
    770                 775

<210> 27
<211> 493
<212> PRT
<213> Artificial Sequence

<220>
<223> Description of Artificial Sequence:BlastP
alignment Pyrococcus furiosus DNA polymerase (Pfu)
query sequence

<400> 27
Met Ile Leu Asp Val Asp Tyr Ile Thr Glu Glu Gly Lys Pro Val Ile
1               5                   10                  15

Arg Leu Phe Lys Lys Glu Asn Gly Lys Phe Lys Ile Glu His Asp Arg
            20                  25                  30

Thr Phe Arg Pro Tyr Ile Tyr Ala Leu Leu Arg Asp Asp Ser Lys Ile
        35                  40                  45

Glu Glu Val Lys Lys Ile Thr Gly Glu Arg His Gly Lys Ile Val Arg
    50                  55                  60

Ile Val Asp Val Glu Lys Val Glu Lys Lys Phe Leu Gly Lys Pro Ile
65                  70                  75                  80

Thr Val Trp Lys Leu Tyr Leu Glu His Pro Gln Asp Val Pro Thr Ile
                85                  90                  95

Arg Glu Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Lys Ala Trp Glu Ser Gly Glu Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Val Ala
        355                 360                 365

Pro Asn Lys Pro Ser Glu Glu Glu Tyr Gln Arg Arg Leu Arg Glu Ser
    370                 375                 380

Tyr Thr Gly Gly Phe Val Lys Glu Pro Glu Lys Gly Leu Trp Glu Asn
385                 390                 395                 400

Ile Val Tyr Leu Asp Phe Arg Ala Leu Tyr Pro Ser Ile Ile Ile Thr
                405                 410                 415

His Asn Val Ser Pro Asp Thr Leu Asn Leu Glu Gly Cys Lys Asn Tyr
            420                 425                 430

Asp Ile Ala Pro Gln Val Gly His Lys Phe Cys Lys Asp Ile Pro Gly
        435                 440                 445

Phe Ile Pro Ser Leu Leu Gly His Leu Leu Glu Glu Arg Gln Lys Ile
    450                 455                 460

Lys Thr Lys Met Lys Glu Thr Gln Asp Pro Ile Glu Lys Ile Leu Leu
465                 470                 475                 480

Asp Tyr Arg Gln Lys Ala Ile Lys Leu Leu Ala Asn Ser
                485                 490


<210> 28
<211> 283
<212> PRT
<213> Artificial Sequence

<220>
<223> Description of Artificial Sequence:BlastP
      alignment Pyrococcus furiosus DNA polymerase (Pfu)
      query sequence
<400> 28
Asn Ser Phe Tyr Gly Tyr Tyr Gly Tyr Ala Lys Ala Arg Trp Tyr Cys
  1               5                  10                  15

Lys Glu Cys Ala Glu Ser Val Thr Ala Trp Gly Arg Lys Tyr Ile Glu
             20                  25                  30

Leu Val Trp Lys Glu Leu Glu Glu Lys Phe Gly Phe Lys Val Leu Tyr
         35                  40                  45

Ile Asp Thr Asp Gly Leu Tyr Ala Thr Ile Pro Gly Gly Glu Ser Glu
     50                  55                  60

Glu Ile Lys Lys Lys Ala Leu Glu Phe Val Lys Tyr Ile Asn Ser Lys
65                  70                  75                  80

Leu Pro Gly Leu Leu Glu Leu Glu Tyr Glu Gly Phe Tyr Lys Arg Gly
                85                  90                  95

Phe Phe Val Thr Lys Lys Arg Tyr Ala Val Ile Asp Glu Glu Gly Lys
            100                 105                 110

Val Ile Thr Arg Gly Leu Glu Ile Val Arg Arg Asp Trp Ser Glu Ile
        115                 120                 125

Ala Lys Glu Thr Gln Ala Arg Val Leu Glu Thr Ile Leu Lys His Gly
    130                 135                 140

Asp Val Glu Glu Ala Val Arg Ile Val Lys Glu Val Ile Gln Lys Leu
145                 150                 155                 160

Ala Asn Tyr Glu Ile Pro Pro Glu Lys Leu Ala Ile Tyr Glu Gln Ile
                165                 170                 175

Thr Arg Pro Leu His Glu Tyr Lys Ala Ile Gly Pro His Val Ala Val
            180                 185                 190

Ala Lys Lys Leu Ala Ala Lys Gly Val Lys Ile Lys Pro Gly Met Val
        195                 200                 205

Ile Gly Tyr Ile Val Leu Arg Gly Asp Gly Pro Ile Ser Asn Arg Ala
    210                 215                 220

Ile Leu Ala Glu Glu Tyr Asp Pro Lys Lys His Lys Tyr Asp Ala Glu
225                 230                 235                 240

Tyr Tyr Ile Glu Asn Gln Val Leu Pro Ala Val Leu Arg Ile Leu Glu
                245                 250                 255

Gly Phe Gly Tyr Arg Lys Glu Asp Leu Arg Tyr Gln Lys Thr Arg Gln
            260                 265                 270

Val Gly Leu Thr Ser Trp Leu Asn Ile Lys Lys
        275                 280


<210> 29
<211> 493
<212> PRT
<213> Artificial Sequence

<220>
<223> Description of Artificial Sequence:BlastP
      alignment Pyrococcus sp. GB-D DNA polymerase (Deep
      Vent) subject sequence

<400> 29
Met Ile Leu Asp Ala Asp Tyr Ile Thr Glu Asp Gly Lys Pro Ile Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Lys Asp Asp Ser Gln Ile
         35                  40                  45

Asp Glu Val Arg Lys Ile Thr Ala Glu Arg His Gly Lys Ile Val Arg
     50                  55                  60

Ile Ile Asp Ala Glu Lys Val Arg Lys Lys Phe Leu Gly Arg Pro Ile
 65                  70                  75                  80

Glu Val Trp Arg Leu Tyr Phe Glu His Pro Gln Asp Val Pro Ala Ile
                85                  90                  95

Arg Asp Lys Ile Arg Glu His Ser Ala Val Ile Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Val Ile Arg Glu Lys Asp Pro Asp Val Ile Ile Thr
        195                 200                 205

Tyr Asn Gly Asp Ser Phe Asp Leu Pro Tyr Leu Val Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Ile Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala His Glu Ile Ala Glu Ala Trp Glu Thr Gly Lys Gly
    290                 295                 300

Leu Glu Arg Val Ala Lys Tyr Ser Met Glu Asp Ala Lys Val Thr Tyr
305                 310                 315                 320

Glu Leu Gly Arg Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Tyr Val Lys Glu Pro Glu Lys Gly Leu Trp Glu Gly
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Arg Glu Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Glu Ile
    450                 455                 460

Lys Arg Lys Met Lys Ala Ser Lys Asp Pro Ile Glu Lys Lys Met Leu
465                 470                 475                 480

Asp Tyr Arg Gln Arg Ala Ile Lys Ile Leu Ala Asn Ser
                485                 490


<210> 30
<211> 283
<212> PRT
<213> Artificial Sequence

<220>
<223> Description of Artificial Sequence:BlastP
      alignment Pyrococcus sp. GB-D DNA polymerase (Deep
      Vent) subject sequence

<400> 30
Asn Ser Tyr Tyr Gly Tyr Tyr Gly Tyr Ala Lys Ala Arg Trp Tyr Cys
  1               5                  10                  15

Lys Glu Cys Ala Glu Ser Val Thr Ala Trp Gly Arg Glu Tyr Ile Glu
             20                  25                  30

Phe Val Arg Lys Glu Leu Glu Glu Lys Phe Gly Phe Lys Val Leu Tyr
         35                  40                  45


Ile Asp Thr Asp Gly Leu Tyr Ala Thr Ile Pro Gly Ala Lys Pro Glu
     50                  55                  60

Glu Ile Lys Lys Lys Ala Leu Glu Phe Val Asp Tyr Ile Asn Ala Lys
 65                  70                  75                  80

Leu Pro Gly Leu Leu Glu Leu Glu Tyr Glu Gly Phe Tyr Val Arg Gly
                 85                  90                  95

Phe Phe Val Thr Lys Lys Lys Tyr Ala Leu Ile Asp Glu Glu Gly Lys
            100                 105                 110

Ile Ile Thr Arg Gly Leu Glu Ile Val Arg Arg Asp Trp Ser Glu Ile
        115                 120                 125

Ala Lys Glu Thr Gln Ala Lys Val Leu Glu Ala Ile Leu Lys His Gly
    130                 135                 140

Asn Val Glu Glu Ala Val Lys Ile Val Lys Glu Val Thr Glu Lys Leu
145                 150                 155                 160

Ser Lys Tyr Glu Ile Pro Pro Glu Lys Leu Val Ile Tyr Glu Gln Ile
                165                 170                 175

Thr Arg Pro Leu His Glu Tyr Lys Ala Ile Gly Pro His Val Ala Val
            180                 185                 190

Ala Lys Arg Leu Ala Ala Arg Gly Val Lys Val Arg Pro Gly Met Val
        195                 200                 205

Ile Gly Tyr Ile Val Leu Arg Gly Asp Gly Pro Ile Ser Lys Arg Ala
    210                 215                 220

Ile Leu Ala Glu Glu Phe Asp Leu Arg Lys His Lys Tyr Asp Ala Glu
225                 230                 235                 240

Tyr Tyr Ile Glu Asn Gln Val Leu Pro Ala Val Leu Arg Ile Leu Glu
                245                 250                 255

Ala Phe Gly Tyr Arg Lys Glu Asp Leu Arg Trp Gln Lys Thr Lys Gln
            260                 265                 270

Thr Gly Leu Thr Ala Trp Leu Asn Ile Lys Lys
        275                 280

<210> 31
<211> 493
<212> PRT
<213> Artificial Sequence

<220>
<223> Description of Artificial Sequence:BlastP
      alignment consensus sequence

<220>
<221> MOD_RES
<222> (1)..(493)
<223> Xaa = unknown amino acid

<400> 31
Met Ile Leu Asp Xaa Asp Tyr Ile Thr Glu Xaa Gly Lys Pro Xaa Ile
  1               5                  10                  15

Arg Xaa Phe Lys Lys Glu Asn Gly Xaa Phe Lys Xaa Glu Xaa Asp Arg
             20                  25                  30

Xaa Phe Arg Pro Tyr Ile Tyr Ala Leu Leu Xaa Asp Asp Ser Xaa Ile
         35                  40                  45

Xaa Glu Val Xaa Lys Ile Thr Xaa Glu Arg His Gly Lys Ile Val Arg
     50                  55                  60

Ile Xaa Asp Xaa Glu Lys Val Xaa Lys Lys Phe Leu Gly Xaa Pro Ile
 65                  70                  75                  80

Xaa Val Trp Xaa Leu Tyr Xaa Glu His Pro Gln Asp Val Pro Xaa Ile
                 85                  90                  95

Arg Xaa Lys Xaa Arg Glu His Xaa Ala Val Xaa Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Xaa Glu Glu Leu Lys Xaa Leu Ala Phe Asp Ile Glu Thr
    130                 135                 140

Leu Tyr His Glu Gly Glu Glu Phe Xaa Lys Gly Pro Ile Ile Met Ile
145                 150                 155                 160

Ser Tyr Ala Asp Glu Xaa Glu Ala Lys Val Ile Thr Trp Lys Xaa Ile
                165                 170                 175

Asp Leu Pro Tyr Val Glu Val Val Ser Ser Glu Arg Glu Met Ile Lys
            180                 185                 190

Arg Phe Leu Xaa Xaa Ile Arg Glu Lys Asp Pro Asp Xaa Ile Xaa Thr
        195                 200                 205

Tyr Asn Gly Asp Ser Phe Asp Xaa Pro Tyr Leu Xaa Lys Arg Ala Glu
    210                 215                 220

Lys Leu Gly Ile Lys Leu Xaa Xaa Gly Arg Asp Gly Ser Glu Pro Lys
225                 230                 235                 240

Met Gln Arg Xaa Gly Asp Met Thr Ala Val Glu Xaa Lys Gly Arg Ile
                245                 250                 255

His Phe Asp Leu Tyr His Val Ile Xaa Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Xaa Glu Ile Ala Xaa Ala Trp Glu Xaa Gly Xaa Xaa
    290                 295                 300

Leu Glu Arg Val Ala Lys Tyr Ser Met Glu Asp Ala Lys Xaa Thr Tyr
305                 310                 315                 320

Glu Leu Gly Xaa Glu Phe Xaa Pro Met Glu Xaa Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Xaa Leu Leu Arg Lys Ala Tyr Glu Arg Asn Glu Xaa Ala
        355                 360                 365

Pro Asn Lys Pro Xaa Glu Xaa Glu Tyr Xaa Arg Arg Leu Arg Glu Ser
    370                 375                 380

Tyr Xaa Gly Gly Xaa Val Lys Glu Pro Glu Lys Gly Leu Trp Glu Xaa
385                 390                 395                 400

Xaa Val Xaa Leu Asp Phe Arg Xaa Leu Tyr Pro Ser Ile Ile Ile Thr
                405                 410                 415

His Asn Val Ser Pro Asp Thr Leu Asn Xaa Glu Gly Cys Xaa Xaa Tyr
            420                 425                 430

Asp Xaa Ala Pro Xaa Val Gly His Lys Phe Cys Lys Asp Xaa Pro Gly
        435                 440                 445

Phe Ile Pro Ser Leu Leu Xaa Xaa Leu Leu Xaa Glu Arg Gln Xaa Ile
    450                 455                 460

Lys Xaa Lys Met Lys Xaa Xaa Xaa Asp Pro Ile Glu Lys Xaa Xaa Leu
465                 470                 475                 480

Asp Tyr Arg Gln Xaa Ala Ile Lys Xaa Leu Ala Asn Ser
                485                 490


<210> 32
<211> 283
<212> PRT
<213> Artificial Sequence

<220>
<223> Description of Artificial Sequence:BlastP
      alignment consensus sequence

<220>
<221> MOD_RES
<222> (1)..(283)
<223> Xaa = unknown amino acid

<400> 32
Asn Ser Xaa Tyr Gly Tyr Tyr Gly Tyr Ala Lys Ala Arg Trp Tyr Cys
  1               5                  10                  15

Lys Glu Cys Ala Glu Ser Val Thr Ala Trp Gly Arg Xaa Tyr Ile Glu
             20                  25                  30

Xaa Val Xaa Lys Glu Leu Glu Glu Lys Phe Gly Phe Lys Val Leu Tyr
         35                  40                  45

Ile Asp Thr Asp Gly Leu Tyr Ala Thr Ile Pro Gly Xaa Xaa Xaa Glu
    50                  55                  60

Glu Ile Lys Lys Lys Ala Leu Glu Phe Val Xaa Tyr Ile Asn Xaa Lys
65                  70                  75                  80

Leu Pro Gly Leu Leu Glu Leu Glu Tyr Glu Gly Phe Tyr Xaa Arg Gly
                85                  90                  95

Phe Phe Val Thr Lys Lys Xaa Tyr Ala Xaa Ile Asp Glu Glu Gly Lys
            100                 105                 110

Xaa Ile Thr Arg Gly Leu Glu Ile Val Arg Arg Asp Trp Ser Glu Ile
        115                 120                 125

Ala Lys Glu Thr Gln Ala Xaa Val Leu Glu Xaa Ile Leu Lys His Gly
    130                 135                 140

Xaa Val Glu Glu Ala Val Xaa Ile Val Lys Glu Val Xaa Xaa Lys Leu
145                 150                 155                 160

Xaa Xaa Tyr Glu Ile Pro Pro Glu Lys Leu Xaa Ile Tyr Glu Gln Ile
                165                 170                 175

Thr Arg Pro Leu His Glu Tyr Lys Ala Ile Gly Pro His Val Ala Val
            180                 185                 190

Ala Lys Xaa Leu Ala Ala Xaa Gly Val Lys Xaa Xaa Pro Gly Met Val
        195                 200                 205

Ile Gly Tyr Ile Val Leu Arg Gly Asp Gly Pro Ile Ser Xaa Arg Ala
    210                 215                 220

Ile Leu Ala Glu Glu Xaa Asp Xaa Xaa Lys His Lys Tyr Asp Ala Glu
225                 230                 235                 240

Tyr Tyr Ile Glu Asn Gln Val Leu Pro Ala Val Leu Arg Ile Leu Glu
                245                 250                 255

Xaa Phe Gly Tyr Arg Lys Glu Asp Leu Arg Xaa Gln Lys Thr Xaa Gln
            260                 265                 270

Xaa Gly Leu Thr Xaa Trp Leu Asn Ile Lys Lys
        275                 280


<210> 33
<211> 480
<212> PRT
<213> Artificial Sequence

<220>
<223> Description of Artificial Sequence:Pyrococcus
      furiosus DNA polymerase (Pfu) alignment sequence

<400> 33
Met Ile Leu Asp Val Asp Tyr Ile Thr Glu Glu Gly Lys Pro Val Ile
  1               5                  10                  15

Arg Leu Phe Lys Lys Glu Asn Gly Lys Phe Lys Ile Glu His Asp Arg
             20                  25                  30

Thr Phe Arg Pro Tyr Ile Tyr Ala Leu Leu Arg Asp Asp Ser Lys Ile
         35                  40                  45
Glu Glu Val Lys Lys Ile Thr Gly Glu Arg His Gly Lys Ile Val Arg
     50                  55                  60

Ile Val Asp Val Glu Lys Val Glu Lys Lys Phe Leu Gly Lys Pro Ile
 65                  70                  75                  80

Thr Val Trp Lys Leu Tyr Leu Glu His Pro Gln Asp Val Pro Thr Ile
                85                  90                  95

Arg Glu Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Lys Ala Trp Glu Ser Gly Glu Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Val Ala
        355                 360                 365

Pro Asn Lys Pro Ser Glu Glu Glu Tyr Gln Arg Arg Leu Arg Glu Ser
    370                 375                 380

Tyr Thr Gly Gly Phe Val Lys Glu Pro Glu Lys Gly Leu Trp Glu Asn
385                 390                 395                 400

Ile Val Tyr Leu Asp Phe Arg Ala Leu Tyr Pro Ser Ile Ile Ile Thr
                405                 410                 415

His Asn Val Ser Pro Asp Thr Leu Asn Leu Glu Gly Cys Lys Asn Tyr
            420                 425                 430

Asp Ile Ala Pro Gln Val Gly His Lys Phe Cys Lys Asp Ile Pro Gly
        435                 440                 445

Phe Ile Pro Ser Leu Leu Gly His Leu Leu Glu Glu Arg Gln Lys Ile
    450                 455                 460

Lys Thr Lys Met Lys Glu Thr Gln Asp Pro Ile Glu Lys Ile Leu Leu
465                 470                 475                 480


<210> 34
<211> 480
<212> PRT
<213> Artificial Sequence

<220>
<223> Description of Artificial Sequence:Pyrococcus sp.
      GD-B (Deep Vent) DNA polymerase alignment sequence

<400> 34
Met Ile Leu Asp Ala Asp Tyr Ile Thr Glu Asp Gly Lys Pro Ile Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Lys Asp Asp Ser Gln Ile
         35                  40                  45

Asp Glu Val Arg Lys Ile Thr Ala Glu Arg His Gly Lys Ile Val Arg
     50                  55                  60

Ile Ile Asp Ala Glu Lys Val Arg Lys Lys Phe Leu Gly Arg Pro Ile
 65                  70                  75                  80

Glu Val Trp Arg Leu Tyr Phe Glu His Pro Gln Asp Val Pro Ala Ile
                 85                  90                  95

Arg Asp Lys Ile Arg Glu His Ser Ala Val Ile Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160
Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Val Ile Arg Glu Lys Asp Pro Asp Val Ile Ile Thr
        195                 200                 205

Tyr Asn Gly Asp Ser Phe Asp Leu Pro Tyr Leu Val Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Ile Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala His Glu Ile Ala Glu Ala Trp Glu Thr Gly Lys Gly
    290                 295                 300

Leu Glu Arg Val Ala Lys Tyr Ser Met Glu Asp Ala Lys Val Thr Tyr
305                 310                 315                 320

Glu Leu Gly Arg Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Tyr Val Lys Glu Pro Glu Lys Gly Leu Trp Glu Gly
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Arg Glu Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Glu Ile
    450                 455                 460

Lys Arg Lys Met Lys Ala Ser Lys Asp Pro Ile Glu Lys Lys Met Leu
465                 470                 475                 480


<210> 35
<211> 480
<212> PRT
<213> Artificial Sequence

<220>
<223> Description of Artificial Sequence:designed hybrid
      polymerase alignment sequence

<220>
<221> MOD_RES
<222> (1)..(480)
<223> Xaa = unknown amino acid

<400> 35
Met Ile Leu Asp Xaa Asp Tyr Ile Thr Glu Xaa Gly Lys Pro Xaa Ile
  1               5                  10                  15

Arg Xaa Phe Lys Lys Glu Asn Gly Xaa Phe Lys Xaa Glu Xaa Asp Arg
             20                  25                  30

Xaa Phe Arg Pro Tyr Ile Tyr Ala Leu Leu Xaa Asp Asp Ser Xaa Ile
         35                  40                  45

Xaa Glu Val Xaa Lys Ile Thr Xaa Glu Arg His Gly Lys Ile Val Arg
     50                  55                  60

Ile Xaa Asp Xaa Glu Lys Val Xaa Lys Lys Phe Leu Gly Xaa Pro Ile
 65                  70                  75                  80

Xaa Val Trp Xaa Leu Tyr Xaa Glu His Pro Gln Asp Val Pro Xaa Ile
                 85                  90                  95

Arg Xaa Lys Xaa Arg Glu His Xaa Ala Val Xaa Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Xaa Glu Glu Leu Lys Xaa Leu Ala Phe Asp Ile Glu Thr
    130                 135                 140

Leu Tyr His Glu Gly Glu Glu Phe Xaa Lys Gly Pro Ile Ile Met Ile
145                 150                 155                 160

Ser Tyr Ala Asp Glu Xaa Glu Ala Lys Val Ile Thr Trp Lys Xaa Ile
                165                 170                 175

Asp Leu Pro Tyr Val Glu Val Val Ser Ser Glu Arg Glu Met Ile Lys
            180                 185                 190

Arg Phe Leu Xaa Xaa Ile Arg Glu Lys Asp Pro Asp Xaa Ile Xaa Thr
        195                 200                 205

Tyr Asn Gly Asp Ser Phe Asp Xaa Pro Tyr Leu Xaa Lys Arg Ala Glu
    210                 215                 220

Lys Leu Gly Ile Lys Leu Xaa Xaa Gly Arg Asp Gly Ser Glu Pro Lys
225                 230                 235                 240

Met Gln Arg Xaa Gly Asp Met Thr Ala Val Glu Xaa Lys Gly Arg Ile
                245                 250                 255

His Phe Asp Leu Tyr His Val Ile Xaa Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Xaa Glu Ile Ala Xaa Ala Trp Glu Xaa Gly Xaa Xaa
    290                 295                 300

Leu Glu Arg Val Ala Lys Tyr Ser Met Glu Asp Ala Lys Xaa Thr Tyr
305                 310                 315                 320

Glu Leu Gly Xaa Glu Phe Xaa Pro Met Glu Xaa Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Xaa Leu Leu Arg Lys Ala Tyr Glu Arg Asn Glu Xaa Ala
        355                 360                 365

Pro Asn Lys Pro Xaa Glu Xaa Glu Tyr Xaa Arg Arg Leu Arg Glu Ser
    370                 375                 380

Tyr Xaa Gly Gly Xaa Val Lys Glu Pro Glu Lys Gly Leu Trp Glu Xaa
385                 390                 395                 400

Xaa Val Xaa Leu Asp Phe Arg Xaa Leu Tyr Pro Ser Ile Ile Ile Thr
                405                 410                 415

His Asn Val Ser Pro Asp Thr Leu Asn Xaa Glu Gly Cys Xaa Xaa Tyr
            420                 425                 430

Asp Xaa Ala Pro Xaa Val Gly His Lys Phe Cys Lys Asp Xaa Pro Gly
        435                 440                 445

Phe Ile Pro Ser Leu Leu Xaa Xaa Leu Leu Xaa Glu Arg Gln Xaa Ile
    450                 455                 460

Lys Xaa Lys Met Lys Xaa Xaa Xaa Asp Pro Ile Glu Lys Xaa Xaa Leu
465                 470                 475                 480

<210> 36
<211> 151
<212> PRT
<213> Escherichia coli

<220>
<223> mesophile E. coli deoxyuridine 5'-triphosphate
      nucleotidohydrolase (dUTPase or Dut) (ECD)

<400> 36
Met Lys Lys Ile Asp Val Lys Ile Leu Asp Pro Arg Val Gly Lys Glu
1               5                   10                  15

Phe Pro Leu Pro Thr Tyr Ala Thr Ser Gly Ser Ala Gly Leu Asp Leu
            20                  25                  30
Arg Ala Cys Leu Asn Asp Ala Val Glu Leu Ala Pro Gly Asp Thr Thr
        35                  40                  45

Leu Val Pro Thr Gly Leu Ala Ile His Ile Ala Asp Pro Ser Leu Ala
    50                  55                  60

Ala Met Met Leu Pro Arg Ser Gly Leu Gly His Lys His Gly Ile Val
65                  70                  75                  80

Leu Gly Asn Leu Val Gly Leu Ile Asp Ser Asp Tyr Gln Gly Gln Leu
                85                  90                  95

Met Ile Ser Val Trp Asn Arg Gly Gln Asp Ser Phe Thr Ile Gln Pro
            100                 105                 110

Gly Glu Arg Ile Ala Gln Met Ile Phe Val Pro Val Val Gln Ala Glu
        115                 120                 125

Phe Asn Leu Val Glu Asp Phe Asp Ala Thr Asp Arg Gly Glu Gly Gly
    130                 135                 140

Phe Gly His Ser Gly Arg Gln
145                 150

<210> 37
<211> 150
<212> PRT
<213> Aquifex aeolicus

<220>
<223> deoxyuridine 5'-triphosphate nucleotidohydrolase
      (dUTPase or Dut) (AAD)

<400> 37
Met Ser Lys Val Ile Leu Lys Ile Lys Arg Leu Pro His Ala Gln Asp
1               5                   10                  15

Leu Pro Leu Pro Ser Tyr Ala Thr Pro His Ser Ser Gly Leu Asp Leu
            20                  25                  30

Arg Ala Ala Ile Glu Lys Pro Leu Lys Ile Lys Pro Phe Glu Arg Val
        35                  40                  45

Leu Ile Pro Thr Gly Leu Ile Leu Glu Ile Pro Glu Gly Tyr Glu Gly
    50                  55                  60

Gln Val Arg Pro Arg Ser Gly Leu Ala Trp Lys Lys Gly Leu Thr Val
65                  70                  75                  80

Leu Asn Ala Pro Gly Thr Ile Asp Ala Asp Tyr Arg Gly Glu Val Lys
                85                  90                  95

Val Ile Leu Val Asn Leu Gly Asn Glu Glu Val Val Ile Glu Arg Gly
            100                 105                 110

Glu Arg Ile Ala Gln Leu Val Ile Ala Pro Val Gln Arg Val Glu Val
        115                 120                 125

Val Glu Val Glu Glu Val Ser Gln Thr Gln Arg Gly Glu Gly Gly Phe
    130                 135                 140

Gly Ser Thr Gly Thr Lys
145                 150

<210> 38
<211> 149
<212> PRT
<213> Artificial Sequence

<220>
<223> Description of Artificial Sequence:E. coli
      deoxyuridine 5'-triphosphate nucleotidohydrolase
      (dUTPase or Dut) (ECD) BlastP alignment sequence

<400> 38
Met Lys Lys Ile Asp Val Lys Ile Leu Asp Pro Arg Val Gly Lys Glu
  1               5                  10                  15

Phe Pro Leu Pro Thr Tyr Ala Thr Ser Gly Ser Ala Gly Leu Asp Leu
             20                  25                  30

Arg Ala Cys Leu Asn Asp Ala Val Glu Leu Ala Pro Gly Asp Thr Thr
         35                  40                  45

Leu Val Pro Thr Gly Leu Ala Ile His Ile Ala Asp Pro Ser Leu Ala
     50                  55                  60

Ala Met Met Leu Pro Arg Ser Gly Leu Gly His Lys His Gly Ile Val
 65                  70                  75                  80

Leu Gly Asn Leu Val Gly Leu Ile Asp Ser Asp Tyr Gln Gly Gln Leu
                 85                  90                  95

Met Ile Ser Val Trp Asn Arg Gly Gln Asp Ser Phe Thr Ile Gln Pro
            100                 105                 110

Gly Glu Arg Ile Ala Gln Met Ile Phe Val Pro Val Val Gln Ala Glu
        115                 120                 125

Phe Asn Leu Val Glu Asp Phe Asp Ala Thr Asp Arg Gly Glu Gly Gly
    130                 135                 140

Phe Gly His Ser Gly
145

<210> 39
<211> 148
<212> PRT
<213> Artificial Sequence

<220>
<223> Description of Artificial Sequence:Aquifex
      aeolicus deoxyuridine 5'-triphosphate
      nucleotidohydrolase (dUTPase or Dut) (AAD) BlastP
      alignment sequence

<400> 39
Met Ser Lys Val Ile Leu Lys Ile Lys Arg Leu Pro His Ala Gln Asp
1               5                   10                  15

Leu Pro Leu Pro Ser Tyr Ala Thr Pro His Ser Ser Gly Leu Asp Leu
            20                  25                  30

Arg Ala Ala Ile Glu Lys Pro Leu Lys Ile Lys Pro Phe Glu Arg Val
        35                  40                  45

Leu Ile Pro Thr Gly Leu Ile Leu Glu Ile Pro Glu Gly Tyr Glu Gly
    50                  55                  60

Gln Val Arg Pro Arg Ser Gly Leu Ala Trp Lys Lys Gly Leu Thr Val
65                  70                  75                  80

Leu Asn Ala Pro Gly Thr Ile Asp Ala Asp Tyr Arg Gly Glu Val Lys
                85                  90                  95

Val Ile Leu Val Asn Leu Gly Asn Glu Glu Val Val Ile Glu Arg Gly
            100                 105                 110

Glu Arg Ile Ala Gln Leu Val Ile Ala Pro Val Gln Arg Val Glu Val
        115                 120                 125

Val Glu Val Glu Glu Val Ser Gln Thr Gln Arg Gly Glu Gly Gly Phe
    130                 135                 140

Gly Ser Thr Gly
145


<210> 40
<211> 6
<212> PRT
<213> Artificial Sequence

<220>
<223> Description of Artificial Sequence:consensus
      sequence

<400> 40
Gly Leu Asp Leu Arg Ala
1               5


<210> 41
<211> 4
<212> PRT
<213> Artificial Sequence

<220>
<223> Description of Artificial Sequence:consensus
      sequence

<400> 41
Pro Thr Gly Leu
1


<210> 42
<211> 5
<212> PRT
<213> Artificial Sequence

<220>
<223> Description of Artificial Sequence:consensus
      sequence

<400> 42
Pro Arg Ser Gly Leu
1               5


<210> 43
<211> 6
<212> PRT
<213> Artificial Sequence

<220>
<223> Description of Artificial Sequence:consensus
      sequence

<400> 43
Gly Glu Arg Ile Ala Gln
1               5


<210> 44
<211> 7
<212> PRT
<213> Artificial Sequence

<220>
<223> Description of Artificial Sequence:consensus
      sequence

<400> 44
Arg Gly Glu Gly Gly Phe Gly
1               5


<210> 45
<211> 459
<212> DNA
<213> Artificial Sequence

<220>
<223> Description of Artificial Sequence:consensus
      sequence, minimal encoding sequence using
      preferred E. coli codons

<400> 45
atgarmaaar ttrwtstgaa aattmwgsrt cygcstswtg scmaagawtt wccgctgccg 60
asctatgcga ccyctsrcag ckcaggcctg gatctgcgtg cgkscmttra saawscgstg 120
raamttrmgc cgkktgawas grygctgrtt ccgaccggcc tgrycmttsa wattscggaw 180
ssttmtctgg mggsgmwgrt gckgccgcgt agcggcctgg scyrkaaama kggcwtaryg 240
stgskgaacs ygsygggcmy gattgatrsc gattatcrgg gcsaastgaw grttakcstg 300
kkgaacckgg gcmasgawkm akttrygatt sagcsgggcg aacgtattgc gcagmtgrtt 360
wttgygccgg tgswgcrggy ggaakttrwt swggtggaag awkttkmtsm gaccsakcgt 420
ggcgaaggcg gctttggcym tascggcasa magtaatga                        459
<210> 46
<211> 151
<212> PRT
<213> Artificial Sequence

<220>
<223> Description of Artificial Sequence:consensus
      sequence, minimal encoding sequence translation

<220>
<221> MOD_RES
<222> (2)
<223> Xaa = Lys, Ser, Arg or Asn

<220>
<221> MOD_RES
<222> (4)

<223> Xaa = Ile or Val

<220>
<221> MOD_RES
<222> (5)
<223> Xaa = Asp, Ile, Val or Asn

<220>
<221> MOD_RES
<222> (6)
<223> Xaa = Val or Leu

<220>
<221> MOD_RES
<222> (9)
<223> Xaa = Leu, Lys, Gln or Met

<220>
<221> MOD_RES
<222> (10)
<223> Xaa = Asp, Arg, Gly or His

<220>
<221> MOD_RES
<222> (11)
<223> Xaa = Pro or Leu

<220>
<221> MOD_RES
<222> (12)
<223> Xaa = Arg or Pro

<220>
<221> MOD_RES
<222> (13)
<223> Xaa = Val, His, Asp or Leu

<220>
<221> MOD_RES
<222> (14)
<223> Xaa = Gly or Ala

<220>
<221> MOD_RES
<222> (15)
<223> Xaa = Lys or Gln

<220>
<221> MOD_RES
<222> (16)
<223> Xaa = Glu or Asp

<220>
<221> MOD_RES
<222> (17)
<223> Xaa = Phe or Leu

<220>
<221> MOD_RES
<222> (21)
<223> Xaa = Thr or Ser

<220>
<221> MOD_RES
<222> (25)
<223> Xaa = Ser or Pro

<220>
<221> MOD_RES

<222> (26)
<223> Xaa = Gly, His, Asp or Arg

<220>
<221> MOD_RES
<222> (28)
<223> Xaa = Ala or Ser

<220>
<221> MOD_RES
<222> (35)
<223> Xaa = Cys, Ala, Ser or Gly

<220>
<221> MOD_RES
<222> (36)
<223> Xaa = Leu or Ile

<220>
<221> MOD_RES
<222> (37)
<223> Xaa = Asn, Glu, Lys or Asp

<220>
<221> MOD_RES
<222> (38)
<223> Xaa = Asp, Lys, Asn or Glu

<220>
<221> MOD_RES
<222> (39)
<223> Xaa = Ala or Pro

<220>
<221> MOD_RES
<222> (40)
<223> Xaa = Val or Leu

<220>
<221> MOD_RES
<222> (41)
<223> Xaa = Glu or Lys

<220>
<221> MOD_RES
<222> (42)
<223> Xaa = Leu or Ile

<220>
<221> MOD_RES
<222> (43)
<223> Xaa = Ala, Lys, Glu or Thr

<220>
<221> MOD_RES
<222> (45)
<223> Xaa = Gly, Phe, Val or Cys

<220>
<221> MOD_RES
<222> (46)
<223> Xaa = Asp or Glu

<220>
<221> MOD_RES
<222> (47)
<223> Xaa = Thr or Arg

<220>

<221> MOD_RES
<222> (48)
<223> Xaa = Thr, Val, Met or Ala

<220>
<221> MOD_RES
<222> (50)
<223> Xaa = Val or Ile

<220>
<221> MOD_RES
<222> (55)
<223> Xaa = Ala, Ile, Val or Thr

<220>
<221> MOD_RES
<222> (56)
<223> Xaa = Ile or Leu

<220>
<221> MOD_RES
<222> (57)
<223> Xaa = His, Glu, Asp or Gln

<220>
<221> MOD_RES
<222> (59)
<223> Xaa = Ala or Pro

<220>
<221> MOD_RES
<222> (60)
<223> Xaa = Asp or Glu

<220>
<221> MOD_RES
<222> (61)
<223> Xaa = Pro, Gly, Arg or Ala

<220>
<221> MOD_RES
<222> (62)
<223> Xaa = Ser or Tyr

<220>
<221> MOD_RES
<222> (64)
<223> Xaa = Ala or Glu

<220>
<221> MOD_RES
<222> (65)
<223> Xaa = Ala or Gly

<220>
<221> MOD_RES
<222> (66)
<223> Xaa = Met, Gln, Lys or Leu

<220>
<221> MOD_RES
<222> (67)
<223> Xaa = Met or Val

<220>
<221> MOD_RES
<222> (68)
<223> Xaa = Leu or Arg

<220>
<221> MOD_RES
<222> (74)
<223> Xaa = Gly or Ala

<220>
<221> MOD_RES
<222> (75)
<223> Xaa = His, Trp, Arg, Gln, Tyr or stop codon

<220>
<221> MOD_RES
<222> (77)
<223> Xaa = His, Lys, Gln or Asn


<220>
<221> MOD_RES
<222> (79)
<223> Xaa = Ile or Leu

<220>
<221> MOD_RES
<222> (80)
<223> Xaa = Val, Thr, Ala or Met

<220>
<221> MOD_RES
<222> (81)
<223> Xaa = Leu or Val

<220>
<221> MOD_RES
<222> (82)
<223> Xaa = Gly, Leu, Val or Arg

<220>
<221> MOD_RES
<222> (84)
<223> Xaa = Leu, Ala, Pro or Val

<220>
<221> MOD_RES
<222> (85)
<223> Xaa = Val, Pro, Ala or Leu

<220>
<221> MOD_RES
<222> (87)
<223> Xaa = Leu, Thr, Pro or Met

<220>
<221> MOD_RES
<222> (90)
<223> Xaa = Ser, Ala, Thr or Gly

<220>
<221> MOD_RES
<222> (93)
<223> Xaa = Gln or Arg

<220>
<221> MOD_RES
<222> (96)
<223> Xaa = Leu or Val

<220>
<221> MOD_RES
<222> (97)
<223> Xaa = Met or Lys

<220>
<221> MOD_RES
<222> (98)
<223> Xaa = Ile or Val

<220>
<221> MOD_RES
<222> (99)
<223> Xaa = Ser or Ile

<220>
<221> MOD_RES
<222> (100)
<223> Xaa = Val or Leu

<220>
<221> MOD_RES
<222> (101)
<223> Xaa = Trp, Asn, Leu or Gly

<220>
<221> MOD_RES
<222> (103)
<223> Xaa = Arg or Leu

<220>
<221> MOD_RES
<222> (105)
<223> Xaa = Gln, Asn, His or Lys

<220>
<221> MOD_RES
<222> (106)
<223> Xaa = Asp or Glu

<220>
<221> MOD_RES
<222> (107)
<223> Xaa = Ser, Glu, Ala or stop codon

<220>
<221> MOD_RES
<222> (108)
<223> Xaa = Phe or Val

<220>
<221> MOD_RES
<222> (109)
<223> Xaa = Thr, Val, Met or Ala

<220>
<221> MOD_RES
<222> (112)
<223> Xaa = Pro or Arg

<220>
<221> MOD_RES
<222> (119)
<223> Xaa = Met or Leu

<220>
<221> MOD_RES
<222> (120)
<223> Xaa = Ile or Val

<220>
<221> MOD_RES

<222> (121)
<223> Xaa = Phe or Ile

<220>
<221> MOD_RES
<222> (122)
<223> Xaa = Val or Ala

<220>
<221> MOD_RES
<222> (125)
<223> Xaa = Val, Gln, Glu or Leu

<220>
<221> MOD_RES
<222> (126)
<223> Xaa = Gln or Arg

<220>
<221> MOD_RES
<222> (127)
<223> Xaa = Ala or Val

<220>
<221> MOD_RES
<222> (129)
<223> Xaa = Phe or Val

<220>
<221> MOD_RES
<222> (130)
<223> Xaa = Asn, Val, Ile or Asp

<220>
<221> MOD_RES
<222> (131)
<223> Xaa = Leu, Glu, Gln or Val

<220>
<221> MOD_RES
<222> (134)
<223> Xaa = Asp or Glu

<220>
<221> MOD_RES
<222> (135)
<223> Xaa = Phe or Val

<220>
<221> MOD_RES
<222> (136)
<223> Xaa = Asp, Ser, Ala or Tyr

<220>
<221> MOD_RES
<222> (137)
<223> Xaa = Ala, Gln, Glu or Pro


<220>
<221> MOD_RES
<222> (139)
<223> Xaa = Asp, Gln, Glu or His

<220>
<221> MOD_RES
<222> (147)
<223> Xaa = His, Ser, Pro or Tyr

<220>

<221> MOD_RES
<222> (148)
<223> Xaa = Ser or Thr

<220>
<221> MOD_RES
<222> (150)
<223> Xaa = Arg or Thr

<220>
<221> MOD_RES
<222> (151)
<223> Xaa = Gln or Lys

<400> 46
Met Xaa Lys Xaa Xaa Xaa Lys Ile Xaa Xaa Xaa Xaa Xaa Xaa Xaa Xaa
 1               5                  10                  15

Xaa Pro Leu Pro Xaa Tyr Ala Thr Xaa Xaa Ser Xaa Gly Leu Asp Leu
            20                  25                  30

Arg Ala Xaa Xaa Xaa Xaa Xaa Xaa Xaa Xaa Xaa Pro Xaa Xaa Xaa Xaa
        35                  40                  45

Leu Xaa Pro Thr Gly Leu Xaa Xaa Xaa Ile Xaa Xaa Xaa Xaa Leu Xaa
    50                  55                  60

Xaa Xaa Xaa Xaa Pro Arg Ser Gly Leu Xaa Xaa Lys Xaa Gly Xaa Xaa
65                  70                  75                  80

Xaa Xaa Asn Xaa Xaa Gly Xaa Ile Asp Xaa Asp Tyr Xaa Gly Glx Xaa
                85                  90                  95

Xaa Xaa Xaa Xaa Xaa Asn Xaa Gly Xaa Xaa Xaa Xaa Xaa Ile Glx Xaa
            100                 105                 110

Gly Glu Arg Ile Ala Gln Xaa Xaa Xaa Xaa Pro Val Xaa Xaa Xaa Glu
        115                 120                 125

Xaa Xaa Xaa Val Glu Xaa Xaa Xaa Xaa Thr Xaa Arg Gly Glu Gly Gly
    130                 135                 140

Phe Gly Xaa Xaa Gly Xaa Xaa
145                 150


<210> 47
<211> 456
<212> DNA
<213> Artificial Sequence
<220>
<223> Description of Artificial Sequence:consensus
      sequence, minimal encoding sequence after removal
      of non-similar degeneracies and selection of AAD
      parent residues

<400> 47
atgarmaaar ttrwtstgaa aattmwgcgt cygcstcatg scmaagawtt wccgctgccg 60
asctatgcga ccyctcacag ckcaggcctg gatctgcgtg cgkscmttra saawscgstg 120
raamttrmgc cgkktgawas grygctgrtt ccgaccggcc tgatcmttsa wattscggaw 180
ggttmtgmgg sgcagrtgck gccgcgtagc ggcctggsct ggaaamakgg cwtarygstg 240
ctgaacgcgs ygggcmygat tgatrscgat tatcrgggcs aastgawgrt takcstggtg 300
aacckgggcm asgawgaakt trygattsag csgggcgaac gtattgcgca gmtgrttwtt 360
gygccggtgs wgcrggygga akttrwtswg gtggaagawk tttctcagac csakcgtggc 420
gaaggcggct ttggctctas cggcasamag taatga                           456


<210> 48
<211> 150
<212> PRT

<213> Artificial Sequence

<220>
<223> Description of Artificial Sequence:consensus
      sequence, minimal encoding sequence translation
      after removal of non-similar degeneracies and
      selection of AAD parent residues

<220>
<221> MOD_RES
<222> (2)
<223> Xaa = Lys, Ser, Arg or Asn

<220>
<221> MOD_RES
<222> (4)
<223> Xaa = Ile or Val

<220>
<221> MOD_RES
<222> (5)
<223> Xaa = Asp, Ile, Val or Asn

<220>
<221> MOD_RES
<222> (6)
<223> Xaa = Val or Leu

<220>
<221> MOD_RES
<222> (9)
<223> Xaa = Leu, Lys, Gln or Met

<220>
<221> MOD_RES
<222> (11)
<223> Xaa = Pro or Leu


<220>
<221> MOD_RES
<222> (12)
<223> Xaa = Arg or Pro

<220>
<221> MOD_RES
<222> (14)
<223> Xaa = Gly or Ala

<220>
<221> MOD_RES
<222> (15)
<223> Xaa = Lys or Gln

<220>
<221> MOD_RES
<222> (16)
<223> Xaa = Glu or Asp

<220>
<221> MOD_RES
<222> (17)
<223> Xaa = Phe or Leu

<220>
<221> MOD_RES
<222> (21)
<223> Xaa = Thr or Ser

<220>

<221> MOD_RES
<222> (25)
<223> Xaa = Ser or Pro

<220>
<221> MOD_RES
<222> (28)
<223> Xaa = Ala or Ser

<220>
<221> MOD_RES
<222> (35)
<223> Xaa = Cys, Ala, Ser or Gly

<220>
<221> MOD_RES
<222> (36)
<223> Xaa = Leu or Ile

<220>
<221> MOD_RES
<222> (37)
<223> Xaa = Asn, Glu, Lys or Asp

<220>
<221> MOD_RES
<222> (38)
<223> Xaa = Asp or Lys

<220>
<221> MOD_RES
<222> (39)
<223> Xaa = Ala or Pro

<220>
<221> MOD_RES
<222> (40)
<223> Xaa = Val or Leu

<220>
<221> MOD_RES
<222> (41)
<223> Xaa = Glu or Lys

<220>
<221> MOD_RES
<222> (42)
<223> Xaa = Leu or Ile

<220>
<221> MOD_RES
<222> (43)
<223> Xaa = Ala, Lys, Glu or Thr

<220>
<221> MOD_RES
<222> (45)
<223> Xaa = Gly, Phe, Val or Cys

<220>
<221> MOD_RES
<222> (46)
<223> Xaa = Asp or Glu

<220>
<221> MOD_RES
<222> (47)
<223> Xaa = Thr or Arg

<220>
<221> MOD_RES
<222> (48)
<223> Xaa = Thr, Val, Met or Ala

<220>
<221> MOD_RES
<222> (50)
<223> Xaa = Val or Ile

<220>
<221> MOD_RES
<222> (56)
<223> Xaa = Ile or Leu

<220>
<221> MOD_RES
<222> (57)
<223> Xaa = His, Glu, Asp or Gln

<220>
<221> MOD_RES
<222> (59)
<223> Xaa = Ala or Pro

<220>
<221> MOD_RES
<222> (60)
<223> Xaa = Asp or Glu

<220>
<221> MOD_RES
<222> (62)
<223> Xaa = Ser or Tyr

<220>
<221> MOD_RES
<222> (63)
<223> Xaa = Ala or Glu

<220>
<221> MOD_RES
<222> (64)
<223> Xaa = Ala or Gly

<220>
<221> MOD_RES
<222> (66)
<223> Xaa = Met or Val

<220>
<221> MOD_RES
<222> (67)
<223> Xaa = Leu or Arg

<220>
<221> MOD_RES
<222> (73)
<223> Xaa = Gly or Ala

<220>
<221> MOD_RES
<222> (76)
<223> Xaa = His, Lys, Gln or Asn

<220>
<221> MOD_RES
<222> (78)
<223> Xaa = Ile or Leu

<220>
<221> MOD_RES
<222> (79)
<223> Xaa = Val, Thr, Ala or Met

<220>
<221> MOD_RES
<222> (80)
<223> Xaa = Leu or Val

<220>
<221> MOD_RES
<222> (84)
<223> Xaa = Val, Pro, Ala or Leu

<220>
<221> MOD_RES
<222> (86)
<223> Xaa = Leu, Thr, Pro or Met

<220>
<221> MOD_RES
<222> (89)
<223> Xaa = Ser, Ala, Thr or Gly

<220>
<221> MOD_RES
<222> (92)
<223> Xaa = Gln or Arg

<220>
<221> MOD_RES
<222> (95)
<223> Xaa = Leu or Val

<220>
<221> MOD_RES
<222> (96)
<223> Xaa = Met or Lys

<220>
<221> MOD_RES
<222> (97)
<223> Xaa = Ile or Val

<220>
<221> MOD_RES
<222> (98)
<223> Xaa = Ser or Ile

<220>
<221> MOD_RES
<222> (99)
<223> Xaa = Val or Leu

<220>
<221> MOD_RES
<222> (102)
<223> Xaa = Arg or Leu

<220>
<221> MOD_RES
<222> (104)
<223> Xaa = Gln, Asn, His or Lys

<220>
<221> MOD_RES
<222> (105)

<223> Xaa = Asp or Glu

<220>
<221> MOD_RES
<222> (107)
<223> Xaa = Phe or Val

<220>
<221> MOD_RES
<222> (108)
<223> Xaa = Thr, Val, Met or Ala

<220>
<221> MOD_RES
<222> (111)
<223> Xaa = Pro or Arg

<220>
<221> MOD_RES
<222> (118)
<223> Xaa = Met or Leu

<220>
<221> MOD_RES
<222> (119)
<223> Xaa = Ile or Val

<220>
<221> MOD_RES
<222> (120)
<223> Xaa = Phe or Ile

<220>
<221> MOD_RES
<222> (121)
<223> Xaa = Val or Ala

<220>
<221> MOD_RES
<222> (124)
<223> Xaa = Val, Gln, Glu or Leu

<220>
<221> MOD_RES
<222> (125)
<223> Xaa = Gln or Arg

<220>
<221> MOD_RES
<222> (126)
<223> Xaa = Ala or Val

<220>
<221> MOD_RES
<222> (128)
<223> Xaa = Phe or Val

<220>
<221> MOD_RES
<222> (129)
<223> Xaa = Asn, Val, Ile or Asp

<220>
<221> MOD_RES
<222> (130)
<223> Xaa = Leu, Glu, Gln or Val

<220>

<221> MOD_RES
<222> (133)
<223> Xaa = Asp or Glu

<220>
<221> MOD_RES
<222> (134)
<223> Xaa = Phe or Val

<220>
<221> MOD_RES
<222> (138)
<223> Xaa = Asp, Gln, Glu or His

<220>
<221> MOD_RES
<222> (147)
<223> Xaa = Ser or Thr

<220>
<221> MOD_RES
<222> (149)
<223> Xaa = Arg or Thr

<220>
<221> MOD_RES
<222> (150)
<223> Xaa = Gln or Lys

<400> 48
Met Xaa Lys Xaa Xaa Xaa Lys Ile Xaa Arg Xaa Xaa His Xaa Xaa Xaa
1               5                   10                  15

Xaa Pro Leu Pro Xaa Tyr Ala Thr Xaa His Ser Xaa Gly Leu Asp Leu
            20                  25                  30

Arg Ala Xaa Xaa Xaa Xaa Xaa Xaa Xaa Xaa Xaa Pro Xaa Xaa Xaa Xaa
        35                  40                  45

Leu Xaa Pro Thr Gly Leu Ile Xaa Xaa Ile Xaa Xaa Gly Xaa Xaa Xaa
    50                  55                  60

Gln Xaa Xaa Pro Arg Ser Gly Leu Xaa Trp Lys Xaa Gly Xaa Xaa Xaa
65                  70                  75                  80

Leu Asn Ala Xaa Gly Xaa Ile Asp Xaa Asp Tyr Xaa Gly Glx Xaa Xaa
                85                  90                  95

Xaa Xaa Xaa Asn Asn Xaa Gly Xaa Xaa Glu Xaa Xaa Ile Glx Xaa Gly
            100                 105                 110

Glu Arg Ile Ala Gln Xaa Xaa Xaa Xaa Pro Val Xaa Xaa Xaa Glu Xaa
        115                 120                 125

Xaa Xaa Val Glu Xaa Xaa Ser Gln Thr Xaa Arg Gly Glu Gly Gly Phe
    130                 135                 140

Gly Ser Xaa Gly Xaa Xaa
145                 150

<210> 49
<211> 487
<212> DNA
<213> Artificial Sequence

<220>
<223> Description of Artificial Sequence:consensus
      sequence, minimal encoding sequence after removal

of non-similar degeneracies, selection of AAD parent
      residues, priming and restriction sites added to ends
      and codon usage changed to add restriction sites

<400> 49
ttggtaccaa gcttcatatg armaaarttr wtstgaaaat tmwgcgtcyg cstcatgscm 60
aagawttwcc gctgccgasc tatgcgaccy ctcacagckc aggcctggat ctgcgtgcgk 120
scmttrasra wscgstgraa mttrmgccgk ktgawasgry gctgrttccg accggtctga 180
tcmttsawat tscggawggt tmtgmggsgc agrtgckgcc gcgtagcggc ctggsctgga 240
aamakggcwt arygstgctg aacgcgsygg gcmygatcga trscgattat crgggcsaas 300
tgawgrttak cstggtgaac ckgggcmasg awgaakttry gattsagcsg ggcgaacgta 360
ttgcgcagmt grttwttgyg ccggtgswgc rggyggaakt trwtswggtg gaagawkttt 420
ctcagaccsa kcgtggcgaa ggcggctttg gctctascgg casamagtaa tgaggatccg 480
aattctt                                                           487


<210> 50
<211> 150
<212> PRT
<213> Artificial Sequence

<220>
<223> Description of Artificial Sequence:consensus
      sequence, minimal encoding sequence translation
      after removal of non-similar degeneracies and
      selection of AAD parent residues

<220>
<221> MOD_RES
<222> (2)
<223> Xaa = Lys, Ser, Arg or Asn

<220>
<221> MOD_RES
<222> (4)
<223> Xaa = Ile or Val

<220>
<221> MOD_RES
<222> (5)
<223> Xaa = Asp, Ile, Val or Asn

<220>
<221> MOD_RES
<222> (6)
<223> Xaa = Val or Leu
<220>
<221> MOD_RES
<222> (9)
<223> Xaa = Leu, Lys, Gln or Met

<220>
<221> MOD_RES
<222> (11)
<223> Xaa = Pro or Leu

<220>
<221> MOD_RES
<222> (12)
<223> Xaa = Arg or Pro

<220>
<221> MOD_RES
<222> (14)
<223> Xaa = Gly or Ala

<220>
<221> MOD_RES
<222> (15)
<223> Xaa = Lys or Gln

<220>
<221> MOD_RES
<222> (16)
<223> Xaa = Glu or Asp

<220>
<221> MOD_RES
<222> (17)
<223> Xaa = Phe or Leu

<220>
<221> MOD_RES
<222> (21)
<223> Xaa = Thr or Ser

<220>
<221> MOD_RES
<222> (25)
<223> Xaa = Ser or Pro

<220>
<221> MOD_RES
<222> (28)
<223> Xaa = Ala or Ser

<220>
<221> MOD_RES
<222> (35)
<223> Xaa = Cys, Ala, Ser or Gly

<220>
<221> MOD_RES
<222> (36)
<223> Xaa = Leu or Ile

<220>
<221> MOD_RES
<222> (37)
<223> Xaa = Asn, Glu, Lys or Asp

<220>
<221> MOD_RES
<222> (38)
<223> Xaa = Asp, Lys, Asn or Glu

<220>
<221> MOD_RES
<222> (39)
<223> Xaa = Ala or Pro

<220>
<221> MOD_RES
<222> (40)
<223> Xaa = Val or Leu

<220>
<221> MOD_RES
<222> (41)
<223> Xaa = Glu or Lys

<220>
<221> MOD_RES
<222> (42)
<223> Xaa = Leu or Ile

<220>
<221> MOD_RES
<222> (43)

<223> Xaa = Ala, Lys, Glu or Thr

<220>
<221> MOD_RES
<222> (45)
<223> Xaa = Gly, Phe, Val or Cys

<220>
<221> MOD_RES
<222> (46)
<223> Xaa = Asp or Glu

<220>
<221> MOD_RES
<222> (47)
<223> Xaa = Thr or Arg

<220>
<221> MOD_RES
<222> (48)
<223> Xaa = Thr, Val, Met or Ala

<220>
<221> MOD_RES
<222> (50)
<223> Xaa = Val or Ile


<220>
<221> MOD_RES
<222> (56)
<223> Xaa = Ile or Leu

<220>
<221> MOD_RES
<222> (57)
<223> Xaa = His, Glu, Asp or Gln

<220>
<221> MOD_RES
<222> (59)
<223> Xaa = Ala or Pro

<220>
<221> MOD_RES
<222> (60)
<223> Xaa = Asp or Glu

<220>
<221> MOD_RES
<222> (62)
<223> Xaa = Ser or Tyr

<220>
<221> MOD_RES
<222> (63)
<223> Xaa = Ala or Glu

<220>
<221> MOD_RES
<222> (64)
<223> Xaa = Ala or Gly

<220>
<221> MOD_RES
<222> (66)
<223> Xaa = Met or Val

<220>
<221> MOD_RES

<222> (67)
<223> Xaa = Leu or Arg

<220>
<221> MOD_RES
<222> (73)
<223> Xaa = Gly or Ala

<220>
<221> MOD_RES
<222> (76)
<223> Xaa = His, Lys, Gln or Asn

<220>
<221> MOD_RES
<222> (78)
<223> Xaa = Ile or Leu

<220>
<221> MOD_RES
<222> (79)
<223> Xaa = Val, Thr, Ala or Met

<220>
<221> MOD_RES
<222> (80)
<223> Xaa = Leu or Val

<220>
<221> MOD_RES
<222> (84)
<223> Xaa = Val, Pro, Ala or Leu

<220>
<221> MOD_RES
<222> (86)
<223> Xaa = Leu, Thr, Pro or Met

<220>
<221> MOD_RES
<222> (89)
<223> Xaa = Ser, Ala, Thr or Gly

<220>
<221> MOD_RES
<222> (92)
<223> Xaa = Gln or Arg

<220>
<221> MOD_RES
<222> (95)
<223> Xaa = Leu or Val

<220>
<221> MOD_RES
<222> (96)
<223> Xaa = Met or Lys

<220>
<221> MOD_RES
<222> (97)
<223> Xaa = Ile or Val

<220>
<221> MOD_RES
<222> (98)
<223> Xaa = Ser or Ile

<220>

<221> MOD_RES
<222> (99)
<223> Xaa = Val or Leu

<220>
<221> MOD_RES
<222> (102)
<223> Xaa = Arg or Leu

<220>
<221> MOD_RES
<222> (104)
<223> Xaa = Gln, Asn, His or Lys

<220>
<221> MOD_RES
<222> (105)
<223> Xaa = Asp or Glu

<220>
<221> MOD_RES
<222> (107)
<223> Xaa = Phe or Val

<220>
<221> MOD_RES
<222> (108)
<223> Xaa = Thr, Val, Met or Ala

<220>
<221> MOD_RES
<222> (111)
<223> Xaa = Pro or Arg

<220>
<221> MOD_RES
<222> (118)
<223> Xaa = Met or Leu

<220>
<221> MOD_RES
<222> (119)
<223> Xaa = Ile or Val

<220>
<221> MOD_RES
<222> (120)
<223> Xaa = Phe or Ile

<220>
<221> MOD_RES
<222> (121)
<223> Xaa = Val or Ala

<220>
<221> MOD_RES
<222> (124)
<223> Xaa = Val, Gln, Glu or Leu

<220>
<221> MOD_RES
<222> (125)
<223> Xaa = Gln or Arg

<220>
<221> MOD_RES
<222> (126)
<223> Xaa = Ala or Val

<220>
<221> MOD_RES
<222> (128)
<223> Xaa = Phe or Val

<220>
<221> MOD_RES
<222> (129)
<223> Xaa = Asn, Val, Ile or Asp

<220>
<221> MOD_RES
<222> (130)
<223> Xaa = Leu, Glu, Gln or Val

<220>
<221> MOD_RES
<222> (133)
<223> Xaa = Asp or Glu

<220>
<221> MOD_RES
<222> (134)
<223> Xaa = Phe or Val

<220>
<221> MOD_RES
<222> (138)
<223> Xaa = Asp, Gln, Glu or His

<220>
<221> MOD_RES
<222> (147)
<223> Xaa = Ser or Thr

<220>
<221> MOD_RES
<222> (149)
<223> Xaa = Arg or Thr

<220>
<221> MOD_RES
<222> (150)
<223> Xaa = Gln or Lys

<400> 50
Met Xaa Lys Xaa Xaa Xaa Lys Ile Xaa Arg Xaa Xaa His Xaa Xaa Xaa
1               5                   10                  15

Xaa Pro Leu Pro Xaa Tyr Ala Thr Xaa His Ser Xaa Gly Leu Asp Leu
            20                  25                  30

Arg Ala Xaa Xaa Xaa Xaa Xaa Xaa Xaa Xaa Xaa Pro Xaa Xaa Xaa Xaa
        35                  40                  45

Leu Xaa Pro Thr Gly Leu Ile Xaa Xaa Ile Xaa Xaa Gly Xaa Xaa Xaa
    50                  55                  60

Gln Xaa Xaa Pro Arg Ser Gly Leu Xaa Trp Lys Xaa Gly Xaa Xaa Xaa
65                  70                  75                  80

Leu Asn Ala Xaa Gly Xaa Ile Asp Xaa Asp Tyr Xaa Gly Glx Xaa Xaa
                85                  90                  95

Xaa Xaa Xaa Asn Asn Xaa Gly Xaa Xaa Glu Xaa Xaa Ile Glx Xaa Gly
            100                 105                 110

Glu Arg Ile Ala Gln Xaa Xaa Xaa Xaa Pro Val Xaa Xaa Xaa Glu Xaa
        115                 120                 125

Xaa Xaa Val Glu Xaa Xaa Ser Gln Thr Xaa Arg Gly Glu Gly Gly Phe
    130                 135                 140

Gly Ser Xaa Gly Xaa Xaa
145                 150


<210> 51
<211> 2349
<212> DNA
<213> Artificial Sequence

<220>
<223> Description of Artificial Sequence:Pfu/Deep Vent
      hybrid DNA polymerase minimal encoding sequence

<400> 51
atgatcctgg atgytgacta catcactgaa gamggcaaac cgrttatccg tmtsttcaaa 60
aaagagaacg gcraatttaa grttgagyat gatcgcamct ttcgtccata catttacgct 120
ctgctgarag atgattctma gattgakgaa gttaraaaaa tcactgstga gcgccatggc 180
aagattgttc gtatcrttga tgyggaaaag gtarrgaaga aatttctggg caraccaatc 240
rmggtgtgga ractgtatyt cgaacatcca caagatgttc cgaytattcg cgakaaartt 300
cgcgaacaty ctgcagttrt tgacatcttc gaatacgata ttccatttgc aaagcgttac 360
ctcatcgaca aaggcctgat accaatggag ggcgakgaag aactcaagmt cctggcgttc 420
gatatagaaa ccctctatca cgaaggcgaa gagtttgsta aaggcccaat tataatgatt 480
agctatgcag atgaaramga agcaaaggtg attacttgga aaaamataga tctcccatac 540
gttgaggttg tatcttccga gcgcgagatg attaagcgct ttctcarart tatccgcgag 600
aaggatccgg acrttatcrt tacttataac ggcgactctt ttgacytccc atatctggyg 660
aaacgcgcag aaaaactcgg tattaaactg mctmtcggcc gtgatggttc cgagccgaag 720
atgcagcgtm tcggcgatat gaccgctgta gaarttaagg gtcgtatcca tttcgacctg 780
tatcatgtaa ttmsccgtac tattaacctc ccgacttaca ctctcgaggc tgtatatgaa 840
gcaatttttg gtaagccgaa ggagaaggta tacgccsatg agattgcara ggcgtgggaa 900
wccggtragr rcctcgagcg tgttgcaaaa tactccatgg aagatgcaaa ggygacttat 960
gaactcggca ragaattcyt cccaatggaa rytcagctct ctcgcctggt tggccaacca 1020
ctgtgggatg tttctcgttc ttccaccggt aacctcgtag agtggtwtct cctgcgcaaa 1080
gcgtacgaac gcaacgaast ggctccgaac aagccakmcg aasragagta tsaacgccgt 1140
ctccgcgagt cttacrctgg tggctwtgtt aaagagccag aaaagggcct ctgggaarrc 1200
mtcgtgtmcc tcgattttcg ckctctgtat ccgtctatta tcattaccca caacgtgtct 1260
ccggatactc tcaacckcga gggctgcara ramtatgatr ttgctccgsa agtaggccac 1320
aagttctgca aggacwtccc gggctttatt ccgtctctcc tgrrgcrtct gctcgakgaa 1380
cgccaaraga ttaagmscaa aatgaaggmg wccmaggatc cgattgaaaa aawamtgctc 1440
gactatcgcc aaaragcgat taaamtcctc gcaaactctt wttacggcta ttatggctat 1500
gcaaaagcac gctggtactg taaggagtgt gctgagtccg ttactgcttg gggtcgcraa 1560
tacatcgagy tcgtgyggaa ggagctcgaa gaaaagtttg gctttaaagt tctctacatt 1620
gacactgatg gtctctatgc gactattccg ggtgstragy ctgaggaaat taagaaaaag 1680
gctctcgaat ttgtgaaata cattaackcg aagctcccgg gtctcctgga gctcgaatat 1740
gaaggctttt atrwgcgcgg cttcttcgtt accaagaaga ratatgcgst gattgatgaa 1800
gaaggcaaar ttattactcg tggtctcgag attgtgcgcc gtgattggag cgaaattgcg 1860
aaagaaactc aagctaragt tctcgagrct attctcaaac acggcracgt tgaagaagct 1920
gtgaraattg taaaagaagt aaycsaaaag ctckctaamt atgaaattcc gccagagaag 1980
ctcgygattt atgagcagat tactcgcccg ctgcatgagt ataaggcgat tggtccgcac 2040
gtggctgttg caaagaract ggctgctara ggcgtgaaar ttaraccggg tatggtaatt 2100
ggctacattg tactccgcgg cgatggtccg attagcaamc gtgcaattct agctgaggaa 2160
twcgatcyga raaagcacaa gtatgacgca gaatattaca ttgagaacca ggtgctcccg 2220
gcggtactcc gtattctgga ggstttggc taccgtaagg aagacctccg ctrscaaaag 2280
actaracagr ytggcctcac tkcttggctc aacattaaaa aatccggtac ccactagtgc 2340
tagcatgac                                                         2349


<210> 52
<211> 778
<212> PRT
<213> Artificial Sequence

<220>
<223> Description of Artificial Sequence:Pfu/Deep Vent
      hybrid DNA polymerase minimal encoding sequence
      translation

<220>
<221> MOD_RES
<222> (5)
<223> Xaa = Val or Ala

<220>
<221> MOD_RES
<222> (11)
<223> Xaa = Glu or Asp

<220>
<221> MOD_RES
<222> (15)
<223> Xaa = Val or Ile

<220>
<221> MOD_RES
<222> (18)
<223> Xaa = Leu or Ile

<220>
<221> MOD_RES
<222> (25)
<223> Xaa = Lys or Glu

<220>
<221> MOD_RES
<222> (28)
<223> Xaa = Ile or Val

<220>
<221> MOD_RES
<222> (30)
<223> Xaa = His or Tyr

<220>
<221> MOD_RES
<222> (33)
<223> Xaa = Asn or Thr

<220>
<221> MOD_RES
<222> (43)
<223> Xaa = Arg or Lys
<220>
<221> MOD_RES
<222> (47)
<223> Xaa = Lys or Gln

<220>
<221> MOD_RES
<222> (49)
<223> Xaa = Glu or Asp

<220>
<221> MOD_RES
<222> (52)
<223> Xaa = Lys or Arg

<220>
<221> MOD_RES
<222> (56)
<223> Xaa = Gly or Ala

<220>
<221> MOD_RES
<222> (66)
<223> Xaa = Ile or Val

<220>

<221> MOD_RES
<222> (68)
<223> Xaa = Val or Ala

<220>
<221> MOD_RES
<222> (72)
<223> Xaa = Gly, Glu, Arg or Lys

<220>
<221> MOD_RES
<222> (78)
<223> Xaa = Lys or Arg

<220>
<221> MOD_RES
<222> (81)
<223> Xaa = Lys, Thr, Glu or Ala

<220>
<221> MOD_RES
<222> (84)
<223> Xaa = Lys or Arg

<220>
<221> MOD_RES
<222> (87)
<223> Xaa = Leu or Phe

<220>
<221> MOD_RES
<222> (95)
<223> Xaa = Thr or Ala

<220>
<221> MOD_RES
<222> (98)
<223> Xaa = Glu or Asp

<220>
<221> MOD_RES
<222> (100)
<223> Xaa = Val or Ile

<220>
<221> MOD_RES
<222> (104)
<223> Xaa = Pro or Ser

<220>
<221> MOD_RES
<222> (107)
<223> Xaa = Val or Ile

<220>
<221> MOD_RES
<222> (132)
<223> Xaa = Glu or Asp

<220>
<221> MOD_RES
<222> (137)
<223> Xaa = Ile or Leu

<220>
<221> MOD_RES
<222> (153)
<223> Xaa = Gly or Ala

<220>
<221> MOD_RES
<222> (166)
<223> Xaa = Lys, Asn, Glu or Asp

<220>
<221> MOD_RES
<222> (175)
<223> Xaa = Lys or Asn

<220>
<221> MOD_RES
<222> (196)
<223> Xaa = Arg or Lys

<220>
<221> MOD_RES
<222> (197)
<223> Xaa = Val or Ile

<220>
<221> MOD_RES
<222> (205)
<223> Xaa = Val or Ile

<220>
<221> MOD_RES
<222> (207)
<223> Xaa = Val or Ile

<220>
<221> MOD_RES
<222> (216)
<223> Xaa = Phe or Leu

<220>
<221> MOD_RES
<222> (220)
<223> Xaa = Val or Ala

<220>
<221> MOD_RES
<222> (231)
<223> Xaa = Thr or Pro

<220>
<221> MOD_RES
<222> (232)
<223> Xaa = Ile or Leu

<220>
<221> MOD_RES
<222> (244)
<223> Xaa = Ile or Leu

<220>
<221> MOD_RES
<222> (252)
<223> Xaa = Val or Ile

<220>
<221> MOD_RES
<222> (265)
<223> Xaa = Thr, Ser, Pro or Arg

<220>
<221> MOD_RES
<222> (293)
<223> Xaa = Asp or His

<220>
<221> MOD_RES
<222> (297)
<223> Xaa = Lys or Glu

<220>
<221> MOD_RES
<222> (301)
<223> Xaa = Thr or Ser

<220>
<221> MOD_RES
<222> (303)
<223> Xaa = Lys or Glu

<220>
<221> MOD_RES
<222> (304)
<223> Xaa = Asn, Gly, Asp or Ser

<220>
<221> MOD_RES
<222> (318)
<223> Xaa = Val or Ala

<220>
<221> MOD_RES
<222> (324)
<223> Xaa = Arg or Lys

<220>
<221> MOD_RES
<222> (327)
<223> Xaa = Leu or Phe

<220>
<221> MOD_RES
<222> (331)
<223> Xaa = Ile, Thr, Val or Ala

<220>
<221> MOD_RES
<222> (356)
<223> Xaa = Phe or Tyr

<220>
<221> MOD_RES
<222> (367)
<223> Xaa = Val or Leu

<220>
<221> MOD_RES
<222> (373)
<223> Xaa = Ser, Tyr, Ala or Asp

<220>
<221> MOD_RES
<222> (375)
<223> Xaa = Arg, Gln, Gly or Glu

<220>
<221> MOD_RES
<222> (386)
<223> Xaa = Thr or Ala

<220>
<221> MOD_RES
<222> (389)

<223> Xaa = Phe or Tyr

<220>
<221> MOD_RES
<222> (400)
<223> Xaa = Asn, Ser, Asp or Gly


<220>
<221> MOD_RES
<222> (401)
<223> Xaa = Ile or Leu

<220>
<221> MOD_RES
<222> (403)
<223> Xaa = Tyr or Ser

<220>
<221> MOD_RES
<222> (408)
<223> Xaa = Ala or Ser

<220>
<221> MOD_RES
<222> (426)
<223> Xaa = Leu or Arg

<220>
<221> MOD_RES
<222> (430)
<223> Xaa = Lys or Arg

<220>
<221> MOD_RES
<222> (431)
<223> Xaa = Lys, Asn, Glu or Asp

<220>
<221> MOD_RES
<222> (434)
<223> Xaa = Val or Ile

<220>
<221> MOD_RES
<222> (446)
<223> Xaa = Ile or Phe

<220>
<221> MOD_RES
<222> (455)
<223> Xaa = Lys, Arg, Glu or Gly

<220>
<221> MOD_RES
<222> (456)
<223> Xaa = Arg or His

<220>
<221> MOD_RES
<222> (459)
<223> Xaa = Glu or Asp

<220>
<221> MOD_RES
<222> (463)
<223> Xaa = Lys or Glu


<220>

<221> MOD_RES
<222> (466)
<223> Xaa = Thr, Ser, Pro or Arg

<220>
<221> MOD_RES
<222> (470)
<223> Xaa = Glu or Ala

<220>
<221> MOD_RES
<222> (471)
<223> Xaa = Thr or Ser

<220>
<221> MOD_RES
<222> (472)
<223> Xaa = Gln or Lys

<220>
<221> MOD_RES
<222> (478)
<223> Xaa = Ile or Lys

<220>
<221> MOD_RES
<222> (479)
<223> Xaa = Met or Leu

<220>
<221> MOD_RES
<222> (485)
<223> Xaa = Arg or Lys

<220>
<221> MOD_RES
<222> (489)
<223> Xaa = Leu or Ile

<220>
<221> MOD_RES
<222> (494)
<223> Xaa = Phe or Tyr

<220>
<221> MOD_RES
<222> (520)
<223> Xaa = Lys or Glu

<220>
<221> MOD_RES
<222> (524)
<223> Xaa = Leu or Phe

<220>
<221> MOD_RES
<222> (526)
<223> Xaa = Trp or Arg

<220>
<221> MOD_RES
<222> (552)
<223> Xaa = Gly or Ala

<220>
<221> MOD_RES
<222> (553)
<223> Xaa = Glu or Lys

<220>
<221> MOD_RES
<222> (554)
<223> Xaa = Ser or Pro

<220>
<221> MOD_RES
<222> (570)
<223> Xaa = Ala or Ser

<220>
<221> MOD_RES
<222> (585)
<223> Xaa = Val, Glu, Met or Lys

<220>
<221> MOD_RES
<222> (594)
<223> Xaa = Arg or Lys

<220>
<221> MOD_RES
<222> (597)
<223> Xaa = Val or Leu

<220>
<221> MOD_RES
<222> (604)
<223> Xaa = Val or Ile

<220>
<221> MOD_RES
<222> (626)
<223> Xaa = Arg or Lys

<220>
<221> MOD_RES
<222> (630)
<223> Xaa = Thr or Ala

<220>
<221> MOD_RES
<222> (642)
<223> Xaa = Arg or Lys

<220>
<221> MOD_RES
<222> (648)
<223> Xaa = Ile or Thr

<220>
<221> MOD_RES
<222> (652)
<223> Xaa = Ala or Ser

<220>
<221> MOD_RES
<222> (653)
<223> Xaa = Lys or Asn

<220>
<221> MOD_RES
<222> (662)
<223> Xaa = Val or Ala

<220>
<221> MOD_RES
<222> (686)
<223> Xaa = Arg or Lys

<220>
<221> MOD_RES
<222> (690)
<223> Xaa = Lys or Arg

<220>
<221> MOD_RES
<222> (694)
<223> Xaa = Val or Ile

<220>
<221> MOD_RES
<222> (695)
<223> Xaa = Arg or Lys

<220>
<221> MOD_RES
<222> (713)
<223> Xaa = Lys or Asn

<220>
<221> MOD_RES
<222> (721)
<223> Xaa = Phe or Tyr

<220>
<221> MOD_RES
<222> (723)
<223> Xaa = Pro or Leu

<220>
<221> MOD_RES
<222> (724)
<223> Xaa = Lys or Arg

<220>
<221> MOD_RES
<222> (748)
<223> Xaa = Gly or Ala

<220>
<221> MOD_RES
<222> (758)
<223> Xaa = Tyr, Trp, stop codon or Cys

<220>
<221> MOD_RES
<222> (762)
<223> Xaa = Lys or Arg

<220>
<221> MOD_RES
<222> (764)
<223> Xaa = Val, Ala, Ile or Thr

<220>
<221> MOD_RES
<222> (768)
<223> Xaa = Ala or Ser

<400> 52
Met Ile Leu Asp Xaa Asp Tyr Ile Thr Glu Xaa Gly Lys Pro Xaa Ile
1               5                   10                  15

Arg Xaa Phe Lys Lys Glu Asn Gly Xaa Phe Lys Xaa Glu Xaa Asp Arg
            20                  25                  30

Xaa Phe Arg Pro Tyr Ile Tyr Ala Leu Leu Xaa Asp Asp Ser Xaa Ile

        35                  40                  45

Xaa Glu Val Xaa Lys Ile Thr Xaa Glu Arg His Gly Lys Ile Val Arg
    50                  55                  60

Ile Xaa Asp Xaa Glu Lys Val Xaa Lys Lys Phe Leu Gly Xaa Pro Ile
65                  70                  75                  80

Xaa Val Trp Xaa Leu Tyr Xaa Glu His Pro Gln Asp Val Pro Xaa Ile
                85                  90                  95

Arg Xaa Lys Xaa Arg Glu His Xaa Ala Val Xaa Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Xaa Glu Glu Leu Lys Xaa Leu Ala Phe Asp Ile Glu Thr
    130                 135                 140

Leu Tyr His Glu Gly Glu Glu Phe Xaa Lys Gly Pro Ile Ile Met Ile
145                 150                 155                 160

Ser Tyr Ala Asp Glu Xaa Glu Ala Lys Val Ile Thr Trp Lys Xaa Ile
                165                 170                 175

Asp Leu Pro Tyr Val Glu Val Val Ser Ser Glu Arg Glu Met Ile Lys
            180                 185                 190

Arg Phe Leu Xaa Xaa Ile Arg Glu Lys Asp Pro Asp Xaa Ile Xaa Thr
        195                 200                 205

Tyr Asn Gly Asp Ser Phe Asp Xaa Pro Tyr Leu Xaa Lys Arg Ala Glu
    210                 215                 220

Lys Leu Gly Ile Lys Leu Xaa Xaa Gly Arg Asp Gly Ser Glu Pro Lys
225                 230                 235                 240

Met Gln Arg Xaa Gly Asp Met Thr Ala Val Glu Xaa Lys Gly Arg Ile
                245                 250                 255

His Phe Asp Leu Tyr His Val Ile Xaa Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Xaa Glu Ile Ala Xaa Ala Trp Glu Xaa Gly Xaa Xaa
    290                 295                 300

Leu Glu Arg Val Ala Lys Tyr Ser Met Glu Asp Ala Lys Xaa Thr Tyr
305                 310                 315                 320

Glu Leu Gly Xaa Glu Phe Xaa Pro Met Glu Xaa Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Xaa Leu Leu Arg Lys Ala Tyr Glu Arg Asn Glu Xaa Ala
        355                 360                 365

Pro Asn Lys Pro Xaa Glu Xaa Glu Tyr Glx Arg Arg Leu Arg Glu Ser
    370                 375                 380

Tyr Xaa Gly Gly Xaa Val Lys Glu Pro Glu Lys Gly Leu Trp Glu Xaa
385                 390                 395                 400

Xaa Val Xaa Leu Asp Phe Arg Xaa Leu Tyr Pro Ser Ile Ile Ile Thr

                405                 410                 415

His Asn Val Ser Pro Asp Thr Leu Asn Xaa Glu Gly Cys Xaa Xaa Tyr
            420                 425                 430

Asp Xaa Ala Pro Glx Val Gly His Lys Phe Cys Lys Asp Xaa Pro Gly
        435                 440                 445

Phe Ile Pro Ser Leu Leu Xaa Xaa Leu Leu Xaa Glu Arg Gln Xaa Ile
    450                 455                 460

Lys Xaa Lys Met Lys Xaa Xaa Xaa Asp Pro Ile Glu Lys Xaa Xaa Leu
465                 470                 475                 480

Asp Tyr Arg Gln Xaa Ala Ile Lys Xaa Leu Ala Asn Ser Xaa Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Xaa Tyr Ile Glu Xaa Val Xaa Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Xaa Xaa Xaa Glu Glu Ile Lys Lys Lys
545                 550                 555                 560

Ala Leu Glu Phe Val Lys Tyr Ile Asn Xaa Lys Leu Pro Gly Leu Leu
                565                 570                 575

Glu Leu Glu Tyr Glu Gly Phe Tyr Xaa Arg Gly Phe Phe Val Thr Lys
            580                 585                 590

Lys Xaa Tyr Ala Xaa Ile Asp Glu Glu Gly Lys Xaa Ile Thr Arg Gly
        595                 600                 605

Leu Glu Ile Val Arg Arg Asp Trp Ser Glu Ile Ala Lys Glu Thr Gln
    610                 615                 620

Ala Xaa Val Leu Glu Xaa Ile Leu Lys His Gly Asx Val Glu Glu Ala
625                 630                 635                 640

Val Xaa Ile Val Lys Glu Val Xaa Glx Lys Leu Xaa Xaa Tyr Glu Ile
                645                 650                 655

Pro Pro Glu Lys Leu Xaa Ile Tyr Glu Gln Ile Thr Arg Pro Leu His
            660                 665                 670

Glu Tyr Lys Ala Ile Gly Pro His Val Ala Val Ala Lys Xaa Leu Ala
        675                 680                 685

Ala Xaa Gly Val Lys Xaa Xaa Pro Gly Met Val Ile Gly Tyr Ile Val
    690                 695                 700

Leu Arg Gly Asp Gly Pro Ile Ser Xaa Arg Ala Ile Leu Ala Glu Glu
705                 710                 715                 720

Xaa Asp Xaa Xaa Lys His Lys Tyr Asp Ala Glu Tyr Tyr Ile Glu Asn
                725                 730                 735

Gln Val Leu Pro Ala Val Leu Arg Ile Leu Glu Xaa Phe Gly Tyr Arg
            740                 745                 750

Lys Glu Asp Leu Arg Xaa Gln Lys Thr Xaa Gln Xaa Gly Leu Thr Xaa
        755                 760                 765

Trp Leu Asn Ile Lys Lys Ser Gly Thr His

770                 775

<210> 53
<211> 783
<212> PRT
<213> Artificial Sequence

<220>
<223> Description of Artificial Sequence:designed hybrid
      polymerase from Figure 11

<220>
<221> MOD_RES
<222> (1)..(783)
<223> Xaa = unknown amino acid

<400> 53
Met Ile Leu Asp Xaa Asp Tyr Ile Thr Glu Xaa Gly Lys Pro Xaa Ile
  1               5                  10                  15

Arg Xaa Phe Lys Lys Glu Asn Gly Xaa Phe Lys Xaa Glu Xaa Asp Arg
             20                  25                  30

Xaa Phe Arg Pro Tyr Ile Tyr Ala Leu Leu Xaa Asp Asp Ser Xaa Ile
         35                  40                  45

Xaa Glu Val Xaa Lys Ile Thr Xaa Glu Arg His Gly Lys Ile Val Arg
     50                  55                  60

Ile Xaa Asp Xaa Glu Lys Val Xaa Lys Lys Phe Leu Gly Xaa Pro Ile
 65                  70                  75                  80

Xaa Val Trp Xaa Leu Tyr Xaa Glu His Pro Gln Asp Val Pro Xaa Ile
                 85                  90                  95

Arg Xaa Lys Xaa Arg Glu His Xaa Ala Val Xaa Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Xaa Glu Glu Leu Lys Xaa Leu Ala Phe Asp Ile Glu Thr
    130                 135                 140

Leu Tyr His Glu Gly Glu Glu Phe Xaa Lys Gly Pro Ile Ile Met Ile
145                 150                 155                 160

Ser Tyr Ala Asp Glu Xaa Glu Ala Lys Val Ile Thr Trp Lys Xaa Ile
                165                 170                 175

Asp Leu Pro Tyr Val Glu Val Val Ser Ser Glu Arg Glu Met Ile Lys
            180                 185                 190

Arg Phe Leu Xaa Xaa Ile Arg Glu Lys Asp Pro Asp Xaa Ile Xaa Thr
        195                 200                 205

Tyr Asn Gly Asp Ser Phe Asp Xaa Pro Tyr Leu Xaa Lys Arg Ala Glu
    210                 215                 220

Lys Leu Gly Ile Lys Leu Xaa Xaa Gly Arg Asp Gly Ser Glu Pro Lys
225                 230                 235                 240

Met Gln Arg Xaa Gly Asp Met Thr Ala Val Glu Xaa Lys Gly Arg Ile
                245                 250                 255

His Phe Asp Leu Tyr His Val Ile Xaa Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Xaa Glu Ile Ala Xaa Ala Trp Glu Xaa Gly Xaa Xaa
    290                 295                 300

Leu Glu Arg Val Ala Lys Tyr Ser Met Glu Asp Ala Lys Xaa Thr Tyr
305                 310                 315                 320

Glu Leu Gly Xaa Glu Phe Xaa Pro Met Glu Xaa Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Xaa Leu Leu Arg Lys Ala Tyr Glu Arg Asn Glu Xaa Ala
        355                 360                 365

Pro Asn Lys Pro Xaa Glu Xaa Glu Tyr Xaa Arg Arg Leu Arg Glu Ser
    370                 375                 380

Tyr Xaa Gly Gly Xaa Val Lys Glu Pro Glu Lys Gly Leu Trp Glu Xaa
385                 390                 395                 400

Xaa Val Xaa Leu Asp Phe Arg Xaa Leu Tyr Pro Ser Ile Ile Ile Thr
                405                 410                 415

His Asn Val Ser Pro Asp Thr Leu Asn Xaa Glu Gly Cys Xaa Xaa Tyr
            420                 425                 430

Asp Xaa Ala Pro Xaa Val Gly His Lys Phe Cys Lys Asp Xaa Pro Gly
        435                 440                 445

Phe Ile Pro Ser Leu Leu Xaa Xaa Leu Leu Xaa Glu Arg Gln Xaa Ile
    450                 455                 460

Lys Xaa Lys Met Lys Xaa Xaa Xaa Asp Pro Ile Glu Lys Xaa Xaa Leu
465                 470                 475                 480

Asp Tyr Arg Gln Xaa Ala Ile Lys Xaa Leu Ala Asn Ser Xaa Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Xaa Tyr Ile Glu Xaa Val Xaa Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Xaa Xaa Xaa Glu Glu Ile Lys Lys Lys
545                 550                 555                 560

Ala Leu Glu Phe Val Lys Tyr Ile Asn Xaa Lys Leu Pro Gly Leu Leu
                565                 570                 575

Glu Leu Glu Tyr Glu Gly Phe Tyr Xaa Arg Gly Phe Phe Val Thr Lys
            580                 585                 590

Lys Xaa Tyr Ala Xaa Ile Asp Glu Glu Gly Lys Xaa Ile Thr Arg Gly
        595                 600                 605

Leu Glu Ile Val Arg Arg Asp Trp Ser Glu Ile Ala Lys Glu Thr Gln
    610                 615                 620

Ala Xaa Val Leu Glu Xaa Ile Leu Lys His Gly Xaa Val Glu Glu Ala
625                 630                 635                 640

Val Xaa Ile Val Lys Glu Val Xaa Xaa Lys Leu Xaa Xaa Tyr Glu Ile
                645                 650                 655

Pro Pro Glu Lys Leu Xaa Ile Tyr Glu Gln Ile Thr Arg Pro Leu His
            660                 665                 670

Glu Tyr Lys Ala Ile Gly Pro His Val Ala Val Ala Lys Xaa Leu Ala
        675                 680                 685

Ala Xaa Gly Val Lys Xaa Xaa Pro Gly Met Val Ile Gly Tyr Ile Val
    690                 695                 700

Leu Arg Gly Asp Gly Pro Ile Ser Xaa Arg Ala Ile Leu Ala Glu Glu
705                 710                 715                 720

Xaa Asp Xaa Xaa Lys His Lys Tyr Asp Ala Glu Tyr Tyr Ile Glu Asn
                725                 730                 735

Gln Val Leu Pro Ala Val Leu Arg Ile Leu Glu Xaa Phe Gly Tyr Arg
            740                 745                 750

Lys Glu Asp Leu Arg Xaa Gln Lys Thr Xaa Gln Xaa Gly Leu Thr Xaa
        755                 760                 765

Trp Leu Asn Ile Lys Lys Ser Gly Thr His Asn Cys Asn His Asp
    770                 775                 780


<210> 54
<211> 845
<212> PRT
<213> Artificial Sequence

<220>
<223> Description of Artificial Sequence:hybrid
      polymerase HyS1 from Figure 11

<220>
<221> MOD_RES
<222> (472)
<223> Xaa = unknown amino acid

<400> 54
Met Ile Leu Asp Ala Asp Tyr Ile Thr Glu Asp Gly Lys Pro Val Ile
  1               5                  10                  15

Arg Leu Phe Lys Lys Glu Asn Gly Glu Phe Lys Ile Glu Tyr Asp Arg
             20                  25                  30

Thr Phe Arg Pro Tyr Ile Tyr Ala Leu Leu Arg Asp Asp Ser Lys Ile
         35                  40                  45

Glu Glu Val Arg Lys Ile Thr Ala Glu Arg His Gly Lys Ile Val Arg
     50                  55                  60

Ile Val Asp Val Glu Lys Val Arg Lys Lys Phe Leu Gly Arg Pro Ile
 65                  70                  75                  80

Lys Val Trp Arg Leu Tyr Phe Glu His Pro Gln Asp Val Pro Thr Ile
                 85                  90                  95

Arg Asp Lys Val Arg Glu His Pro Ala Val Ile Asp Ile Phe Glu Tyr
            100                 105                 110

Asp Ile Ala Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140

Leu Tyr His Gly Ser Glu Glu Phe Gly Lys Gly Pro Ile Ile Met Ile
145                 150                 155                 160

Ser Tyr Ala Asp Glu Asn Glu Ala Lys Val Ile Thr Trp Lys Asn Ile
                165                 170                 175

Asp Leu Pro Tyr Val Glu Val Val Ser Ser Glu Arg Glu Met Ile Lys
            180                 185                 190

Arg Phe Leu Arg Ile Ile Arg Glu Lys Asp Pro Asp Ile Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Leu Gly Arg Asp Gly Cys Glu Ala Lys
225                 230                 235                 240

Met Gln Arg Leu Gly Asp Met Thr Ala Val Glu Val Lys Gly Arg Ile
                245                 250                 255

His Phe Asp Leu Tyr Tyr Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Asp Ile Ala Glu Ala Trp Glu Thr Gly Lys Gly
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Tyr Glu Arg Glu Tyr Glu Arg Arg Leu Arg Glu Ser
    370                 375                 380

Tyr Thr Gly Gly Phe Val Lys Glu Pro Glu Lys Gly Leu Trp Glu Ser
385                 390                 395                 400

Leu Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Ala Gly Leu Thr Ala
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys Asn
        835                 840                 845

<210> 55
<211> 758
<212> PRT
<213> Artificial Sequence

<220>
<223> Description of Artificial Sequence:hybrid
      polymerase Hyb2 from Figure 11

<220>
<221> MOD_RES
<222> (472)
<223> Xaa = unknown amino acid

<400> 55
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Glu Asp Asp Ser Lys Ile
         35                  40                  45

Asp Glu Val Arg Lys Ile Thr Ala Glu Arg His Gly Lys Ile Val Arg
     50                  55                  60

Ile Val Asp Ala Glu Lys Val Glu Lys Lys Phe Leu Gly Arg Pro Ile
 65                  70                  75                  80

Thr Val Trp Lys Leu Tyr Phe Glu His Pro Gln Asp Val Pro Thr Ile
                 85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Phe Asp Leu Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Asn
        755

<210> 56
<211> 758
<212> PRT
<213> Artificial Sequence

<220>
<223> Description of Artificial Sequence:hybrid
      polymerase Hyb3 from Figure 11

<220>
<221> MOD_RES
<222> (472)
<223> Xaa = unknown amino acid

<400> 56
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Glu Asp Asp Ser Lys Ile
         35                  40                  45

Asp Glu Val Arg Lys Ile Thr Ala Glu Arg His Gly Lys Ile Val Arg
     50                  55                  60

Ile Val Asp Ala Glu Lys Val Glu Lys Lys Phe Leu Gly Arg Pro Ile
 65                  70                  75                  80

Thr Val Trp Lys Leu Tyr Phe Glu His Pro Gln Asp Val Pro Thr Ile
                 85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr

195                 200                 205

Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile
    450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu

565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Phe Asp Leu Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Asn
        755


<210> 57
<211> 845
<212> PRT
<213> Artificial Sequence

<220>
<223> Description of Artificial Sequence:hybrid
      polymerase HyS4 from Figure 11

<220>
<221> MOD_RES
<222> (472)
<223> Xaa = unknown amino acid


<400> 57
Met Ile Leu Asp Ala Asp Tyr Ile Thr Glu Glu Gly Lys Pro Val Ile
  1               5                  10                  15

Arg Ile Phe Lys Lys Glu Asn Gly Glu Phe Lys Val Glu Tyr Asp Arg
             20                  25                  30

Asn Phe Arg Pro Tyr Ile Tyr Ala Leu Leu Glu Asp Asp Ser Lys Ile
         35                  40                  45

Asp Glu Val Arg Lys Ile Thr Ala Glu Arg His Gly Lys Ile Val Arg
     50                  55                  60

Ile Val Asp Ala Glu Lys Val Glu Lys Lys Phe Leu Gly Arg Pro Ile
 65                  70                  75                  80

Thr Val Trp Lys Leu Tyr Phe Glu His Pro Gln Asp Val Pro Thr Ile
                85                  90                  95

Arg Glu Lys Ile Arg Glu His Ser Ala Val Val Gly Ile Phe Glu Tyr
            100                 105                 110

Asp Ile Pro Phe Ala Lys Ser Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asp Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Leu Gly Asp Met Thr Ala Val Glu Val Lys Gly Arg Ile
                245                 250                 255

His Phe Asp Leu Tyr His Val Ile Ser Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Gly Ala Trp Glu Thr Gly Glu Asp
    290                 295                 300

Leu Glu Arg Val Ala Lys Tyr Ser Met Glu Asp Ala Lys Ala Ile Tyr
305                 310                 315                 320

Glu Leu Gly Lys Glu Phe Phe Pro Met Glu Val Gln Leu Pro Arg Leu
                325                 330                 335

Val Gly Gln Pro Leu Trp Asp Val Ser Arg Ser Ser Thr Gly Asn Leu
            340                 345                 350

Val Glu Trp Leu Leu Leu Arg Lys Ala Tyr Glu Arg Asn Glu Leu Ala
        355                 360                 365

Pro Asn Lys Pro Ala Glu Gln Glu Tyr Glu Arg Arg Leu Arg Glu Ser
    370                 375                 380

Tyr Thr Gly Gly Phe Val Lys Glu Pro Glu Lys Gly Leu Trp Glu Asp
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Asp Tyr
            420                 425                 430

Asp Ile Ala Pro Glu Val Gly His Lys Phe Cys Lys Asp Phe Leu Gly
        435                 440                 445

Phe Ile Pro Ser Leu Leu Gly His Leu Leu Glu Glu Arg Gln Glu Ile

450                 455                 460

Lys Thr Lys Met Lys Glu Thr Xaa Asp Pro Ile Glu Lys Ile Leu Leu
465                 470                 475                 480

Asp Tyr Arg Gln Lys Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Glu Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Ala Ile Leu Lys His Gly Asn Val Glu Glu Ala
625                 630                 635                 640

Val Lys Ile Val Lys Glu Ile Ile Glu Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Lys Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Lys Arg Ala Ile Leu Ala Glu Glu
705                 710                 715                 720

Phe Asp Pro Lys Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Ala Gly Leu Thr Ala
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro

820                 825                 830

Lys Glu Leu Leu Gln Met Leu Glu Lys Gln Lys Lys Asn
        835                 840                 845


<210> 58
<211> 845
<212> PRT
<213> Artificial Sequence

<220>
<223> Description of Artificial Sequence:hybrid
      polymerase PhS1 from Figure 11

<400> 58
Met Ile Leu Asp Ala Asp Tyr Ile Thr Glu Glu Gly Lys Pro Val Ile
  1               5                  10                  15

Arg Leu Phe Lys Lys Glu Asn Gly Glu Phe Lys Ile Glu His Asp Arg
             20                  25                  30

Thr Phe Arg Pro Tyr Ile Tyr Ala Leu Leu Lys Asp Asp Ser Lys Ile
         35                  40                  45

Glu Glu Val Lys Lys Ile Thr Ala Glu Arg His Gly Lys Ile Val Arg
     50                  55                  60
Ile Val Asp Ala Glu Lys Val Glu Lys Lys Phe Leu Gly Arg Pro Ile
 65                  70                  75                  80

Thr Val Trp Arg Leu Tyr Phe Glu His Pro Gln Asp Val Pro Thr Ile
                 85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Glu Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Lys Ile Ile Arg Glu Lys Asp Pro Asp Ile Ile Ile Thr
        195                 200                 205

Tyr Asn Gly Asp Ser Phe Asp Leu Pro Tyr Leu Ala Lys Arg Ala Glu
    210                 215                 220

Lys Leu Gly Ile Lys Leu Thr Ile Gly Arg Asp Gly Ser Glu Pro Lys
225                 230                 235                 240

Met Gln Arg Ile Gly Asp Met Thr Ala Val Glu Val Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Lys Ala Trp Glu Thr Gly Glu Gly

290                 295                 300

Leu Glu Arg Val Ala Lys Tyr Ser Met Glu Asp Ala Lys Ala Thr Tyr
305                 310                 315                 320

Glu Leu Gly Lys Glu Phe Phe Pro Met Glu Ala Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Leu Ala
        355                 360                 365

Pro Asn Lys Pro Asp Glu Arg Glu Tyr Glu Arg Arg Leu Arg Glu Ser
    370                 375                 380

Tyr Ala Gly Gly Phe Val Lys Glu Pro Glu Lys Gly Leu Trp Glu Asn
385                 390                 395                 400

Ile Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Arg Asn Tyr
            420                 425                 430

Asp Val Ala Pro Glu Val Gly His Lys Phe Cys Lys Asp Phe Pro Gly
        435                 440                 445

Phe Ile Pro Ser Leu Leu Lys Arg Leu Leu Asp Glu Arg Gln Lys Ile
    450                 455                 460

Lys Thr Lys Met Lys Ala Ser Gln Asp Pro Ile Glu Lys Ile Met Leu
465                 470                 475                 480

Asp Tyr Arg Gln Arg Ala Ile Lys Ile Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Ser Glu Glu Ile Lys Lys Lys
545                 550                 555                 560

Ala Leu Glu Phe Val Asp Tyr Ile Asn Ala Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Lys Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala
625                 630                 635                 640

Val Arg Ile Val Lys Glu Val Thr Gln Lys Leu Ser Lys Tyr Glu Ile
                645                 650                 655

Pro Pro Glu Lys Leu Ala Ile Tyr Glu Gln Ile Thr Arg Pro Leu His

660                 665                 670

Glu Tyr Lys Ala Ile Gly Pro His Val Ala Val Ala Lys Arg Leu Ala
        675                 680                 685

Ala Lys Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Pro Arg Lys His Lys Tyr Asp Ala Glu Tyr Tyr Ile Glu Asn
                725                 730                 735

Gln Val Leu Pro Ala Val Leu Arg Ile Leu Glu Gly Phe Gly Tyr Arg
            740                 745                 750

Lys Glu Asp Leu Arg Trp Gln Lys Thr Lys Gln Thr Gly Leu Thr Ser
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys Asn
        835                 840                 845

<210> 59
<211> 845
<212> PRT
<213> Artificial Sequence

<220>
<223> Description of Artificial Sequence:hybrid
      polymerase PhS2 from Figure 11

<400> 59
Met Ile Leu Asp Val Asp Tyr Ile Thr Glu Glu Gly Lys Pro Val Ile
  1               5                  10                  15

Arg Leu Phe Lys Lys Glu Asn Gly Glu Phe Lys Val Glu Tyr Asp Arg
             20                  25                  30

Thr Phe Arg Pro Tyr Ile Tyr Ala Leu Leu Lys Asp Asp Ser Lys Ile
         35                  40                  45

Asp Glu Val Arg Lys Ile Thr Gly Glu Arg His Gly Lys Ile Val Arg
     50                  55                  60

Ile Ile Asp Ala Glu Lys Val Arg Lys Lys Phe Leu Gly Lys Pro Ile
 65                  70                  75                  80

Glu Val Trp Lys Leu Tyr Phe Glu His Pro Gln Asp Val Pro Thr Ile
                 85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Ile Leu Ala Phe Asp Ile Glu Thr
    130                 135                 140
Leu Tyr His Glu Gly Glu Glu Phe Gly Lys Gly Pro Ile Ile Met Ile
145                 150                 155                 160
Ser Tyr Ala Asp Glu Asn Glu Ala Lys Val Ile Thr Trp Lys Lys Ile
                165                 170                 175
Asp Leu Pro Tyr Val Glu Val Val Ser Ser Glu Arg Glu Met Ile Lys
            180                 185                 190
Arg Phe Leu Lys Val Ile Arg Glu Lys Asp Pro Asp Ile Ile Val Thr
        195                 200                 205
Tyr Asn Gly Asp Ser Phe Asp Phe Pro Tyr Leu Ala Lys Arg Ala Glu
    210                 215                 220
Lys Leu Gly Ile Lys Leu Pro Ile Gly Arg Asp Gly Ser Glu Pro Lys
225                 230                 235                 240
Met Gln Arg Ile Gly Asp Met Thr Ala Val Glu Val Lys Gly Arg Ile
                245                 250                 255
His Phe Asp Leu Tyr His Val Ile Arg Arg Thr Ile Asn Leu Pro Thr
            260                 265                 270
Tyr Thr Leu Glu Ala Val Tyr Glu Ala Ile Phe Gly Lys Pro Lys Glu
        275                 280                 285
Lys Val Tyr Ala His Glu Ile Ala Glu Ala Trp Glu Ser Gly Glu Gly
    290                 295                 300
Leu Glu Arg Val Ala Lys Tyr Ser Met Glu Asp Ala Lys Ala Thr Tyr
305                 310                 315                 320
Glu Leu Gly Lys Glu Phe Phe Pro Met Glu Ile Gln Leu Ser Arg Leu
                325                 330                 335
Val Gly Gln Pro Leu Trp Asp Val Ser Arg Ser Ser Thr Gly Asn Leu
            340                 345                 350
Val Glu Trp Phe Leu Leu Arg Lys Ala Tyr Glu Arg Asn Glu Leu Ala
        355                 360                 365
Pro Asn Lys Pro Ser Glu Arg Glu Tyr Glu Arg Arg Leu Arg Glu Ser
    370                 375                 380
Tyr Thr Gly Gly Tyr Val Lys Glu Pro Glu Lys Gly Leu Trp Glu Asn
385                 390                 395                 400
Ile Val Tyr Leu Asp Phe Arg Ser Leu Tyr Pro Ser Ile Ile Ile Thr
                405                 410                 415
His Asn Val Ser Pro Asp Thr Leu Asn Leu Glu Gly Cys Lys Glu Tyr
            420                 425                 430
Asp Val Ala Pro Glu Val Gly His Lys Phe Cys Lys Asp Ile Pro Gly
        435                 440                 445
Phe Ile Pro Ser Leu Leu Gly His Leu Leu Glu Glu Arg Gln Lys Ile
    450                 455                 460
Lys Arg Lys Met Lys Ala Ser Lys Asp Pro Ile Glu Lys Ile Leu Leu
465                 470                 475                 480
Asp Tyr Arg Gln Arg Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Leu Val Arg Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Ser Glu Glu Ile Lys Lys Lys
545                 550                 555                 560

Ala Leu Glu Phe Val Asp Tyr Ile Asn Ser Lys Leu Pro Gly Leu Leu
                565                 570                 575

Glu Leu Glu Tyr Glu Gly Phe Tyr Lys Arg Gly Phe Phe Val Thr Lys
            580                 585                 590

Lys Arg Tyr Ala Leu Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Lys Val Leu Glu Thr Ile Leu Lys His Gly Asn Val Glu Glu Ala
625                 630                 635                 640

Val Arg Ile Val Lys Glu Val Thr Gln Lys Leu Ala Lys Tyr Glu Ile
                645                 650                 655

Pro Pro Glu Lys Leu Ala Ile Tyr Glu Gln Ile Thr Pro Pro Leu His
            660                 665                 670

Glu Tyr Lys Ala Ile Gly Pro His Val Ala Val Ala Lys Arg Leu Ala
        675                 680                 685

Ala Arg Gly Val Lys Ile Lys Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Leu Lys Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg
            740                 745                 750

Lys Glu Asp Leu Arg Tyr Gln Lys Thr Lys Gln Val Asp Leu Thr Ala
        755                 760                 765

Cys Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys Asn
        835                 840                 845

<210> 60
<211> 145

<212> PRT
<213> Artificial Sequence

<220>
<223> Description of Artificial Sequence:hybrid
      polymerase PhS3 from Figure 11

<400> 60
Met Ile Leu Asp Ala Asp Tyr Ile Thr Glu Glu Gly Lys Pro Ile Ile
  1               5                  10                  15

Arg Leu Phe Lys Lys Glu Asn Gly Lys Phe Lys Val Glu Tyr Asp Arg
             20                  25                  30

Thr Phe Arg Pro Tyr Ile Tyr Ala Leu Leu Lys Asp Asp Ser Lys Ile
         35                  40                  45

Asp Glu Val Arg Lys Ile Thr Gly Glu Arg His Gly Lys Ile Val Arg
     50                  55                  60

Ile Val Asp Ala Glu Lys Val Glu Lys Lys Phe Leu Gly Lys Pro Ile
 65                  70                  75                  80

Glu Val Trp Lys Leu Tyr Leu Glu His Pro Gln Asp Val Pro Thr Ile
                 85                  90                  95

Arg Glu Lys Ile Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Val Arg Tyr Arg Asn Pro Leu Ser Arg Arg Arg Arg Val
    130                 135                 140

Trp
145


<210> 61
<211> 844
<212> PRT
<213> Artificial Sequence
<220>
<223> Description of Artificial Sequence:hybrid
      polymerase PhS4 from Figure 11

<400> 61
Met Ile Leu Asp Ala Asp Tyr Ile Thr Glu Glu Gly Lys Pro Val Ile
  1               5                  10                  15

Arg Leu Phe Lys Lys Glu Asn Gly Glu Phe Lys Val Glu Tyr Asp Arg
             20                  25                  30

Asn Phe Arg Pro Tyr Ile Tyr Ala Leu Leu Arg Asp Asp Ser Gln Ile
         35                  40                  45

Asp Glu Val Arg Lys Ile Thr Gly Glu Arg His Gly Lys Ile Val Arg
     50                  55                  60

Ile Val Asp Ala Glu Lys Val Glu Lys Lys Phe Leu Gly Arg Pro Ile
 65                  70                  75                  80

Glu Val Trp Lys Leu Tyr Leu Glu His Pro Gln Asp Val Pro Ala Ile
                 85                  90                  95

Arg Glu Lys Val Arg Glu His Ser Ala Val Val Asp Ile Phe Glu Tyr
            100                 105                 110

Asp Ile Pro Phe Ala Lys Arg Tyr Leu Ile Asp Lys Gly Leu Ile Pro

115                 120                 125

Met Glu Gly Glu Glu Glu Leu Lys Leu Leu Ala Phe Asp Ile Glu Thr
    130                 135                 140

Leu Tyr His Glu Gly Glu Glu Phe Ala Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Val Ile Val Thr
        195                 200                 205

Tyr Asn Gly Asp Ser Phe Asp Leu Pro Tyr Leu Val Lys Arg Ala Glu
    210                 215                 220

Lys Leu Gly Ile Lys Leu Pro Ile Gly Arg Asp Gly Ser Glu Pro Lys
225                 230                 235                 240

Met Gln Arg Ile Gly Asp Met Thr Ala Val Glu Ile Lys Gly Arg Ile
                245                 250                 255

His Phe Asp Leu Tyr His Val Ile Arg Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala His Glu Ile Ala Lys Ala Trp Glu Ser Gly Glu Gly
    290                 295                 300
Leu Glu Arg Val Ala Lys Tyr Ser Met Glu Asp Ala Lys Val Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Phe Leu Leu Arg Lys Ala Tyr Glu Arg Asn Glu Val Ala
        355                 360                 365

Pro Asn Lys Pro Ser Glu Glu Glu Tyr Glu Arg Arg Leu Arg Glu Ser
    370                 375                 380

Tyr Ala Gly Gly Tyr Val Lys Glu Pro Glu Lys Gly Leu Trp Glu Asn
385                 390                 395                 400

Ile Val Ser Leu Asp Phe Arg Ser Leu Tyr Pro Ser Ile Ile Ile Thr
                405                 410                 415

His Asn Val Ser Pro Asp Thr Leu Asn Arg Glu Gly Cys Lys Asn Tyr
            420                 425                 430

Asp Ile Ala Pro Gln Val Gly His Lys Phe Cys Lys Asp Ile Pro Gly
        435                 440                 445

Phe Ile Pro Ser Leu Leu Lys His Leu Leu Asp Glu Arg Gln Lys Ile
    450                 455                 460

Lys Arg Lys Met Lys Glu Ser Gln Asp Pro Ile Glu Lys Lys Met Leu
465                 470                 475                 480

Asp Tyr Arg Gln Arg Ala Ile Lys Leu Leu Ala Asn Ser Tyr Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Phe Val Arg Lys Glu
        515                 520                 525

Leu Glu Glu Lys Phe Gly Phe Lys Val Leu Tyr Ile Asp Thr Gly Leu
    530                 535                 540

Tyr Ala Thr Ile Pro Gly Ala Lys Ser Glu Glu Ile Lys Lys Lys Ala
545                 550                 555                 560

Leu Glu Phe Val Lys Tyr Ile Asn Ser Lys Leu Pro Gly Leu Leu Glu
                565                 570                 575

Leu Glu Tyr Glu Gly Phe Tyr Val Arg Gly Phe Phe Val Thr Lys Lys
            580                 585                 590

Arg Tyr Ala Leu Ile Asp Glu Glu Gly Lys Ile Ile Thr Arg Gly Leu
        595                 600                 605

Glu Ile Val Arg Arg Asp Trp Ser Glu Ile Ala Lys Glu Thr Gln Ala
    610                 615                 620

Arg Val Leu Glu Thr Ile Leu Lys His Gly Asn Val Glu Glu Ala Val
625                 630                 635                 640

Arg Ile Val Lys Glu Val Thr Lys Lys Leu Ser Asn Tyr Glu Ile Pro
                645                 650                 655

Pro Glu Lys Leu Ala Ile Tyr Glu Gln Ile Thr Arg Pro Leu His Glu
            660                 665                 670

Tyr Lys Ala Ile Gly Pro His Val Ala Val Ala Lys Arg Leu Ala Ala
        675                 680                 685

Lys Gly Val Lys Ile Arg Pro Gly Met Val Ile Gly Tyr Ile Val Leu
    690                 695                 700

Arg Gly Asp Gly Pro Ile Ser Asn Arg Ala Ile Leu Ala Glu Glu Tyr
705                 710                 715                 720

Asp Pro Lys Lys His Lys Tyr Asp Ala Glu Tyr Tyr Ile Glu Asn Gln
                725                 730                 735

Val Leu Pro Ala Val Leu Arg Ile Leu Glu Ala Phe Gly Tyr Arg Lys
            740                 745                 750

Glu Asp Leu Arg Trp Gln Lys Thr Lys Gln Val Gly Leu Thr Ala Trp
        755                 760                 765

Leu Asn Ile Lys Lys Ser Gly Thr Gly Gly Gly Gly Ala Thr Val Lys
    770                 775                 780

Phe Lys Tyr Lys Gly Glu Glu Lys Glu Val Asp Ile Ser Lys Ile Lys
785                 790                 795                 800

Lys Val Trp Arg Val Gly Lys Met Ile Ser Phe Thr Tyr Asp Glu Gly
                805                 810                 815

Gly Gly Lys Thr Gly Arg Gly Ala Val Ser Glu Lys Asp Ala Pro Lys
            820                 825                 830

Glu Leu Leu Gln Met Leu Glu Lys Gln Lys Lys Asn
        835                 840

<210> 62
<211> 845

<212> PRT
<213> Artificial Sequence

<220>
<223> Description of Artificial Sequence:hybrid
      polymerase PhS5 from Figure 11

<400> 62
Met Ile Leu Asp Ala Asp Tyr Ile Thr Glu Asp Gly Lys Pro Ile Ile
  1               5                  10                  15

Arg Leu Phe Lys Lys Glu Asn Gly Glu Phe Lys Val Glu Tyr Asp Arg
             20                  25                  30

Asn Phe Arg Pro Tyr Ile Tyr Ala Leu Leu Arg Asp Asp Ser Gln Ile
         35                  40                  45
Asp Glu Val Lys Lys Ile Thr Ala Glu Arg His Gly Lys Ile Val Arg
     50                  55                  60

Ile Ile Asp Ala Glu Lys Val Glu Lys Lys Phe Leu Gly Arg Pro Ile
 65                  70                  75                  80

Thr Val Trp Arg Leu Tyr Phe Glu His Pro Gln Asp Val Pro Ala Ile
                 85                  90                  95

Arg Asp Lys Val Arg Glu His Pro Ala Val Val Asp Ile Phe Glu Tyr
            100                 105                 110

Asp Ile Pro Phe Ala Lys Arg Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Ile Ile Ile Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Ile Gly Asp Met Thr Ala Val Glu Ile Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Glu Ala Trp Glu Ser Gly Lys Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Asp Glu Glu Glu Tyr Glu Arg Arg Leu Arg Glu Ser
    370                 375                 380

Tyr Thr Gly Gly Tyr Val Lys Glu Pro Glu Lys Gly Leu Trp Glu Asn
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Glu Tyr
            420                 425                 430

Asp Ile Ala Pro Gln Val Gly His Lys Phe Cys Lys Asp Phe Pro Gly
        435                 440                 445

Phe Ile Pro Ser Leu Leu Lys His Leu Leu Asp Glu Arg Gln Glu Ile
    450                 455                 460

Lys Arg Lys Met Lys Ala Ser Lys Asp Pro Ile Glu Lys Lys Met Leu
465                 470                 475                 480

Asp Tyr Arg Gln Arg Ala Ile Lys Leu Leu Ala Asn Ser Phe Tyr Gly
                485                 490                 495

Tyr Tyr Gly Tyr Ala Lys Ala Arg Trp Tyr Cys Lys Glu Cys Ala Glu
            500                 505                 510

Ser Val Thr Ala Trp Gly Arg Glu Tyr Ile Glu Leu Val Trp Lys Glu
        515                 520                 525

Leu Glu Glu Lys Phe Gly Phe Lys Val Leu Tyr Ile Asp Thr Asp Gly
    530                 535                 540

Leu Tyr Ala Thr Ile Pro Gly Gly Lys Pro Glu Glu Ile Lys Lys Lys
545                 550                 555                 560

Ala Leu Glu Phe Val Lys Tyr Ile Asn Ser Lys Leu Pro Gly Leu Leu
                565                 570                 575

Glu Leu Glu Tyr Glu Gly Phe Tyr Val Arg Gly Phe Phe Val Thr Lys
            580                 585                 590

Lys Arg Tyr Ala Val Ile Asp Glu Glu Gly Lys Ile Ile Thr Arg Gly
        595                 600                 605

Leu Glu Ile Val Arg Arg Asp Trp Ser Glu Ile Ala Lys Glu Thr Gln
    610                 615                 620

Ala Arg Val Leu Glu Ala Ile Leu Lys His Gly Asn Val Glu Glu Ala
625                 630                 635                 640

Val Lys Ile Val Lys Glu Val Thr Gln Lys Leu Ala Lys Tyr Glu Ile
                645                 650                 655

Pro Pro Glu Lys Leu Ala Ile Tyr Glu Gln Ile Thr Arg Pro Leu His
            660                 665                 670

Glu Tyr Lys Ala Ile Gly Pro His Val Ala Val Ala Lys Arg Leu Ala
        675                 680                 685

Ala Arg Gly Val Lys Val Arg Pro Gly Met Val Ile Gly Tyr Ile Val
    690                 695                 700

Leu Arg Gly Asp Gly Pro Ile Ser Asn Arg Ala Ile Leu Ala Glu Glu
705                 710                 715                 720

Tyr Asp Leu Lys Lys His Lys Tyr Asp Ala Glu Tyr Tyr Ile Glu Asn
                725                 730                 735

Gln Val Leu Pro Ala Val Leu Arg Ile Leu Glu Ala Phe Gly Tyr Arg
            740                 745                 750

Lys Glu Asp Leu Arg Trp Gln Lys Thr Lys Gln Val Gly Leu Thr Ser
        755                 760                 765

Trp Leu Asn Ile Lys Lys Ser Gly Thr Gly Gly Gly Gly Ala Thr Val
    770                 775                 780

Lys Phe Lys Tyr Lys Gly Glu Glu Lys Glu Val Asp Ile Ser Lys Ile
785                 790                 795                 800

Lys Lys Val Trp Arg Val Gly Lys Met Ile Ser Phe Thr Tyr Asp Glu
                805                 810                 815

Gly Gly Gly Lys Thr Gly Arg Gly Ala Val Ser Glu Lys Asp Ala Pro
            820                 825                 830

Lys Glu Leu Leu Gln Met Leu Glu Lys Gln Lys Lys Asn
        835                 840                 845


<210> 63
<211> 472
<212> PRT
<213> Artificial Sequence

<220>
<223> Description of Artificial Sequence:hybrid
      polymerase PhS6 from Figure 11

<400> 63
Met Ile Leu Asp Ala Asp Tyr Ile Thr Glu Asp Gly Lys Pro Ile Ile
  1               5                  10                  15

Arg Leu Phe Lys Lys Glu Asn Gly Glu Phe Lys Val Glu Tyr Asp Arg
             20                  25                  30

Asn Phe Arg Pro Tyr Ile Tyr Ala Leu Leu Arg Asp Asp Ser Gln Ile
         35                  40                  45

Asp Glu Val Lys Lys Ile Thr Ala Glu Arg His Gly Lys Ile Val Arg
     50                  55                  60

Ile Ile Asp Ala Glu Lys Val Glu Lys Lys Phe Leu Gly Arg Pro Ile
 65                  70                  75                  80

Thr Val Trp Arg Leu Tyr Phe Glu His Pro Gln Asp Val Pro Ala Ile
                 85                  90                  95

Arg Asp Lys Val Arg Glu His Pro Ala Val Val Asp Ile Phe Glu Tyr
            100                 105                 110
Asp Ile Pro Phe Ala Lys Arg Tyr Leu Ile Asp Lys Gly Leu Ile Pro
        115                 120                 125

Met Glu Gly Glu Glu Glu Leu Lys Leu Leu Ala Phe Asp Ile Glu Thr
    130                 135                 140

Leu Tyr His Glu Gly Glu Glu Phe Gly Lys Gly Pro Ile Ile Met Ile
145                 150                 155                 160

Ser Tyr Ala Asp Glu Asn Glu Ala Lys Val Ile Thr Trp Lys Lys Ile
                165                 170                 175

Asp Leu Pro Tyr Val Glu Val Val Ser Ser Glu Arg Glu Met Ile Lys
            180                 185                 190

Arg Phe Leu Arg Val Ile Arg Glu Lys Asp Pro Asp Ile Ile Ile Thr
        195                 200                 205

Tyr Asn Gly Asp Ser Phe Asp Phe Pro Tyr Leu Ala Lys Arg Ala Glu
    210                 215                 220

Lys Leu Gly Ile Lys Leu Pro Leu Gly Arg Asp Gly Ser Glu Pro Lys
225                 230                 235                 240

Met Gln Arg Ile Gly Asp Met Thr Ala Val Glu Ile Lys Gly Arg Ile
                245                 250                 255

His Phe Asp Leu Tyr His Val Ile Thr Arg Thr Ile Asn Leu Pro Thr
            260                 265                 270

Tyr Thr Leu Glu Ala Val Tyr Glu Ala Ile Phe Gly Lys Pro Lys Glu
        275                 280                 285

Lys Val Tyr Ala Asp Glu Ile Ala Glu Ala Trp Glu Ser Gly Lys Asn
    290                 295                 300

Leu Glu Arg Val Ala Lys Tyr Ser Met Glu Asp Ala Lys Ala Thr Tyr
305                 310                 315                 320

Glu Leu Gly Lys Glu Phe Leu Pro Met Glu Ile Gln Leu Ser Arg Leu
                325                 330                 335

Val Gly Gln Pro Leu Trp Asp Val Ser Arg Ser Ser Thr Gly Asn Leu
            340                 345                 350

Val Glu Trp Tyr Leu Leu Arg Lys Ala Tyr Glu Arg Asn Glu Val Ala
        355                 360                 365

Pro Asn Lys Pro Asp Glu Glu Glu Tyr Glu Arg Arg Leu Arg Glu Ser
    370                 375                 380

Tyr Thr Gly Gly Tyr Val Lys Glu Pro Glu Lys Gly Leu Trp Glu Asn
385                 390                 395                 400

Leu Val Ser Leu Asp Phe Arg Ala Leu Tyr Pro Ser Ile Ile Ile Thr
                405                 410                 415

His Asn Val Ser Pro Asp Thr Leu Asn Arg Glu Gly Cys Lys Glu Tyr
            420                 425                 430

Asp Val Ala Pro Gln Val Gly His Lys Phe Cys Lys Asp Phe Pro Gly
        435                 440                 445

Phe Ile Pro Ser Leu Leu Lys Arg Leu Leu Glu Glu Arg Gln Lys Ile
    450                 455                 460

Lys Arg Lys Met Lys Ala Thr Asn
465                 470

<210> 64
<211> 60
<212> PRT
<213> Artificial Sequence

<220>
<223> Description of Artificial Sequence:region
      containing invariable sequence element from
      parental Pfu polymerase containing nucleotide
      binding motif

<400> 64
Asp Tyr Arg Gln Lys Ala Ile Lys Leu Leu Ala Asn Ser Phe Tyr Gly
1               5                   10                  15

Tyr Tyr Gly Tyr Ala Lys Ala Arg Trp Tyr Cys Lys Glu Cys Ala Glu
            20                  25                  30

Ser Val Thr Ala Trp Gly Arg Lys Tyr Ile Glu Leu Val Trp Lys Glu
        35                  40                  45

Leu Glu Glu Lys Phe Gly Phe Lys Val Leu Tyr Ile
    50                  55                  60


<210> 65
<211> 60
<212> PRT
<213> Artificial Sequence

<220>
<223> Description of Artificial Sequence:region
      containing invariable sequence element from
      parental Deep Vent polymerase containing
      nucleotide binding motif

<400> 65
Asp Tyr Arg Gln Arg Ala Ile Lys Ile Leu Ala Asn Ser Tyr Tyr Gly
1               5                   10                  15

Tyr Tyr Gly Tyr Ala Lys Ala Arg Trp Tyr Cys Lys Glu Cys Ala Glu
            20                  25                  30

Ser Val Thr Ala Trp Gly Arg Glu Tyr Ile Glu Phe Val Arg Lys Glu
        35                  40                  45

Leu Glu Glu Lys Phe Gly Phe Lys Val Leu Tyr Ile
    50                  55                  60


<210> 66
<211> 60
<212> PRT
<213> Artificial Sequence

<220>
<223> Description of Artificial Sequence:region
      containing invariable sequence element from
      dedigned hybrid polymerase containing nucleotide
      binding motif

<220>
<221> MOD_RES
<222> (1)..(60)
<223> Xaa = unknown amino acid

<400> 66
Asp Tyr Arg Gln Xaa Ala Ile Lys Xaa Leu Ala Asn Ser Xaa Tyr Gly
1               5                   10                  15

Tyr Tyr Gly Tyr Ala Lys Ala Arg Trp Tyr Cys Lys Glu Cys Ala Glu
            20                  25                  30

Ser Val Thr Ala Trp Gly Arg Xaa Tyr Ile Glu Xaa Val Xaa Lys Glu
        35                  40                  45

Leu Glu Glu Lys Phe Gly Phe Lys Val Leu Tyr Ile
    50                  55                  60

<210> 67
<211> 60
<212> PRT
<213> Artificial Sequence

<220>
<223> Description of Artificial Sequence:region
      containing invariable sequence elements from HyS1,
      Hyb2, Hyb3 and HyS4 hybrid polymerase containing
      nucleotide binding motif

<400> 67
Asp Tyr Arg Gln Lys Ala Ile Lys Leu Leu Ala Asn Ser Tyr Tyr Gly
  1               5                  10                  15

Tyr Tyr Gly Tyr Ala Lys Ala Arg Trp Tyr Cys Lys Glu Cys Ala Glu
             20                  25                  30

Ser Val Thr Ala Trp Gly Arg Glu Tyr Ile Glu Phe Val Trp Lys Glu
         35                  40                  45

Leu Glu Glu Lys Phe Gly Phe Lys Val Leu Tyr Ile
     50                  55                  60


<210> 68
<211> 60
<212> PRT
<213> Artificial Sequence


<220>
<223> Description of Artificial Sequence:region
      containing invariable sequence element from PhS1
      hybrid polymerase containing nucleotide binding
      motif

<400> 68
Asp Tyr Arg Gln Arg Ala Ile Lys Ile Leu Ala Asn Ser Tyr Tyr Gly
  1               5                  10                  15

Tyr Tyr Gly Tyr Ala Lys Ala Arg Trp Tyr Cys Lys Glu Cys Ala Glu
             20                  25                  30

Ser Val Thr Ala Trp Gly Arg Glu Tyr Ile Glu Phe Val Trp Lys Glu
         35                  40                  45

Leu Glu Glu Lys Phe Gly Phe Lys Val Leu Tyr Ile
     50                  55                  60


<210> 69
<211> 60
<212> PRT
<213> Artificial Sequence

<220>
<223> Description of Artificial Sequence:region
      containing invariable sequence element from PhS2
      polymerase containing nucleotide binding motif

<400> 69
Asp Tyr Arg Gln Arg Ala Ile Lys Leu Leu Ala Asn Ser Phe Tyr Gly
  1               5                  10                  15

Tyr Tyr Gly Tyr Ala Lys Ala Arg Trp Tyr Cys Lys Glu Cys Ala Glu
             20                  25                  30

Ser Val Thr Ala Trp Gly Arg Glu Tyr Ile Glu Leu Val Arg Lys Glu

35                  40                  45

Leu Glu Glu Lys Phe Gly Phe Lys Val Leu Tyr Ile
    50                  55                  60


<210> 70
<211> 60
<212> PRT
<213> Artificial Sequence

<220>
<223> Description of Artificial Sequence:region
      containing invariable sequence element from PhS4
      hybrid polymerase containing nucleotide binding
      motif

<400> 70
Asp Tyr Arg Gln Arg Ala Ile Lys Leu Leu Ala Asn Ser Tyr Tyr Gly
  1               5                  10                  15

Tyr Tyr Gly Tyr Ala Lys Ala Arg Trp Tyr Cys Lys Glu Cys Ala Glu
             20                  25                  30
Ser Val Thr Ala Trp Gly Arg Glu Tyr Ile Glu Phe Val Arg Lys Glu
         35                  40                  45

Leu Glu Glu Lys Phe Gly Phe Lys Val Leu Tyr Ile
    50                  55                  60


<210> 71
<211> 60
<212> PRT
<213> Artificial Sequence

<220>
<223> Description of Artificial Sequence:region
      containing invariable sequence element from PhS5
      hybrid polymerase containing nucleotide binding
      motif

<400> 71
Asp Tyr Arg Gln Arg Ala Ile Lys Leu Leu Ala Asn Ser Phe Tyr Gly
  1               5                  10                  15

Tyr Tyr Gly Tyr Ala Lys Ala Arg Trp Tyr Cys Lys Glu Cys Ala Glu
             20                  25                  30

Ser Val Thr Ala Trp Gly Arg Glu Tyr Ile Glu Leu Val Trp Lys Glu
         35                  40                  45

Leu Glu Glu Lys Phe Gly Phe Lys Val Leu Tyr Ile
    50                  55                  60


<210> 72
<211> 60
<212> PRT
<213> Artificial Sequence

<220>
<223> Description of Artificial Sequence:region
      containing invariable sequence element from PhS7
      polymerase containing nucleotide binding motif

<400> 72
Asp Tyr Arg Gln Lys Ala Ile Lys Ile Leu Ala Asn Ser Phe Tyr Gly
  1               5                  10                  15

Tyr Tyr Gly Tyr Ala Lys Ala Arg Trp Tyr Cys Lys Glu Cys Ala Glu

20                   25                   30

Ser Val Thr Ala Trp Gly Arg Lys Tyr Ile Glu Phe Val Arg Lys Glu
        35                  40                  45

Leu Glu Glu Lys Phe Gly Phe Lys Val Leu Tyr Ile
    50                  55                  60


<210> 73
<211> 6
<212> PRT
<213> Artificial Sequence

<220>
<223> Description of Artificial Sequence:6-His
      polyhistidine epitope tag, metal chelate affinity
      ligand

<400> 73
His His His His His His
  1               5


<210> 74
<211> 8
<212> PRT
<213> Artificial Sequence

<220>
<223> Description of Artificial Sequence:anti-DYKDDDDK
      epitope tag

<400> 74
Asp Tyr Lys Asp Asp Asp Asp Lys
  1               5


<210> 75
<211> 21
<212> DNA
<213> Artificial Sequence

<220>
<223> Description of Artificial Sequence:Fwd 1 PCR
      primer oligonucleotide

<400> 75
ttggtaccaa gcttcatatg a                                           21


<210> 76
<211> 51
<212> DNA
<213> Artificial Sequence

<220>
<223> Description of Artificial Sequence:Fwd 2 PCR
      primer oligonucleotide

<400> 76
ccgctgccga sctatgcgac cyctcacagc kcaggcctgg atctgcgtgc g          51


<210> 77
<211> 69
<212> DNA
<213> Artificial Sequence

<220>

<223> Description of Artificial Sequence:Fwd 3 PCR
      primer oligonucleotide

<400> 77
ttccgaccgg tctgatcmtt sawattscgg awggttmtgm ggsgcagrtg ckgccgcgta 60
gcggcctgg                                                          69

<210> 78
<211> 98
<212> DNA
<213> Artificial Sequence

<220>
<223> Description of Artificial Sequence:Fwd 4 PCR
      primer oligonucleotide

<400> 78
ttttgatcga trscgattat crgggcsaas tgawgrttak cstggtgaac ckgggcmasg 60
awgaakttry gattsagcsg ggcgaacgta ttgcgcag                          98

<210> 79
<211> 56
<212> DNA
<213> Artificial Sequence

<220>
<223> Description of Artificial Sequence:Fwd 5 PCR
      primer oligonucleotide

<400> 79
cgtggcgaag gcggctttgg ctctascggc asamagtaat gaggatccga attctt      56

<210> 80
<211> 89
<212> DNA
<213> Artificial Sequence

<220>
<223> Description of Artificial Sequence:Rev A PCR
      primer oligonucleotide

<400> 80
ggtcgcatag stcggcagcg gwaawtcttk gscatgasgc rgacgcwkaa ttttcasawy 60
aaytttkytc atatgaagct tggtaccaa                                    89

<210> 81
<211> 83
<212> DNA
<213> Artificial Sequence

<220>
<223> Description of Artificial Sequence:Rev B PCR
      primer oligonucleotide

<400> 81
gatcagaccg gtcggaayca gcrycstwtc ammcggckya akttycascg swtystyaak 60
gsmcgcacgc agatccaggc ctg                                          83

<210> 82
<211> 68
<212> DNA
<213> Artificial Sequence

<220>
<223> Description of Artificial Sequence:Rev C PCR
      primer oligonucleotide

<400> 82
ttttatcgat crkgcccrsc gcgttcagca scrytawgcc mtktttccag sccaggccgc 60
tacgcggc                                                          68


<210> 83
<211> 107
<212> DNA
<213> Artificial Sequence

<220>
<223> Description of Artificial Sequence:Rev D PCR
      primer oligonucleotide

<400> 83
tagagccaaa gccgccttcg ccacgmtsgg tctgagaaam wtcttccacc wsawyaamtt 60
ccrccygcws caccggcrca awaaycakct gcgcaatacg ttcgccc               107


<210> 84
<211> 22
<212> DNA
<213> Artificial Sequence

<220>
<223> Description of Artificial Sequence:Rev D PCR
      primer oligonucleotide

<400> 84
aagaattcgg atcctcatta ct                                          22


<210> 85
<211> 45
<212> DNA
<213> Artificial Sequence

<220>
<223> Description of Artificial Sequence:primer to
      measure exonuclease activity

<220>
<221> modified_base
<222> (1)
<223> n = t modified by 6-carboxy-fluorescein (FAM)

<220>
<221> modified_base
<222> (45)
<223> n = t amino-linked to quencher
      4-(4-dimethylaminophenylazo)benzoyl group (dabcyl,
      DAB)
<400> 85
ntttttgagg tgtgtcctac acagcggagt gtaggacaca cctcn                 45

## Description

### CROSS-REFERENCES TO RELATED APPLICATIONS

This application claims the benefit of U.S. Provisional Application No. 60/398,687, filed July 25, 2002; U.S. Provisional Application No. 60/463,781, filed April 17, 2003; and U.S. Provisional Application 60/483,287, filed June 27, 2003, each of which applications is herein incorporated by reference.

### FIELD OF THE INVENTION

This invention relates to methods for the facilitated evolution of proteins and novel polypeptides obtained by using the methods.

### BACKGROUND OF THE INVENTION

This invention provides methods of creating hybrid proteins to identify proteins with enhanced activity. A number of methods of generating hybrid sequences to enhance protein function are known *(see, e.g.,* U.S. Patent No. 6,132,970). However, these methods rely on recombinational techniques that shuffle the sequences to create new proteins. There is a need for additional techniques to facilitate identification of proteins with an enhanced function. This invention addresses that need and further, provides polypeptides, *e.g*., polymerases, that are obtained using the methodt

Polymerases catalyze the formation of biological polymers. Polymerases are useful for the synthesis of DNA from deoxyribonucleoside triphosphates in the presence of a nucleic acid template and a nucleic acid primer; the synthesis of RNA from ribonucleotides and a DNA or RNA template; DNA replication and repair; and *in vitro* DNA or RNA amplification.

The 3' to 5' exonuclease activity, commonly referred to as "proofreading" activity, is an important characteristic of some DNA polymerases and is present in *Pyrococcus* species family B polymerases such as *Pyrococcus furiosus* PolI (referred to herein as "Pfu" and described in US Patent 5,948,663; commercially available from Stratagene, San Diego, CA) and *Pyrococcus* strain GB-D PolI (referred to herein as "Deep Vent®" and described US Patent 5,834,285; commercially available from New England Biolabs, Beverly MA). The essential function of the 3' to 5' exonuclease is to recognize and cleave a non-base-paired terminus. Enzymes with high exonuclease activity, however, are not commonly used in reactions relying on polymerase activity because they have poor processivity. For example, if used in PCR, it is often in combination with *Thermus aquaticus* DNA PolI, (Taq), an enzyme with higher processivity but no 3' to 5' exonuclease activity, in order to improve the fidelity of the PCR reaction. Improved processivity in polymerases with high 3' to 5' exonuclease activity would greatly increase the reliability of reactions relying on the use of polymerases and would eliminate, in some cases, the need for Taq polymerase. Accordingly, a need exists for creating improved polymerases with 3' to 5' exonuclease activity.

This invention addresses this and other needs by providing novel polymerase compositions.

### BRIEF SUMMARY OF THE INVENTION

The invention provides a method of generating polypeptides with enhanced function. The method comprises creating hybrid proteins having a common biological activity comprising the steps of: (a) creating a library of 32 or more nucleic acids encoding a plurality of hybrid protein members, wherein the members differ from a set of at least two starting proteins with corresponding amino acids, and i ) where the starting proteins are homologous proteins having greater than 60% amino acids pair-wise similarity to each other and having at least one common biological activity, ii.) where a majority of the encoding library members have a greater than 60% amino acid similarity to any of the starting proteins, and iii.) where the majority of differences between the encoded library members and the starting proteins are confined to those corresponding amino acids that differ among the starting proteins; (b) expressing protein from at least one library member to create at least one hybrid protein; and (c) selecting at least one protein having a common biological activity of the starting proteins.

In another aspect, the invention provides a library of nucleic acids encoding a plurality of hybrid protein members, wherein the members differ from a set of at least two starting proteins with corresponding amino acids, and i) where the starting proteins are homologous proteins having greater than 60% amino acid sequence similarity to each other and having at least one common biological activity, ii) where a majority of the library members have a greater than 60% amino acid similarity to any of the parent proteins, and iii) where the majority of differences between the library members and the starting proteins are confined to those corresponding amino acids that differ among the starting proteins. In some embodiments, the parent proteins are enzymes, *e.g*., polymerases, biosynthetic and catabolic enzymes. Parent enzymes can also be isozymes. Parent proteins can also be non-enzymatic proteins, *e.g*., proteins that bind to another molecule, with or without allosteric effect, such as hormones, receptors, antibodies and the like. Often, the parent protein have greater than 80% amino acid similarity to each other and the majority of the library members have greater than 80% amino acid similarity to any of the starting proteins.

In another aspect, the invention provides a synthetic hybrid protein comprising greater than 60% amino acid similarity to each member of a set of at least two starting proteins, where each starting protein in the set shares greater than 60% amino acid similarity and at least one common biological activity with each member of the set, and wherein the hybrid protein: (a) shares at least one biological activity with all members of the set; (b) has a minimum of 5 amino acid residue differences from any member of the set; and (c) comprises no more than 24% of amino acids not corresponding to any member of the set.

In some embodiments, the starting parent proteins are enzymes, *e.g*. polymerases. The parents may also be isozymes.

Often, the parent proteins have greater than 80% similarity to each other and the majority of the library members have greater than 80% similarity to any of the parent proteins. hybrid protein.

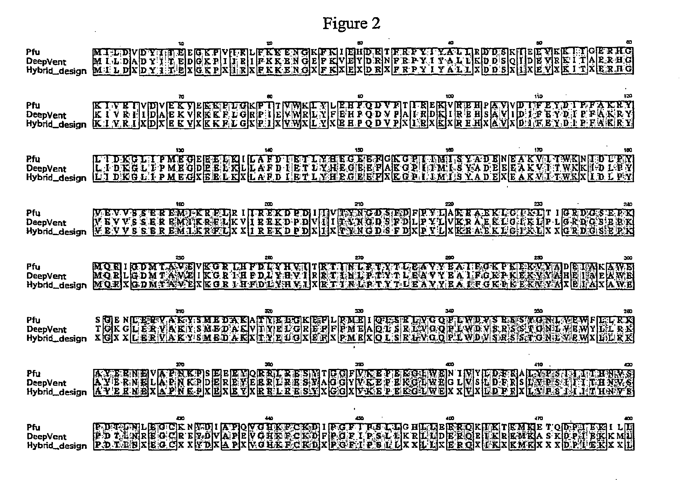

In some embodiments, wherein the set of parent proteins comprises the *Pyrococcus furiosus* family B DNA polymerase (Pfu) and Deep Vent® DNA Polymerase and the differences from any member of the set comprise at least 10 of the mismatches selected from the group shown in Figure 2.

The invention relates to generating hybrid polypeptides that comprise alterations in less conserved regions of parent proteins and, surprisingly, provides hybrid proteins that have improvement in desired properties relative to parent proteins. A protein of the invention can be designated as a variable residue altered hybrid protein (VRAHP), as described broadly. More specifically, a VRAHP contains alterations at non-conserved positions of the parent proteins, *i.e.,* variable residues, where the altered residue is an amino acid that occurs at that position in one of the parent proteins. Such alterations are typically present throughout the protein, for example occurring in at least 1 of every 30 or 50 amino acid residues, rather than concentrated in one region of the protein.

Typically, according to the invention, each parent protein in a set of at least two parent proteins shares greater than 60% amino acid similarity and at least one common biological activity with each member of the set. A typical hybrid protein according to the present invention will comprise greater than 60% amino acid similarity to each member of a set of at least two parent proteins, and will share at least one biological activity with all members of the set.

Furthermore, the aforementioned set of parent proteins necessarily comprises a subset of invariant amino acids that are identical among all members of the set. A typical hybrid protein according to the present invention comprises at least 95% of the subset of invariant amino acids.

Finally, the set of parent proteins necessarily comprises a subset of variable amino acids that differ among at least some members of the set. A typical hybrid protein according to the present invention will comprise a minimum of 5 amino acid residue differences from any member of the set, corresponding to members of the subset of variable amino acids. A typical hybrid protein according to the present invention will also comprise a subset of at least 5 amino acid residues from among the subset of variable amino acids, where each of the at least 5 amino acids is identical to a corresponding amino acid in at least one of the members in the parental set, and each of the subset of at least 5 amino acids, in order from N-terminus to C-terminus, is identical to a corresponding amino acid from a different one of the set of parent proteins from the previous member of the subset of at least 5 variable amino acid residues. In other words, the typical hybrid protein contains at least 5 variable amino acid residues corresponding to alternating parent proteins.

In another aspect, the invention provides hybrid polymerase polypeptides having residues from multiple parent polymerases. The invention also provides nucleic acids encoding such proteins. Thus, in one aspect, the invention provides a hybrid polymerase having polymerase activity, wherein the polymerase comprises SEQ ID NO:23 and is at least 80% identical over 700 contiguous amino acids of the *Pyrococcus furiosus* (*Pfu*) sequence set forth in SEQ ID NO: 24 or at least 80% identical over 700 contiguous amino acids of the Deep Vent® sequence set forth in SEQ ID NO:25, with the proviso that (a) when the polymerase is at least 85% identical to SEQ ID NO:24, the sequence comprises at least one hybrid position that is mutated from the native *Pfu* residue to the residue that occurs at the corresponding position of SEQ ID NO:25, wherein the hybrid position is one of the residues designated as "X" in SEQ ID NO:26; or (b) when the polymerase is at least 85% identical to SEQ ID NO:25, the sequence comprises at least one hybrid position that is mutated from the native Deep Vent® residue to the residue that occurs at the corresponding position of SEQ ID NO:24, wherein the hybrid position is one of the residues designated as "X" in SEQ ID NO:26. In some embodiments, the polymerase is at least 90% identical over 700 contiguous amino acids of the Pfu sequence set forth in SEQ ID NO:24 or at least 90% identical over 700 contiguous amino acids of the Deep Vent® sequence set forth in SEQ ID NO:25.

In some embodiments, the hybrid polymerase comprises at least ten hybrid positions, typically twenty hybrid positions, thirty hybrid positions, forty hybrid positions, or fifty or more hybrid positions, that are mutated from the native reside of SEQ ID NO:24 or SEQ ID NO:25 to the corresponding residue of SEQ ID NO:25 or SEQ ID NO:24, respectively.

In other embodiments, the hybrid polymerase comprises an amino acid sequence of SEQ ID NO:2, SEQ ID NO:12, SEQ ID NO:16, or SEQ ID NO:18; or the polymerase region of SEQ ID NO:4, SEQ ID NO:6, SEQ ID NO:8, SEQ ID NO:10, SEQ ID NO:14, or SEQ ID NO:20

The invention also includes embodiments in which the hybrid polymerase further comprises a DNA binding domain, often Sso7d, Sac7d, and Sac7e. Often, the DNA binding domain is conjugated to the polymerase. In some embodiments, the polymerase DNA binding domain conjugate comprises an amino acid sequence of SEQ ID NO:4, SEQ ID NO:6, SEQ ID NO:8, SEQ ID NO:10, SEQ ID NO:14, or SEQ ID NO:20.

The invention also provides isolated nucleic acids encoding the hybrid polymerases, and conjugates comprising the hybrid polymerase linked to a DNA binding domain; and expression vectors and host cells comprising the nucleic acids.

In another aspect, the invention provides an isolated nucleic acid encoding a polypeptide comprising an amino acid sequence at least 94% identical to SEQ ID NO:2, wherein the polypeptide exhibits polymerase activity. In typical embodiments, the polypeptide comprises SEQ ID NO:2. In some embodiments, the isolated nucleic acid comprises SEQ ID NO:1.

The invention also provides embodiments, wherein the polypeptide encoded by the nucleic acid further comprises a DNA binding domain, which is often selected from the group consisting of Sso7d, Sac7d, and Sac7e. The nucleic acid can encode a polypeptide comprising SEQ ID NO:4. In one embodiment, the nucleic acid comprises SEQ ID NO:3.

In other aspects, the invention provides expression vectors and host cells comprising the nucleic acids.

In another aspect, the invention provides an isolated polypeptide comprising an amino acid sequence at least 94% identical to SEQ ID NO:2, wherein the polypeptide has polymerase activity. In one embodiment, the polypeptide comprises SEQ ID NO:2.

In some embodiments, the polypeptide further comprises a DNA-binding domain, *e.g*., Sso7d, Sac7d, or Sac7e. The DNA-binding domain can be fused to the carboxy-terminus of the polypeptide. In one embodiment, the polypeptide comprises SEQ ID NO:4.

In another aspect, the invention provides an isolated nucleic acid encoding a polypeptide comprising an amino acid sequence at least 94% identical to SEQ ID NO:12, SEQ ID NO:16, or SEQ ID NO:18; or the polymerase region of SEQ ID NO:6, SEQ ID NO:8, SEQ ID NO:10, SEQ ID NO:14, or SEQ ID NO:20, wherein the polypeptide exhibits polymerase activity. In typical embodiments, the polypeptide comprises SEQ ID NO:12, SEQ ID NO:16, or SEQ ID NO:18; or the polymerase region of SEQ ID NO:6, SEQ ID NO:8, SEQ ID NO:10, SEQ ID NO:14, or SEQ ID NO:20. In some embodiments, the isolated nucleic acid comprises SEQ ID NO:11, SEQ ID NO:15, or SEQ ID NO:17; or the polymerase region of SEQ ID NO:5, SEQ m NO:7, SEQ ID NO:9, SEQ ID NO:13, or SEQ ID NO:19.

The invention also provides embodiments, wherein the polypeptide encoded by the nucleic acid further comprises a DNA binding domain, which is often selected from the group consisting of Sso7d, Sac7d, and Sac7e. The nucleic acid can encode a polypeptide comprising SEQ ID NO:6, SEQ ID NO:8, SEQ ID NO:10, SEQ ID NO:14, or SEQ ID NO:20. In one embodiment, the nucleic acid comprises SEQ ID NO:5, SEQ ID NO:7, SEQ ID NO:9, SEQ ID NO:13, or SEQ ID NO:1.

In other aspects, the invention provides expression vectors and host cells comprising the nucleic acids.

In another aspect, the invention provides an isolated polypeptide comprising an amino acid sequence at least 94% identical to SEQ ID NO:12, SEQ ID NO:16, or SEQ ID NO:18; or the polymerase region of SEQ ID NO:6, SEQ ID NO:8, SEQ ID NO:10, SEQ ID NO:14, or SEQ ID NO:20, wherein the polypeptide has polymerase activity. In one embodiment, the polypeptide comprises SEQ ID NO:12, SEQ ID NO:16, or SEQ ID NO:18; or the polymerase region of SEQ ID NO:6, SEQ ID NO:8, SEQ ID NO:10, SEQ ID NO:14, or SEQ ID NO:20.

In some embodiments, the further comprising a DNA binding domain, *e.g*., Sso7d, Sac7d, or Sac7e. The DNA binding domain can be fused to the carboxy-terminus of the polypeptide. In one embodiment, the polypeptide comprises SEQ ID NO:6, SEQ ID NO:8, SEQ ID NO:10, SEQ ID NO:14, or SEQ ID NO:20.

### BRIEF DESCRIPTION OF THE DRAWINGS

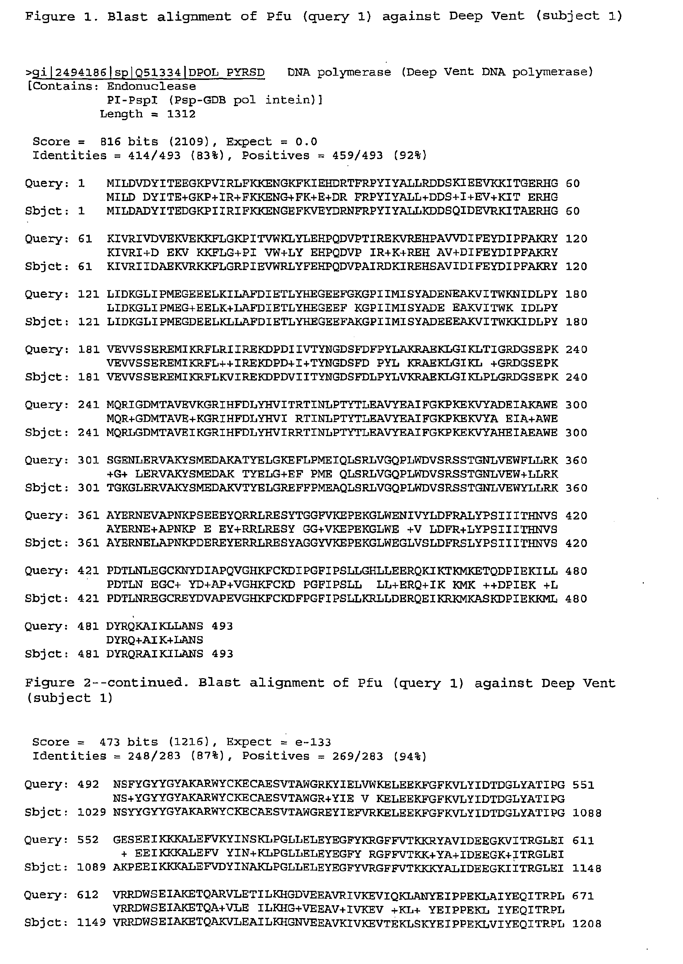

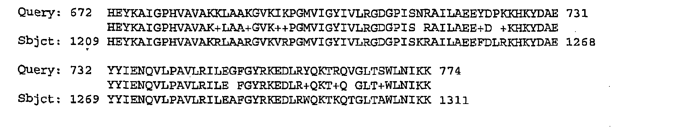

Figure 1 shows a BlastP alignment of *Pyrococcus furiosus* polymerase (Pfu) (query 1) against *Pyrococcus sp* GB-D polymerase (Deep Vent®) (subject 1).

Figure 2 shows an alignment of the parent Pfu and Deep Vent® polymerase sequences. The hybrid protein design polymerase sequence shows the positions that vary, between the two parent sequences, which are designated by an X. "Corresponding residues" in the sequences are those residues that occur in the same position as shown in the alignment.

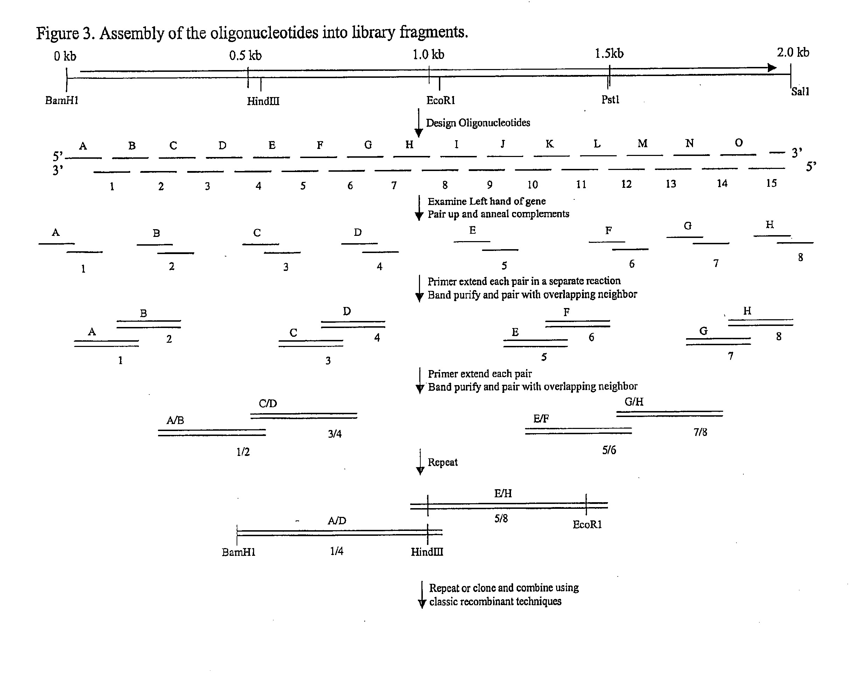

Figure 3 shows an example of assembly PCR. In this example, 100 base pair degenerate oligonucleotides are subj ected to rounds of annealing and primer extension until fragments of approximately 500 base pairs are obtained. These fragment libraries are sufficiently large in size to be easily manipulated and assembled into full length clones or libraries of full length clones by conventional molecular cloning techniques.

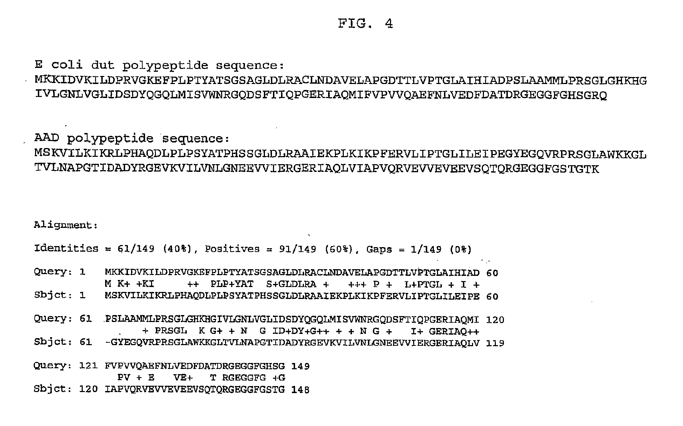

Figure 4 shows the sequences of the parental Dut proteins and the BLASTP alignment of the parent sequences.

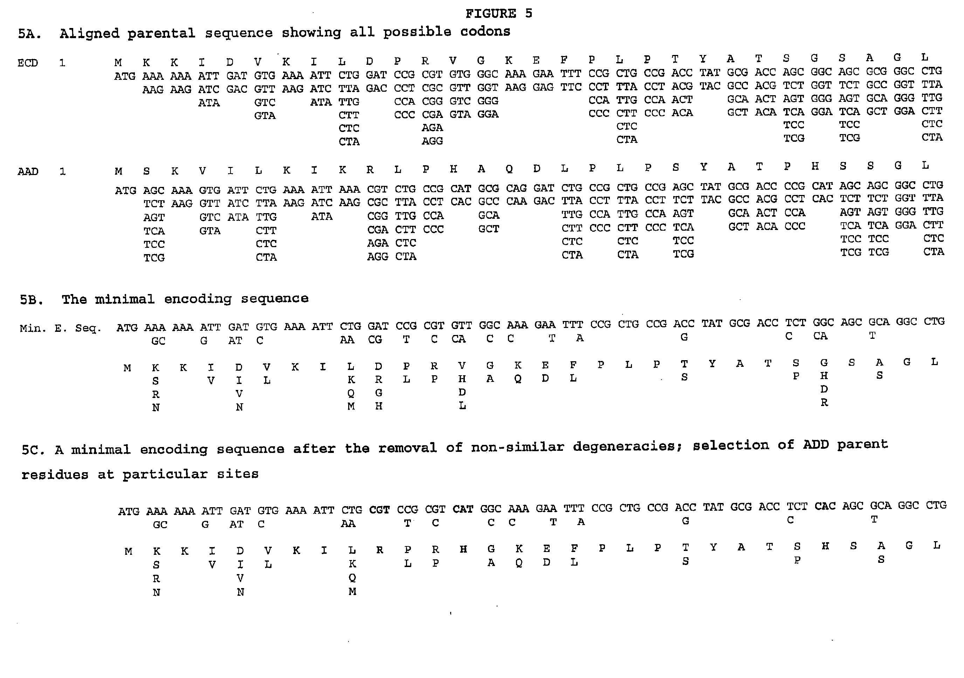

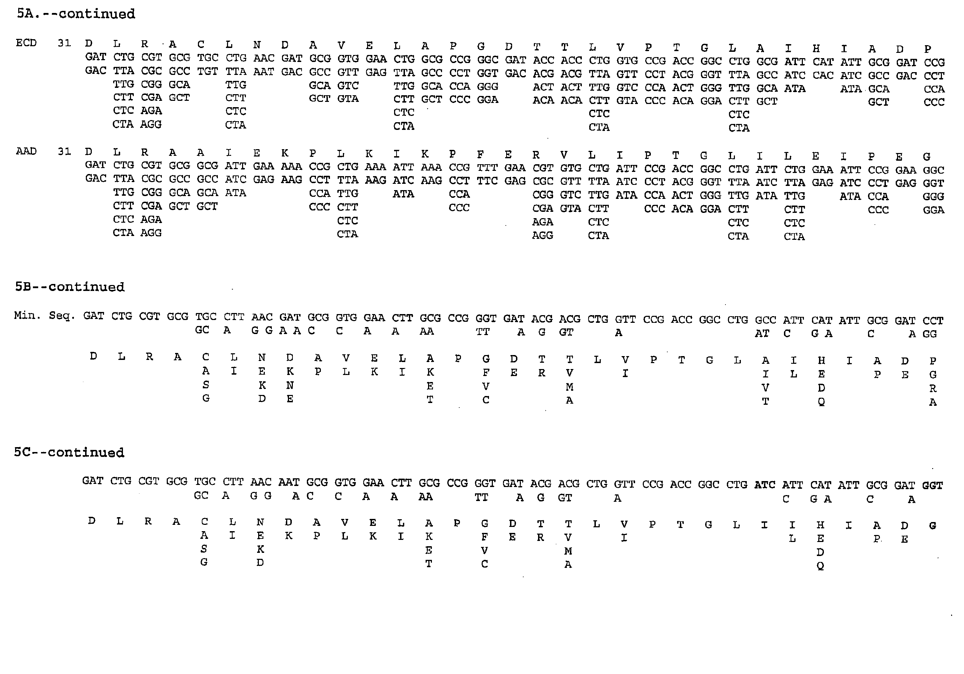

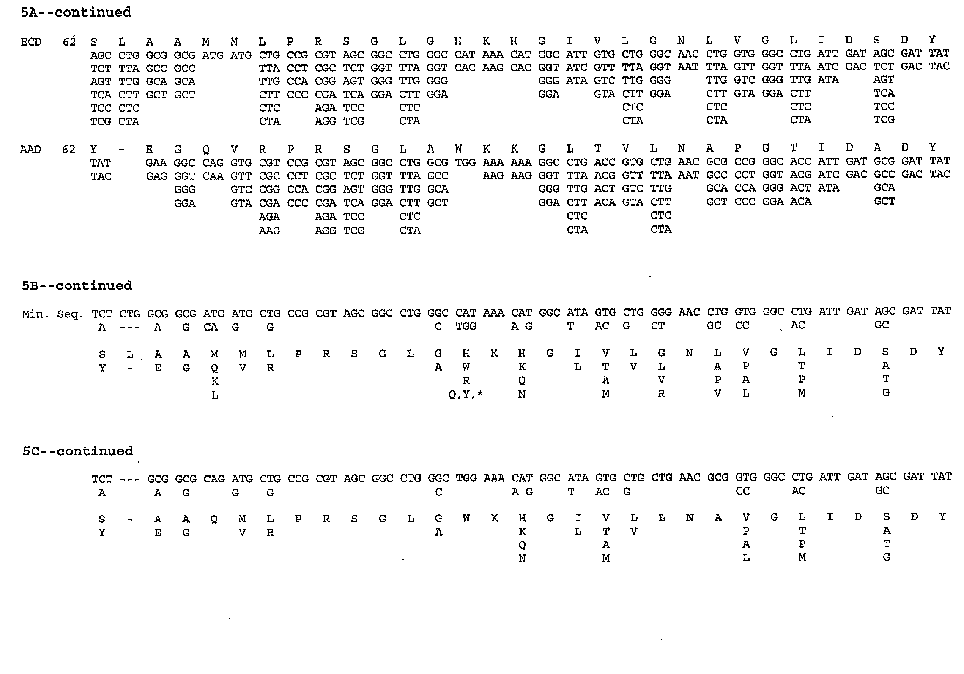

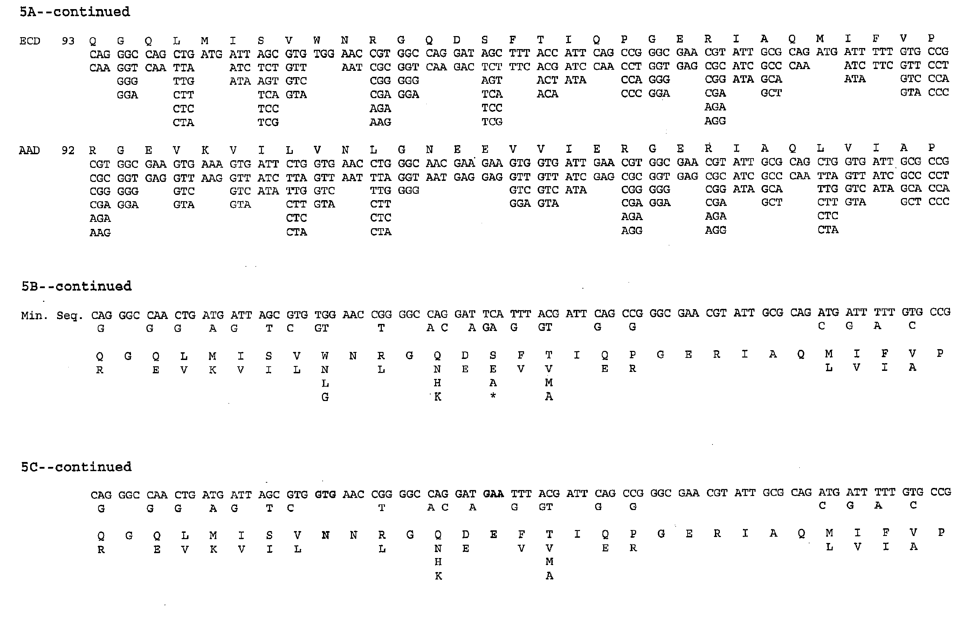

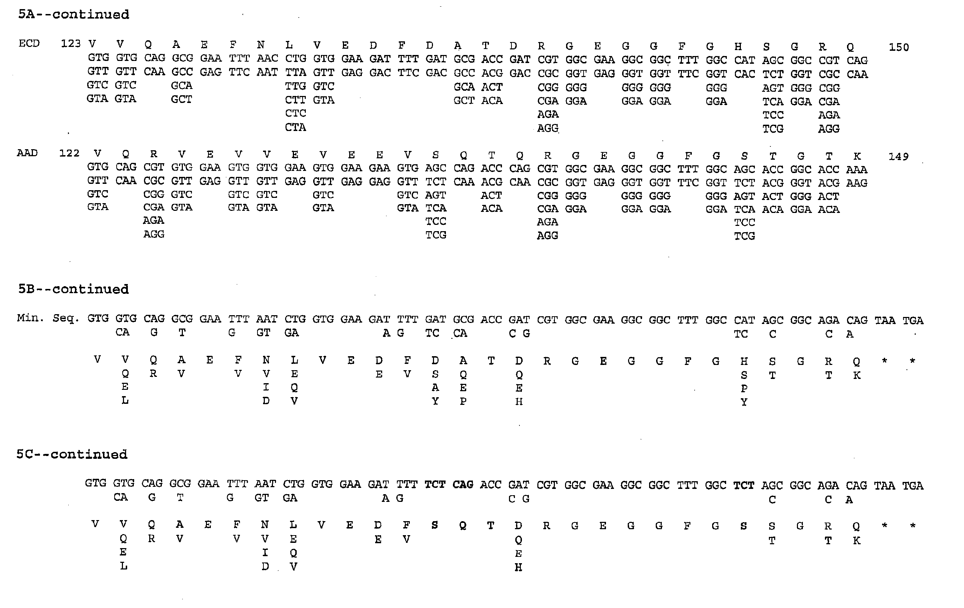

Figure 5 shows the degeneracies at the positions that differ in the parental Dut proteins. 5A.: Aligned parental sequence showing all possible codons in order of frequency of use by *E. coli.* 5B: Consensus sequence is derived by finding codons that will encode both sequences with a minimal number of degeneracies. Codons frequently used by *E. coli* are preferred. 5C: Nucleic acid degeneracies that incorporate amino acid sequences not similar (BLOSUM 62 number is <0) to either parental amino acid sequence are removed; in this example the nucleic acid encoding the thermal stable protein sequence, AAD is used instead. These are indicated in bold. The sequence of the thermal stable enzyme is also used in deciding to retain the one gap and eliminate the 2 cases where terminating codons could be incorporated in the sequence.

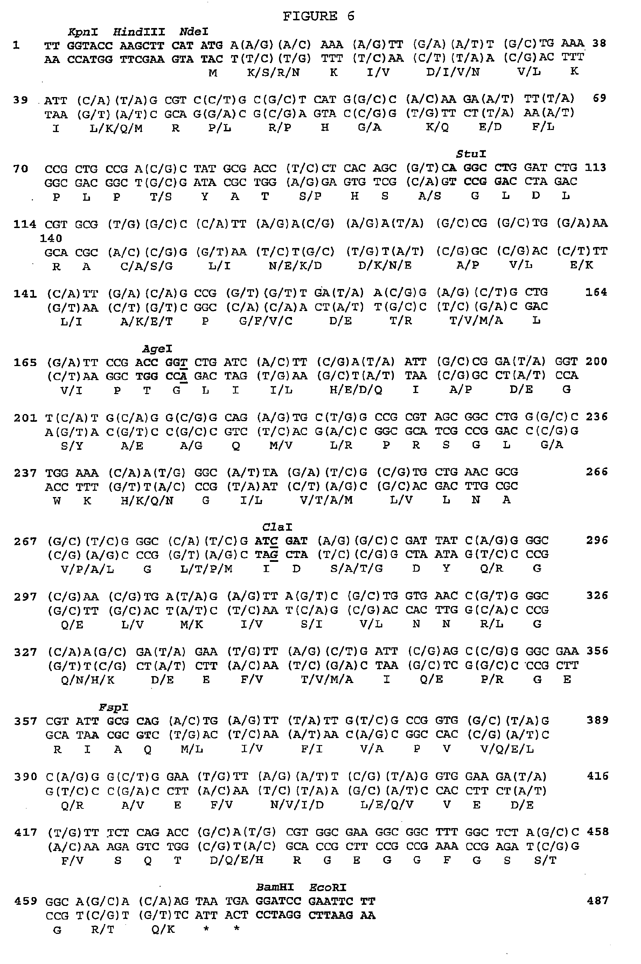

Figure 6 shows the priming and restriction sites (bold) that were added to the ends of the sequence. In two cases, codon usage was changed to add restriction sites (underlined and in italics). The amino acids encoded by the sequence are indicated below the codons.

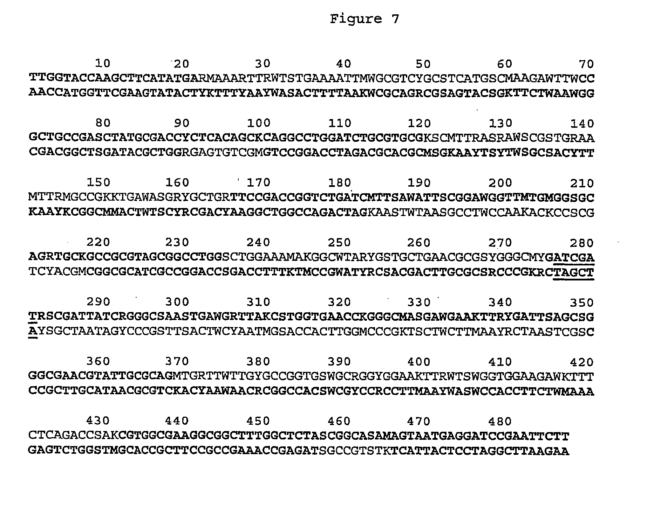

Figure 7 shows the minimal encoding oligonucleotide sequence to be synthesized to assemble the Dut hybrid library. The DNA sequence was converted to single letter nucleotide code using standard designations and oligonucleotide sequences were selected (below in bold). Selections were made such that minimal degeneracies exist where primers were expected to anneal to each other during assembly. In one stretch of sequence there was no region where reasonably sized annealable oligonucleotide sequences could be selected. In this example, the ClaI site (underlined) inserted in the previous step is used to assemble a full-length protein-encoding library from 2 restriction fragments.

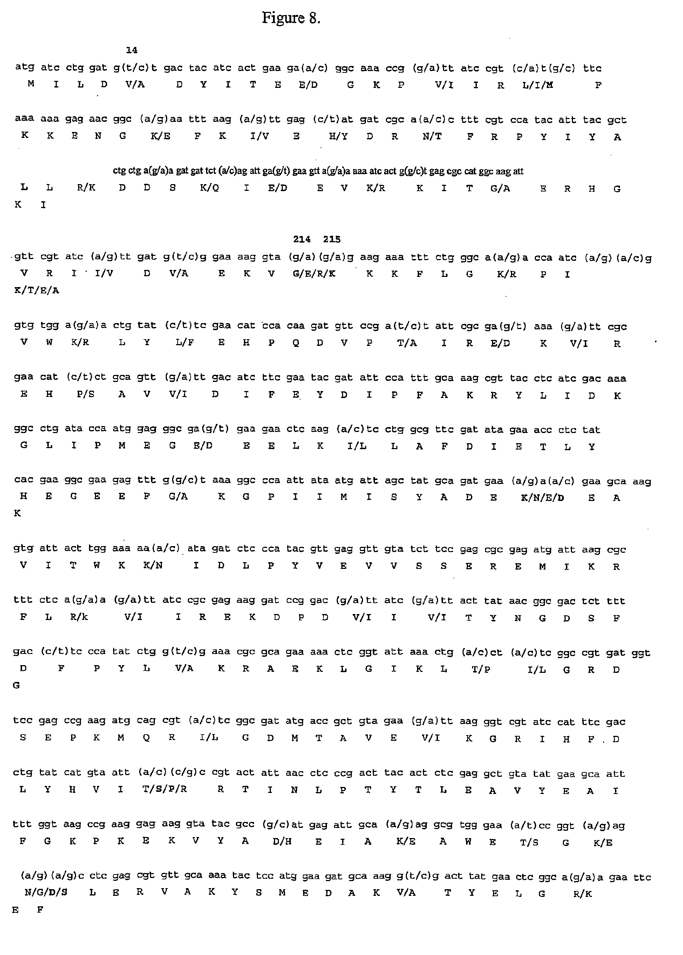

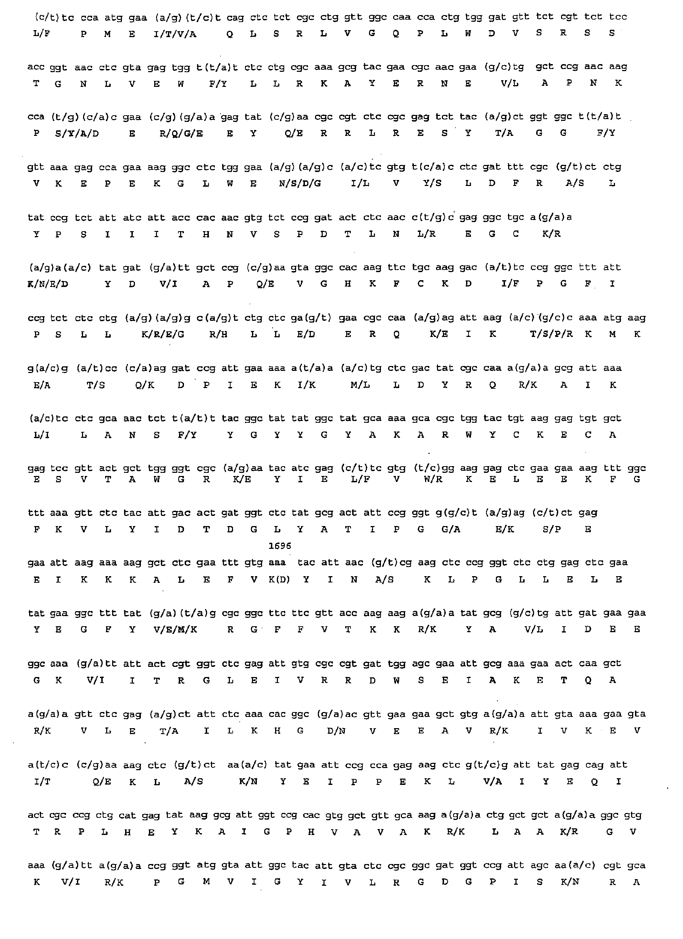

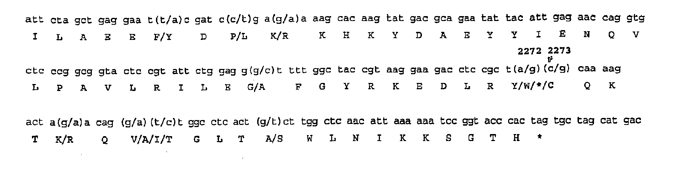

Figure 8 shows the minimal encoding sequence used to generate oligonucleotides encoding a Pfu/ Deep Vent® Hybrid DNA polymerase as explained in example 2. The degenerate nucleotides are in parenthesis. The amino acid sequences that differ between the parent proteins (the "mismatches") are indicated. Non-parental amino acids are indicated in bold. Examples mentioned in the text are numbered.

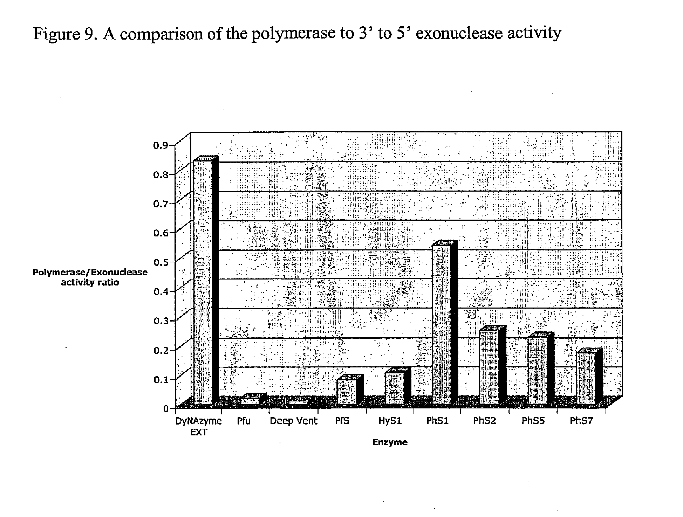

Figure 9 shows a comparison of the polymerase to 3' exonuclease ratios for several commercially available enzymes, including the parental proteins, and isolates from the hybrid library.

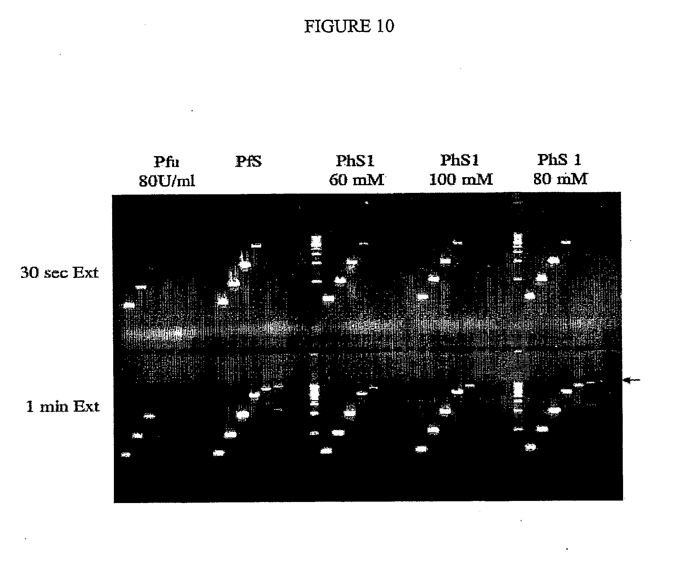

Figure 10 shows the results of a comparison of hybrid and parent polymerases. The enzymes were tested for the ability to amplify bacteriophage lambda DNA amplicons of a range of sizes, given a 30 sec or 1 min extension time. The sizes of the amplicons, in kilobases, are listed across the bottom of the lanes. Twenty units of enzyme per ml were used unless otherwise specified.

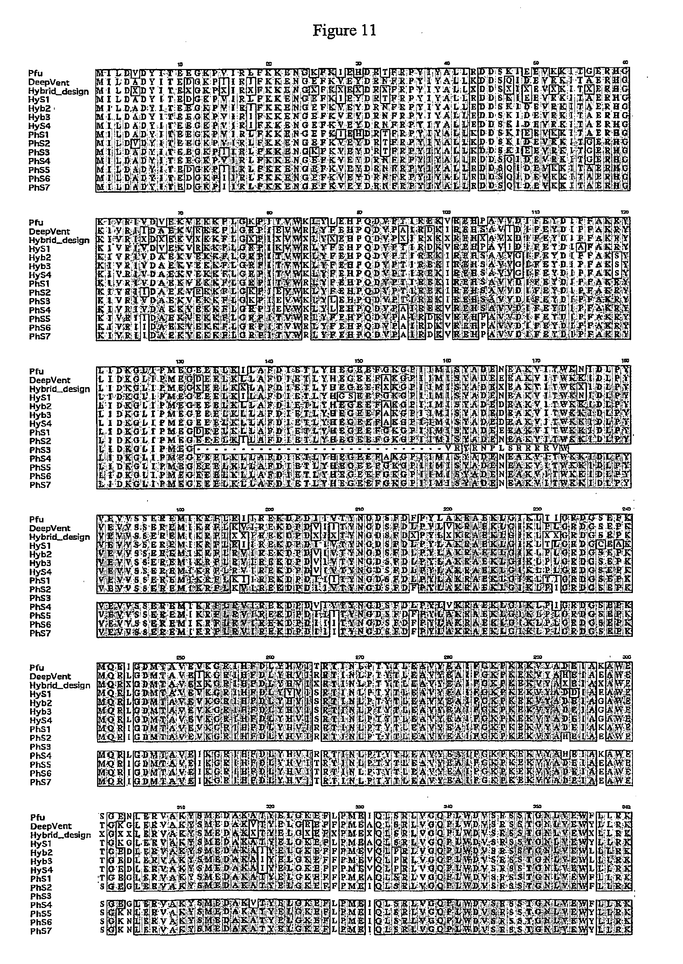

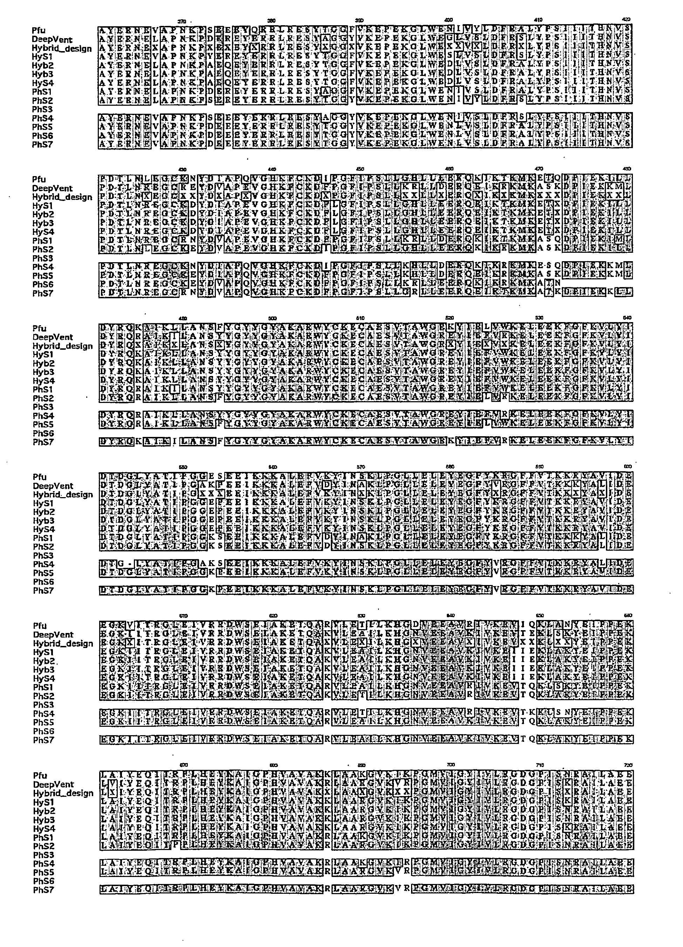

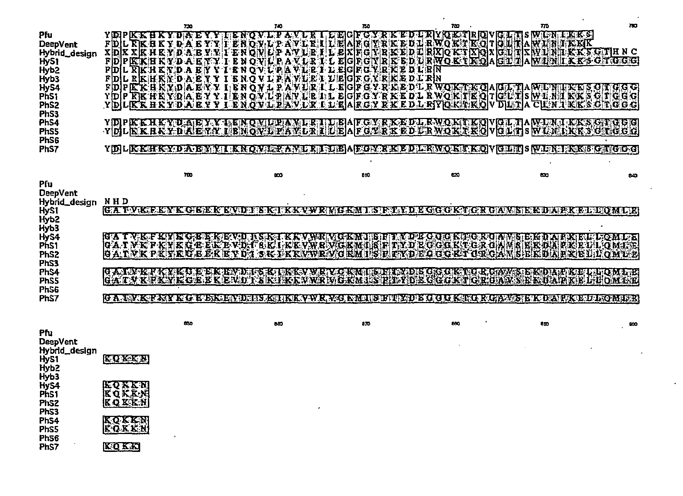

Figure 11 shows a comparison of the sequences of parent and hybrid polymerase proteins.

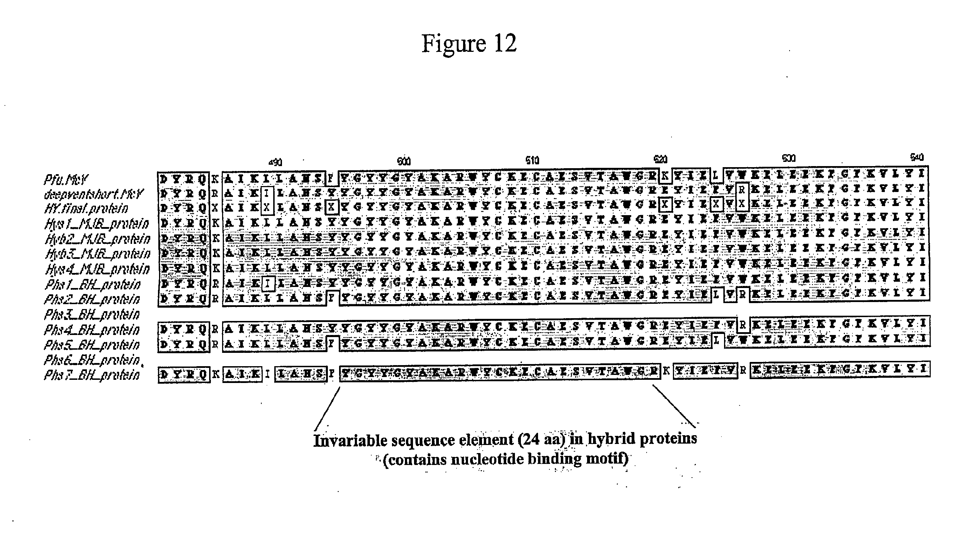

Figure 12 shows a sequence element that is common to the parental and hybrid sequences.

### DETAILED DESCRIPTION OF THE INVENTION

### A. General Overview

This invention provides methods of creating hybrid proteins having a desired phenotype. In general it is often desirable to create new proteins with functions that are similar to, but altered from, the functions of known existing proteins, *e.g*., it may be desirable to create proteins with enhanced stability, enhanced or decreased enzymatic activity towards particular substrates, enhanced or decreased affinity for particular ligands, etc. For example, a DNA polymerase enzyme may have both polymerase and exonuclease activities, and it may be useful to create new enzymes with different ratios of those two activities. This invention provides methods of producing large numbers of proteins that can be screened for desirable properties.

With the sequencing of the human, mouse, and many invertebrate and microbial genomes essentially complete, a wide variety of gene and deduced protein sequences are available. The invention therefore also provides a method of using the raw sequence information about protein variation as a source for generating useful variant proteins.

In particular, the invention provides methods of synthesizing a nucleic acid library that encodes hybrids of two or more parent proteins and selecting for hybrid proteins having a desired phenotype or activity. Typically, the library will comprise 32 or more hybrid proteins. The invention also provides a library of hybrid nucleic acids encoding a plurality of hybrid proteins and synthetic hybrid proteins comprising greater than 60% amino acid similarity, often greater than 60% identity, to each member of a set of at least two parent proteins.

The practice of this invention involves the construction of recombinant proteins and their expression in host cells. Molecular cloning techniques to achieve these ends are known in the art. A wide variety of cloning and *in vitro* amplification methods suitable for the construction of recombinant nucleic acids such as expression vectors are well-known to persons of skill. General texts which describe molecular biological techniques, useful herein, include Sambrook & Russell, Molecular Cloning, A Laboratory Manual (3rd Ed, 2001) ("Sanibrook"); Kriegler, Gene Transfer and Expression: A Laboratory Manual (1990); and Current Protocols in Molecular Biology, Ausubel et al., eds., 1994-1999, John Wiley & Sons, Inc ("Ausubel").

Parental sequences for generating hybrid proteins for a protein of interest may be identified by various amino acid sequence comparison methods. Using these techniques, one of skill can identify conserved regions in the nucleic acids encoding the proteins of the invention to prepare appropriate oligonucleotides that can be used to generate the hybrid proteins.

Oligonucleotides can be custom-made and ordered from a variety of commercial sources known in the art. Those that are not commercially available can be chemically synthesized using a variety of chemistries, *e.g*., the solid phase phosphoramidite triester method first described by Beaucage & Caruthers, Tetrahedron Letts. 22:1859-1862 (1981), using an automated synthesizer, as described in Van Devanter et. al., Nucleic Acids Res. 12:6159-6168 (1984). Purification of oligonucleotides is performed using known methods, *e.g*., by native acrylamide gel electrophoresis or by anion-exchange HPLC as described in Pearson & Reanier, J. Chrom. 255:137-149 (1983).

The nucleic acids encoding the hybrid proteins or segments of the hybrid proteins can be amplified from nucleic acid samples, *e.g*., oligonucleotide segments, using various amplification/extension techniques. For instance, polymerase chain reaction (PCR) technology may be used to obtain nucleic acid sequences that code for hybrid proteins to be expressed, to make nucleic acids to use as probes for detecting the presence of the desired nucleic acid sequences in samples, for nucleic acid sequencing, or for other purposes. For a general overview of PCR see PCR Protocols: A Guide to Methods and Applications. (Innis, M, Gelfand, D., Sninsky, J. and White, T., eds.), Academic Press, San Diego (1990).

Nucleic acid sequences encoding the hybrid proteins of the invention can be cloned into expression vectors to generate a library of sequences encoding individual hybrid proteins.

The following discussion provides details on how to select and align the parent starting proteins, how to create the library of nucleic acids encoding hybrid polypeptides derived from the parent proteins, and how to evaluate proteins obtained from the library.

The present invention also provides hybrid polymerase polypeptide and nucleic cid sequences that were generated using the methods described herein. In some embodiments, the polypeptides further comprise a DNA binding domain, *e.g*., an Archaeal small basic protein, such as an Sso7d, Sac7d, or Sac7e DNA binding domain, which is fused to the polypeptide. The DNA binding domain typically increases the binding affinity of the enzyme to nucleic acid and can enhance the processivity of the polymerases.

Polymerases of the invention includes polymerases identical or substantially identical to the polymerase sequences disclosed in SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, or SEQ ID NO:20. Such polymerases often exhibit altered activity compared to that of wild type polymerase Pfu or Deep Vent® polymerase.

### B. Definitions

The term "hybrid protein" is used herein to describe a polypeptide that comprises amino acid residues from multiple parent polypeptide sequences.

The term "hybrid position" refers to a position that differs between parent polypeptide sequences, or subsequences.

The term "amplification" refers to a process whereby the number of copies of a nucleic acid fragment is increased.

A "parent sequence" indicates a starting or reference amino acid or nucleic acid sequence prior to a manipulation of the invention. The term is used interchangeably with "starting sequence". Parent sequences may be wild-type proteins, hybrid proteins, proteins containing mutations, or other engineered proteins. Parent sequences can be full-length proteins, protein subunits, protein domains, amino acid motifs, protein active sites, or any polypeptide sequence or subset of polypeptide sequences, whether continuous or interrupted by other polypeptide sequences.

The term "wild-type" refers to a polynucleotide or polypeptide sequence that does not comprise mutations. A "wild-type" protein refers to a protein active at a level of activity found in nature and that typically comprises the amino acid sequence found in nature.

A "native" polypeptide sequence refers to a parent polypeptide sequence, typically a "wildtype" sequence.

The term "mutations" refers to changes in the sequence of a wild-type nucleic acid sequence or changes in the sequences of a peptide. Such mutations may be point mutations such as transitions or transversions, or deletions, insertions, or duplications.

The term "naturally occurring" as used herein refers to a nucleic acid or polypeptide that can be found in nature. For example, a polypeptide or polynucleotide sequence that is present in an organism that can be isolated from a source in nature and which has not been intentionally modified in a laboratory is naturally-occurring.

A "common biological" activity refers to an activity that is shared by two or more proteins wherein the common biological activity is an activity that is found in nature. Biological activity of a protein can be assessed using standard means known in the art for determining the function of a protein.

The term "DNA binding domain" refers to a protein domain which binds with significant affinity to DNA, for which there is no known nucleic acid which binds to the protein domain with more than 100-fold more affinity than another nucleic acid with the same nucleotide composition but a different nucleotide sequence.

The term "Sso7d" or "Sso7d DNA binding domain" or "Sso7d-like DNA binding domain" or "Sso7d binding protein" refers to nucleic acid and polypeptide polymorphic variants, alleles, mutants, and interspecies homologs that: (1) have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 15, 25, 35, 50, or more amino acids, to an Sso7d sequence of SEQ ID NO:22; (2) bind to antibodies, *e.g*., polyclonal antibodies, raised against an immunogen comprising an amino acid sequence of SEQ ID NO:22 and conservatively modified variants thereof; (3) specifically hybridize under stringent hybridization conditions to a Sso7d nucleic acid sequence of SEQ ID NO:21 and conservatively modified variants thereof; or (4) have a nucleic acid sequence that has greater than about 90%, preferably greater than about 96%, 97%, 98%, 99%, or higher nucleotide sequence identity, preferably over a region of at least about 50, 100, 150, or more nucleotides, to SEQ ID NO:21. The term includes both full-length Sso7d polypeptides and fragments of the polypeptides that have sequence non-specific double-stranded binding activity. Sso7d-like proteins include Sac7d and Sac7e.

"Domain" refers to a unit of a protein or protein complex; comprising a polypeptide subsequence, a complete polypeptide sequence, or a plurality of polypeptide sequences where that unit has a defined function. The function is understood to be broadly defined and can be ligand binding, catalytic activity or can have a stabilizing effect on the structure of the protein.

An "Sso7d polymerase conjugate" refers to a modified polymerase comprising at least one Sso7D DNA binding domain joined to a polymerase domain, or a catalytic subunit of the polymerase domain.

"Enhances" in the context of an enzyme refers to improving the activity of the enzyme, *i.e*., increasing the amount of product per unit enzyme per unit time.

"Fused" refers to linkage by covalent bonding.

"Heterologous", when used with reference to portions of a protein, indicates that the protein comprises two or more domains that are not found in the same relationship to each other in nature. Such a protein, *e.g*., a fusion protein, contains two or more domains from unrelated proteins arranged to make a new functional protein.

A polynucleotide sequence is "heterologous to" an organism or a second polynucleotide sequence if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is different from any naturally occurring allelic variants.

"Join" refers to any method known in the art for functionally connecting protein domains, including without limitation recombinant fusion with or without intervening domains, intein-mediated fusion, non-covalent association, and covalent bonding, including disulfide bonding; hydrogen bonding; electrostatic bonding; and conformational bonding, *e.g*., antibody-antigen, and biotin-avidin associations.

"Polymerase" refers to an enzyme that performs template-directed synthesis of polynucleotides. The term encompasses both the full length polypeptide and a domain that has polymerase activity.

"Efficiency" in the context of a polymerase of this invention refers to the ability of the enzyme to perform its catalytic function under specific reaction conditions. Typically, "efficiency" as defined herein is indicated by the amount of product generated under given reaction conditions.

"Processivity" refers to the ability of a polymerase to remain bound to the template or substrate and perform polynucleotide synthesis. Processivity is measured by the number of catalytic events that take place per binding event.

"Thermally stable polymerase" as used herein refers to any enzyme that catalyzes polynucleotide synthesis by addition of nucleotide units to a nucleotide chain using DNA or RNA as a template and has an optimal activity at a temperature above 45°C.

"Polymerase chain reaction" or "PCR" refers to a method whereby a specific segment or subsequence of a target double-stranded DNA, is amplified in a geometric progression. PCR is well known to those of skill in the art; see, e.g., U.S. Patents 4,683,195 and 4,683,202; and PCR Protocols: A Guide to Methods and Applications, Innis et al., eds, 1990. Exemplary PCR reaction conditions typically comprise either two or three step cycles. Two step cycles have a denaturation step followed by a hybridization/elongation step. Three step cycles comprise a denaturation step followed by a hybridization step followed by a separate elongation step.

"Amplifying" refers to a step of submitting a solution to conditions sufficient to allow for amplification of a polynucleotide if all of the components of the reaction are intact. Components of an amplification reaction include, e.g., primers, a polynucleotide template, polymerase, nucleotides, and the like. The term "amplifying" typically refers to an "exponential" increase in target nucleic acid. However, "amplifying" as used herein can also refer to linear increases in the numbers of a select target sequence of nucleic acid, such as is obtained with cycle sequencing.

The term "amplification reaction mixture" refers to an aqueous solution comprising the various reagents used to amplify a target nucleic acid. These include enzymes, aqueous buffers, salts, amplification primers, target nucleic acid, and nucleoside triphosphates. Depending upon the context, the mixture can be either a complete or incomplete amplification reaction mixture

"Long PCR" refers to the amplification of a DNA fragment of 5 kb or more in length. Long PCR is typically performed using specially-adapted polymerases or polymerase mixtures (*see, e.g.,* U.S. Patent Nos. 5,436,149 and 5,512,462) that are distinct from the polymerases conventionally used to amplify shorter products.

PCR "sensitivity" refers to the ability to amplify a target nucleic acid that is present in low copy number. "Low copy number" refers to 10⁵, often 10⁴, 10³, 10², 10¹ or fewer, copies of the target sequence in the nucleic acid sample to be amplified.

The term "polymerase primer/template binding specificity" as used herein refers to the ability of a polymerase to discriminate between correctly matched primer/templates and mismatched primer templates. An "increase in polymerase primer/template binding specificity" in this context refers to an increased ability of a polymerase of the invention to discriminate between matched primer/template in comparison to a wild type polymerase protein.

"Nucleic acid" and "polynucleotide" are used interchangeably herein to refer to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, and peptide-nucleic acids (PNAs).

"Polypeptide," "peptide" and "protein" are used interchangeably herein to refer to a polymer of amino acid residues. The terms apply to naturally occurring amino acid polymers, as well as amino acid polymers in which one or more amino acid residue is an artificial chemical mimetic of a corresponding naturally occurring amino acid.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

For example, substitutions may be made wherein an aliphatic amino acid (G, A, I, L, or V) is substituted with another member of the group. Similarly, an aliphatic polaruncharged group such as C, S, T, M, N, or Q, may be substituted with another member of the group; and basic residues, *e.g*., K, R, or H, may be substituted for one another. In some embodiments, an amino acid with an acidic side chain, E or D, may be substituted with its uncharged counterpart, Q or N, respectively; or vice versa. Each of the following eight groups contains other exemplary amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(see, *e.g*., Creighton, Proteins (1984)).

A "polymerase nucleic acid" or "polymerase polynucleotide" is a polynucleotide sequence or subsequence encoding a protein comprising a polymerase domain. Nucleic acids encoding exemplary embodiments of the polymerases of the invention are identical or substantially identical to a nucleic acid encoding a polymerase disclosed herein, *e.g*., a sequence set forth in SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, or SEQ ID NO:19; which encodes a polymerase polypeptide identical or substantially identical to SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, or SEQ ID NO:20.

A "polymerase polypeptide" of the present invention is a protein comprising a polymerase domain. The polymerase polypeptide may also comprise additional domains including a DNA binding domain, e.g., Sso7D. DNA polymerases are well-known to those skilled in the art, e.g., *Pyrococcus furiosus, Thermococcus litoralis,* and *Thennotoga maritima.* They include both DNA-dependent polymerases and RNA-dependent polymerases such as reverse transcriptase. At least five families of DNA-dependent DNA polymerases are known, although most fall into families A, B and C. There is little or no sequence similarity among the various families. Most family A polymerases are single chain proteins that can contain multiple enzymatic functions including polymerase, 3' to 5' exonuclease activity and 5' to 3' exonuclease activity. Family B polymerases typically have a single catalytic domain with polymerase and 3' to 5' exonuclease activity, as well as accessory factors. Family C polymerases are typically multi-subunit proteins with polymerizing and 3' to 5' exonuclease activity. In *E*. *coli,* three types of DNA polymerases have been found, DNA polymerases I (family A), II (family B), and III (family C). In eukaryotic cells, three different family B polymerases, DNA polymerases α, δ, and ε, are implicated in nuclear replication, and a family A polymerase, polymerase , is used for mitochondrial DNA replication. Other types of DNA polymerases include phage polymerases. Similarly, RNA polymerases typically include eukaryotic RNA polymerases I, II, and III, and bacterial RNA polymerases as well as phage and viral polymerases. RNA polymerases can be DNA-dependent and RNA-dependent.

Exemplary embodiments of polymerases of the present invention include a polymerase identical or substantially identical to SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, or SEQ ID NO:20. A skilled practitioner will understand that specific amino acid residues within the polymerases can be modified, e.g., conservatively modified, without significantly affecting the improved polymerase ability. On average, there are at least 6 amino acids per 100 that can be modified. They include, for example, replacing glycine at position 12 with alanine, methionine at position 1 with valine, isoleucine at position 2 with leucine, isoleucine at position 8 with valine, or threonine at position 33 with serine.
(Positions are indicated with reference to SEQ ID NO:26.)

The polymerases of the present invention may be identified by their ability to bind to antibodies, *e.g*., polyclonal antibodies, raised against an immunogen comprising an amino acid sequence of SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, or SEQ ID NO:20 and conservatively modified variants thereof.

Polypeptide polymerases of the present invention have polymerase activity. Using the assays described herein, the activity of the polypeptides of the present invention can be measured. Some polymerase polypeptides of the invention exhibit improved polymerase activity as compared to wild type polymerases in the assays described herein.

Two nucleic acid sequences or polypeptides are said to be "identical" if the sequence of nucleotides or amino acid residues, respectively, in the two sequences is the same when aligned for maximum correspondence as described below. The terms "identical" or percent "identity," in the context of two or more nucleic acids or polypeptide sequences, refer to two or more sequences or subsequences that are the same or have a specified percentage of amino acid residues or nucleotides that are the same, when compared and aligned for maximum correspondence over a comparison window, as measured using one of the sequence comparison algorithms described later or by manual alignment and visual inspection.

When referring to proteins or peptides and for purposes of aligning polypeptides, it is recognized that residue positions that are not identical often differ by conservative amino acid substitutions, where amino acids residues are substituted for other amino acid residues with similar chemical properties (*e.g*. charge or hydrophobicity) and do not necessarily change the functional properties of the molecule. The scoring of conservative substitutions for the purposes of this patent is based on the BLOSUM62 matrix (Henikoff & Henikoff, Proc. Natl. Acad. Sci. USA 89:10915, 1989).

The term "sequence similarity" or "similar" may also be used with respect to amino acid sequences. This term encompasses conservative substitutions, as described above. For purposes of determining percent similarity, two amino acids are considered similar if they are given a value greater than zero (0) on the BLOSUM62 substitution matrix. Optimal alignment for determining percent sequence similarity may be conducted using various algorithms as further explained hereinbelow. In cases where an optimal alignment of two sequences requires the insertion of a gap in one or both of the sequences, an amino acid residue in one sequence that aligns with a gap in the other sequence is counted as a mismatch for purposes of determining percent identity. Gaps can be internal or external, *i.e*., a truncation.

The term "absolute percent identity" refers to a percentage of sequence identity determined by scoring identical amino acids as 1 and any substitution as zero, regardless of the similarity of mismatched amino acids. In a typical sequence alignment, *e.g*. a BLAST alignment, the "absolute percent identity" of two sequences is presented as a percentage of amino acid "identities." As used herein, where a sequence is defined as being "at least X% identical" to a reference sequence, *e.g*. "a polypeptide at least 90% identical to SEQ ID NO:2," it is to be understood that "X% identical" refers to absolute percent identity, unless otherwise indicated. In cases where an optimal alignment of two sequences requires the insertion of a gap in one or both of the sequences, an amino acid residue in one sequence that aligns with a gap in the other sequence is counted as a mismatch for purposes of determining percent identity. Gaps can be internal or external, *i*.*e*., a truncation.

The term "substantial identity" or "substantial similarity" of polynucleotide sequences means that a polynucleotide comprises a sequence that has at least 60% sequence identity, or sequence similarity, respectively. Alternatively, percent identity or percent similarity can be any integer from at least 60% to 100% (*e.g*. at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%).

In some embodiments, *e.g*., polymerases sequences disclosed herein, substantially identical polymerase sequences have 80%, 85%, 90%, 94%, 95%, 96%, 97%, 98%, or 99% compared to a reference sequence (*e.g*., a polymerase of SEQ ID NO:12, SEQ ID NO:16, or SEQ ID NO:18; or the polymerase region of SEQ ID NO:6, SEQ ID NO:8, SEQ ID NO:10, SEQ ID NO:14, or SEQ ID NO:20) using the programs described herein; preferably BLAST using standard parameters and procedures, as described below. One of skill will recognize that these values can be appropriately adjusted to determine corresponding identity or similarity of proteins encoded by two nucleotide sequences by taking into account codon degeneracy, amino acid similarity, reading frame positioning and the like, as further described below.

One of skill in the art will recognize that two polypeptides can also be "substantially identical" if the two polypeptides are immunologically similar. Thus, overall protein structure may be similar while the primary structure of the two polypeptides display significant variation. Therefore a method to measure whether two polypeptides are substantially identical involves measuring the binding of monoclonal or polyclonal antibodies to each polypeptide. Two polypeptides are substantially identical if the antibodies specific for a first polypeptide bind to a second polypeptide with an affinity of at least one third of the affinity for the first polypeptide.

Another indication that two nucleic acid sequences are substantially identical is that the two molecules or their complements hybridize to each other under stringent conditions, as described below. The phrase "selectively (or specifically) hybridizes to" refers to the binding, duplexing, or hybridizing of a molecule only to a particular nucleotide sequence under stringent hybridization conditions when that sequence is present in a complex mixture (*e.g*. total cellular or library DNA or RNA).

The phrase "stringent hybridization conditions" refers to conditions under which a probe will hybridize to its target subsequence, typically in a complex mixture of nucleic acid, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, highly stringent conditions are selected to be about 5-10°C lower than the thermal melting point (Tm) for the specific sequence at a defined ionic strength pH. Low stringency conditions are generally selected to be about 15-30°C below the Tm. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (*e.g*. 10 to 50 nucleotides) and at least about 60°C for long probes (*e.g*. greater than 50 nucleotides). Stringent conditions may also be achieved with the addition of destabilizing agents such as forrnamide. For selective or specific hybridization, a positive signal is at least two times background, preferably 10 times background hybridization.

In the present invention, nucleic acids encoding polypeptides of the invention can be identified in standard Southern blots under stringent conditions using the nucleic acid sequences disclosed here. For the purposes of this disclosure, suitable stringent conditions for such hybridizations are those which include a hybridization in a buffer of 40% formamide, 1 M NaCl, 1% SDS at 37°C, and at least one wash in 0.2X SSC at a temperature of at least about 50°C, usually about 55°C to about 60°C or 60°C, for 20 minutes, or equivalent conditions. A positive hybridization is at least twice background. Those of ordinary skill will readily recognize that alternative hybridization and wash conditions can be utilized to provide conditions of similar stringency.

A further indication that two polynucleotides are substantially identical is if the reference sequence, amplified by a pair of oligonucleotide primers, can then be used as a probe under stringent hybridization conditions to isolate the test sequence from a cDNA or genomic library, or to identify the test sequence in, *e.g*. a northern or Southern blot.

The terms "library members," "members of a the library" and the like refer to those nucleic acids present in a nucleic acid library that have the intended characteristics described herein; that is, nucleic acids that encode hybrid polypeptides. Small library members, *e.g*., comprising sequences encoding polypeptide domains, can of course be joined with other library members or parental or non-parental sequences to encode full length proteins..It is recognized that libraries may in addition contain other nucleic acids, either as intentional additions or unintended contaminants; these additional nucleic acids are not considered "members".

The terms 'minimal encoding sequence', 'minimal encoding oligonucleotide sequence', and 'minimal encoding nucleotide sequence' refer to nucleotide sequences that encode a library of hybrid sequences. It is the result of examining two or more different amino acid sequences and deducing a single degenerate nucleotide sequence that will encode a library of proteins that includes hybrid proteins derived from the two different amino acid sequences. Minimal encoding sequences can refer to a single codon, several codons or enough codons to encode an entire protein. Minimal encoding sequences need not be continuous. Minimal encoding sequences may encode non-parental amino acids both similar and dissimilar to parental sequences. Often, it is possible to deduce multiple minimal encoding sequences that can encode the same parental amino acids.

### C. Parent Protein Selection and alignment of sequences

In the methods of the invention, at least two polynucleotide sequences encoding polypeptides with a common biological activity (*e.g,*. deoxyuridine triphosphate nucleotidohydrolases, or DNA polymerases) are recombined to produce a library of hybrid polynucleotides. The library is then screened to identify functional hybrid proteins with an altered phenotype relative to the parent polypeptides.

The parent proteins can show substantial sequence or secondary structural similarity with each other, but they should also differ in at least 5 positions and may differ by as many as 100, 200, or more positions. The percent similarity or percent identity between the parent proteins can be any number from at least 60% to 99%. In comparing the initial sequences, there may be more than two parents. The multiple sequences may be divergent at a single position or at different positions. For example, there may be three related sequences that are the parents for generating hybrid molecules. One sequence may differ from the second at a single position, and the second may differ from the third at a different single position.

The majority of differences, *e.g*., greater than 50%, often greater than 75% or 90% of the differences, between the library members are typically confined to those corresponding amino acids that differ between the parent proteins. A corresponding amino acid refers to an amino acid residue of a parent sequence that occurs at a particular position when the parent sequences are maximally aligned. It should be understood that such position designations do not indicate the number of amino acids in the parent sequences per se, but indicate where in the parent sequence the residues occur. Alignment can be performed either manually or using a sequence comparison algorithm, as further explained below. For example, Figure 1 shows the amino acid sequences of two wild-type proteins that can be used in the methods of the present invention, Pfu and Deep Vent® polymerases. Figure 2 indicates the positions of amino acids that differ between the two parent proteins. Typically, the hybrid proteins created by the methods of the invention will differ from each other in the positions that differ between their parents.

The initial differences in sequence between the parent proteins are typically, but not necessarily, the result of natural variation. For example, the parent proteins can be variant forms that are obtained from different individuals or strains of an organism, *e.g.,* the parent proteins can be related sequences from the same organism (*e.g*., paralogs or allelic variations), or can be homologs from different organisms (interspecies homologs).

Accordingly, the parent polypeptides used in the methods of this invention are any set of two or more homologous proteins that share a common biological activity. Biological activity is not always shown directly, but may be inferred from sequence similarity or identity to known proteins of demonstrated activity. Biological activity may refer to a single enzyme activity even if a particular protein may have more than one enzymatic activity. Also, biological activity may refer to non-enzymatic activities such as binding to another molecule with allosteric effect, as with a hormone or a receptor; or binding to another molecule without allosteric effect, as for certain antibodies; or binding to another molecule with the effect of neutralization or sequestration.

Biological activity of a protein can be assessed using standard means known in the art for determining the function of a protein. For example, in some embodiments, the parent proteins will be enzymes and will share a common enzymatic activity. Exemplary enzymes include polymerases, ligases, lipases, dehydrogenases, RNAses, DNAses, proteases, kinases, caspases, methylases, transcription factors, and restriction endonucleases. In other embodiments, the parent proteins will be other proteins, *e.g*., receptors, hormones, immunoglobulins, or chromophores. The biological activity of these types of proteins can be assessed using known assays. The skilled practitioner will understand that any protein set, where the protein members are homologous proteins having at least 60% amino acid similarity, and often at least 60% identity, to each other and having a common biological activity, can be used as parental polypeptides.

The parent sequences are aligned according to standard alignment methods. The sequences are compared and aligned for maximum correspondence over a comparison window, or designated region as measured using one of the sequence comparison algorithms described below or by manual alignment and visual inspection. The parent protein sequences can be aligned using any of the known algorithms suitable for determining percent sequence identity and sequence similarity. For purposes of this patent, percent amino acid identity and percent amino acid similarity is determined by the default parameters of BLASTP using the Blosum62 similarity matrix, an expectation of 10, a word size of 3, and a gap costs setting of existence 11/extension 1 (Altschul et al., Nuc. Acids Res. 25:3389-3402 (1977)).

For sequence comparison, either nucleic acid or protein, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities or sequence similarities for the test sequences relative to the reference sequence, based on the program parameters.

The comparison window includes reference to a segment of any one of the number of contiguous positions selected from the group consisting of from 10 to 600, usually about 50 to about 200, more usually about 100 to about 150 in which a sequence maybe compared to a reference sequence of the same number of contiguous positions after the two sequences are optimally aligned. Methods of alignment of sequences for comparison are well-known in the art. Optimal alignment of sequences for comparison can be conducted, *e.g*. by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'1. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e.g*. Current Protocols in Molecular Biology (Ausubel et al., eds. 1995 supplement)).

An example of an algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. Software for performing BLAST analyses is publicly available through the National Center for Biotechnology Information (http://www.ncbi.nhn.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negativescoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

The BLAST algorithm also performs a statistical analysis of the similarity between two sequences (*see, e.g.* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

Proteins that are useful as parent proteins for generating protein hybrids typically have greater than 60% amino acid similarity, often greater than 60% identity, to each other. In some embodiments, the chosen parent sequences can be aligned without gaps. In other embodiments, the alignment must accommodate the presence of a gap or deletion in the amino acid sequence of one of the parent proteins.

Methods of aligning proteins containing gaps or deletions are known in the art. In some embodiments, the gap may be a result of a loop linking alpha helices or a turn in beta sheets. Typically, the gap or deletion will not affect the shared enzymatic activity between the two parent proteins. In some embodiments of the present invention, the starting sequences will be aligned in such a way as to include the gap or deletion. Standard programs for protein structure modeling can be used to help determine whether to omit or include an amino acid where a gap exists in an alignment, *e.g*. BLASTP.

For example, after inputting a protein sequence into BLASTP, a list of proteins is created with percent identities, similarities, and gaps indicated across the top of each pairwise comparison. If a gap is indicated, a library can be made to have some representatives with the gap and some without the gap.

In some embodiments, the gap may be of significant size, *e.g*. 2-50 amino acids, or have a loop with an enzymatic role. By aligning the two parent proteins to include the diversity of a gap or loop, larger diversity can be achieved.

Related proteins frequently have differences in length at their amino and carboxyl termini. Thus, the parental protein sequences may have length differences at their amino and carboxyl termini. The additional amino acids at either end may or may not contain a motif essential for function. For example, the carboxyl end of some type B polymerases contains a proliferating cell nuclear antigen (PCNA) binding motif. In some embodiments, one or more of the parent proteins will retain its C or N terminus motif. A library can be made to have some representatives with a C or N terminus tail and some without a C or N terminus tail.

Accordingly, the present invention provides methods of aligning parent sequences with or without gaps, deletions, or differences in their amino and carboxy terminals and combining them to construct a hybrid protein library and hybrid proteins of the present invention.

### D. Creating Polynucleotide Hybrids

After the parent proteins have been selected and aligned, the mismatches between the sequences are identified. Hybrid oligonucleotide sequences are then generated that contain a mixture of parental residues at the mismatched sites, *i.e.,* for any given hybrid sequences, some of the residues at some of the mismatched sites are from one parent; residues at other of the mismatched sites are from another parent. A library comprising hybrid sequences can thereby be created. Considerations in generating the hybrid molecule libraries are set forth below.

### Codon election

After the amino acid sequences of the homologous parent proteins are aligned, the amino acids residues that are different between the sequences are identified. For each set of different amino acid residues, the codons that encoded the differing residues are compared and a minimal encoding sequence is derived. Preferably, codons that encode the different residues and only differ by one nucleotide are then selected as a point of degeneracy, *i.e*., a point at which nucleotide variation results in codons encoding only one or the other parental amino acid.

Typically, the derivation of the minimal encoding sequence is also governed by the codon usage of a particular host. For example, if a nucleic acid encoding a hybrid protein is to be expressed in *E*. *coli,* the codon usage of *E. coli* can be used to derive a polynucleotide sequence that comprises preferred *E*. *coli* codons. An *E*. *coli* codon usage table can thus be used to compare the various codons that can encode two amino acids that differ.

In the simplest, and typically most common, case a single nucleic acid degeneracy can encode both amino acids that differ at a particular position in the parent sequences. For example, two homologous proteins may differ at a particular position where one parent has a valine at the position and the other has an isoleucine residue. Valine can be encoded by a number of different codons, one of which is GTT. Isoleucine can be encoded by a number of different residues, one of which is ATT. Therefore a minimal encoding sequence is (G/A)TT (or RTT using the standard single letter code). The first nucleotide of the codon is the site of degeneracy. Oligonucleotide synthesis machines can readily be directed to produce product with half G and half A at a particular position. Individual nucleic acid molecules generated during the synthesis will therefore have either G or A at that particular site and the library of hybrid sequences will have some sequences with a G for this codon and some with an A. Accordingly, the proteins encoded by the individual library members will have either a valine or an isoleucine at that site. The degeneracy created at this site is independent of degeneracies created at other sites. This results in a library with a large number of variants, but that is constrained by the sequences of the parental polypeptides.

In comparing some of the differences where a position in the aligned sequences has two different amino acids, a minimal encoding sequence may require two nucleotides to be changed in order to encode the two parental residues at that position. This can result in a situation where two non-parental amino acid sequences may also be encoded by the degenerate codon. For example, two parental sequences may differ at a particular position, where one residue is a lysine and the other parental residue is an alanine. Lysine is encoded by AAR and Ala by GCN. The minimal encoding sequence (A/G)(A/C)G may therefore be used to encode both lysine and alanine. However, such a degenerate codon also can encode threonine (ACN) and glutamine (GAR) in addition to Lys and Ala. In some instances, a hybrid protein may tolerate an amino acid residue that is not in either parent, especially if the non-parental amino acid is similar to the one of the parental amino acids. In other instances, for example, if the sequence occurs in a domain that is known to be important for protein activity, it may not be desirable to introduce non-parental amino acid residues. Moreover, in some cases, the degenerate codon could result in the introduction of a stop codon, which would result in a library in which a portion of the sequences are not useful. Typically, one of the parent residues is selected for this position.

The purpose of producing the hybrid library should be considered in making the decision as to which parent residue to choose. For instance, if a desired function such as thermal stability, or exonuclease activity level, is greater in one parent than another, the choice should favor the parent with the desired characteristic. All things being equal, in cases where there are more than two parental protein sequences or there are additional isozymes, homologues, or related sequences, decisions on whether to include a particular amino acid may be made by "voting" - for example, if fewer than a threshold fraction of the parental sequences differ in one position from an amino acids present in a majority of the sequences, then the rare amino acid may be ignored.

This situation may also be addressed by generating two different nucleic acid sequences, *e.g*., by synthesizing two different oligonucleotides, one of which encodes one of the parent residues, the other of which encodes the different parent residue. For the purposes of generating a library, mixing the two oligonucleotides in equal amounts will effectively produce a degenerate oligonucleotide encoding the two amino acids exclusively. This mixed nucleic acid sequence may then be used for assembly of nucleic acids encoding a hybrid protein library.

In some cases, the minimal encoding sequence is more likely to encode non-parental amino acid sequences because they share no codon sequences in common (*e.g*., Met and Asp). In this case again, the non-parental amino acids can be accepted as mutations in the library, or one of the parent codon sequence can be selected for incorporation into the hybrid protein library at this position, or two libraries can be constructed and combined as described above, or, if more than two parental sequences are used, or homologues are known, then the decision may be made by "voting".

In comparing two homologous sequences, gaps and deletions may also occur. As the parental proteins share a common activity, the gaps typically do not significantly affect activity. For example, the homologous proteins may include loops linking alpha helices or turns in beta sheets. The absolute size of these links and turns are often not consequential. In general, a gap may be accommodated in generating the hybrid library by generating two versions of the nucleic acid sequence, *e.g*. synthesizing two oligonucleotides, mixing the two sequences, and using the mixture to construct the library. Alternatively, the hybrid library can be constructed so that the gap is present in all of the members or absent in all of the members. Similarly, related proteins frequently have differences in length at their amino and carboxyl termini. Again, two sequence may be generated, one with a longer end and one without the longer end, and then combined; or a library can be generated that includes the additional length or omits the additional length; or, if more than two parental sequences are used, then the decision may be made by "voting".

Often, it may be desirable to introduce restriction endonuclease sites into the sequences of the library, for example, in order to facilitate assembly of the sequence encoding the protein, or domain exchange. One of skill in the art understands that such sites are typically relatively infrequent, *e.g*., have a 6 base pair recognition site. The restriction sites are often introduced into the nucleic acids by modifying codons without changing the amino acid encoded by the codon. The restriction sites are typically introduced into regions of the two parent sequences that are identical, although this need not be the case.

### Preparation of hybrid sequences and librasy production

After the minimal encoding sequence is selected, the library is constructed using techniques well known in the art. Typically, the nucleic acids to be incorporated into the library are synthesized as oligonucleotides that are assembled to form a sequence encoding the hybrid polypeptide. Procedures for performing this are well known in the art. Oligonucleotides of about 50-100 bases are typically synthesized. The oligonucleotides are designed such that they overlap, *e.g*., by 10 to 50 bases, to provide adequate annealing and specificity despite the sequence differences. As appreciated by one of skill, the 3' ends often are in regions in which there are minimal or no differences between the parent sequences.

The completed gene is then assembled, *e.g.,* by primer extension *(see, e.g.,* Figure 3). In such an assembling procedure overlapping oligonucleotides are annealed to one another and extended using a high fidelity thermostable polymerase. Large amounts of primer and minimal cycles (usually between 0 and 5) are used in assembling segments. The products are then purified and used for the next cycle of pairing and primer extension.

The resulting reassembled polynucleotide can be of various lengths. Preferably the reassembled sequences are from about 50 bp to about 10 kb.

As appreciated by one of skill, the gene encoding the hybrid polypeptide can also be assembled by ligating the appropriate fragments. Further, the full-length hybrid polypeptide can be assembled by ligating together the appropriate smaller fragments. If the hybrid polypeptide is a portion of a larger protein, incorporation into the larger protein can also occur at this step. Often, restriction endonuclease sites can be incorporated into the primers to improve the efficiency of the ligation step.

In some instances, it is desirable to prepare two libraries and then combine them, for example, in instances in which there is a gap in the parental sequences or two amino acid residues that differ in the parent sequences differ in their codons at all three nucleotide position.

As appreciated by those in the art, the hybrid molecules can further be used as substrates to generate additional diversity by using various techniques such as recursive recombination *(see, e.g.,* U.S. Patent No. 6,180406, and related patents); and various other mutagenesis procedures, *e.g*., error-prone PCR, cassette mutagenesis. These techniques may be performed on all of the library members or selected subpopulation or individual library members.

In some recombination techniques, polynucleotide fragments are recombined by linking overlapping single stranded segments and then contacting the resulting linked segments with a polymerase. *See*, *e.g.* U. S. Patent No. 6,150,111.

In other techniques, recombination is independent of natural restriction sites or *in vitro* ligation (Ma et al., Gene 58:201-216 (1989); Oldenburg et al., Nucleic Acids Research 25:451-452 (1997)). In some of these methods, an *in vivo* method for plasmid construction takes advantage of the double-stranded break repair pathway in a cell such as a yeast cell to achieve precision joining of DNA fragments. This method involves synthesis of linkers, *e.g*. 60-140 base pairs, from short oligonucleotides and requires assembly by enzymatic methods into the linkers needed (Raymond et al., BioTechniques 26(1):134-141 (1999)).

In some techniques, short random or non-random oligonucleotide sequences are recombined with polynucleotide segments derived from polynucleotides encoding functional polymerases.

Modifications may also be introduced into the polynucleotide segments or the assembled polynucleotides encoding the hybrid proteins using other known mutagenesis techniques. For example, the polynucleotides can be submitted to one or more rounds of error-prone PCR (*e.g*. Leung, D. W. et al., Technique 1:11-15 (1989); Caldwell, R. C. and Joyce, G. F. PCR Methods and Applications 2:28-33 (1992); Gramm, H. et al., Proc. Natl. Acad. Sci. USA 89:3576-3580 (1992)), thereby introducing variation into the polynucleotides. Alternatively, cassette mutagenesis (*e.g*. Stemmer, W. P. C. et al., Biotechniques 14:256-265 (1992); Arkin, A. and Youvan, D. C. Pro. Natl. Acad. Sci. USA 89:7811-7815 (1992); Oliphant, A. R. et al., Gene 44:177-183 (1986); Hermes, J. D. et al., Proc. Natl. Acad. Sci. USA 87:696-700 (1990)), in which the specific region to be optimized is replaced with a synthetically mutagenized oligonucleotide, can be used. Mutator strains of host cells can also be employed to add to mutational frequency (Greener and Callahan, Strategies in Mol. Biol. 7: 32 (1995)).

Site directed mutagenesis is well known in the art and may also be used to introduce further diversity into the sequences. Such techniques include site directed mutagenesis as described, *e.g*., in Ling et al. (1997) Anal Biochem. 254(2): 157-178; Dale et al. (1996) Methods Mol. Biol. 57:369-374; Smith (1985) Ann. Rev. Genet. 19:423-462; Botstein & Shortle (1985) Science 229:1193-1201; Carter (1986) Biochem. J. 237:1-7; and Kunkel (1987) "The efficiency of oligonucleotide directed mutagenesis" in Nucleic Acids & Molecular Biology (Eckstein, F. and Lilley, D.M.J. eds., Springer Verlag, Berlin)); mutagenesis using uracil containing templates (Kunkel (1985 Proc. Natl. Acad. Sci. USA 82:488-492; Kunkel et al. (1987) Methods in Enzymol. 154, 367-382; and Bass et al. (1988) Science 242:240-245); oligonucleotide-directed mutagenesis (Methods in Enzymol. 100: 468-500 (1983); Methods in Enzyinol. 154: 329-350 (1987); Zoller & Smith (1982) Nucleic Acids Res. 10:6487-6500; Zoller & Smith (1983) Methods in Enzymol. 100:468-500; and Zoller & Smith (1987) Methods in Enzymol. 154:329-350); phosphorothioate-modified DNA mutagenesis (Taylor et al. (1985) Nucl. Acids Res. 13: 8749-8764; Taylor et al. (1985) Nucl. Acids Res. 13: 8765-8787 (1985); Nakamaye & Eckstein (1986) Nucl. Acids Res. 14: 9679-9698; Sayers et al. (1988) Nucl. Acids Res. 16:791-802; and Sayers et al. (1988) Nucl. Acids Res. 16: 803-814); mutagenesis using gapped duplex DNA (Kramer et al. (1984) Nucl. Acids Res. 12: 9441-9456; Kramer & Fritz (1987) Methods in Enzymol. 154:350-367; Kramer et al. (1988) Nucl. Acids Res. 16: 7207; and Fritz et al. (1988) Nucl. Acids Res. 16: 6987-6999).

An additional modification method well known in the art is point mismatch repair, *e.g*. (Kramer et al. (1984) Cell 38:879-887), mutagenesis using repair-deficient host strains (Carter et al. (1985) Nucl. Acids Res. 13: 4431-4443; and Carter (1987) Methods in Enzymol. 154: 382-403), deletion mutagenesis (Eghtedarzadeh & Henikoff (1986) Nucl. Acids Res. 14: 5115), restriction-selection and restriction-selection and restriction-purification (Wells et al. (1986) Phil. Trans. R. Soc. Lond. A 317: 415-423), mutagenesis by total gene synthesis (Nambiar et al. (1984) Science 223: 1299-1301; Sakamar and Khorana (1988) Nucl. Acids Res. 14: 6361-6372; Wells et al. (1985) Gene 34:315-323; and Grundström et al. (1985) Nucl. Acids Res. 13: 3305-3316), double-strand break repair (Mandecki (1986); Arnold (1993) Current Opinion in Bioteclanology 4:450-455; Proc. Natl. Acad. Sci. USA, 83:7177-7181). Additional details on many of the above methods can be found in Metlrods in Enzymology Volume 154, which also describes useful controls for trouble-shooting problems with various mutagenesis methods.

The assembled gene fragments may then be cloned into any of a number of vectors to generate a library comprising individual hybrid molecules that comprise residues from the parent sequences.

### Expression of hybrid proteins and protein libraries

There are many expression systems for producing the hybrid polypeptides and polypeptide libraries that are well know to those of ordinary skill in the art. *(See, e.g.,* Gene Expression Systems, Fernandez and Hoeffler, Eds. Academic Press, 1999; Sambrook & Russell, *supra*; and Ausubel *et al, supra*.) Typically, the polynucleotide that encodes a hybrid polypeptide is placed under the control of a promoter that is functional in the desired host cell. An extremely wide variety of promoters are available, and can be used in the expression vectors of the invention, depending on the particular application. Ordinarily, the promoter selected depends upon the cell in which the promoter is to be active. Other expression control sequences such as ribosome binding sites, transcription termination sites and the like are also optionally included.

Commonly used prokaryotic control sequences, which are defined herein to include promoters for transcription initiation, optionally with an operator, along with ribosome binding site sequences, include such commonly used promoters as the beta-lactamase (penicillinase) and lactose (*lac*) promoter systems (Change et al., Nature (1977) 198: 1056), the tryptophan (*tip*) promoter system (Goeddel et al., Nucleic Acids Res. (1980) 8: 4057), the *tac* promoter (DeBoer, et al., Proc. Natl. Acad. Sci. U.S.A. (1983) 80:21-25); and the lambda-derived P_{L} promoter and N-gene ribosome binding site (Shimatake et al., Nature (1981) 292: 128). The particular promoter system is not critical to the invention, any available promoter that functions in prokaryotes can be used. Exemplary bacterial expression vectors include plasmids such as pBR322-based plasmids, *e.g*., pBLUESCRIPT™, pSKF, pET23D, λ-page derived vectors, and fusion expression systems such as GST and LacZ. Epitope tags can also be added to recombinant proteins to provide convenient methods of isolation, *e.g*., c-myc, HA-tag, 6-His tag, maltose binding protein, VSV-G tag, anti-DYKDDDDK tag, or any such tag, a large number of which are well known to those of skill in the art.

For expression of hybrid polypeptides in prokaryotic cells other than *E*. *coli,* a promoter that functions in the particular prokaryotic species is required. Such promoters can be obtained from genes that have been cloned from the species, or heterologous promoters can be used. For example, the hybrid *trp-lac* promoter functions in *Bacillus* in addition to *E*. *coli.* These and other suitable bacterial promoters are well known in the art and are described, *e.g*., in Sambrook *et al.* and Ausubel *et al.* Bacterial expression systems for expressing the proteins of the invention are available in, *e.g., E. coli, Bacillus sp.,* and *Salmonella* (Palva et al., Gene 22:229-235 (1983); Mosbach et al., Nature 302:543-545 (1983). Kits for such expression systems are commercially available.

Eukaryotic expression systems for mammalian cells, yeast, and insect cells are well known in the art and are also commercially available. In yeast, vectors include Yeast Integrating plasmids (*e.g*., YIp5) and Yeast Replicating plasmids (the YRp series plasmids) and pGPD-2. Expression vectors containing regulatory elements from eukaryotic viruses are typically used in eukaryotic expression vectors, *e.g*., SV40 vectors, papilloma virus vectors, and vectors derived from Epstein-Barr virus. Other exemplary eukaryotic vectors include pMSG, pAV009/A+, pMT010/A+, pMAMneo-5, baculovirus pDSVE, and any other vector allowing expression of proteins under the direction of the CMV promoter, SV40 early promoter, SV40 later promoter, metallothionein promoter, murine mammary tumor virus promoter, Rous sarcoma virus promoter, polyhedrin promoter, or other promoters shown effective for expression in eukaryotic cells.

Either constitutive or regulated promoters can be used in the present invention. Regulated promoters can be advantageous because the host cells can be grown to high densities before expression of the polypeptides is induced. Further, high level expression of heterologous proteins may slow cell growth in some situations. An inducible promoter is a promoter that directs expression of a gene where the level of expression is alterable by environmental or developmental factors such as, for example, temperature, pH, anaerobic or aerobic conditions, light, transcription factors and chemicals.

For *E*. *coli* and other bacterial host cells, inducible promoters are known to those of skill in the art. These include, for example, the *lac* promoter, the bacteriophage lambda P_{L} promoter, the hybrid *trp-lac* promoter (Amann et al. (1983) Gene 25: 167; de Boer et al. (1983) Proc. Nat'l. Acad. Sci. USA 80: 21), and the bacteriophage T7 promoter (Studier et al. (1986) J. Mol. Biol.; Tabor et al. (1985) Proc. Nat'l. Acad. Sci. USA 82: 1074-8). These promoters and their use are discussed in Sambrook *et al., supra.*

Inducible promoters for other organisms are also well known to those of skill in the art. These include, for example, the metallothionein promoter, the heat shock promoter, as well as many others.

Translational coupling may also be used to enhance expression. The strategy uses a short upstream open reading frame derived from a highly expressed gene native to the translational system, which is placed downstream of the promoter, and a ribosome binding site followed after a few amino acid codons by a termination codon. Just prior to the termination codon is a second ribosome binding site, and following the termination codon is a start codon for the initiation of translation. The system dissolves secondary structure in the RNA, allowing for the efficient initiation of translation. See Squires, et. al. (1988), J. Biol. Chent. 263: 16297-16302.

The construction of polynucleotide constructs generally requires the use of vectors able to replicate in bacteria. Such vectors are commonly used in the art. A plethora of kits are commercially available for the purification of plasmids from bacteria (for example, EasyPrepJ, FlexiPrepJ, from Pharmacia Biotech; StrataCleanJ, from Stratagene; and, QIAexpress Expression System, Qiagen). The isolated and purified plasmids can then be further manipulated to produce other plasmids, and used to transform cells.

The hybrid polypeptides can be expressed intracellularly, or can be secreted from the cell. Intracellular expression often results in high yields. If necessary, the amount of soluble, active polypeptide may be increased by performing refolding procedures (*see, e.g*., Sambrook *et al., supr-a*.; Marston et al., Bio/Technology (1984) 2: 800; Schoner et al., Bio/Technology (1985) 3: 151). Fusion polypeptides of the invention can be expressed in a variety of host cells, including *E*. *coli,* other bacterial hosts, yeast, and various higher eukaryotic cells such as the COS, CHO and HeLa cells lines and myeloma cell lines. The host cells can be mammalian cells, insect cells, or microorganisms, such as, for example, yeast cells, bacterial cells, or fungal cells.

Once expressed, the hybrid polypeptides can be purified according to standard procedures of the art, including ammonium sulfate precipitation, affinity columns, column chromatography, gel electrophoresis and the like (*see*, generally, R. Scopes, Protein Purification, Springer-Verlag, N.Y. (1982), Deutscher, Methods in Enzynology Vol. 182: Guide to Protein Purification., Academic Press, Inc. N.Y. (1990)). Substantially pure compositions of at least about 90 to 95% homogeneity are preferred, and 98 to 99% or more homogeneity are most preferred. Once purified, partially or to homogeneity as desired, the polypeptides may then be used (*e.g*., as immunogens for antibody production).

To facilitate purification of the hybrid polypeptides of the invention, the nucleic acids that encode the fusion polypeptides can also include a coding sequence for an epitope or "tag" for which an affinity binding reagent is available. Examples of suitable epitopes include the myc and V-5 reporter genes; expression vectors useful for recombinant production of fusion polypeptides having these epitopes are commercially available (*e.g*., Invitrogen (Carlsbad CA) vectors pcDNA3.1/Myc-His and pcDNA3.1/VS-His are suitable for expression in mammalian cells). Additional expression vectors suitable for attaching a tag to the fusion proteins of the invention, and corresponding detection systems are known to those of skill in the art, and several are commercially available (*e.g*., FLAG" (Kodak, Rochester NY). Another example of a suitable tag is a polyhistidine sequence, which is capable of binding to metal chelate affinity ligands. Typically, six adjacent histidines are used, although one can use more or less than six. Suitable metal chelate affinity ligands that can serve as the binding moiety for a polyhistidine tag include nitrilo-tri-acetic acid (NTA) (Hochuli, E. (1990) "Purification of recombinant proteins with metal chelating adsorbents" In Genetic Engineering: Principles and Methods, J.K. Setlow, Ed., Plenum Press, NY; commercially available from Qiagen (Santa Clarita, CA)).

### E. Characterization of Hybrid Proteins

After the nucleic acid library is created using the methods described above, the library is screened for functional hybrids and/or hybrids that possess improved activity over their parents. Assays known in the art are used to compare the activity of a hybrid protein to its wild-type counterpart.

The nature of screening or selection depends on the property or characteristic that is to be improved or acquired. A detailed example explaining the evaluation of hybrid polymerases is provided below. Of course, hybrid proteins, *e.g*., receptor molecules, may be tested for an improved or acquired activity such as signaling or ligand binding using assays appropriate to the protein. It is not usually necessary to understand the molecular basis by which particular products of recombination (recombinant segments) have acquired new or improved properties or characteristics relative to the starting substrates.

Depending on the particular screening protocol used for a desired property, initial round(s) of screening can sometimes be performed using bacterial cells due to high transfection efficiencies and ease of culture. However, for eukaryotic proteins, bacterial expression is often not practical, and yeast, fungal or other eukaryotic systems are used for library expression and screening. Similarly, other types of screening which are not amenable to screening in bacterial or simple eukaryotic library cells, are performed in cells selected for use in an environment close to that of their intended use. Final rounds of screening can be performed in the precise cell type of intended use.

If further diversity is desired, at least one, and usually a collection, of hybrid sequences that are identified in an initial screen/selection may be subjected to an additional round of hybrid generation or an additional procedure to generate diversity. For example, in generating the initial library, not all of the residues that are identified as differing in parental sequences may be the subject of hybrid generation, *i.e.,* particular amino acids present in a subset of the parental sequences may be selected. Subsequent rounds may be directed to generating hybrids comprising such additional residues. Further, an additional round of hybrid generation may be performed using a different parent sequence, *i.e*., a parent sequence that was not included in the initial alignment. Lastly, a different diversity-generating procedure, *e.g*., recursive recombination, may be used.

The second round of diversity generation can be followed by a further round of screening/selection according to the principles discussed above for the first round. The stringency of screening/selection may be increased between rounds. Also, the nature of the screen and the property being screened for may vary between rounds if improvement in more than one property is desired or if acquiring more than one new property is desired. Additional rounds of hybrid/diversity generation and screening can then be performed until the recombinant segments have sufficiently evolved to acquire the desired new or improved property or function.

### F. Hybrid Polymerases

In some embodiments of the invention, the parental sequences are polymerases and the hybrid proteins are selected for improved polymerase function, *e.g*, processivity or error-correcting activity. These properties can be measured and compared to the parent polymerase activities using methodology well known in the art *(see, e.g.,* WO0192501), examples of which follow.

### Assays to evaluate polymerase activity

Activity of a polymerase can be measured using a variety of assays that can be used to determine processivity or modification activity of a polymerase. Improvement in activity may include both increased processivity and increased efficiency.

The polymerases of the present invention, e.g. SEQ ID NO:2 and SEQ ID NO:4, exhibit polymerase activity, e.g., processivity, primer/template binding specificity, and 3' to 5' exonuclease activity. The activities can be measured using techniques that are standard in the art.

For example, polymerase processivity can be measured by a variety of methods known to those of ordinary skill in the art. Polymerase processivity is generally defined as the number of nucleotides incorporated during a single binding event of a modifying enzyme to a primed template. For example, a 5' FAM-labeled primer is annealed to circular or linearized ssM13mp18 DNA to form a primed template. In measuring processivity, the primed template usually is present in significant molar excess to the polymerase so that the chance of any primed template being extended more than once by the polymerase is minimized. The primed template is therefore mixed with the polymerase at a ratio such as approximately 4000:1 (primed DNA:DNA polymerase) in the presence of buffer and dNTPs. MgCl₂ is added to initiate DNA synthesis. Samples are quenched at various times after initiation, and analyzed on a sequencing gel. At a polymerase concentration where the median product length does not change with time or polymerase concentration, the length corresponds to the processivity of the enzyme. The processivity of a protein of the invention, e.g., SEQ ID NO:2 or SEQ ID NO:4, is then compared to the processivity of a wild type enzyme.

Efficiency can be demonstrated by measuring the ability of an enzyme to produce product. Increased efficiency can be demonstrated by measuring the increased ability of an enzyme to produce product. Such an analysis measures the stability of the double-stranded nucleic acid duplex indirectly by determining the amount of product obtained in a reaction. For example, a PCR assay can be used to measure the amount of PCR product obtained with a short, *e.g*., 12 nucleotide in length, primer annealed at an elevated temperature, *e.g*., 50°C. In this analysis, enhanced efficiency is shown by the ability of a polymerase to produce more product in a PCR reaction using the 12 nucleotide primer annealed at 504°C.

Efficiency can also be measured, *e.g*., in a real-time PCR. The Ct value represents the number of cycles required to generate a detectable amount of DNA (a "detectable" amount of DNA is typically 2X, usually 5X, 10X, 100X or more above background). An efficient polymerase may be able to produce a detectable amount of DNA in a smaller number of cycles by more closely approaching the theoretical maximum amplification efficiency of PCR. Accordingly, a lower Ct value reflects a greater amplification efficiency for the enzyme.

Long PCR may be used as another method of demonstrating enhanced efficiency. For example, an enzyme with enhanced efficiency typically allows the amplification of a long amplicon (> 5 kb) in a shorter extension time compared to an enzyme with relatively lower efficiency.

Assays such as salt sensitivity can also be used to demonstrate improvement in efficiency or equivalent efficiency of a polymerase of the invention. A polymerase of the present invention may exhibit increased tolerance to high salt concentrations, *i.e*., a processive enzyme with increased processivity can produce more product in higher salt concentrations. For example, a PCR analysis can be performed to determine the amount of product obtained in a reaction using a polymerase of the present invention compared to a wild type polymerase in reaction conditions with high salt, *e.g*., 80 mM.

Other methods of assessing efficiency of the polymerases of the invention can be determined by those of ordinary skill in the art using standard assays of the enzymatic activity of a given modification enzyme.

Primer/template specificity is the ability of an enzyme to discriminate between matched primer/template duplexes and mismatched primer/template duplexes. Specificity can be determined, for example, by comparing the relative yield of two reactions, one of which employs a matched primer, and one of which employs a mismatched primer. An enzyme with increased discrimination will have a higher relative yield with the matched primer than with the mismatched primer, *i.e*., the ratio of the yield in the reaction using the matched primer vs. the reaction using the mismatched primer is about 1 or above. This ratio can then be compared to the yield obtained in a parallel set of reactions employing a wild type polymerase.

In other assays for improvement, the exonuclease activity of a polymerase can also be measured, as described in the "Examples" section. In some instances, desired improvements may take into account multiple functions of a polymerase. For example, one may want to tailor the ratio of exonuclease activity to polymerization activity.

### Polymerase-DNA binding domain conjguates

In some embodiments, the novel polymerases are conjugated to a DNA binding domain. A DNA binding domain is a protein, or a defined region of a protein, that binds to nucleic acid in a sequence-independent matter, *e.g*., binding does not exhibit a gross preference for a particular sequence. DNA binding domains may be single or double stranded.

The DNA binding proteins are preferably thermostable. Examples of such proteins include, but are not limited to, the Archaeal small basic DNA binding proteins Sso7D and Sso7D-like proteins(*see*, *e.g.,* Choli et al., Biochinzica et Biophysica Acta 950:193-203, 1988; Baumann et al., Structural Biol. 1:808-819, 1994; and Gao et al, Nature Struc. Biol. 5:782-786, 1998), archaeal HMf-like proteins (*see*, *e.g.,* Starich et al., J. Molec. Biol. 255:187-203, 1996; Sandman et al., Gene 150:207-208, 1994), and PCNA homologs *(see, e.g.,* Cann et al., J. Bacteriology 181:6591-6599, 1999; Shamoo and Steitz, Cell:99, 155-166, 1999; De Felice et al., J. Molec. Biol. 291, 47-57, 1999; and Zhang et al., Biochemistry 34:10703-10712, 1995).

Sso7d and Sso7d-like proteins, Sac7d and Sac7d-like proteins, *e.g*., Sac7a, Sac7b, Sac7d, and Sac73 are small (about 7,000 kd MW), basic chromosomal proteins from the hyperthermophilic archaeabacteria *Sulfolobus solfataricus* and *S*. *acidocaldarius,* respectively. These proteins are lysine-rich and have high thermal, acid and chemical stability. They bind DNA in a sequence-independent manner and when bound, increase the T_{M} of DNA by up to 40° C under some conditions (McAfee et al., Biochemistry 34:10063-10077, 1995). These proteins and their homologs are typically believed to be involved in stabilizing genomic DNA at elevated temperatures. Suitable Sso7d-like DNA binding domains for use in the invention can be modified based on their sequence homology to Sso7d. Typically, DNA binding domains that are identical to or substantially identical to a known DNA binding protein over a comparison window of about 25 amino acids, optionally about 50-100 amino acids, or the length of the entire protein, can be used in the invention. The sequence can be compared and aligned for maximum correspondence over a comparison window, or designated region as measured using one of the described comparison algorithms or by manual alignment and visual inspection. For purposes of this patent, percent amino acid identity is determined by the default parameters of BLAST.

The HMf-like proteins are archaeal histones that share homology both in amino acid sequences and in structure with eukaryotic H4 histones, which are thought to interact directly with DNA. The HMf family of proteins form stable dimers in solution, and several HMf homologs have been identified from thermostable species (*e.g., Methanothennus fervidus* and *Pyrococcus* strain GB-3a). The HMf family of proteins, once joined to Taq DNA polymerase or any DNA modifying enzyme with a low intrinsic processivity, can enhance the ability of the enzyme to slide along the DNA substrate and thus increase its processivity. For example, the dimeric HMf-like protein can be covalently linked to the N terminus of Taq DNA polymerase, *e.g*., via chemical modification, and thus improve the processivity of the polymerase.

Certain helix-hairpin-helix motifs have been shown to bind DNA nonspecifically and enhance the processivity of a DNA polymerase to which it is fused (Pavlov et al., Proc Natl Acad Sci U S A. 99:13510-5, 2002).

Many but not all family B DNA polymerases interact with accessory proteins to achieve highly processive DNA synthesis. A particularly important class of accessory proteins is referred to as the sliding clamp. Several characterized sliding clamps exist as trimers in solution, and can form a ring-like structure with a central passage capable of accommodating double-stranded DNA. The sliding clamp forms specific interactions with the amino acids located at the C terminus of particular DNA polymerases, and tethers those polymerases to the DNA template during replication. The sliding clamp in eukarya is referred to as the proliferating cell nuclear antigen (PCNA), while similar proteins in other domains are often referred to as PCNA homologs. These homologs have marked structural similarity but limited sequence similarity.

Recently, PCNA homologs have been identified from thermophilic Archaea (*e.g*., *Pyroccocus furiosus*). Some family B polymerases in Archaea have a C terminus containing a consensus PCNA-interacting amino acid sequence and are capable of using a PCNA homolog as a processivity factor (*see, e.g.,* Cann et al., J. Bacteriol. 181:6591-6599, 1999 and De Felice et al., J. Mol. Biol. 291:47-57, 1999). These PCNA homologs are useful DNA binding domains for the invention. For example, a consensus PCNA-interacting sequence can be joined to a polymerase that does not naturally interact with a PCNA homolog, thereby allowing a PCNA homolog to serve as a processivity factor for the polymerase. By way of illustration, the PCNA-interacting sequence from *Pyrococcus furiosus* PolII (a heterodimeric DNA polymerase containing two family B-like polypeptides) can be covalently joined to *Pyrococcus furiosus* PolI (a monomeric family B polymerase that does not normally interact with a PCNA homolog). The resulting fusion protein can then be allowed to associate non-covalently with the *Pyrococcus furiosus* PCNA homolog to generate a novel heterologous protein with increased processivity relative to the unmodified *Pyrococcus furiosus* PolI.

Additional DNA binding domains suitable for use in the invention can be identified by homology with known DNA binding proteins and/or by antibody cross-reactivity, or may be found by means of a biochemical assay. DNA binding domains may be synthesized or isolated using the techniques described above.

The DNA binding domain and the polymerase domain of the conjugate or fusion proteins of the invention can be joined by methods well known to those of skill in the art. These methods include both chemical and recombinant means, which are described in WO0192501.

### EXAMPLES

These examples describe the generation of hybrid libraries and the isolation of hybrid proteins from the libraries.

### Example 1. Generation of hybrid Dut proteins

In this example the invention is to be used to isolate hybrid proteins with varying temperature optima. The model proteins are a mesophilic and thermophilic deoxyuridine 5'-triphosphate nucleotidohydrolase (dUTPase or Dut). Sequences of the mesophile E. *coli* Dut (ECD) and the thermophile *Aquifex aeolicus* Dut (AAD) genes were aligned using BlastP. The sequences are 40% identical and 60% similar as defined by the Blast default parameters. The sequences and Blast alignment are shown in Figure 4.

The aligned parental sequences and all possible codons in order of frequency of use by E. *coli* are shown in Figure 5A. A minimal encoding sequence was derived by identifying codons that will encode both sequences with a minimal number of degeneracies (Figure 5B). Codons frequently used by *E. coli* are preferred. There are 90 differences between the two sequences. Of these, 49 can be encoded by incorporating a single degeneracy in the DNA sequence. Most of the others, 38 of them, require two degeneracies and 1 requires three. There is one gap. Two of the degeneracies could result in termination codons being incorporated into the sequence. Nucleic acid degeneracies that might incorporate termination sites or amino acids not similar (BLOSUM 62 number is <0) to either parental amino acid sequence were removed (Figure 5C) and replaced with sequences of the more thermal stabile AAD, in keeping with the experimental purpose. If this was not done, up to 24% of the amino acids incorporated into the hybrid protein may have been non-parental; some of those with no similarity to either parent. The elimination of the non-similar sequences reduced the maximum number of non-parental amino acids to 14%, all of which would be similar to at least one parent.

The double-stranded nucleic acid sequence showing the degeneracies and encoded amino acid residues is shown in Figure 6. Priming and restriction sites were added to the ends (shown in bold). In two cases, codon usage was changed to add restriction sites (underlined and in italics). The amino acids encoded by the sequence are indicted below the codons.

Figure 7 shows the full length hybrid library nucleic acid sequence. The degenerate positions are represented using standard single letter code. Oligonucleotides sequences were selected for synthesis (shown in bold). Selections were made such that minimal degeneracies exist where primers were expected to anneal to one another during assembly. In one portion of the sequence, there were no regions where reasonably-sized (about 10 to 50 bases), annealable oligonucleotides sequences could be selected. In this example, the *Cla*I site (underlined) inserted in the previous step is used to assemble a full length protein encoding library.

A hybrid library encoding a small protein such as DUT can be constructed by synthesizing the oligonucleotides so that there are no gaps once the primers are annealed. In this case, ligation rather than assembly PCR may be used to construct the hybrid library. The oligonucleotides are simply sequentially annealed, ligated, purified, than annealed again.

The final primers chosen in this example are indicated below. Assembly would occur as follows: Fwd1 primer is annealed to RevA primer. In separate tubes, Fwd2 is annealed to RevB, Fwd3 to RevC, Fwd4 to RevD, and Fwd5 is annealed to Rev5. The products of the five annealing reactions are primer extended with a DNA dependent DNA polymerase with proof reading activity, typically *E*. *coli* DNA polymerase I Klenow fragment, or the thermal stable Phusion polymerase (MJ Research, Inc.). If Phusion polymerase is used, it is possible to thermally cycle the primer extension reaction. The products of the Fwd1/RevA reaction are annealed to the products of the Fwd/2/RevB reaction and extension is repeated. Similarly, the products of the Fwd4/RevD reaction are annealed to the Fwd5/RevE reaction and extended. Finally, the Fwd1/RevA/Fwd2/RevB products are annealed to the Fwd3/RevC products and extended.

### Oligonucleotides:

Fwd 1: 5'-TTGGTACCAA GCTTCATATG A-3' 
Fwd 2: 5'-CC GCTGCCGASC TATGCGACCY CTCACAGCKC AGGCCTGGAT
CTGCGTGCG-3'
 
Fwd 3: 5'-TTCCG ACCGGTCTGA TCMTTSAWAT TSCGGAWGGT TMTGMGGSGC
AGRTGCKGCC GCGTAGCGGC CTGG-3'
 
Fwd 4: 5'-TTTTGATCGA TRSCGATTAT CRGGGCSAAS TGAWGRTTAK
CSTGGTGAAC CKGGGCMASG AWGAAKTTRY GATTSAGCSG GGCGAACGTA
TTGCGCAG-3'
 
Fwd 5: 5'-CGTGGCGAA GGCGGCTTTG GCTCTASCGG CASAMAGTAA
TGAGGATCCG AATTCTT-3'
 
Rev A: 5'-
GGTCGCATAGSTCGGCAGCGGWAAWTCTTKGSCATGASGCRGACGCWKAATTTT
CASAWYAAYTTTKYTCATATGAAGCTTGGTACCAA-3'
 
Rev B: 5'-
GATCAGACCGGTCGGAAYCAGCRYCSTWTCAMMCGGCKYAAKTTYCASCGSWT
YSTYAAKGSMCGCACGCAGATCCAGGCCTG-3'
 
Rev C: 5'-
TTTTATCGATCRKGCCCRSCGCGTTCAGCASCRYTAWGCCMTKTTTCCAGSCCAG
GCCGCTACGCGGC-3'
 
Rev D: 5'-
TAGAGCCAAAGCCGCCTTCGCCACGMTSGGTCTGAGAAAMWTCTTCCACCWSA
WYAAMTTCCRCCYGCWSCACCGGCRCAAWAAYCAKCTGCGCAATACGTTCGCC
C-3'
Rev E: 5'-AAGAATTCGGATCCTCATTACT-3'

The two resulting fragments are sub-libraries that can now be combined using classical molecular biology techniques. For example, the Fwd1/RevA/Fwd2/RevB/Fwd3/RevC fragment (the amino-encoding half) can be cloned using *Nde*I and *Cla*I*.* The Fwd4/RevD/Fwd5/RevE fragment (the carboxyl half) can be cloned using *Cla*I and *Bam*HI*.* The fragments can be cloned separately than combined to form a full-length hybrid library. Alternatively, the fragments can be combined in a single step in a three-fragment forced cloning ligation.

If the vector used in the cloning is an expression vector such as pET11c, the protein can be expressed from the T7 promoter (Studier, et al., Methods in Enzymology 185:60-89, 1990) and protein can be isolated and assayed for the desired characteristic. In this example, a thermal stable parent protein was "mixed" with a mesophilic homologue. One skilled in the art can purify these proteins (Hoffinann, et al., Eur. J Biochem. 164, 45-51, 1987) and assay them for their temperature optima. Differences in the sequences between proteins with different temperature optima will lead to a better understanding of the factors important in protein stabilization at high temperatures.

### Example 2. Generation of hybrid polymerase proteins

Those skilled in the art will recognize that this example represents a much more complex application of the present invention than Example 1. Pfu polymerase is a commercially available (Stratagene, La Jolla, CA) family B DNA polymerase isolated from *Pyrococcus furiosus.* Deep Vent^{®} is a commercially available (New England Biolabs, Beverly, MA) family B DNA polymerase isolated from *Pyrococcus sp.* GB-D. Being 775 amino acids in length, these proteins are twice as large as a typical protein and five times as large as Dut. They share a variety of activities including DNA binding, nucleotide binding, nucleotide addition, pyrophosphorolysis, and 3' to 5' exonuclease (proofreading) activities. The invention can be applied to any one of the activities encoded by these large proteins by being applied to one domain of the protein. In this example, the invention was applied to each of the different enzymatic activities, by making a hybrid library for the entire protein. Thus, this example represents at least two independent tests of the method, for the two activities assayed (polymerase activity and proofreading exonuclease activity).

The protein sequence of Pfu polymerase and Deep Vent polymerase were aligned. A BlastP alignment is shown in Figure 1. The amino acid sequences differ from one another at 115 locations. The sequences are 85% identical over the complete sequence. One 18-amino-acid-region is only 56% identical.

As stated, the alignment found 115 differences between the Pfu and Deep Vent amino acid sequences. The alignment and a consensus hybrid protein sequence, in which X indicates the residues at which the parents differ, are shown in Figure 2. Figure 8 shows the minimal encoding sequence used to generate oligonucleotides encoding a Pfu/ Deep Vent® Hybrid DNA polymerase. The An E. *coli* codon usage table was then used to compare the various codons that can encode the amino acids and deduce an minimal encoding sequence. In many instances, a single nucleic acid degeneracy could encode both amino acids. For example, the parent proteins differ at amino acid position 15 where Pfu has a valine (Val) and Deep Vent an isoleucine (Ile). It is possible to encode Val using GTT and Ile using ATT. The oligonucleotide synthesis machine was therefore programmed to produce product with half G and half A at nucleotide position 43 of the protein-coding DNA. Thus, a codon with a RTT where either a G or an A is introduced into the first nucleotide position of the codon will provide a pool of oligonucleotides, some of which have a GTT at that position; the others of which have an ATT at that position.

In the alignment of Pfu and Deep Vent, 98 of the 115 differences could be simply incorporated into the library by introducing a single degeneracy at one nucleotide residue of the codon that encoded the different amino acids.

The remaining 17 differences required two nucleotides to be changed in order to encode the two parental sequences. These changes forced the possibility that two non-parental amino acid sequences would exist in the resulting library. An example of this is residue 72, at which Pfu has a glutamate (Glu) and the Deep Vent has an arginine (Arg). Glu is encoded by GAR and Arg by CGN or AGR. The minimal encoding sequence (A/G)(A/G)G was selected to potentially encode the parent sequences at position 214 through 216 of the hybrid protein-coding region. This combination will also generate nucleotides encoding glycine (GGG) and lysine (AAG). This situation was determined to be tolerable even though glycine is not similar to either parental amino acid because such situations were rare relative to the size of the protein.

Incorporation of a potential stop codon at amino acid residue 758 (nucleic acid residues 2272 and 2273) was also deemed to be tolerable. This stop codon made 1/4 of the library useless. Amino acid residue 566 (nucleotides 1696 through 1698) was made a lysine by mistake (Figure 8); it should have contained a nucleotide degeneracy that encoded lysine or aspartic acid. Figure 8 shows the minimal encoding sequence used to generate oligonucleotides encoding a Pfu/ Deep Vent® Hybrid DNA polymerase as explained in example 2. The degenerate nucleotides are in parenthesis. The amino acid sequences that differ between the parent proteins (the "mismatches") are indicated. Non-parental amino acids are indicated in bold. Examples mentioned in the text are numbered.

Hybrid Deep Vent^{®}/Pfu proteins were produced by creating a collection of oligonucleotides that encodes a blend of sequences from the two parents and then assembling the oligonucleotides in a library of full-length polymerase proteins. For each strand of the minimal encoding sequence, a set of degenerate oligonucleotides of approximately 100 bases in length, and separated by gaps of 40 bases, was synthesized. The oligonucleotide sequences on the two strands were arranged so that the oligonucleotides from the first strand spanned the gaps on the second strand and overlapped the oligonucleotides of the second strand by 30 bases (Figure 3). This oligonucleotide set was used in assembly PCR as follows. Overlapping oligonucleotides were paired, annealed to each other, and extended using a thermostable high fidelity polymerase. High concentrations of oligonucleotide and a minimal number of thermal cycles (no more than 5) were used. The products of the first cycle were double-stranded fragments of approximately 170 base pairs in length. These fragments were band-purified from a gel and used for the next cycle of pairing and primer extension to generate a new double-stranded fragment of about 310 base pairs in length. This cycle was repeated until the entire sequence was obtained as a collection of fragments of about 500 bases in length. At this point, particular fragments were selected and sequenced to assess the integrity of the procedure. It was found that the oligonucleotides purchased were of low quality, resulting in excessive unintended mutations. A number of segments containing no unintended mutations were chosen and used to assemble full-length genes using restriction sites that had been incorporated at the ends of each fragment and conventional molecular biology techniques. Four full-length clones were assembled and the encoded proteins were expressed in pET11 (Novogene, Madison, Wi). Expression by all four clones was confirmed by SDS-PAGE. These clones were names Hyb1 to Hyb4.

A second collection of libraries was constructed on a custom basis by Blue Heron Biotechnology (Bothell, Washington) using "Genemaker" technology. The complete coding sequence was delivered as four fragment libraries that could be assembled into a full-length hybrid genes. Two full-length assembled clones were obtained and sequenced to verify validity of the library. These clones were named Phy1 and Phy2. Clones from this library contained only proper hybrid sequences including the degeneracies at position 566 (lysine/aspartic acid) and 758 (tyrosine/tryptophan) discussed earlier. The full-length sequences were cloned into expression vectors and protein of the expected size were produced.

Hybrid polymerase protein was expressed and purified from each of the six clones from the two libraries. Purification was performed as follows.

### Purification of hybrid polymerases

This section describes methodology for isolating a hybrid polymerase. Following induction of expression in E. *coli,* the cells were centrifuged and the pellets stored at -20°C to -80°C. One milliliter of Buffer A (Buffer: 50 mM Tris (8.0); 50 mM Dextrose; 1 mM EDTA) was added for every 100 ml of starting culture and the cells were lysed with 4 mg/ml of powdered lysozyme at 72°C. MgCl₂ and CaCl₂ were added to a concentration of 2 mM, followed by the addition of 1 unit/ml of DNase I. The sample was shaken slowly for 10 min at room temperature. One ml of Buffer B (10 mM Tris (8.0); 50 mM KCl; 1 mM EDTA; 0.5% Tween 20; 0.5% NP40) was added per 100 mls starting culture and the sample then shaken slowly at room temperature for 15 min. The sample was transferred to a centrifuge tube and incubated at 72°C for 1 hour followed by centrifugation at 4000 x g at 4°C for 15 min. The supernatant was collected and 0.476 gm/ml of (NH₄)₂SO₄ was added and the sample was mixed slowly at 4°C for 1 hour and then centrifuged at 15,000 x g at 4°C for 15 min.

The pellet was resuspended in, and dialyzed against HiTrap Q 'A' Buffer (20 mM Tris (7.9); 50 mM NaCl; 5 mM β-mercaptoethanol). The suspension was then loaded onto a ÄKTAprime HiTrap Q chromatography column (Amersham Biosciences) equilibrated and run using method #2 per the manufacturers instructions using HiTrap Q buffers 'A' and 'B' ('A' buffer with 1 M NaCl). Fractions containing the polymerase were combined and dialyzed against P-11 Loading Buffer (20 mM Tris (7.9); 50 mM NaCl). The sample was bound to a liquid chromatography column of P-11 resin (Amersham Biosciences), washed with P-11 Buffer 'B' (20 mM Tris (7.9); 150 mM NaCl), then eluted using P-11 Elution Buffer (20 mM Tris (7.9); 400 mM NaCl). The eluted fractions were dialyzed against HiTrap SP 'A' buffer (20 mM Tris (6.8); 50 mM NaCl; 5 mM β-mercaptoethanol) then injected onto a ÄKTAprime HiTrap SP chromatography column equilibrated and run using method #2 per the manufacturers instructions using HiTrap SP 'A' and 'B' Buffer ('A' buffer with 1 M NaCl). Fractions containing PhS1 were concentrated using a YM-30 Centricon protein concentrator (Millipore). The sample was then dialyzed against buffer containing 50 mM Tris (pH 8.2); 0.1 mM EDTA; 1mM DTT; 0.1% NP40; 0.1% Tween 20. The final volume was then measured and 1.47X 85% glycerol, and 0.015X 10% NP-40 and 10% Tween 20 added. The sample was stored at-20°C.

Of the six hybrid polymerase proteins generated from the two libraries, all had DNA polymerase activity.

Sso7d fusion polymerases (*see, e.g.,* WO0192501) were prepared using some of the hybrid polymerase proteins and compared to the parental Pfu polymerase with and without Sso7d (designated as "Pfu" and "PfS", respectively) in exonuclease assays and extension assays. Sso7d fusions of Hyb clones are designated HyS; Sso7d fusions of the Phy clones are designated PhS. The most thoroughly studied hybrid protein was PhS1.

To measure exonuclease activity, a 45 base long primer with the following sequence was synthesized: 5'-FAM-TTTTTTGAGGTGTGTCCTACACAGCGGAGTGTAGGA CACACCTCT* 3', wherein T*= is an amino-link dT with the quencher, DAB (dabcyl) attached. The sequence forms a 16 base pair stem loop structure with a T:T* mismatch at the quencher-labeled base. The 5' unbase-paired poly T sequence keeps FAM (6 carboxy-fluorescein) in close proximity to the quenching dye so the FAM, if excited, it will not fluoresce.

The oligonucleotide was combined with buffer and the enzyme and incubated in a real time detection instrument, the DNA Engine Opticon System (MJ Research, Inc.). This instrument excites the FAM and detects any fluorescence if present. In the absence of 3' to 5' exonuclease activity, there is only background fluorescence because FAM is quenched by DAB. However if the enzyme does have 3' to 5' exonuclease activity, the T:T* mismatch is recognized and the 3'-T* is removed. The DAB is released and will no longer quench the FAM fluorescence. The Opticon System will detect the increase in fluorescence with increasing time (readings were taken every 10 sec at 65°C). The rate of fluorescence increase directly reflects the amount of 3' to 5' exonuclease activity. An increase in fluorescence greater than control levels shows that the enzyme has 3' to 5' exonuclease activity. The results (Figure 9) of this analysis are discussed below.

Figure 10 shows results of a comparison of a hybrid and a parent polymerase in extension assays. Even with excess enzyme (80 U/ml), Pfu could not amplify any amplicon longer than 2 kb. An Sso7d fusion to Pfu polymerase (PfS) amplified a 10 kb fragment given a 1 min extension time. PhS1 amplified a 15 kb fragment (arrow) in 80 mM KCl with a 1 minute extension time. Further, PhS1 was also able to perform long PCR under a variety of salt conditions.

### Characterization of additional hybrid polymerases

Five additional hybrid clones were isolated from the second library directly as Sso7d fusions and were designated PhS3 to PhS7. The polymerases were tested for polymerase and exonuclease activity. Table 1 summarizes characteristics of the various hybrid proteins analyzed in this example. PhS2 has two mutations at sites other than a target site. PhS3 is truncated due to an early stop codon. PhS4 has one deletion and one mutation. The "Hyb" and "HyS" polymerases also comprise mutations at positions other than the target sites, probably due to faulty oligonucleotide synthesis.

**Table 1**

| Pol | Activity | Full-length | KCL Opt | Temp. Stab. | Processivity | Number Pfu parent residues | Number D. vent parent residues | Relative specific activity |
|---|---|---|---|---|---|---|---|---|
| PhS 1 | Yes | Yes | 80-100 mM | 3 hr, 97.5 | 26-30 | 55 | 60 | 1.5 |
| PhS2 | Yes | Yes | 160-180 mM | 3 hr+, 97.5 | 24-28 | 64 | 51 | 4 |
| PhS 3 | No | No | N/A | N/A | N/A | N/A | N/A | n.d. |
| PhS 4 | No | No; minus one Pfu/DV amino acid | N/A | N/A | N/A | 56 | 58 | n.d. |
| PhS 5 | Yes | Yes | 40-80 mM | 3 hr, 97.5 | nd | 52 | 63 | 1 |
| PhS 6 | No | No | N/A | N/A | N/A | 55 | 60 | n.d. |
| PhS 7 | Yes | Yes | 40-80 mM | 3 hr, 97.5 | nd | 54 | 61 | 2 |
| | | | | | | | | |
| Hyb1 | Yes | Yes | nd | 10 min* | 2-4 nt | 59 | 46 | n.d. |
| Hys1 | Yes | Yes | 90-100mM | 8-14 min* | 11 nt | 59 | 46 | 2 |
| Hyb2** | Yes | No | nd | n.d. | n.d. | 50 | 53 | n.d. |
| Hyb3** | Yes | No | nd | n.d. | n.d. | 51 | 47 | n.d. |
| Hys4 | Yes | Yes | 80-90 mM | < 1min* | n.d. | 51 | 50 | n.d. |

| | | | | | | | | |
|---|---|---|---|---|---|---|---|---|
| All polymerases designated "PhS" are Sso7d fusions. "Hys1" is Hyb1 with Sso7d at the C-terminus. "Hys4" has Sso7d at the C-terminus. | | | | | | | | |

The exonuclease activity of various hybrid polymerases was also evaluated as described above. The polymerase to 3'-exonuclease ratios for several commercially available enzymes, including the parental proteins and isolates from the hybrid library, were compared. DyNAzyme EXT, an enzyme used in long accurate PCR, is a blend of a Family B polymerase with 3' to 5' exonuclease activity, and a Family A polymerase that lacks any proofreading activity. Too much exonuclease activity is detrimental because it digests primers instead of extending them. Pfu and Deep Vent are the parental Family B polymerases which both have high exonuclease activity. Pfs (a Pfu-Sso7d fusion enzyme) has increased polymerase activity. HyS1, PhS1, PhS2, PhS5, and PhS7 are isolates from the hybrid libraries. Surprisingly, the results (Figure 9) show that the hybrid proteins vary greatly in their polymerase to exonuclease activities, both relative to the parent proteins and each other. PhS1 has a polymerase to exonuclease activity ratio approaching that of the enzyme blend.

A comparison of the sequences of the parent and hybrid proteins is presented in Figure 11. As can be seen, a signature sequence, *i.e*., an invariable sequence element, is present in all of the proteins. This element (Figure 12) contains the nucleotide binding motif and is characteristic of Pfu/DeepVent polymerases generated using the method described herein. The sites that differ between the parent polymerases are indicated.

These results show that multiple polymerase hybrid isolates from two different libraries were active. Furthermore, the example shows that the method also allows for generating hybrids for different domains, *i.e*., polymerase activity domain vs. exonuclease activity domain. Clearly, the invention could be applied to proteins with very divergent activities.

### Substantially Identical Polymerase Gene Synthesis.

The following is a preferred method of generating polymerase nucleic acids encoding polymerases substantially identical to a polymerase of the invention, *e.g*., SEQ ID N0:2 or SEQ ID N0:4. A set of conservative substitutions are chosen. A degenerate sequence is constructed, where the degenerate positions in the nucleotide encode, in their alternative forms, at least the two amino acids corresponding to the wild-type amino acid and the conservative substitution. For each strand of the degenerate sequence, a set of degenerate oligonucleotides of approximately 100 bases in length, and separated by gaps of 40 bases, is synthesized. The oligonucleotide sequences on the two strands are arranged so that the oligonucleotides from the first strand span the gaps on the second strand and overlap the oligonucleotides of the second strand by 30 bases. This oligonucleotide set is used in assembly PCR as follows. Overlapping oligonucleotides are paired, annealed to each other, and extended using a thermostable high fidelity polymerase. High concentrations of oligonucleotide and a minimal number of thermal cycles (no more than 5) are used whenever possible. The products of the first cycle are double-stranded fragments of length approximately 170 bases. These are band-purified from a gel and used for the next cycle of pairing and primer extension to generate new double-stranded fragments of length approximately 310 bases. This cycle is repeated until the entire sequence has been obtained in a single fragment. If at any point the quantity of the product becomes too low, the amount can be increased by PCR using short (15-30) base primers corresponding to the ends of particular desired fragments. Cloning of partial gene sequences, and/or cutting with restriction enzymes and ligating subfragments together, are additional techniques that may be used to improve the efficiency of the gene construction process. When the entire gene is synthesized, it is cloned into a vector suitable for protein expression. Because the sequence is degenerate, cloning will produce a library of related but different clones, which must be screened to eliminate those clones that do not produce a functional protein or which are not substantially identical to the target polymerase.

It is understood that the examples and embodiments described herein are for illustrative purposes only and that various modifications or changes in light thereof will be suggested to persons skilled in the art and are to be included within the spirit and purview of this application and scope of the appended claims.

All publications, patents, and patent applications cited herein are hereby incorporated by reference in their entirety for all purposes.

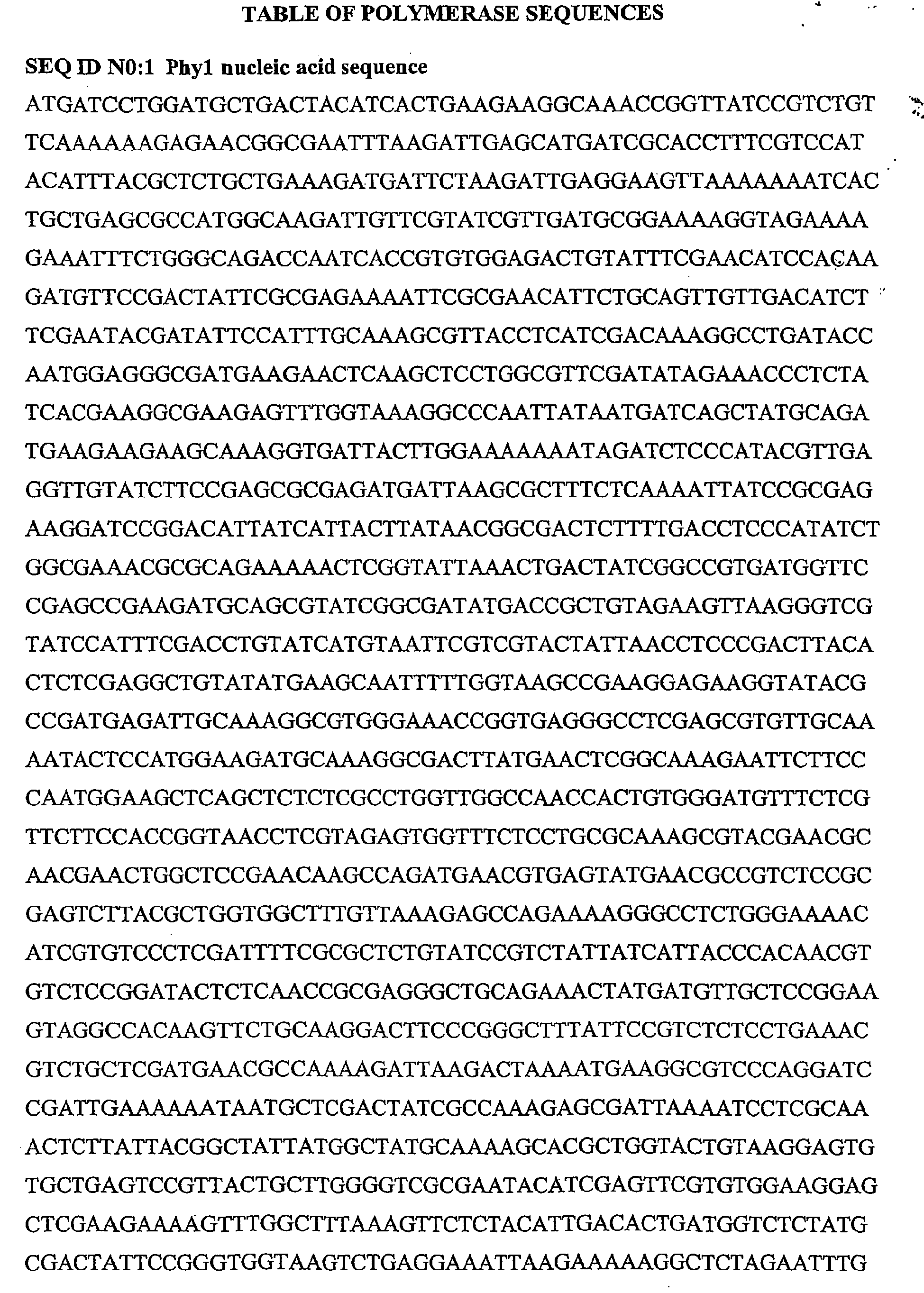

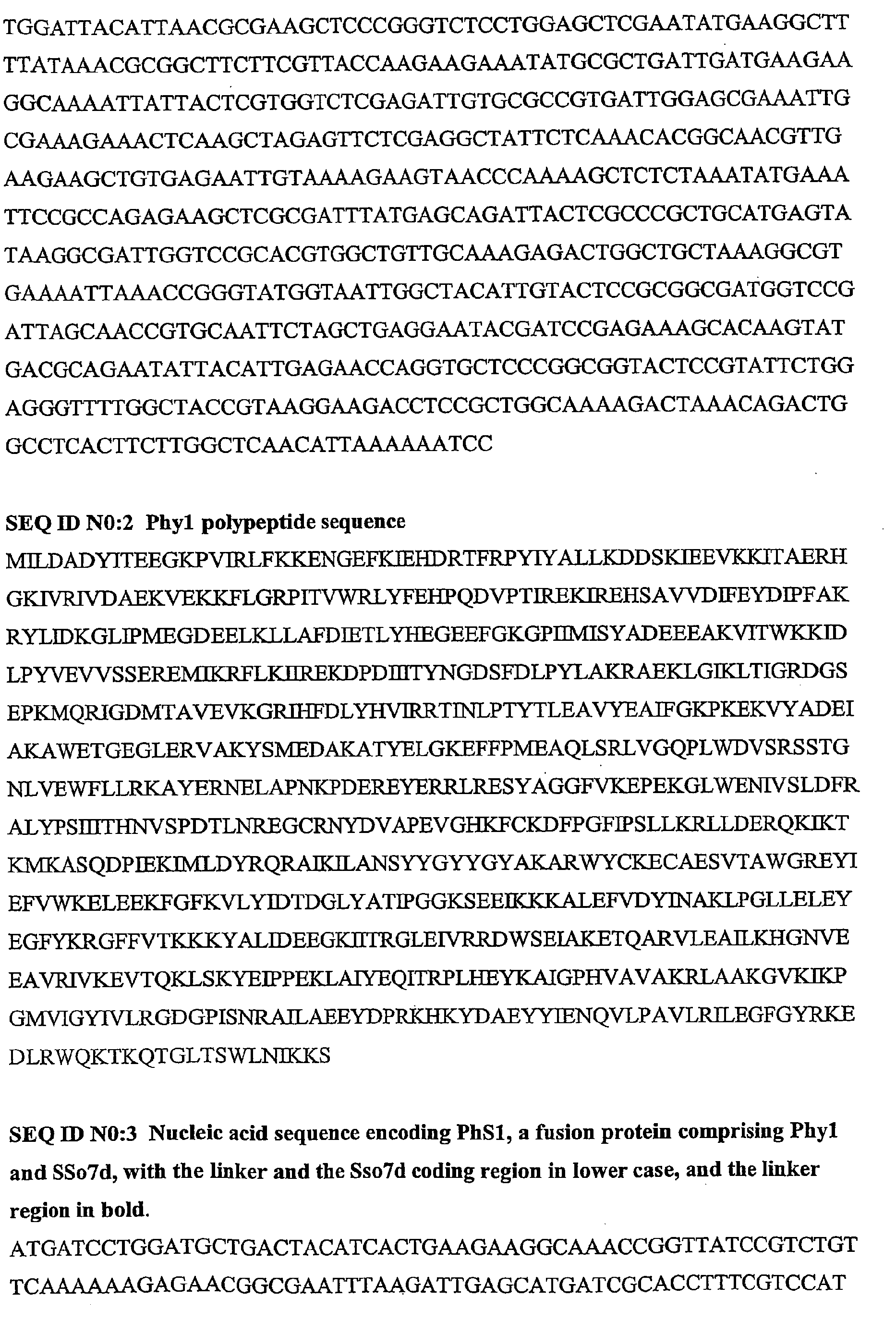

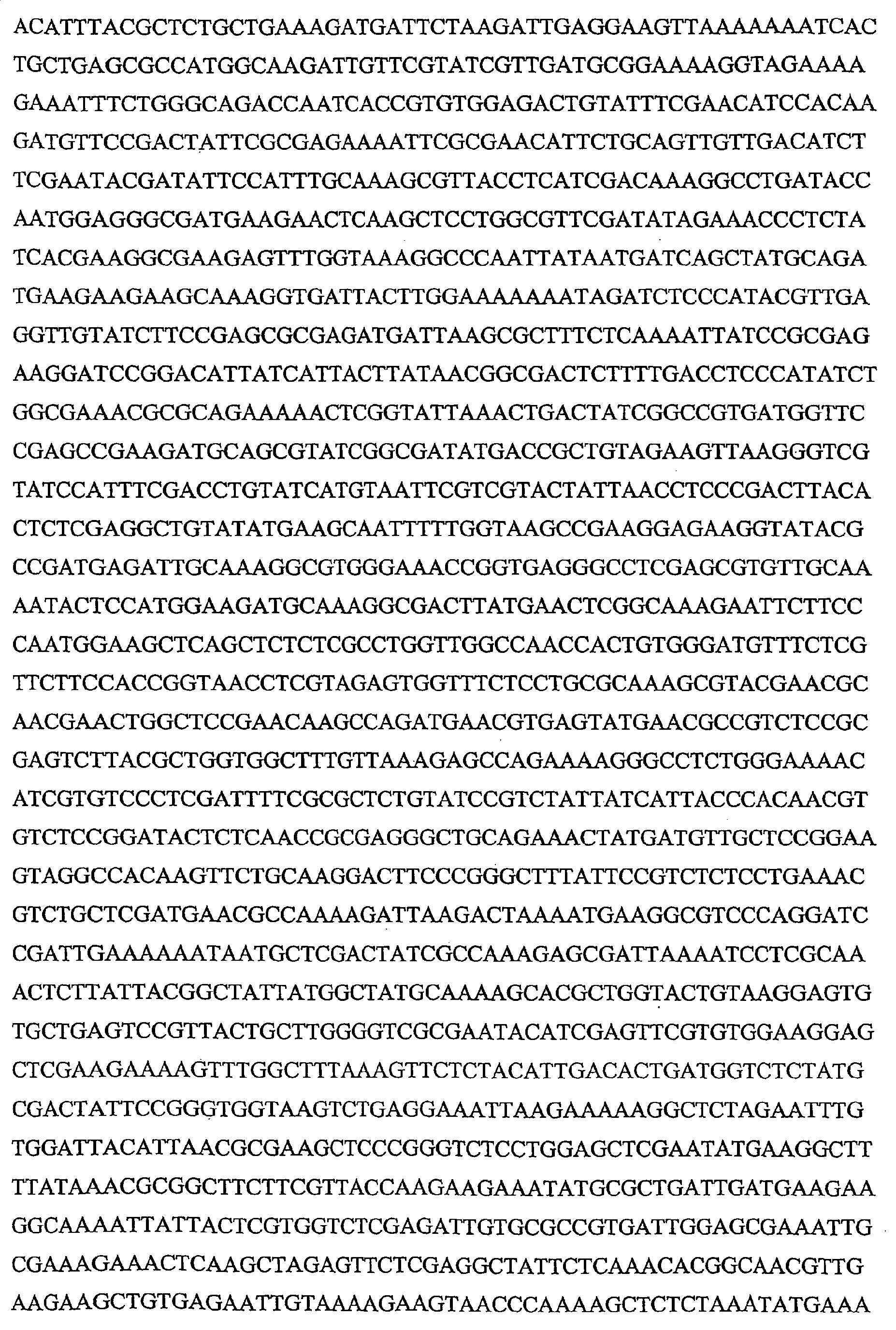

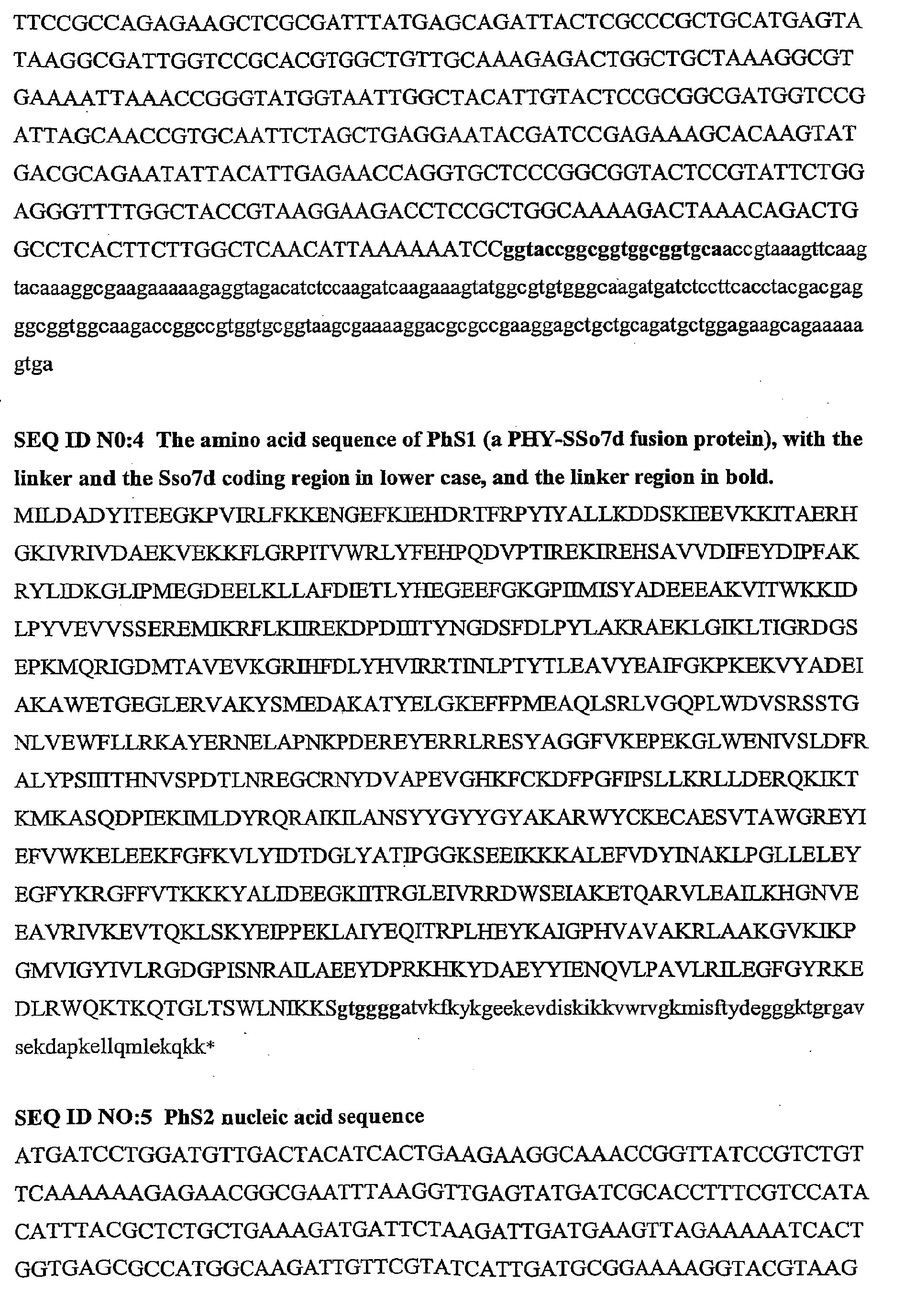

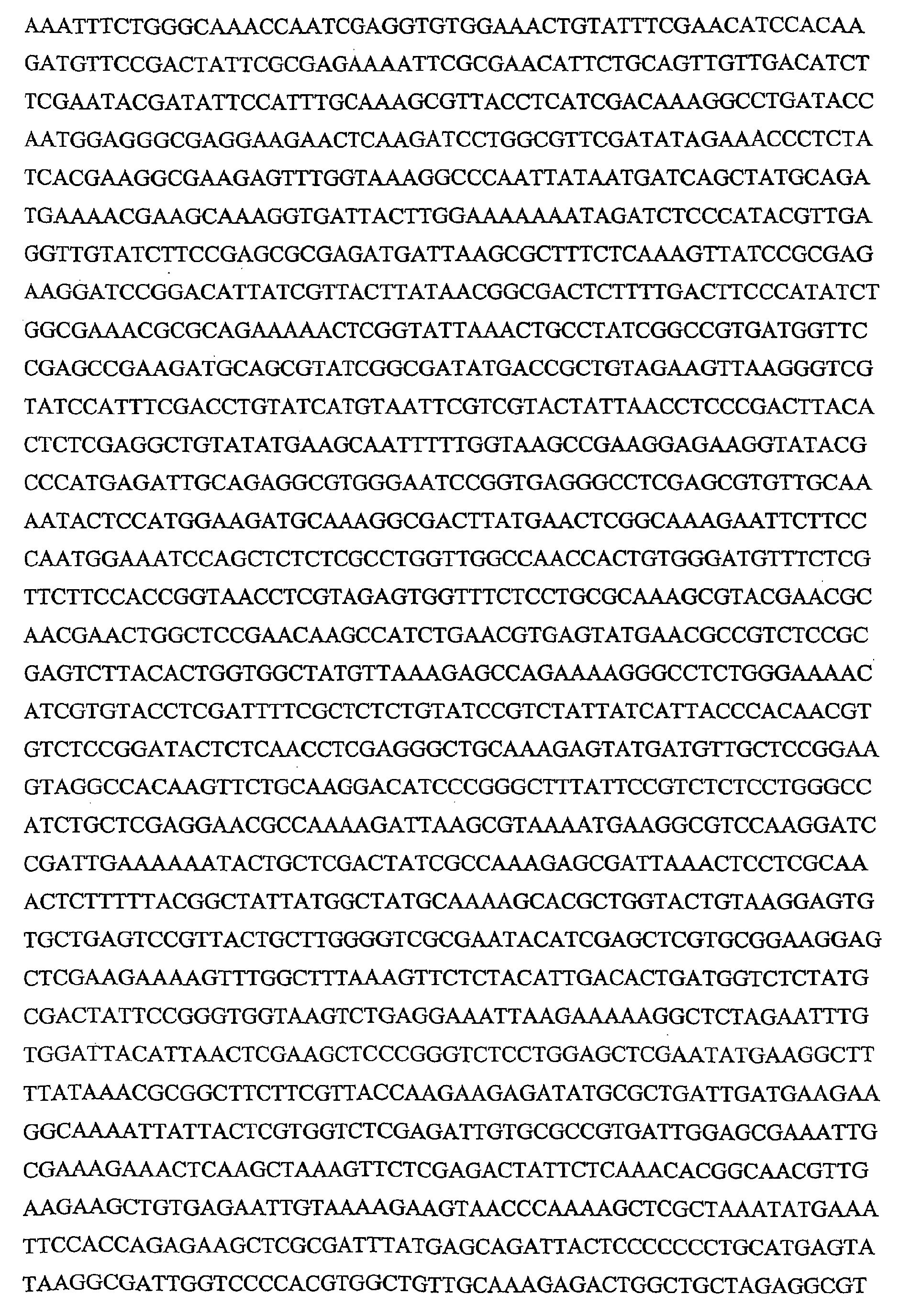

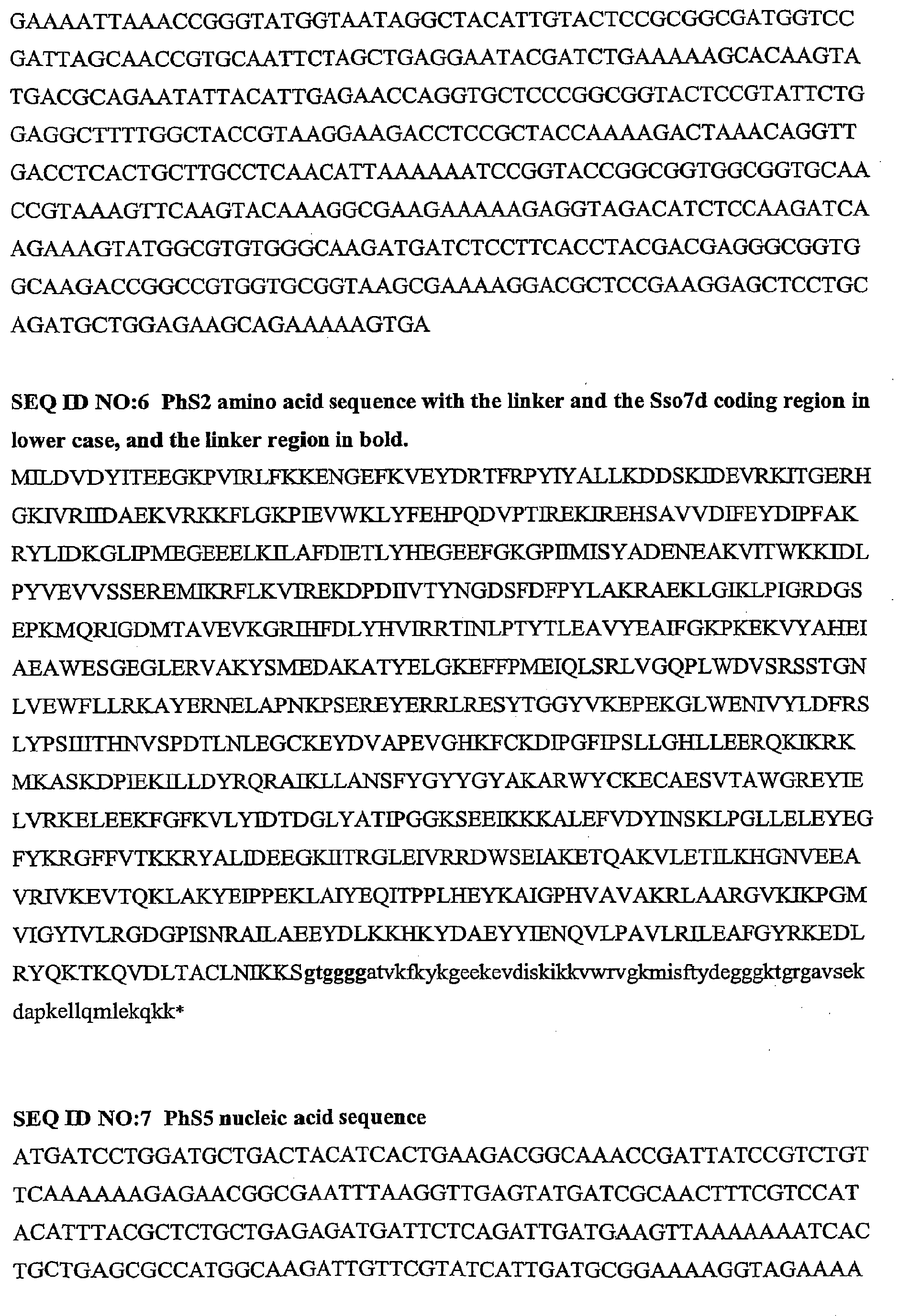

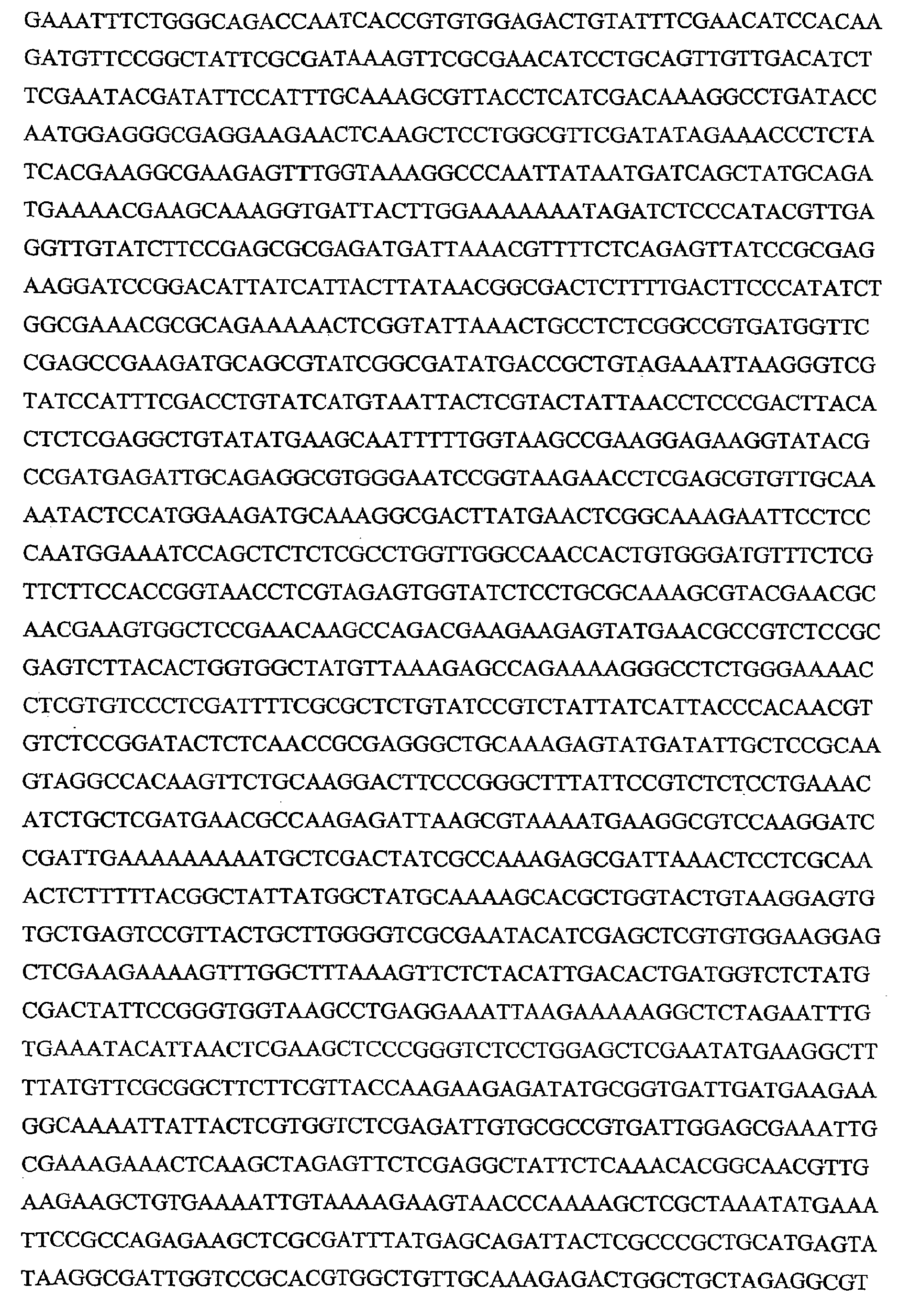

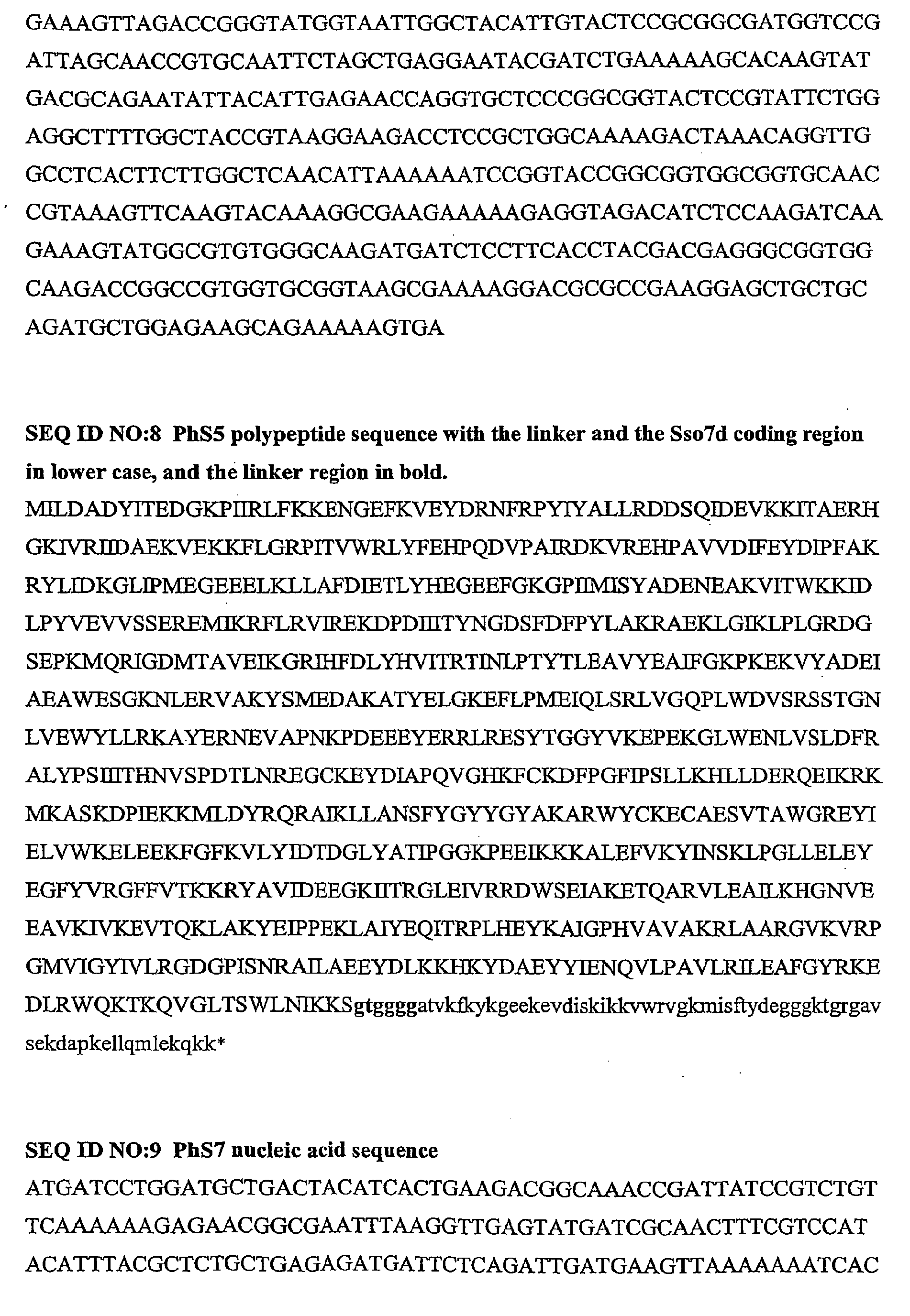

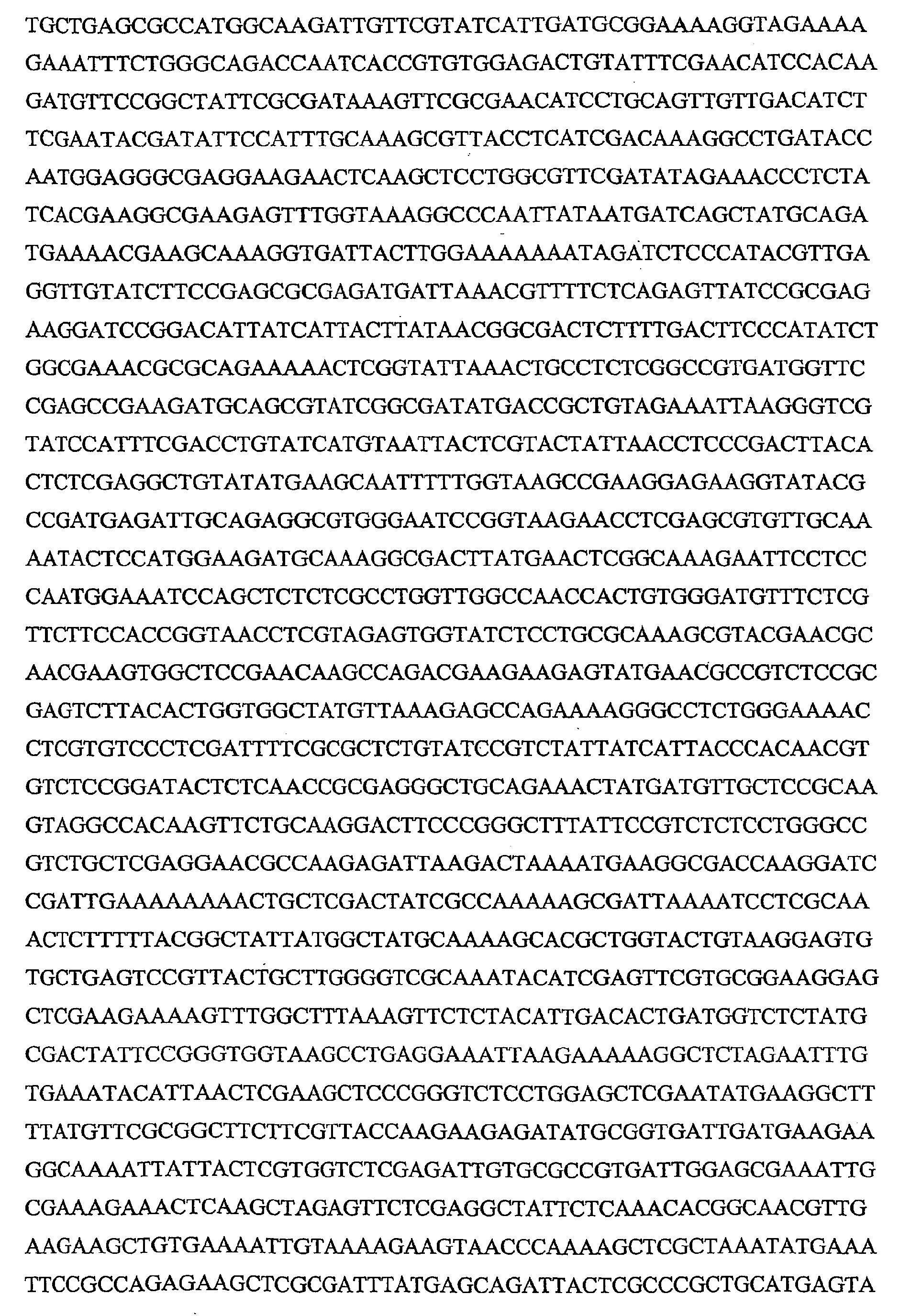

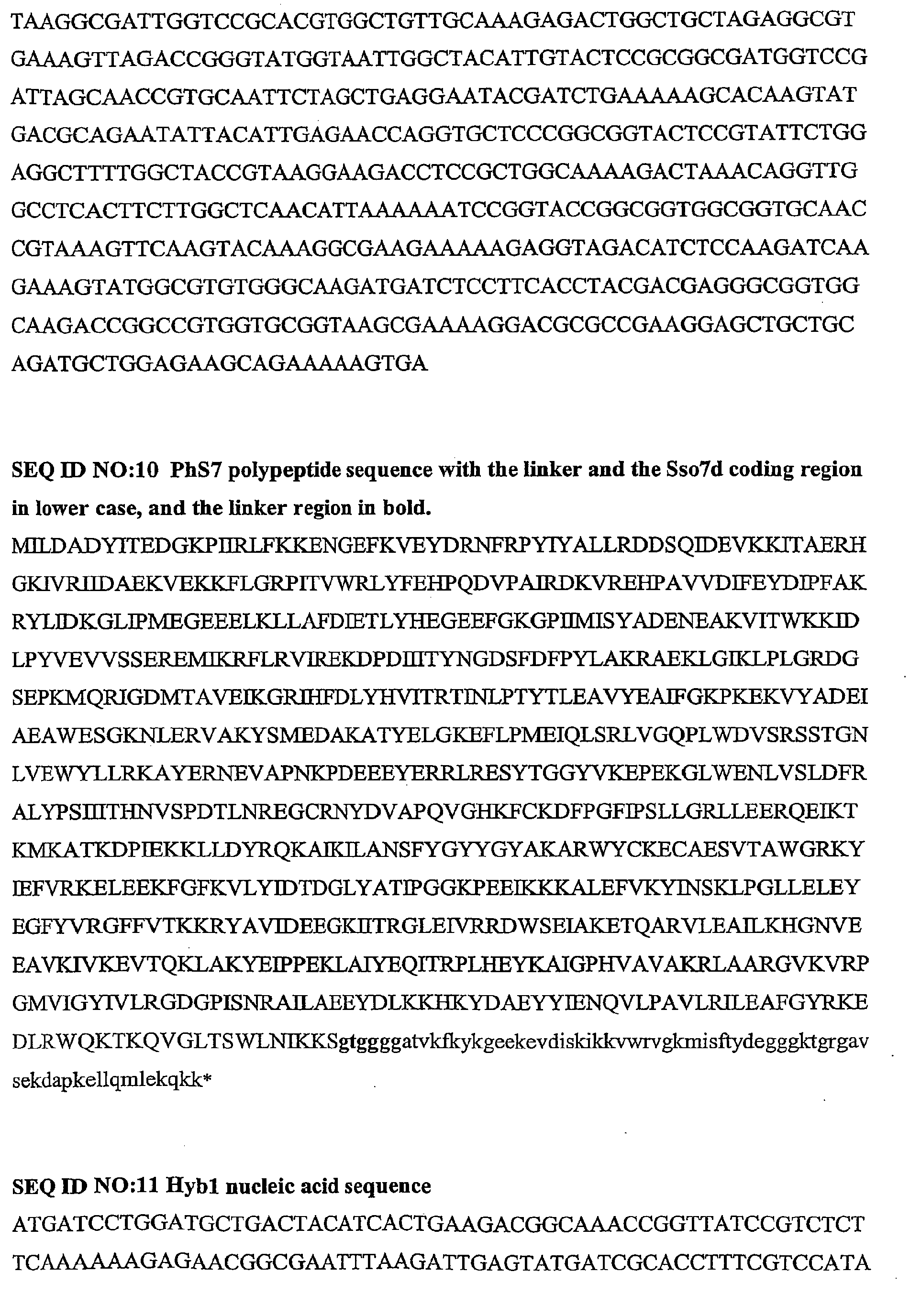

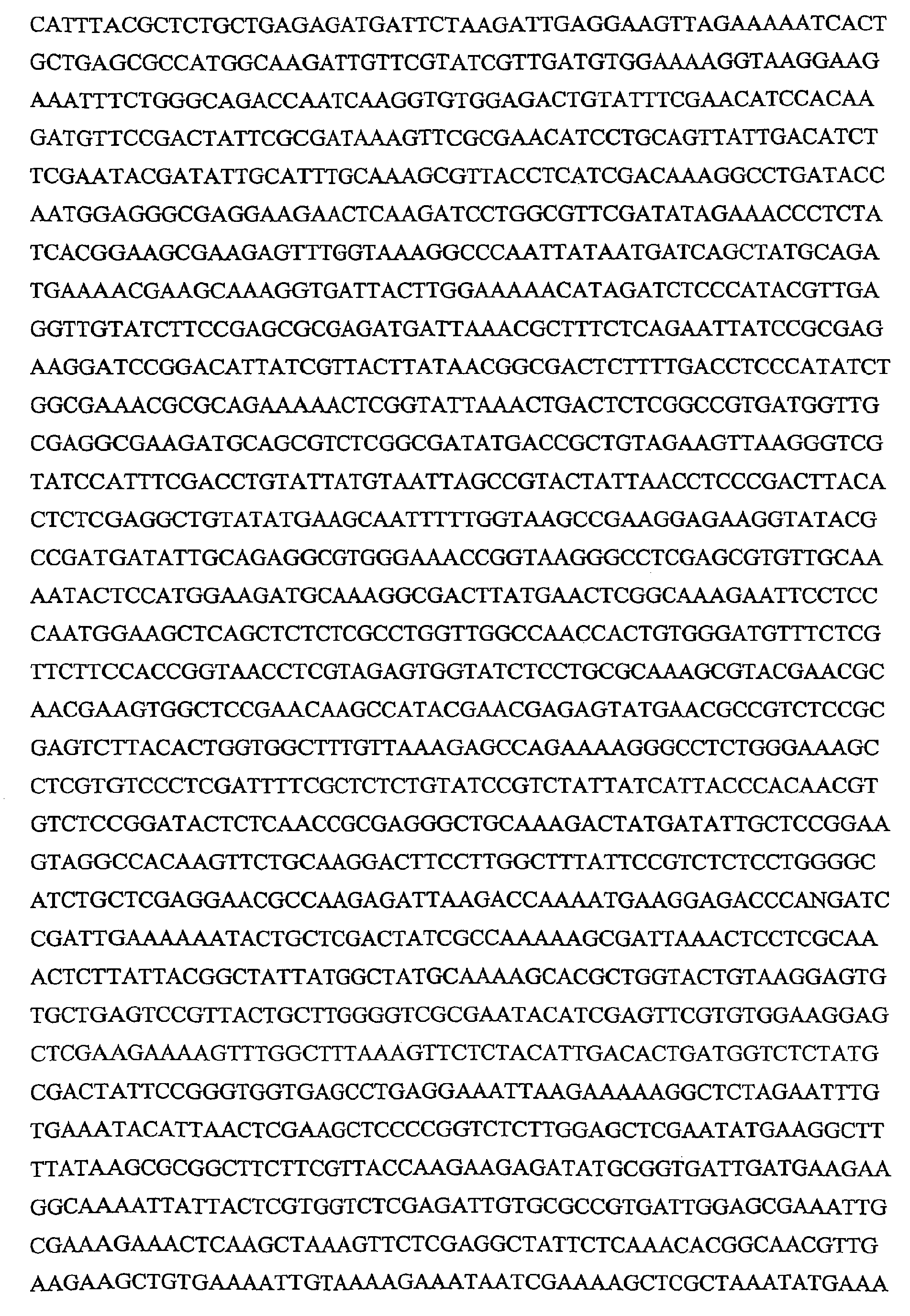

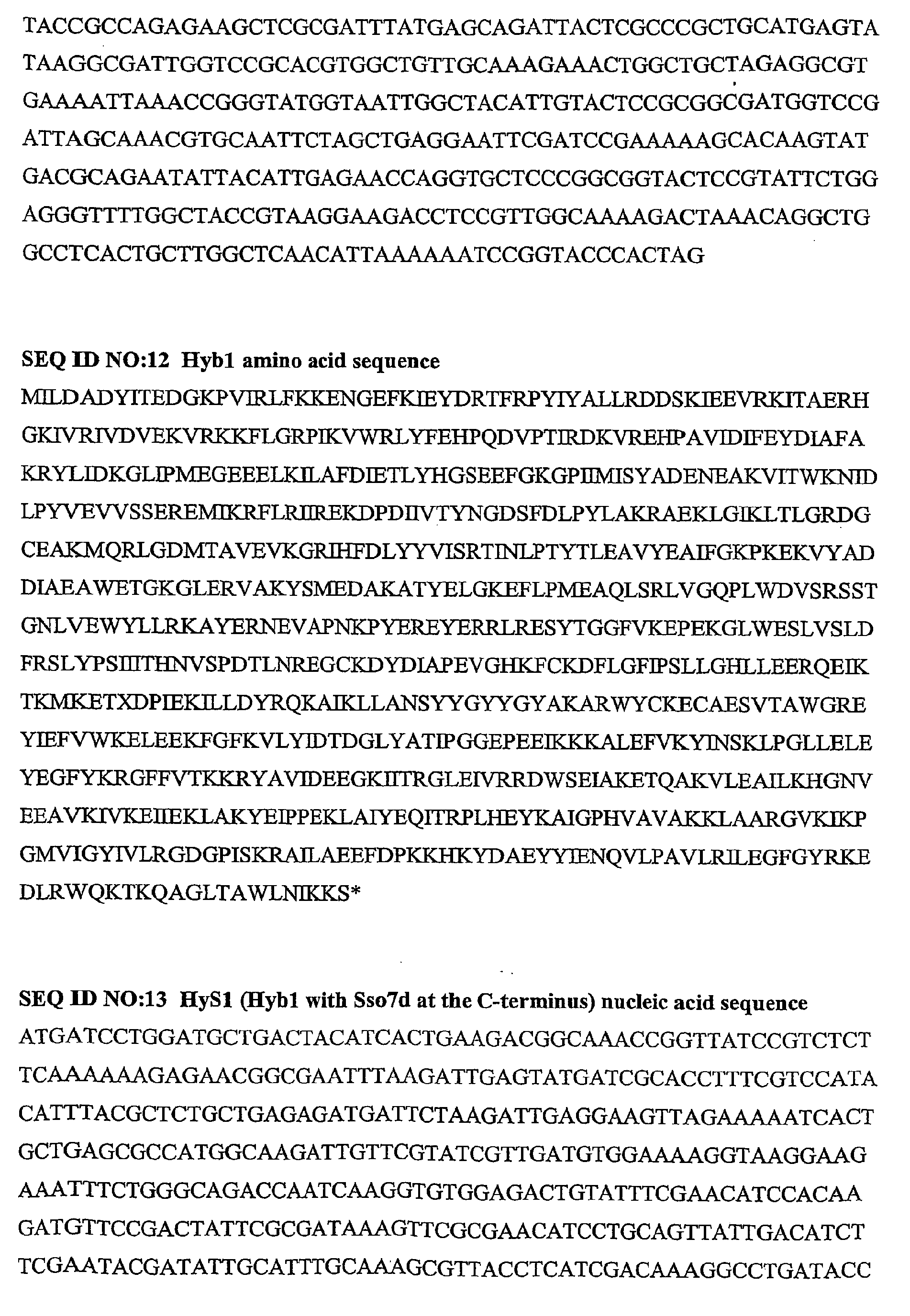

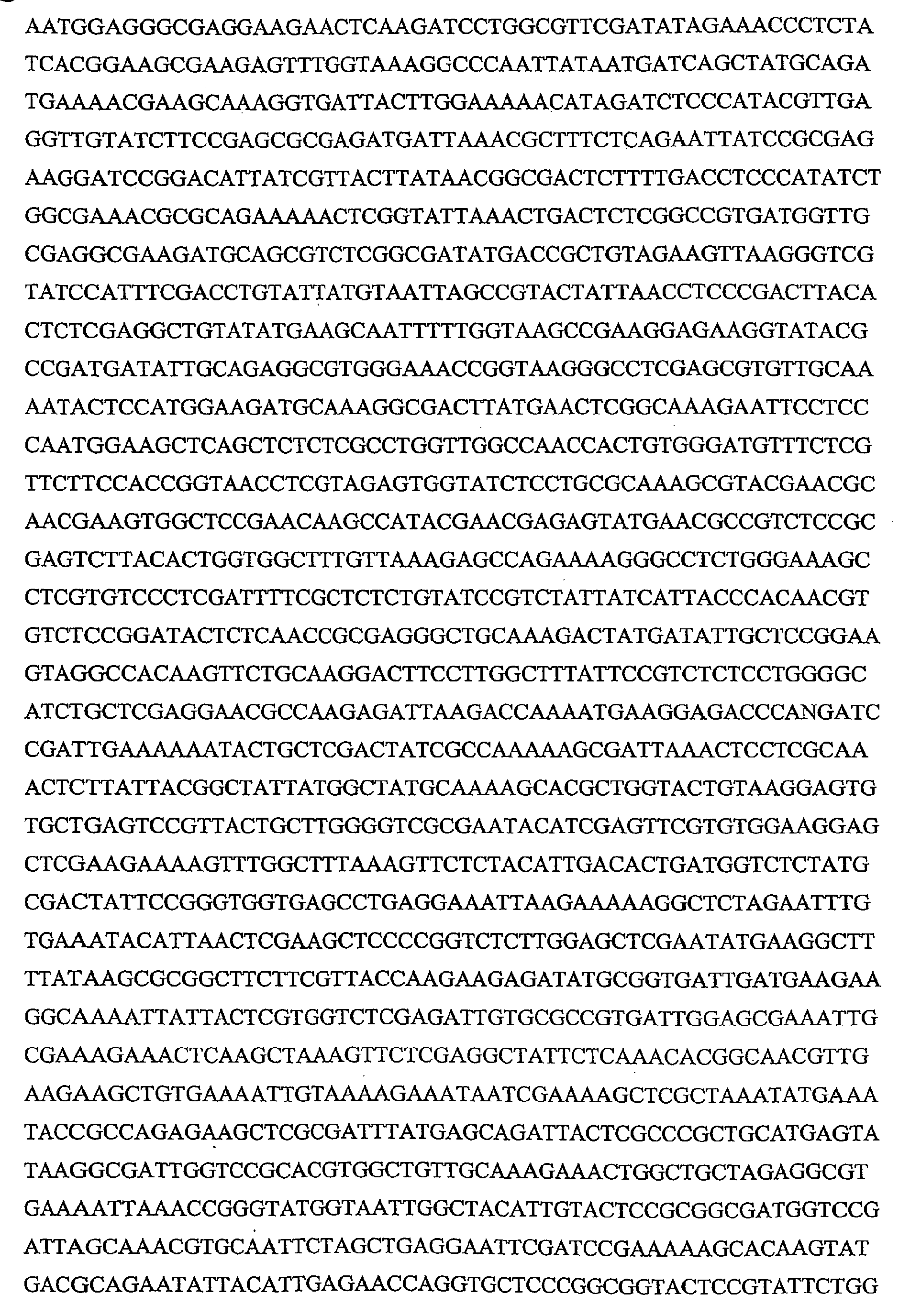

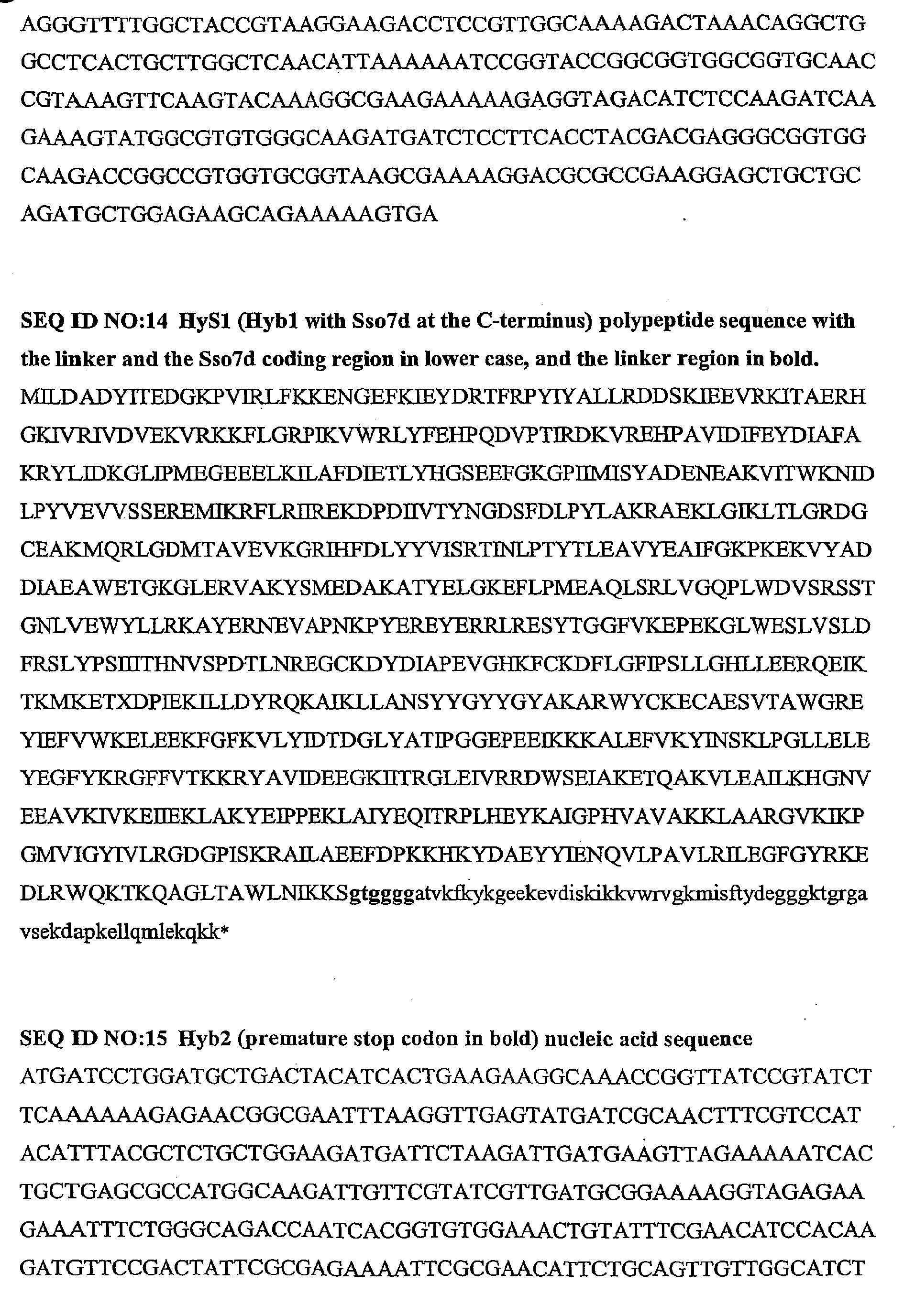

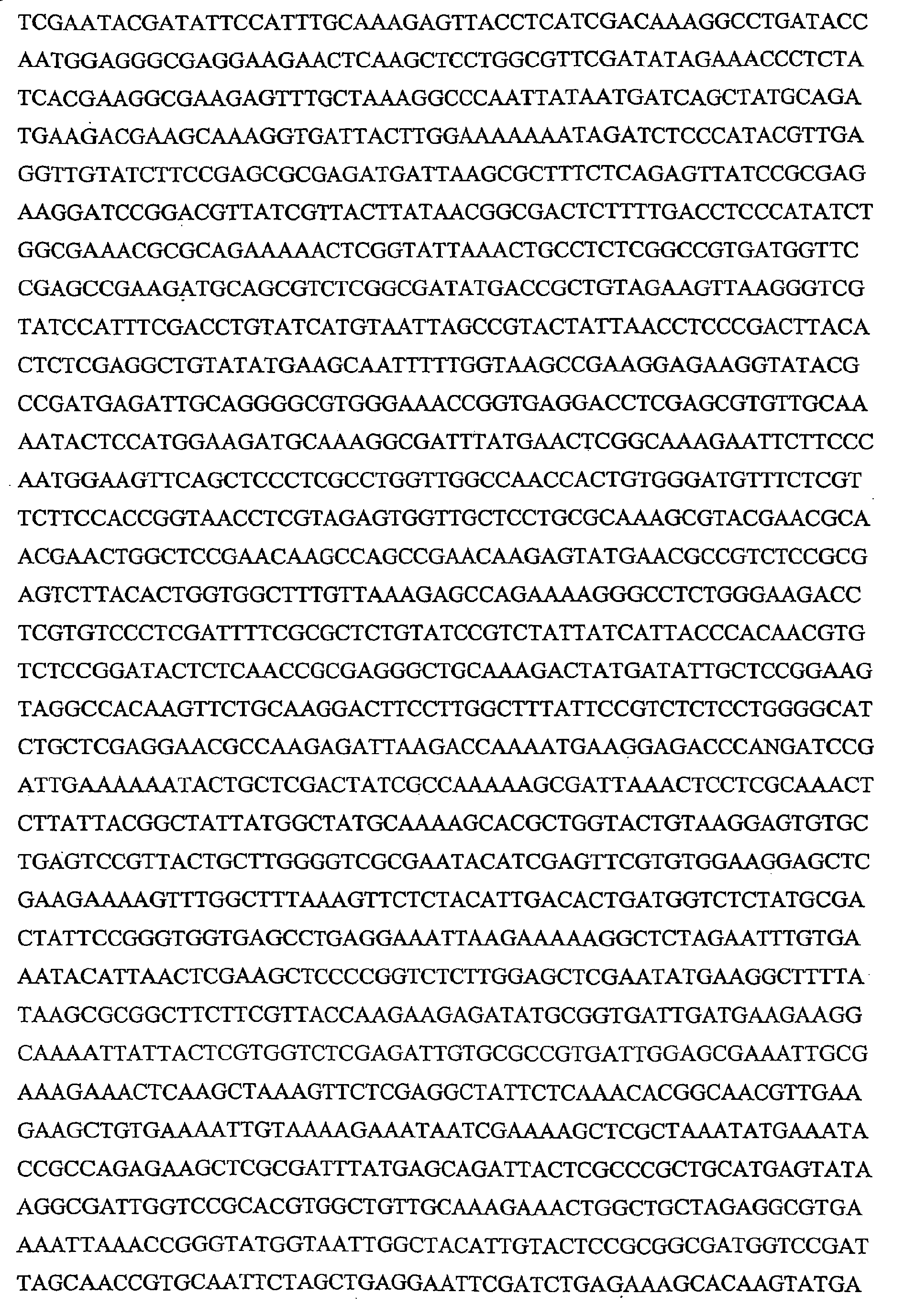

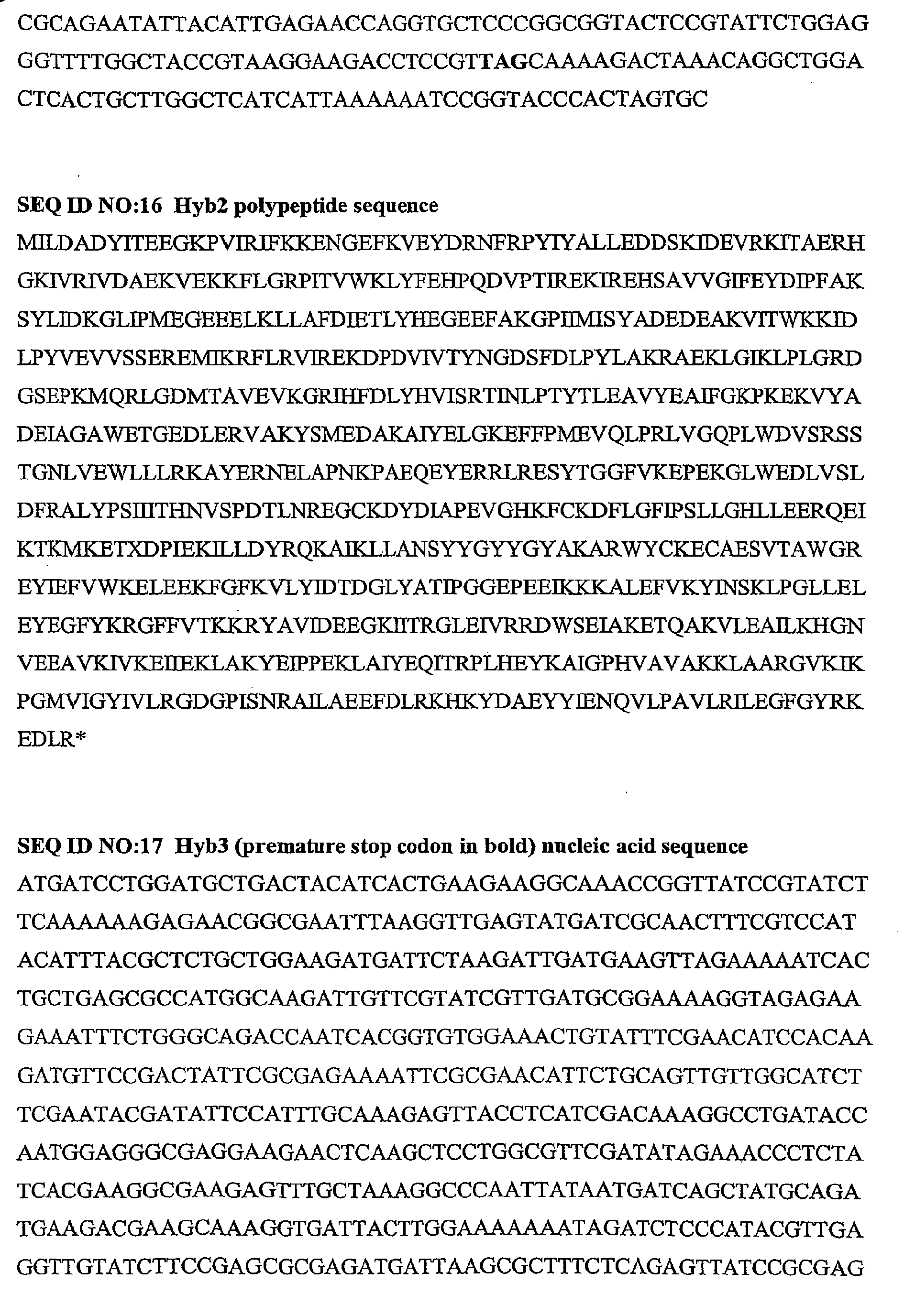

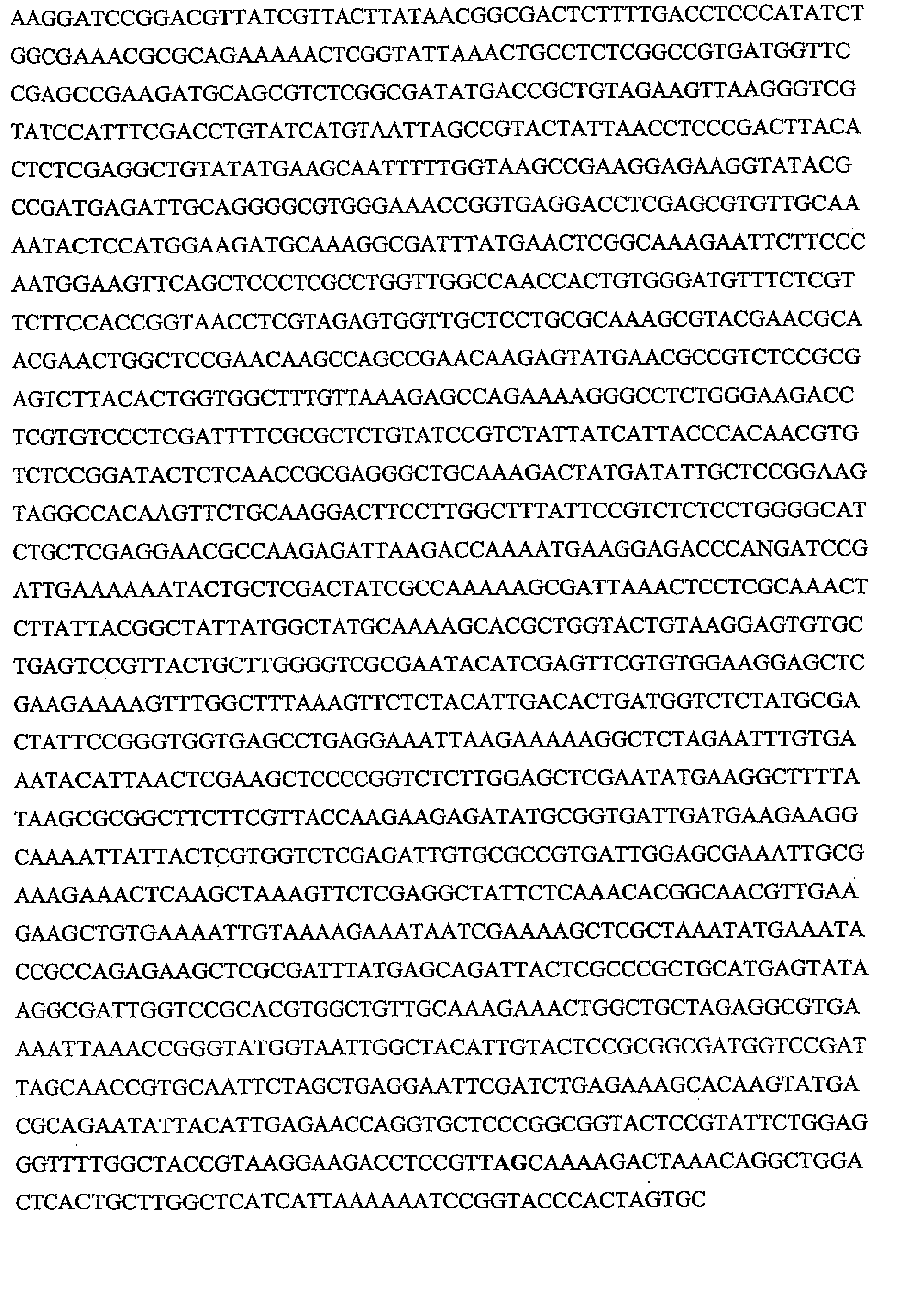

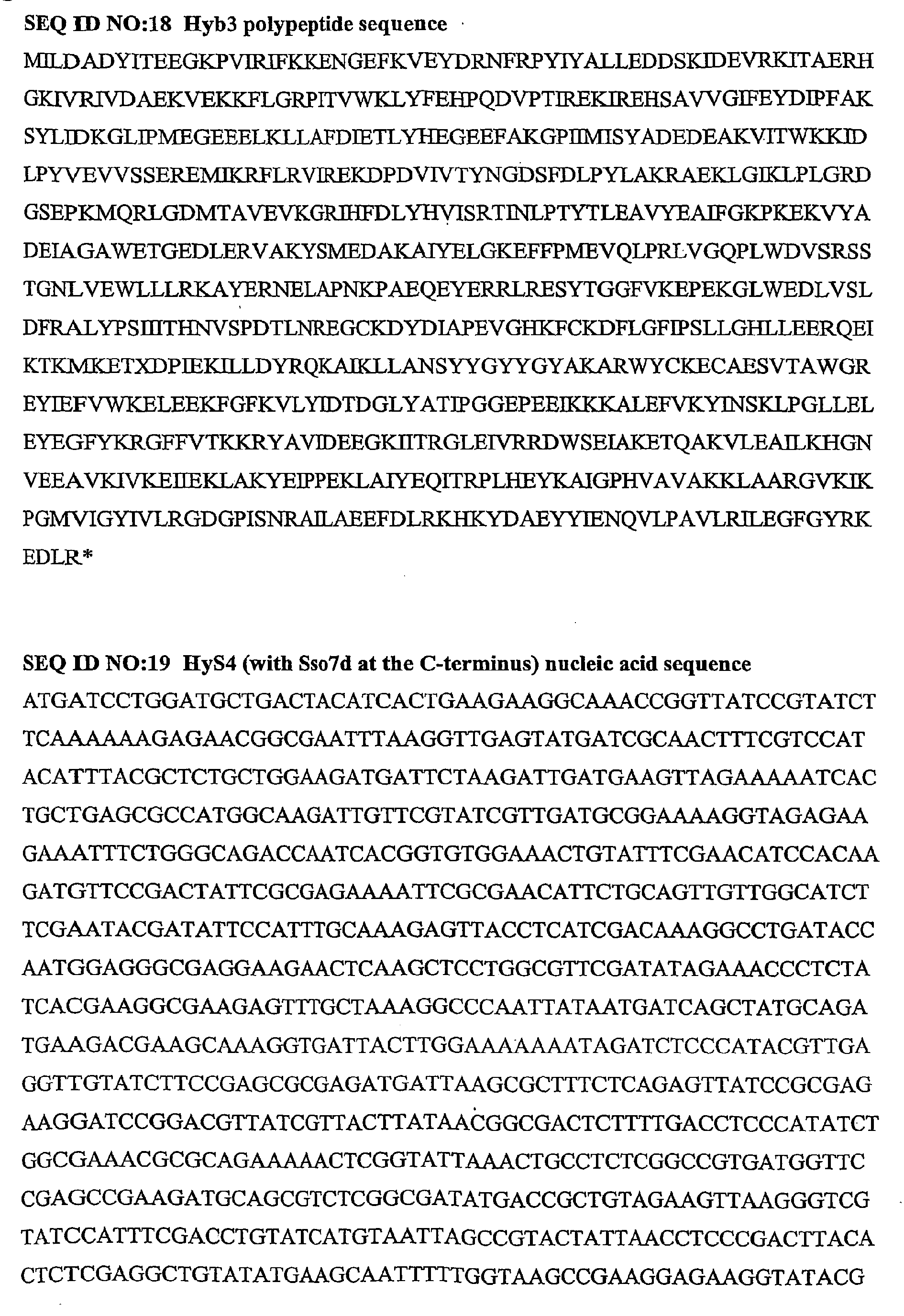

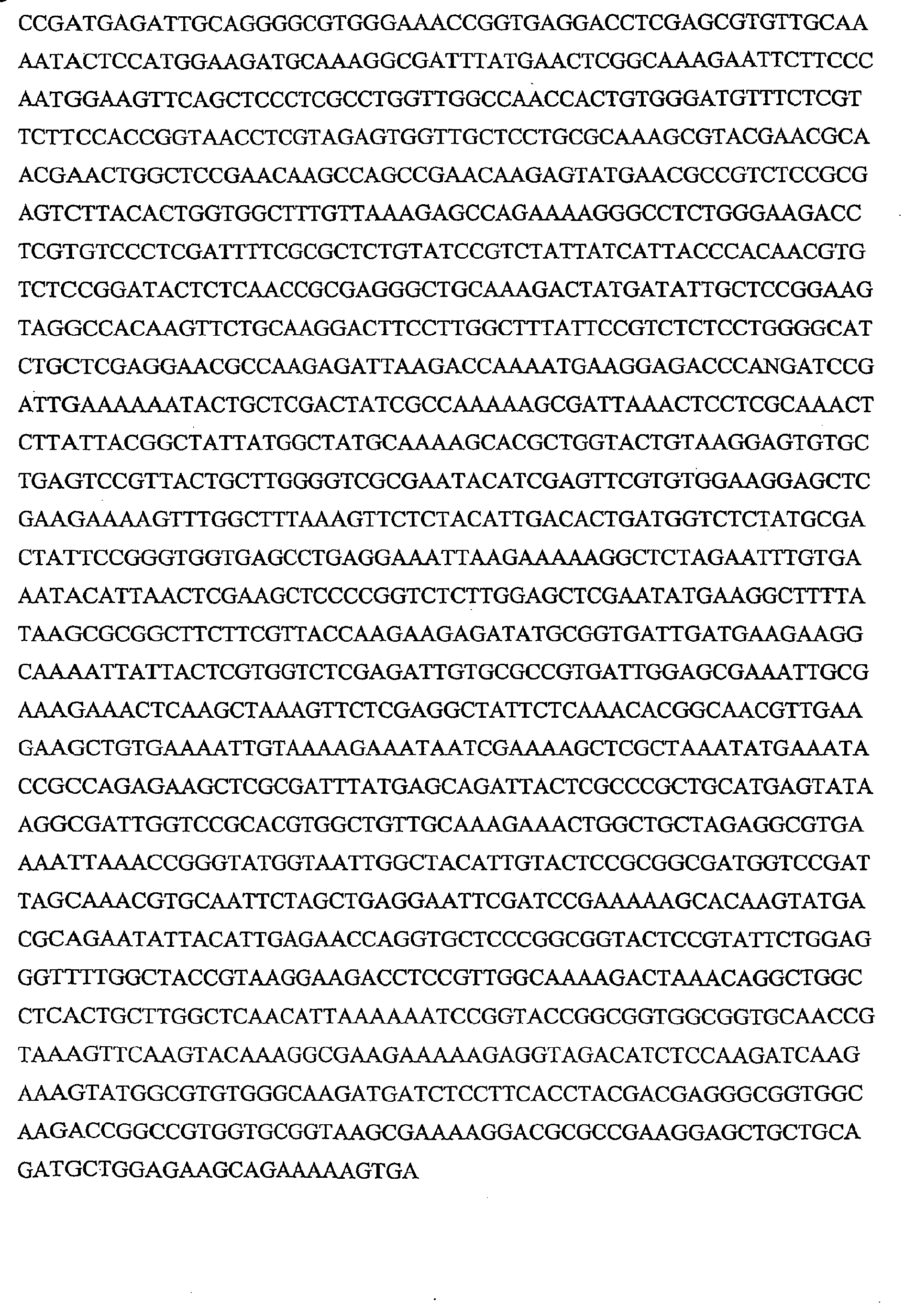

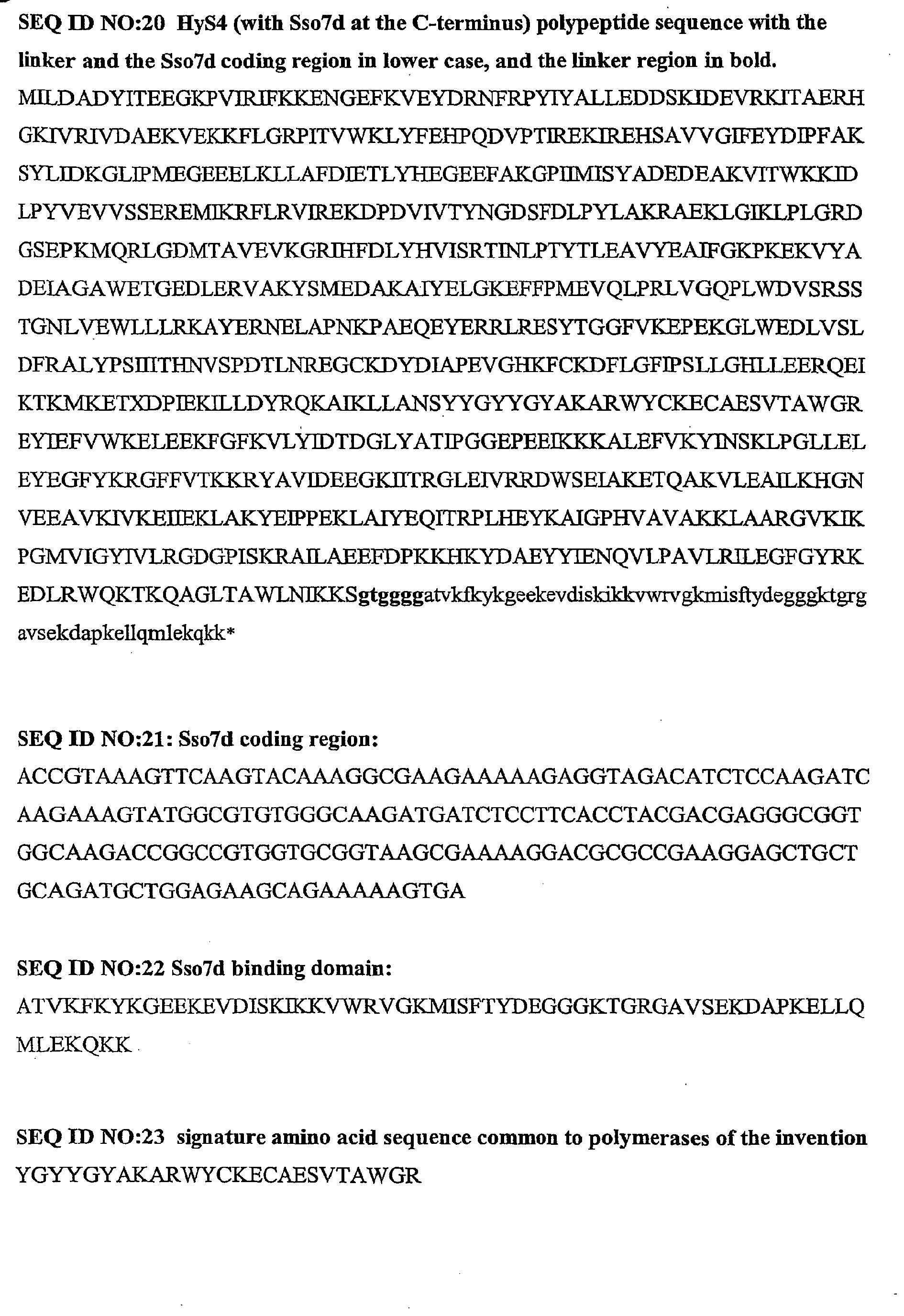

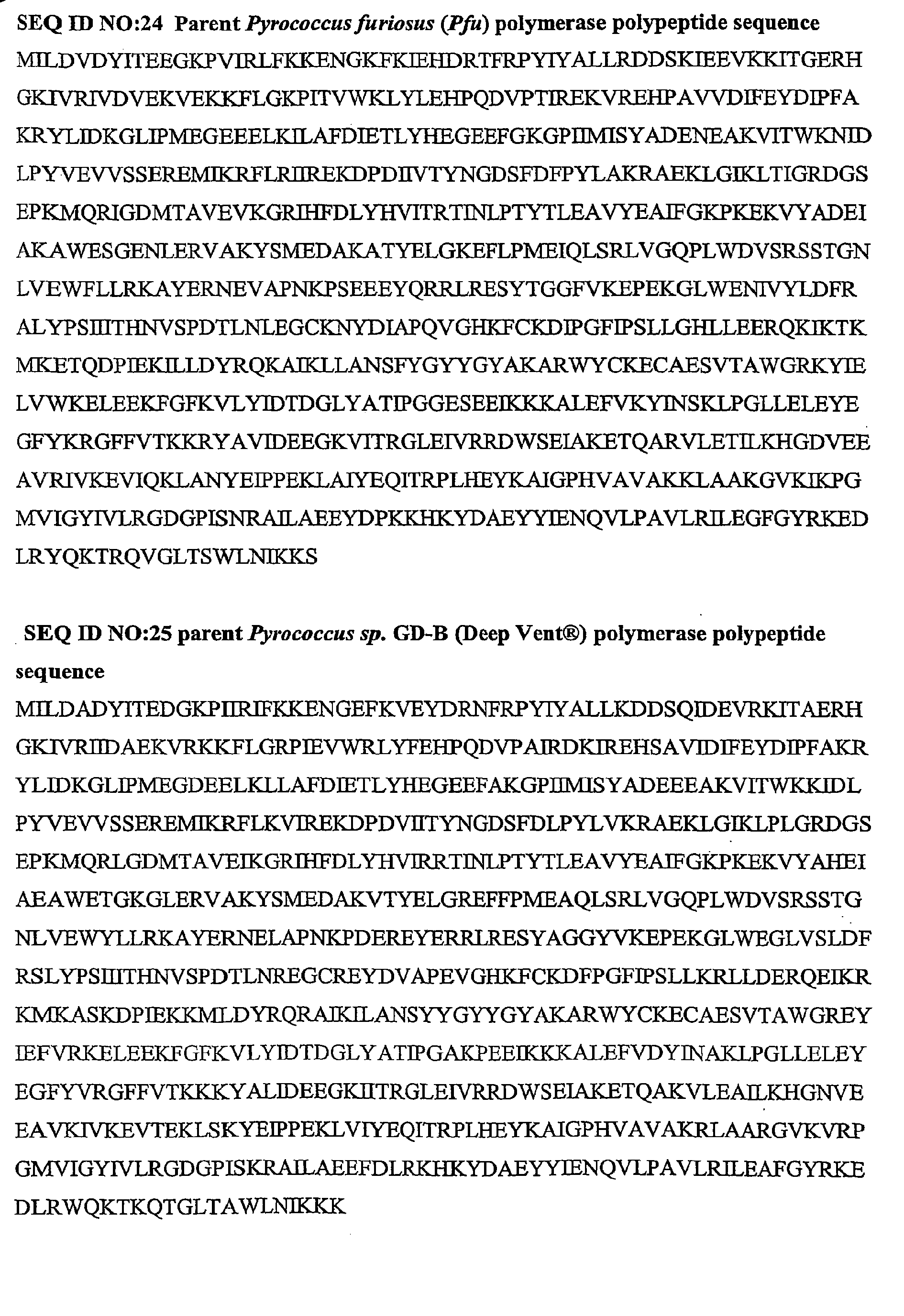

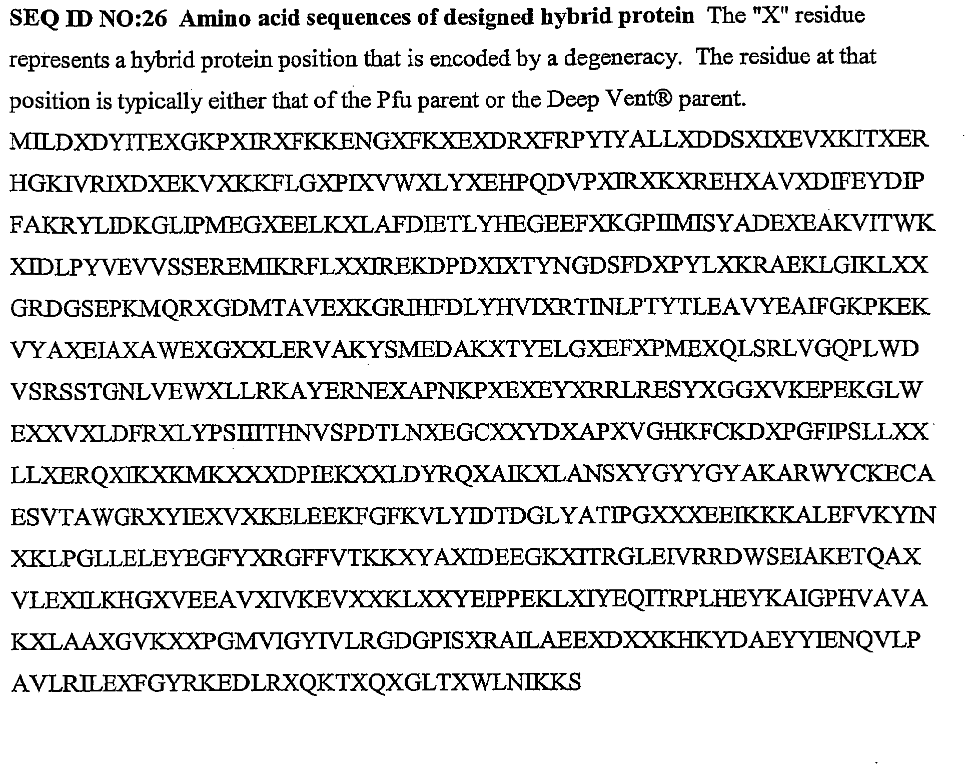

Also described herein are the following aspects:
1. A method of creating hybrid proteins having a common biological activity comprising the steps of:
   (a) creating a library comprising 32 or more nucleic acids encoding a plurality of hybrid protein members, wherein the members differ from a set of at least two parent proteins with corresponding amino acids, and
      i. where the parent proteins are homologous proteins having greater than 60% amino acid similarity to each other and having at least one common biological activity,
      ii. where a majority of the library members have a greater than 60% amino acid similarity to any of the parent proteins, and
      iii. where the majority of differences between the library members and the parent proteins are confined to those corresponding amino acids that differ among the parent proteins;
   (b) expressing protein from at least one library member to create at least one hybrid protein;
   (c) selecting at least one protein having a common biological activity of the parent proteins.
2. The method of aspect 1, wherein the parent proteins are enzymes.
3. The method of aspect 1, wherein the parent proteins are isozymes.
4. The method of aspect 1, wherein the parent proteins are polymerases.
5. The method of aspect 1, wherein the parent proteins have greater than 80% amino acid similarity to each other and the majority of the library members have greater than 80% amino acid similarity to any of the wild-type proteins.
6. A library comprising nucleic acids encoding a plurality of hybrid protein members, wherein the members differ from a set of at least two parent proteins with corresponding amino acids, and
   i. where the parent proteins are homologous proteins having greater than 60% amino acid similarity to each other and having at least one common biological activity,
   ii. where a majority of the library members have a greater than 60% amino acid similarity to any of the parent proteins, and
   iii. where the majority of differences between the library members and their parent proteins are confined to those corresponding amino acids that differ among the parent proteins.
7. The method of aspect 6, wherein the parent proteins are enzymes.
8. The method of aspect 6, wherein the parent proteins are isozymes.
9. The method of aspect 6, wherein the parent proteins are polymerases.
10. The method of aspect 6, wherein the parent proteins have greater than 80% amino acid similarity to each other and the majority of the library members have greater than 80% amino acid similarity to any of the parent proteins.
11. A synthetic hybrid protein comprising greater than 60% amino acid similarity to each member of a set of at least two parent proteins, where each parent protein in the set shares greater than 60% amino acid similarity and at least one common biological activity with each member of the set, and wherein the set comprises a subset of invariant amino acids that are identical among all members of the set, and wherein the hybrid protein:
   (a) shares at least one biological activity with all members of the set;
   (b) comprises at least 95% of the subset of invariant amino acids
   (c) has a minimum of 5 amino acid residue differences from any member of the set; and
   (d) contains at least 5 variable amino acid residues corresponding to alternating parent proteins.
12. The synthetic hybrid protein of aspect 11, wherein the parent protein is an enzyme.
13. The synthetic hybrid protein of aspect 11, wherein the parent protein is an isozyme.
14. The synthetic hybrid protein of aspect 11, wherein the parent protein is a polymerase.
15. The synthetic hybrid protein of aspect 11, wherein the synthetic protein comprises greater than 80% amino acid similarity to each member of the set and wherein each wild-type protein in the set shares greater than 80% amino acid similarity with each member of the set.
16. The synthetic hybrid protein of aspect 11, wherein the set of parent proteins comprises the *Pyroccus furiosus* family B DNA polymerase and *Pyrococcu ssp.* GB-D DNA Polymerase and the differences from any member of the set comprise at least 10 of the mutations selected from the group shown in Figure 2.
17. A hybrid polymerase having polymerase activity, wherein the polymerase comprises SEQ ID NO:23 and is at least 80% identical over 700 contiguous amino acids of the *Pyrococcus furiosus* (*Pfu*) polymerase sequence set forth in SEQ ID NO: 24 or at least 80% identical over 700 contiguous amino acids of the *Pyrococcus sp.* GB-D polymerase sequence set forth in SEQ ID NO:25, with the *proviso* that
   (a) when the polymerase is at least 85% identical to SEQ ID NO:24, the sequence comprises at least one hybrid position that is mutated from the native *Pfu* residue to the residue that occurs at the corresponding position of SEQ ID NO:25, wherein the hybrid position is one of the residues designated as "X" in SEQ ID NO:26; or
   (b) when the polymerase is at least 85% identical to SEQ ID NO:25, the sequence comprises at least one hybrid position that is mutated from the native *Pyrococcus sp.* GB-D residue to the residue that occurs at the corresponding position of SEQ ID NO:24, wherein the hybrid position is one of the residues designated as "X" in SEQ ID NO:26.
18. The hybrid polymerase of aspect 17, wherein the polymerase is at least 90% identical over 700 contiguous amino acids of the *Pfu* sequence set forth in SEQ ID NO:24 or at least 90% identical over 700 contiguous amino acids of the *Pyrococcus sp.* GB-D sequence set forth in SEQ ID NO:25.
19. The hybrid polymerase of aspect 17, wherein the hybrid polymerase comprises at least ten hybrid positions that are mutated from the native reside of SEQ ID NO:24 or SEQ ID NO:25 to the corresponding residue of SEQ ID NO:25 or SEQ ID NO:24.
20. The hybrid polymerase of aspect 17, wherein the hybrid polymerase comprises at least twenty hybrid positions that are mutated from the native reside of SEQ ID NO:24 or SEQ ID NO:25 to the corresponding residue of SEQ ID NO:25 or SEQ ID NO:24.
21. The hybrid polymerase of aspect 17, wherein the hybrid polymerase comprises at least forty hybrid positions that are mutated from the native reside of SEQ ID NO:24 or SEQ ID NO:25 to the corresponding residue of SEQ ID NO:25 or SEQ ID NO:24.
22. The hybrid polymerase of aspect 17, wherein the hybrid polymerase comprises at least fifty hybrid positions that are mutated from the native reside of SEQ ID NO:24 or SEQ ID NO:25 to the corresponding residue of SEQ ID NO:25 or SEQ ID NO:24.
23. The hybrid polymerase of aspect 17, wherein the hybrid polymerase comprises an amino acid sequence of SEQ ID NO:2, SEQ ID NO:12, SEQ ID NO:16, or SEQ ID NO:18; or the polymerase region of SEQ ID NO:4, SEQ ID NO:6, SEQ ID NO:8, SEQ ID NO:10, SEQ ID NO:14, or SEQ ID NO:20.
24. The hybrid polymerase of aspect 17, further comprising a DNA binding domain.
25. The hybrid polymerase of aspect 24, wherein the DNA binding domain is selected from the group consisting of Sso7d, Sac7d, and Sac7e.
26. The hybrid polymerase of aspect 25, wherein the DNA binding domain is Sso7d.
27. The hybrid polymerase of aspect 26 comprising an amino acid sequence of SEQ ID NO:4, SEQ ID NO:6, SEQ ID NO:8, SEQ ID NO:10, SEQ ID NO:14, or SEQ ID NO:20.
28. An isolated nucleic acid enclosing a hybrid polymerase as set forth in aspect 17 or aspect 24.
29. An expression vector comprising the nucleic acid of aspect 28.
30. A host cell transfected with the vector of aspect 29.
31. An isolated nucleic acid encoding a polypeptide comprising an amino acid sequence at least 94% identical to SEQ ID NO:2, wherein the polypeptide exhibits polymerase activity.
32. The isolated nucleic acid of aspect 31, wherein the polypeptide comprises SEQ ID NO:2.
33. The isolated nucleic acid of aspect 31, wherein the nucleic acid comprises SEQ ID NO: 1.
34. The isolated nucleic acid of aspect 31, wherein the polypeptide further comprises a DNA binding domain.
35. The isolated nucleic acid of aspect 34, wherein the DNA binding domain is selected from the group consisting of Sso7d, Sac7d, and Sac7e.
36. The isolated nucleic acid of aspect 35, wherein the DNA binding domain is Sso7d.
37. The isolated nucleic acid of aspect 35, wherein the nucleic acid comprises SEQ ID NO:3.
38. The isolated nucleic acid of aspect 35, wherein the nucleic acid encodes a polypeptide comprising SEQ ID NO:4.
39. An expression vector comprising the nucleic acid of aspect 31.
40. A host cell transfected with the vector of aspect 39.
41. An isolated polypeptide, wherein the polypeptide comprises an amino acid sequence at least 94% identical to SEQ ID NO:2, and wherein the polypeptide has polymerase activity.
42. The isolated polypeptide of aspect 41, wherein the polypeptide comprises SEQ ID NO:2.
43. The isolated polypeptide of aspect 41, further comprising a DNA binding domain.
44. The isolated polypeptide of aspect 43, wherein the DNA binding domain is selected from the group consisting of Sso7d, Sac7d, or Sac7e.
45. The isolated polypeptide of aspect43, wherein the DNA binding domain is fused to the carboxy-terminus of the polypeptide.
46. The isolated polypeptide of aspect 45, wherein the DNA binding domain is Sso7d.
47. The isolated polypeptide of aspect 46, wherein the polypeptide comprises SEQ ID NO:4.
48. A method of amplifying a target sequence using a hybrid polymerase, the method comprising the steps of:
   providing a polymerase according to aspect 17 or aspect 41,
   combining the polymerase in an amplification reaction mixture, and
   amplifying the target sequence.

## Claims

1. A hybrid polymerase having at has at least 85% identity to the amino acid sequence of SEQ ID NO: 2; wherein the hybrid polymerase comprises at least 20 positions that are mutated from the native residue of SEQ ID NO: 24 or SEQ ID NO: 25 to the corresponding residue of SEQ ID NO: 25 or SEQ ID NO: 24; and has an increased polymerase to exonuclease activity ratio relative to the parent Pfu polymerase.

2. The hybrid polymerase of claim 1, wherein the hybrid polymerase has at least 90% identity to the amino acid sequence of SEQ ID NO: 2.

3. The hybrid polymerase of claim 1 or claim 2, wherein the hybrid polymerase comprises at least 30 positions that are mutated from the native residue of SEQ ID NO: 24 or SEQ ID NO: 25 to the corresponding residue of SEQ ID NO: 25 or SEQ ID NO: 24.

4. The hybrid polymerase of any one of the preceding claims, further comprising a DNA binding domain that is conjugated to the polymerase, wherein the DNA binding domain is Sso7d, Sac7d, or Sac7e.

5. An isolated nucleic acid encoding a hybrid polymerase as set forth in any one of claims 1 to 4.

6. An expression vector comprising the nucleic acid of claim 5.

7. A host cell transfected with the vector of claim 6.

8. A method of amplifying a target sequence using a hybrid polymerase, the method comprising the steps of:
(a) providing a polymerase according to any one of claims 1 to 4,
(b) combining the polymerase or the polypeptide in an amplification reaction mixture, and
(c) amplifying the target sequence.
